# EUROPEAN PATENT SPECIFICATION

(11) **EP 4 395 627 B1**
(45) Date of publication and mention of the grant of the patent: **18.06.2025**
(21) Application number: 22769639.0
(22) Date of filing: 26.08.2022
(51) Int. Cl.: A61B 5/01, A61B 5/145, A61F 2/26, A61F 2/48, A61B 5/00, A61F 2/00

(54) **IMPLANTABLE ENERGIZED MEDICAL DEVICE, RELATED METHODS AND KIT**
IMPLANTIERBARES ENERGETISIERTES MEDIZINISCHES GERÄT, ZUGEHÖRIGE METHODEN UND KIT
DISPOSITIF MEDICAL IMPLANTABLE SOUS TENSION, PROCEDES ET KIT ASSOCIES

(30) Priority: 30.08.2021 WO PCT/EP2021/073893; 18.02.2022 SE 2250205
(43) Date of publication of application: 10.07.2024
(73) Proprietor: Implantica Patent Ltd, 223 70 Lund (SE)
(72) Inventor: FORSELL, Peter, 223 70 Lund (SE)
(74) Representative: AWA Sweden AB
(86) International application number: PCT/EP2022/073817
(87) International publication number: WO 2023/031048

(56) References cited:
- EP-A1- 2 211 772
- EP-B1- 2 211 772
- US-A1- 2016 074 196
- US-B1- 9 827 430

## Description

### Technical field

The present invention relates to medical implants. More specifically the invention relates to medical implants configured to be kept in place by a body tissue of the patient.

### Background

Medical devices, designed to be implanted in a patient's body, are typically operated by means of electrical power. Such medical devices include electrical and mechanical stimulators, motors, pumps, etc, which are designed to support or stimulate various body functions. Electrical power can be supplied to such an implanted medical device from a likewise implanted battery or from an external energy source that can supply any needed amount of electrical power intermittently or continuously without requiring repeated surgical operations.

An implanted energy receiver or other implanted devices required for the operation of an implanted medical device must in some way be located in the patient's body in a secure and convenient way. It is often the case that the implanted device must be located close to the patient's skin in order to keep the distance between an external device, such as an energy transmitter, and the implanted device to a minimum. In practice, this means subcutaneous placement of the implanted device.

It is also often important that the implanted device is kept in a relatively fixed position so that for example energy transfer can be performed accurately.

There is a need for improved energized medical devices for implantation in a patient.

US 2016/074196 A1 discloses an implanted control unit which comprises a communication part close to the skin with a wireless energy receiver for receiving wireless energy from an external source and an interconnection portion with an electrical lead for transferring the received energy to a main portion of the control unit which is deeper under the skin.

EP 2211772 A1 discloses another implantable energized medical device that receives wireless energy.

US 9827430 B1 discloses an injected conductive tattoo which receives power wirelessly from an external transmitter and then retransmits the power through the body to an implant.

### Summary

It is an object of the present inventive concept to mitigate, alleviate or eliminate one or more of the above-identified deficiencies in the art and disadvantages singly or in combination.

According to one embodiment of the inventive concept, these and other objects are achieved in full, or at least in part, by an implantable energized medical device configured to be held in position by a tissue portion of a patient, the medical device comprising: a first portion configured to be placed on a first side of the tissue portion, the first portion having a first cross-sectional area in a first plane and comprising a first surface configured to face a first tissue surface of the first side of the tissue portion, a second portion configured to be placed on a second side of the tissue portion, the second side opposing the first side, the second portion having a second cross-sectional area in a second plane and comprising a second surface configured to engage a second tissue surface of the second side of the tissue portion, and a connecting portion configured to be placed through a hole in the tissue portion extending between the first and second sides of the tissue portion, the connecting portion having a third cross-sectional area in a third plane and a fourth cross-sectional area in a fourth plane and a third surface configured to engage the first tissue surface of the first side of the tissue portion, wherein the connecting portion is configured to connect the first portion to the second portion, wherein: the first, second, third and fourth planes are parallel to each other, the third cross-sectional area is smaller than the second and fourth cross-sectional areas, such that the first portion, second portion and connecting portion are prevented from travelling through the hole in the tissue portion in a direction perpendicular to the first, second and third planes, and the first portion is detachably connected to at least one of the connecting portion and the second portion.

In some embodiments, the third cross-sectional area is smaller than the first cross-sectional area.

In some embodiments, the third cross-sectional area is equal to or larger than the first cross-sectional area.

In some embodiments, the connecting portion comprises a flange comprising the fourth cross-sectional area, such that the flange is prevented from travelling through the hole in the tissue portion in a direction perpendicular to the first, second and third planes.

In some embodiments, the flange protrudes in a direction parallel to the first, second, third and fourth planes, and perpendicular to a central extension of the connecting portion.

In some embodiments, the flange comprises the third surface configured to engage the first tissue surface of the first side of the tissue portion.

In some embodiments, the connecting portion comprises at least one protruding element comprising the fourth cross-sectional area, such that the at least one protruding element is prevented from travelling through the hole in the tissue portion, such that the second portion and the connecting portion can be held in position by the tissue portion of the patient also when the first portion is disconnected from the connecting portion.

In some embodiments, the at least one protruding element protrudes in a direction parallel to the first, second, third and fourth planes, and perpendicular to a central extension of the connecting portion.

In some embodiments, the at least one protruding element comprises the third surface configured to engage the first tissue surface of the first side of the tissue portion.

In some embodiments, the connecting portion comprises at least two protruding elements comprising the fourth cross-sectional area.

In some embodiments, the at least two protruding elements are symmetrically arranged about a central axis of the connecting portion.

In some embodiments, the at least two protruding elements are asymmetrically arranged about a central axis of the connecting portion.

In some embodiments, at least one of the first, second and third surfaces comprises at least one of ribs, barbs, hooks, a friction enhancing surface treatment, and a friction enhancing material, to facilitate the implantable energized medical device being held in position by the tissue portion.

In some embodiments, the connecting portion comprises a hollow portion.

In some embodiments, the hollow portion provides a passage between the first and second portions.

In some embodiments, the first portion is detachably connected to the connecting portion by at least one of a mechanical connection and a magnetic connection.

In some embodiments, the first portion is detachably connected to the connecting portion by at least one of threads and corresponding grooves, a screw, a self-locking element, a twist and lock fitting, and a spring-loaded locking mechanism.

In some embodiments, the at least one protruding element has a height in a direction perpendicular to the fourth plane being less than a height of the first portion in said direction.

In some embodiments, the at least one protruding element has a height in said direction perpendicular to the fourth plane being less than half of said height of the first portion in said direction.

In some embodiments, the at least one protruding element has a height in said direction perpendicular to the fourth plane being less than a quarter of said height of the first portion in said direction.

In some embodiments, the at least one protruding element has a height in said direction perpendicular to the fourth plane being less than a tenth of said height of the first portion in said direction.

In some embodiments, the at least one protruding element has a diameter in the fourth plane being one of: less than a diameter of the first portion in the first plane, equal to a diameter of the first portion in the first plane, and larger than a diameter of the first portion in the first plane.

In some embodiments, the at least one protruding element has a cross-sectional area in the fourth plane being one of: less than a cross-sectional area of the first portion in the first plane, equal to a cross-sectional area of the first portion in the first plane, and larger than a cross-sectional area of the first portion in the first plane.

In some embodiments, the at least one protruding element has a height in said direction perpendicular to the fourth plane being less than half of a height of the connecting portion in said direction.

In some embodiments, the at least one protruding element has a height in said direction perpendicular to the fourth plane being less than a quarter of said height of the connecting portion in said direction.

In some embodiments, the at least one protruding element has a height in said direction perpendicular to the fourth plane being less than a tenth of said height of the connecting portion in said direction.

According to one embodiment of the inventive concept, these and other objects are achieved in full, or at least in part, by an implantable energized medical device configured to be held in position by a tissue portion of a patient, the medical device comprising: a first portion configured to be placed on a first side of the tissue portion, the first portion having a first cross-sectional area in a first plane and comprising a first surface configured to face a first tissue surface of the first side of the tissue portion, a second portion configured to be placed on a second side of the tissue portion, the second side opposing the first side, the second portion having a second cross-sectional area in a second plane and comprising a second surface configured to engage a second tissue surface of the second side of the tissue portion, and a connecting portion configured to be placed through a hole in the tissue portion extending between the first and second sides of the tissue portion, the connecting portion having a third cross-sectional area in a third plane and being configured to connect the first portion to the second portion, wherein: the first, second, and third planes are parallel to each other, the third cross-sectional area is smaller than the first and second cross-sectional areas, such that the first portion and second portion are prevented from travelling through the hole in the tissue portion in a direction perpendicular to the first, second and third planes, and a connecting interface between the connecting portion and the second portion is excentric with respect to the second portion.

In some embodiments, the connecting interface between the connecting portion and the second portion is excentric, with respect to the second portion, in a first direction, but not in a second direction being perpendicular to the first direction.

In some embodiments, the connecting interface between the connecting portion and the second portion is excentric, with respect to the second portion, in a first direction and in a second direction being perpendicular to the first direction.

In some embodiments, the first direction and second direction are parallel to the second plane.

In some embodiments, the second portion has a first end and a second end opposing the first end, wherein the second portion has a length between the first and second end.

In some embodiments, the first end and second end are separated in a direction parallel to the second plane.

In some embodiments, the second portion is curved along the length.

In some embodiments, the second portion is curved in said first direction and said second direction being perpendicular to the first direction.

In some embodiments, the first and second ends comprise an elliptical point respectively.

In some embodiments, the first and second ends comprise a hemispherical end cap respectively.

In some embodiments, the second portion has at least one circular cross-section along the length between the first and second end.

In some embodiments, the second portion has at least one oval cross-section along the length between the first and second end.

In some embodiments, the second portion has at least one elliptical cross-section along the length between the first and second end.

In some embodiments, the second portion has said length in a direction being different to a central extension of the connecting portion.

In some embodiments, the second portion has a proximal region, an intermediate region, and a distal region.

In some embodiments, the proximal region extends from the first end to an interface between the connecting portion and the second portion, the intermediate region is defined by the connecting interface between the connecting portion and the second portion, and the distal region extends from the interface between the connecting portion and the second portion to the second end.

In some embodiments, the proximal region is shorter than the distal region with respect to the length of the second portion.

In some embodiments, the proximal region and the intermediate region together are shorter than the distal region with respect to the length of the second portion.

In some embodiments, the proximal region and the distal region comprises the second surface configured to engage the second surface of the second side of the tissue portion.

In some embodiments, the second portion has a length x and a width y along respective length and width directions being perpendicular to each other and substantially parallel to the second plane, wherein the connecting interface between the connecting portion and the second portion is contained within a region extending from x>0 to x<x/2 and/or y>0 to y<y/2, x and y and 0 being respective end points of the second portion along said length and width directions.

In some embodiments, the second portion is tapered from the first end to the second end.

In some embodiments, the second portion is tapered from each of the first end and second end towards the intermediate region of the second portion.

In some embodiments, the first portion has a maximum dimension being in the range of 10 to 40 mm, such as in the range of 10 to 30 mm, such as in the range of 15 to 25 mm.

In some embodiments, the first portion has a diameter being in the range of 10 to 40 mm, such as in the range of 10 to 30 mm, such as in the range of 15 to 25 mm.

In some embodiments, the connecting portion has a maximum dimension in the third plane in the range of 2 to 20 mm, such as in the range of 2 to 15 mm, such as in the range of 5 to 10 mm.

In some embodiments, the second portion has a maximum dimension being in the range of 30 to 90 mm, such as in the range of 30 to 70 mm, such as in the range of 35 to 60 mm.

In some embodiments, the first portion has one or more of a spherical shape, an ellipsoidal shape, a polyhedral shape, an elongated shape, and a flat disk shape.

In some embodiments, the connecting portion has one of an oval cross-section, an elongated cross-section, and a circular cross-section, in a plane parallel to the third plane.

In some embodiments, the distal region is configured to be directed downwards in a standing patient.

In some embodiments, the first portion comprises a proximal region extending from an first end to an interface between the connecting portion and the first portion, an intermediate region defined by an connecting interface between the connecting portion and the first portion, and a distal region extending from the interface between the connecting portion and the first portion to a second end of the first portion.

In some embodiments, the first portion has a first height, and the second portion has a second height, both heights being in a direction perpendicular to the first and second planes, wherein the first height is smaller than the second height.

In some embodiments, the first height is less than 2/3 of the second height, such as less than 1/2 of the second height, such as less than 1/3 of the second height.

In some embodiments, the second end of the second portion comprises connections for connecting to an implant being located in a caudal direction from a location of the implantable energized medical device in the patient.

In some embodiments, the first end of the second portion comprises connections for connecting to an implant being located in a cranial direction from a location of the implantable energized medical device in the patient.

According to one embodiment of the inventive concept, these and other objects are achieved in full, or at least in part, by an implantable energized medical device configured to be held in position by a tissue portion of a patient, the medical device comprising: a first portion configured to be placed on a first side of the tissue portion, the first portion having a first cross-sectional area in a first plane and comprising a first surface configured to face a first tissue surface of the first side of the tissue portion, a second portion configured to be placed on a second side of the tissue portion, the second side opposing the first side, the second portion having a second cross-sectional area in a second plane and comprising a second surface configured to engage a second tissue surface of the second side of the tissue portion, and a connecting portion configured to be placed through a hole in the tissue portion extending between the first and second sides of the tissue portion, the connecting portion has a third cross-sectional area in a third plane and is configured to connect the first portion to the second portion, wherein: the first, second and third planes are parallel to each other, the third cross-sectional area is smaller than the first and second cross-sectional areas, such that the first portion and second portion are prevented from travelling through the hole in the tissue portion in a direction perpendicular to the first, second and third planes, the first cross-sectional area has a first cross-sectional distance and a second cross-sectional distance, the first and second cross-sectional distances being perpendicular to each other and the first cross-sectional distance being longer than the second cross-sectional distance, the second cross-sectional area has a first cross-sectional distance and a second cross-sectional distance, the first and second cross-sectional distances being perpendicular to each other and the first cross-sectional distance being longer than the second cross-sectional distance, the first cross-sectional distance of the first cross-sectional area and the first cross-sectional distance of the second cross-sectional area are rotationally displaced in relation to each other with an angle exceeding 45° to facilitate insertion of the second portion through the hole in the tissue portion.

In some embodiments, the first cross-sectional distance of the first cross-sectional area and the first cross-sectional distance of the second cross-sectional area are rotationally displaced in relation to each other with an angle exceeding 60° to facilitate insertion of the second portion through the hole in the tissue portion.

In some embodiments, the first cross-sectional distance of the first cross-sectional area and the first cross-sectional distance of the second cross-sectional area are substantially perpendicular to each other to facilitate insertion of the second portion through the hole in the tissue portion.

In some embodiments, the first cross-sectional distance of the first cross-sectional area and the first cross-sectional distance of the second cross-sectional area are rotationally displaced in relation to each other with an angle exceeding 45° and being less than 135°.

In some embodiments, the cross-sectional area of the first portion is elongated.

In some embodiments, the cross-sectional area of the second portion is elongated.

In some embodiments, the connecting portion is connected eccentrically to the second portion.

In some embodiments, the first cross-sectional distance of the second portion is divided into a first, second and third equal length-portions, and wherein the connecting portion is connected to the second portion along the first length-portion of the first cross-sectional distance.

In some embodiments, the first cross-sectional area of the first portion is elongated.

In some embodiments, the second cross-sectional area of the second portion is elongated.

In some embodiments, the first portion comprises a first wireless energy receiver configured to receive energy transmitted wirelessly from an external wireless energy transmitter.

In some embodiments, the first portion comprises an internal wireless energy transmitter.

In some embodiments, the second portion comprises a second wireless energy receiver.

In some embodiments, the first portion comprises a first energy storage unit.

In some embodiments, the second portion comprises a second energy storage unit.

In some embodiments, at least one of the first and second energy storage unit is a solid-state battery.

In some embodiments, the solid-state battery is a thionyl-chloride battery.

In some embodiments, the first wireless energy receiver is configured to receive energy transmitted wirelessly by the external wireless energy transmitter, and store the received energy in the first energy storage unit, the internal wireless energy transmitter is configured to wirelessly transmit energy stored in the first energy storage unit to the second wireless energy receiver, and the second wireless energy receiver is configured to receive energy transmitted wirelessly by the internal wireless energy transmitter and store the received energy in the second energy storage unit.

In some embodiments, the first portion comprises a first controller comprising at least one processing unit.

In some embodiments, the second portion comprises a second controller comprising at least one processing unit.

In some embodiments, at least one of the first and second controller is connected to a wireless transceiver for communicating wirelessly with an external device.

In some embodiments, the first controller is connected to a first wireless communication receiver in the first portion for receiving wireless communication from an external device, and the first controller is connected to a first wireless communication transmitter in the first portion for transmitting wireless communication to a second wireless communication receiver in the second portion.

In some embodiments, the second controller is connected to the second wireless communication receiver for receiving wireless communication from the first portion.

In some embodiments, the first wireless energy receiver comprises a first coil and the internal wireless energy transmitter comprises a second coil.

In some embodiments, the first portion comprises a combined coil, wherein the combined coil is configured to receive energy wirelessly from an external wireless energy transmitter, and transmit energy wirelessly to the second wireless receiver of the second portion.

In some embodiments, at least one of the coils are embedded in a ceramic material.

In some embodiments, the implantable energized medical device further comprises a housing configured to enclose at least the first portion, and wherein a first portion of the housing is made from titanium and a second portion of the housing is made from a ceramic material.

In some embodiments, the portion of the housing made from a ceramic material comprises at least one coil embedded in the ceramic material.

In some embodiments, the implantable energized medical device further comprises a housing configured to enclose at least the second portion, and wherein a first portion of the housing is made from titanium and a second portion of the housing is made from a ceramic material.

In some embodiments, the portion of the housing made from a ceramic material comprises at least one coil embedded in the ceramic material.

According to one embodiment of the inventive concept, these and other objects are achieved in full, or at least in part, by an implantable energized medical device configured to be held in position by a tissue portion of a patient, the medical device comprising: a first portion configured to be placed on a first side of the tissue portion, the first portion having a first cross-sectional area in a first plane and comprising a first surface configured to face a first tissue surface of the first side of the tissue portion, a second portion configured to be placed on a second side of the tissue portion, the second side opposing the first side, the second portion having a second cross-sectional area in a second plane and comprising a second surface configured to engage a second tissue surface of the second side of the tissue portion, and a connecting portion configured to be placed through a hole in the tissue portion extending between the first and second sides of the tissue portion, the connecting portion having a third cross-sectional area in a third plane and being configured to connect the first portion to the second portion, wherein: the first, second, and third planes are parallel to each other, the third cross-sectional area is smaller than the first and second cross-sectional areas, such that the first portion and second portion are prevented from travelling through the hole in the tissue portion in a direction perpendicular to the first, second and third planes, the first portion comprises a first wireless energy receiver for receiving energy transmitted wirelessly by an external wireless energy transmitter, and an internal wireless energy transmitter configured to transmit energy wirelessly to the second portion, and the second portion comprises a second wireless energy receiver configured to receive energy transmitted wirelessly by the internal wireless energy transmitter.

In some embodiments, the implantable energized medical device further comprises at least one sensor for providing input to at least one of the first and second controller.

In some embodiments, the sensor is a sensor configured to sense a physical parameter of the implantable energized medical device.

In some embodiments, the sensor is a sensor configured to sense at least one of: a temperature of the implantable energized medical device or of a body engaging portion, a parameter related to the power consumption of the implantable energized medical device or of a body engaging portion, a parameter related to a status of at least one of the first and second energy storage unit, a parameter related to the wireless transfer of energy from a source external to the body of the patient, and a hydraulic pressure.

In some embodiments, the sensor is a sensor configured to sense a physiological parameter of the patient.

In some embodiments, the sensor is a sensor configured to sense at least one of: a parameter related to the patient swallowing, a local temperature, a systemic temperature, blood saturation, blood oxygenation, blood pressure, a parameter related to an ischemia marker, and pH.

In some embodiments, the sensor configured to sense a parameter related to the patient swallowing comprises at least one of: a motility sensor, a sonic sensor, an optical sensor, and a strain sensor.

In some embodiments, the sensor configured to sense pH is configured to sense the acidity in the stomach.

In some embodiments, the controller is configured to transmit information based on sensor input to a device external to the body of the patient.

In some embodiments, the second portion comprises at least a portion of an operation device for operating an implantable body engaging portion.

In some embodiments, the second portion comprises at least one electrical motor.

In some embodiments, the second portion comprises a transmission configured to reduce the velocity and increase the force of the movement generated by the electrical motor.

In some embodiments, the transmission is configured to transfer a week force with a high velocity into a stronger force with lower velocity.

In some embodiments, the transmission is configured to transfer a rotating force into a linear force.

In some embodiments, the transmission comprises a gear system.

In some embodiments, the second portion comprises a magnetic coupling for transferring mechanical work from the electrical motor through one of: a barrier separating a first chamber of the second portion from a second chamber of the second portion, a housing enclosing at least the second portion.

In some embodiments, the second portion comprises at least one hydraulic pump.

In some embodiments, the hydraulic pump comprises a pump comprising at least one compressible hydraulic reservoir.

In some embodiments, the implantable energized medical device further comprises a capacitor connected to at least one of the first and second energy storage unit and connected to the electrical motor, wherein the capacitor is configured to: be charged by at least one of the first and second energy storage units, and provide the electrical motor with electrical power.

In some embodiments, at least one of the first and second portion comprises a sensation generator adapted to generate a sensation detectable by a sense of the patient.

In some embodiments, the second portion comprises a force transferring element configured to mechanically transfer force from the second portion to an implanted body engaging portion.

In some embodiments, the second portion comprises a force transferring element configured to hydraulically transfer force from the second portion to an implanted body engaging portion.

In some embodiments, the second portion comprises at least one lead for transferring electrical energy and/or information from the second portion to an implanted body engaging portion.

In some embodiments, the first portion comprises an injection port for injecting fluid into the first portion.

In some embodiments, the connecting portion comprises a conduit for transferring a fluid from the first portion to the second portion.

In some embodiments, the conduit is arranged to extend through the hollow portion of the connecting portion.

In some embodiments, the second portion comprises a first and a second chamber separated from each other, wherein the first chamber comprises a first liquid and the second chamber comprises a second liquid, and wherein the second liquid is a hydraulic liquid configured to transfer force to an implantable element configured to exert force on the body portion of the patient.

In some embodiments, a wall portion of the first chamber is resilient to allow an expansion of the first chamber.

In some embodiments, the second portion comprises a first hydraulic system in fluid connection with a first hydraulically operable implantable element configured to exert force on the body portion of the patient, and a second hydraulic system in fluid connection with a second hydraulically operable implantable element configured to exert force on the body portion of the patient, wherein the first and second hydraulically operable implantable elements are adjustable independently from each other.

In some embodiments, the first hydraulic system comprises a first hydraulic pump and the second hydraulic systems comprises a second hydraulic pump.

In some embodiments, each of the first and second hydraulic systems comprises a reservoir for holding hydraulic fluid.

In some embodiments, the implantable energized medical further comprises a first pressure sensor configured to sense a pressure in the first hydraulic system, and a second pressure sensor configured to sense a pressure in the second hydraulic system.

In some embodiments, the first surface is configured to engage the first tissue surface of the first side of the tissue portion.

In some embodiments, the first, second and third planes are parallel to a major extension plane of the tissue.

According to one embodiment of the inventive concept, these and other objects are achieved in full, or at least in part, by an implantable energized medical device configured to be held in position by a tissue portion of a patient, the medical device comprising: a first portion configured to be placed on a first side of the tissue portion, the first portion having a first cross-sectional area in a first plane and comprising a first surface configured to face a first tissue surface of the first side of the tissue portion, a second portion configured to be placed on a second side of the tissue portion, the second side opposing the first side, the second portion having a second cross-sectional area in a second plane and comprising a second surface configured to engage a second tissue surface of the second side of the tissue portion, and a connecting portion configured to be placed through a hole in the tissue portion extending between the first and second sides of the tissue portion, the connecting portion having a third cross-sectional area in a third plane and being configured to connect the first portion to the second portion, wherein: the first, second, and third planes are parallel to each other, the third cross-sectional area is smaller than the first and second cross-sectional areas, such that the first portion and second portion are prevented from travelling through the hole in the tissue portion in a direction perpendicular to the first, second and third planes, at least one of the first portion and the second portion comprises at least one coil embedded in a ceramic material, the at least one coil being configured for at least one of: receiving energy transmitted wirelessly, transmitting energy wirelessly, receiving wireless communication, and transmitting wireless communication.

In some embodiments, the first portion comprises a first wireless energy receiver configured to receive energy transmitted wirelessly from an external wireless energy transmitter.

In some embodiments, the first portion comprises a first wireless communication receiver.

In some embodiments, the first portion comprises a coil embedded in a ceramic material, hereinafter referred to as a first coil.

In some embodiments, the first wireless energy receiver comprises the first coil.

In some embodiments, the first wireless communication receiver comprises the first coil.

In some embodiments, the first portion comprises a distal end and a proximal end with respect to the connecting portion, along a direction perpendicular to the first plane.

In some embodiments, the first coil is arranged at the distal end of the first portion.

In some embodiments, the first portion comprises an internal wireless energy transmitter.

In some embodiments, the first portion comprises a first wireless communication transmitter.

In some embodiments, the first portion comprises a coil embedded in a ceramic material, hereinafter referred to as a second coil.

In some embodiments, the internal wireless energy transmitter comprises the second coil.

In some embodiments, the first wireless communication transmitter comprises the second coil.

In some embodiments, the second coil is arranged at the proximal end of the first portion.

In some embodiments, the first wireless energy receiver and the internal wireless energy transmitter comprises a single coil embedded in a ceramic material.

In some embodiments, the first wireless communication receiver and the first wireless communication transmitter comprises a single coil embedded in a ceramic material.

In some embodiments, the first wireless energy receiver, the internal wireless energy transmitter, the first wireless communication receiver, and the internal wireless communication transmitter comprises a single coil embedded in a ceramic material.

In some embodiments, the second portion comprises a second wireless energy receiver.

In some embodiments, the second portion comprises a coil embedded in a ceramic material, hereinafter referred to as a third coil, wherein the second wireless energy receiver comprises the third coil.

In some embodiments, the second portion comprises a distal end and a proximal end with respect to the connecting portion, along a direction perpendicular to the first plane.

In some embodiments, the third coil is arranged at the proximal end of the second portion.

In some embodiments, the first portion comprises a first energy storage unit.

In some embodiments, the second portion comprises a second energy storage unit.

In some embodiments, the first wireless energy receiver is configured to receive energy transmitted wirelessly by the external wireless energy transmitter, and store the received energy in the first energy storage unit, the internal wireless energy transmitter is configured to wirelessly transmit energy stored in the first energy storage unit to the second wireless energy receiver, and the second wireless energy receiver is configured to receive energy transmitted wirelessly by the internal wireless energy transmitter and store the received energy in the second energy storage unit.

In some embodiments, the first energy storage unit is configured to store less energy than the second energy storage unit, and configured to be charged faster than the second energy storage unit.

In some embodiments, the first energy storage unit has lower energy density than the second energy storage unit.

In some embodiments, the implantable energized medical device further comprises a housing configured to enclose at least the first portion, and wherein a first portion of the housing is made from titanium and a second portion of the housing is made from a ceramic material.

In some embodiments, the housing made from a ceramic material comprises the at least one coil embedded in the ceramic material.

In some embodiments, the implantable energized medical device further comprises a housing configured to enclose at least the second portion, and wherein a first portion of the housing is made from titanium and a second portion of the housing is made from a ceramic material.

In some embodiments, the portion of the housing made from a ceramic material comprises the at least one coil embedded in the ceramic material.

In some embodiments, the first, second and third planes are parallel to a major extension plane of the tissue.

In some embodiments, the connecting portion further comprises a fourt cross-sectional area in a fourth plane, wherein the fourt plane is parallel to the first, second and third planes, and wherein the third cross-sectional area is smaller than the fourth cross-sectional area.

In some embodiments, the connecting portion comprises a protruding element comprising the fourth cross-sectional area.

In some embodiments, the fourth plane is parallel to a major extension plane of the tissue.

In some embodiments, a connecting interface between the connecting portion and the second portion is excentric with respect to the second portion.

According to an embodiment of the inventive concept, an implantable device for exerting a force on a body portion of a patient is provided, wherein the implantable device comprises: an implantable energized medical device and an implantable element configured to exert a force on a body portion of the patient.

In some embodiments, the implantable element configured to exert a force on a body portion of the patient is an implantable hydraulic constriction device.

In some embodiments, the implantable hydraulic constriction device is configured for constricting a luminary organ of the patient.

In some embodiments, the implantable hydraulic constriction device comprises an implantable hydraulic constriction device for constricting an intestine of the patient.

In some embodiments, the implantable hydraulic constriction device comprises an implantable hydraulic constriction device for constricting a colon or rectum of the patient.

In some embodiments, the implantable hydraulic constriction device comprises an implantable hydraulic constriction device for constricting the intestine at a region of a stoma of the patient.

In some embodiments, the implantable hydraulic constriction device comprises an implantable hydraulic constriction device for constricting a blood vessel of the patient.

In some embodiments, the implantable hydraulic constriction device for constricting a blood vessel of the patient is configured to constrict the venous blood flow leading from an erectile tissue for promoting the engorgement of the erectile tissue.

In some embodiments, the implantable hydraulic constriction device comprises an implantable hydraulic constriction device for constricting a vas deference of the patient.

In some embodiments, the implantable element configured to exert a force on a body portion of the patient is an implantable element for actively emptying the urinary bladder of the patient.

In some embodiments, the implantable element for actively emptying the urinary bladder of the patient is configured to empty the bladder of the patient by compressing the urinary bladder from the outside thereof.

In some embodiments, the implantable element configured to exert a force on a body portion of the patient is an implantable element for actively stretching a stomach wall of the patient to create a feeling of satiety.

According to one embodiment of the inventive concept, these and other objects are achieved in full, or at least in part, by a method of implanting an implantable energized medical device, the method comprising: placing a second portion of an implantable energized medical device between a peritoneum and a layer of muscular tissue of the abdominal wall, placing a first portion of the implantable energized medical device between the skin of the patient and a layer of muscular tissue of the abdominal wall, wherein the first and second portions are configured to be connected by a connecting portion extending through at least one layer of muscular tissue of the abdominal wall, placing a body engaging portion of the implantable energized medical device in connection with a tissue or an organ of the patient which is to be affected by the implantable energized medical device, and placing a transferring member, configured to transfer at least one of energy and force from the second portion to the body engaging portion, at least partially between a peritoneum and a layer of muscular tissue of the abdominal wall, such that at least 1/3 of the length of the transferring member is placed on the outside of the peritoneum.

In some embodiments, the transferring member is configured to transfer mechanical force from the second portion to the body engaging portion.

In some embodiments, the transferring member is configured to transfer hydraulic force from the second portion to the body engaging portion.

In some embodiments, the transferring member is configured to transfer electrical energy force from the second portion to the body engaging portion.

In some embodiments, the transferring member is configured to transfer data between the second portion and the body engaging portion.

In some embodiments, the step of placing the transferring member comprises placing the transferring member at least partially between the peritoneum and the layer of muscular tissue of the abdominal wall, such that at least 1/2 of the length of the transferring member is placed on the outside of the peritoneum of the patient.

In some embodiments, the step of placing the transferring member comprises placing the transferring member at least partially between the peritoneum and the layer of muscular tissue of the abdominal wall, such that at least 2/3 of the length of the transferring member is placed on the outside of the peritoneum of the patient.

In some embodiments, the step of placing the transferring member comprises placing the transferring member entirely outside of the peritoneum of the patient.

In some embodiments, the step of placing the transferring member comprises placing the transferring member such that it extends from the second portion to an area between the rib cage and the peritoneum of the patient, outside of the peritoneum.

In some embodiments, the step of placing the transferring member comprises placing the transferring member such that it extends from the second portion to an area between the stomach and the thoracic diaphragm of the patient.

In some embodiments, the step of placing the transferring member comprises placing the transferring member such that it extends from the second portion to the stomach of the patient.

In some embodiments, the step of placing the transferring member comprises placing the transferring member such that it extends from the second portion to the esophagus of the patient.

In some embodiments, the step of placing the transferring member comprises placing the transferring member such that it extends from the second portion to the retroperitoneal space.

In some embodiments, the step of placing the transferring member comprises placing the transferring member such that it extends from the second portion to an area of the kidneys.

In some embodiments, the step of placing the transferring member comprises placing the transferring member such that it extends from the second portion to the renal arteries.

In some embodiments, the step of placing the transferring member comprises placing the transferring member such that it extends from the second portion to the subperitoneal space, outside of the peritoneum.

In some embodiments, the step of placing the transferring member comprises placing the transferring member such that it extends from the second portion to the urinary bladder, outside of the peritoneum.

In some embodiments, the step of placing the transferring member comprises placing the transferring member such that it extends from the second portion to the urethra, outside of the peritoneum.

In some embodiments, the step of placing the second portion of the implantable energized medical device between the peritoneum and the layer of muscular tissue of the abdominal wall comprises placing the second portion between a first and second layer of muscular tissue of the abdominal wall.

In some embodiments, the step of placing the second portion comprises placing a second portion comprising an electrical motor.

In some embodiments, the step of placing the second portion comprises placing a second portion comprising a hydraulic pump.

In some embodiments, the step of placing the second portion comprises placing a second portion comprising an energy storage unit.

In some embodiments, the step of placing the second portion comprises placing a second portion comprising a receiver for receiving at least one of: energy and communication, wirelessly.

In some embodiments, the step of placing the first portion comprises placing a first portion comprising a transmitter for transmitting at least one of: energy and communication, wirelessly.

In some embodiments, the step of placing the second portion comprises placing a second portion comprising a controller involved in the control of the powered medical device.

In some embodiments, the second portion is elongated and has a length axis extending substantially in the direction of the elongation of the second portion, and wherein the step of placing the second portion comprises placing the second portion such that the length axis is substantially parallel with the cranial-caudal axis of the patient.

In some embodiments, the second portion is elongated and has a length axis extending substantially in the direction of the elongation of the second portion, and wherein the step of placing the second portion comprises placing the second portion such that the length axis is substantially perpendicular with the cranial-caudal axis of the patient.

In some embodiments, the second portion is elongated and has a length axis extending substantially in the direction of the elongation of the second portion, and wherein the step of placing the second portion comprises entering a hole in a layer of muscular tissue of the stomach wall in the direction of the length axis of the second portion and pivoting or angling the second portion after the hole has been entered.

In some embodiments, the step of placing the first portion of the implantable energized medical device between the skin of the patient and a layer of muscular tissue of the abdominal wall comprises placing the first portion in the subcutaneous tissue.

In some embodiments, the step of placing the first portion of the implantable energized medical device between the skin of the patient and a layer of muscular tissue of the abdominal wall comprises placing the first portion between a first and second layer of muscular tissue of the abdominal wall.

In some embodiments, the step of placing the first portion comprises placing a first portion comprising an energy storage unit.

In some embodiments, the step of placing the first portion comprises placing a first portion comprising a receiver for receiving at least one of: energy and communication, wirelessly.

In some embodiments, the step of placing the first portion comprises placing a first portion comprising a transmitter for transmitting at least one of: energy and communication, wirelessly.

In some embodiments, the step of placing the first portion comprises placing a first portion comprising a controller involved in the control of the powered medical device.

In some embodiments, the first portion is elongated and has a length axis extending substantially in the direction of the elongation of the first portion, and wherein the step of placing the first portion comprises placing the first portion such that the length axis is substantially parallel with the cranial-caudal axis of the patient.

In some embodiments, the first portion is elongated and has a length axis extending substantially in the direction of the elongation of the first portion, and wherein the step of placing the first portion comprises placing the first portion such that the length axis is substantially perpendicular with the cranial-caudal axis of the patient.

In some embodiments, the first portion is elongated and has a first portion length axis extending substantially in the direction of the elongation of the first portion, and the second portion is elongated and has a second portion length axis extending substantially in the direction of the elongation of the second portion, and wherein the step of placing the first and second portions comprises placing the first and second portions such that the first portion length axis and the second portion length axis are placed at an angle in relation to each other exceeding 30°.

In some embodiments, the step of placing the first and second portions comprises placing the first and second portions such that the first portion length axis and the second portion length axis are placed at an angle in relation to each other exceeding 45°.

In some embodiments, the method further comprises the step of placing the connecting portion through at least one layer of muscular tissue of the abdominal wall.

In some embodiments, the first portion, the second portion and the connecting portion are portions of a single unit.

In some embodiments, the method further comprises the step of connecting the first portion to the connecting portion, in situ.

In some embodiments, the method further comprises the step of connecting the second portion to the connecting portion, in situ.

In some embodiments, the method further comprises the step of connecting the transferring member to the first portion.

In some embodiments, the method further comprises the step of connecting the transferring member to the body engaging portion.

In some embodiments, the body engaging portion comprises a medical device for stretching the stomach wall such that a sensation of satiety is created.

In some embodiments, the body engaging portion comprises a constriction device configured to constrict a luminary organ of a patient.

In some embodiments, the body engaging portion comprises an implantable constriction device.

In some embodiments, the implantable constriction device comprises an implantable constriction device for constricting a luminary organ of the patient.

In some embodiments, the implantable constriction device comprises an implantable constriction device for constricting an intestine of the patient.

In some embodiments, the implantable constriction device comprises an implantable constriction device for constricting a colon or rectum of the patient.

In some embodiments, the implantable constriction device comprises an implantable constriction device for constricting the intestine at a region of a stoma of the patient.

In some embodiments, the implantable constriction device comprises an implantable constriction device for constricting a blood vessel of the patient.

In some embodiments, the implantable constriction device for constricting a blood vessel of the patient is configured to constrict the venous blood flow leading from an erectile tissue for promoting the engorgement of the erectile tissue.

In some embodiments, the implantable constriction device for constricting a blood vessel of the patient is configured to constrict the blood flow in the renal artery to affect the patients systemic blood pressure.

In some embodiments, the implantable constriction device comprises an implantable constriction device for constricting a vas deference of the patient.

In some embodiments, the body engaging portion comprises an implantable element for actively emptying the urinary bladder of the patient.

In some embodiments, the implantable element for actively emptying the urinary bladder of the patient is configured to empty the bladder of the patient by compressing the urinary bladder from the outside thereof.

In some embodiments, the body engaging comprises an element for electrically stimulating a tissue portion of a patient.

According to one embodiment of the inventive concept, these and other objects are achieved in full, or at least in part, by a kit for assembling an implantable energized medical device configured to be held in position by a tissue portion of a patient, the kit comprising: a group of one or more first portions, a group of one or more second portions, a group of one or more connecting portions, wherein at least one of said groups comprises at least two different types of said respective portions; wherein the medical device is a modular device and, when assembled, comprises a selection, from said groups, of one first portion, one second portion, and one connecting portion, wherein: the first portion is configured to be placed on a first side of the tissue portion, the first portion having a first cross-sectional area in a first plane and comprising a first surface configured to face a first tissue surface of the first side of the tissue portion, the second portion is configured to be placed on a second side of the tissue portion, the second side opposing the first side, the second portion having a second cross-sectional area in a second plane and comprising a second surface configured to engage a second tissue surface of the second side of the tissue portion, and the connecting portion is configured to be placed through a hole in the tissue portion extending between the first and second sides of the tissue portion, the connecting portion having a third cross-sectional area in a third plane and being configured to connect the first portion to the second portion, wherein: the first, second, and third planes are parallel to each other, and the third cross-sectional area is smaller than the first and second cross-sectional areas, such that the first portion and second portion are prevented from travelling through the hole in the tissue portion in a direction perpendicular to the first, second and third planes.

In general, any of the embodiments of the implantable energized medical device disclosed herein may form part of such kit, and any features of such embodiments may be combined to form part of such kit.

In some embodiments, the group of one or more first portions comprises a first portion comprising a first energy storage unit.

In some embodiments, the group of one or more first portions comprises a first portion comprising a first wireless energy receiver unit for receiving energy transmitted wirelessly by an external wireless energy transmitter.

In some embodiments, the first energy storage unit is connected to the first wireless energy receiver, wherein the first wireless energy receiver is configured to receive energy transmitted wirelessly by the external wireless energy transmitter and store the received energy in the first energy storage unit.

In some embodiments, the first wireless energy receiver is configured to be physically connected to a second energy storage unit in the second portion.

In some embodiments, the group of one or more first portions comprises a first portion comprising an internal wireless energy transmitter.

In some embodiments, the group of one or more second portions comprises a second portion comprising a second wireless energy receiver configured to receive energy transmitted wirelessly by the internal wireless energy transmitter.

In some embodiments, the internal wireless energy transmitter is configured to transmit energy wirelessly to the second wireless energy receiver.

In some embodiments, the group of one or more second portions comprises a second portion comprising a second energy storage unit connected to the second wireless energy receiver.

In some embodiments, the second wireless energy receiver is configured to receive energy transmitted wirelessly by the internal wireless energy transmitter and store the received energy in the second energy storage unit.

In some embodiments, the group of one or more first portions comprises a first portion being formed as one integral unit with a connecting portion.

In some embodiments, the group of one or more second portions comprises a second portion being formed as one integral unit with a connecting portion.

In some embodiments, one of the group of one or more first, second or connecting portions comprises a first portion, second portion and connecting portion being formed as one integral unit.

In some embodiments, the group of one or more first portions comprises a first portion having a first height along a direction being perpendicular to the first plane, and a first portion having a second height along said direction being perpendicular to the first plane, wherein the second height is larger than the first height.

In some embodiments, the group of one or more first portions comprises a first portion having a first width and/or length along a direction being parallel to the first plane, and a first portion having a second width and/or length along said direction being parallel to the first plane, wherein the second width and/or length is larger than the first width and/or length.

In some embodiments, the group of one or more second portions comprises a second portion having a first height along a direction being perpendicular to the second plane, and a second portion having a second height along said direction being perpendicular to the second plane, wherein the second height is larger than the first height.

In some embodiments, the group of one or more second portions comprises a second portion having a first width and/or length along a direction being parallel to the second plane, and a second portion having a second width and/or length along said direction being parallel to the second plane, wherein the second width and/or length is larger than the first width and/or length.

In some embodiments, the group of one or more connecting portions comprises a connecting portion having a first height along a direction being perpendicular to the third plane, and a connecting portion having a second height along said direction being perpendicular to the third plane, wherein the second height is larger than the first height.

In some embodiments, the group of one or more connecting portions comprises a connecting portion having a first width and/or length along a direction being parallel to the third plane, and a connecting portion having a second width and/or length along said direction being parallel to the third plane, wherein the second width and/or length is larger than the first width and/or length.

In some embodiments, the group of one or more first portions comprises a first portion comprising an injection port for injecting fluid into the first portion.

In some embodiments, the group of one or more connecting portions comprises a connecting portion comprising a hydraulic fluid conduit for hydraulically connecting the first portion to the second portion.

In some embodiments, the group of one or more first portions comprises a first portion comprising a first controller comprising at least one processing unit.

In some embodiments, the group of one or more second portions comprises a second portion comprising a second controller comprising at least one processing unit.

In some embodiments, at least one of the first and second controller is connected to a wireless transceiver for communicating wirelessly with an external device.

In some embodiments, the first controller is connected to a first wireless communication receiver in the first portion for receiving wireless communication from an external device, the first controller is connected to a first wireless communication transmitter in the first portion for transmitting wireless communication to a second wireless communication receiver in the second portion.

In some embodiments, the second controller is connected to the second wireless communication receiver for receiving wireless communication from the first portion.

In some embodiments, the first wireless energy receiver comprises a first coil and the internal wireless energy transmitter comprises a second coil.

In some embodiments, the group of first portions comprises a first portion comprising a combined coil, wherein the combined coil is configured to receive wireless energy wirelessly from an external wireless energy transmitter, and transmit wireless energy wirelessly to the second wireless receiver of the second portion.

In some embodiments, at least one of the coils are embedded in a ceramic material.

In some embodiments, the group of one or more first portions comprises a first portion comprising a push button and/or a capacitive button for controlling a function of the implantable energized medical device.

The term "body tissue" referred to in the present disclosure may be one or several body tissue groups or layers in a patient, such as muscle tissue, connective tissue, bone, etc.

An external device configured for communication with an implantable medical device, when implanted in a patient, is provided. The external device comprises at least one first wireless transceiver configured for communication with the implantable medical device using a first network protocol, for determining a distance between the external device and the implantable medical device, and at least one second wireless transceiver configured for communication with the implantable medical device using a second network protocol, for transferring data between the external device and the implantable medical device.

According to one embodiment, the first wireless transceiver comprises an UWB transceiver.

According to one embodiment, the first wireless transceiver is configured for transcutaneous energy transfer for at least one of powering an energy consuming component of the implantable medical device and charging an implantable energy storage unit.

According to one embodiment, the second network protocol is a standard network protocol. The standard network protocol may be one from the list of: Radio Frequency type protocol, RFID type protocol, WLAN type protocol, Bluetooth type protocol, BLE type protocol, NFC type protocol, 3G/4G/5G type protocol, and GSM type protocol.

According to one embodiment, the second wireless transceiver comprises a Bluetooth transceiver.

According to one embodiment, the external device is further configured to communicate with a second external device using said at least one wireless transceiver.

According to one embodiment, the external device is configured for determining a distance between the external device and the implantable medical device by determining the RSSI.

According to one embodiment, a communication range of the first network protocol is less than a communication range of the second network protocol.

According to one embodiment, a frequency band of the first network protocol differs from a frequency band of the second network protocol.

According to one embodiment, the external device is configured to authenticate the implantable medical device if the determined distance between the external device and the implantable medical device is less than a predetermined threshold value.

According to one embodiment, the external device is configured to allow the transfer of data between the external device and the implantable medical device after the implantable medical device has been authenticated.

According to one embodiment, the external device is one from the list of: a wearable external device, and a handset.

An implantable medical device configured for communication with an external device is provided. The implantable medical device comprises at least one first wireless transceiver configured for communication with the external device using a first network protocol, for determining a distance between the external device and the implantable medical device, and at least one second wireless transceiver configured for communication with the external device using a second network protocol, for transferring data between the external device and the implantable medical device.

According to one embodiment, the first wireless transceiver comprises an UWB transceiver.

According to one embodiment, the first wireless transceiver is configured for transcutaneous energy transfer for at least one of: powering an energy consuming component of the implantable medical device, and charging an implantable energy storage unit.

According to one embodiment, the second network protocol is a standard network protocol, such as selected from the list of Radio Frequency type protocol, RFID type protocol, WLAN type protocol, Bluetooth type protocol, BLE type protocol, NFC type protocol, 3G/4G/5G type protocol, and GSM type protocol.

According to one embodiment, the second wireless transceiver comprises a Bluetooth transceiver.

According to one embodiment, the implantable medical device is further configured to communicate with a second external device using said at least one wireless transceiver.

According to one embodiment, the implantable medical device is configured for determining a distance between the external device and the implantable medical device by determining the RSSI.

According to one embodiment, a communication range of the first network protocol is less than a communication range of the second network protocol.

According to one embodiment, a frequency band of the first network protocol differs from a frequency band of the second network protocol.

According to one embodiment, the implantable medical device is configured to authenticate the external device if the determined distance between the external device and the implantable medical device is less than a predetermined threshold value.

According to one embodiment, the implantable medical device is configured to allow the transfer of data between the implantable medical device and the external device after the external device has been authenticated.

According to one embodiment, the implantable medical device comprises at least one of:
an external heart compression device,
an apparatus assisting the pump function of a heart of the patient,
an apparatus assisting the pump function comprising a turbine bump placed within a patient's blood vessel for assisting the pump function of the heart,
an operable artificial heart valve,
an operable artificial heart valve for increasing the blood flow to the coronary arteries,
an implantable drug delivery device,
an implantable drug delivery device for injecting directly into a blood vessel and change the position of the injection site, all from within the patient's body,
an implantable drug delivery device for injecting potency enhancing drugs into an erectile tissue of the patient,
a hydraulic, mechanic, and/or electric constriction implant,
an operable volume filling device,
an operable gastric band,
an operable implant for stretching the stomach wall of the patient for creating satiety,
an implant configured to sense the frequency of the patient ingesting food,
an operable cosmetic implant,
an operable cosmetic implant for adjust the shape and/or size in the breast region of a patient,
an implant controlling medical device for the emptying of a urinary bladder,
an implant hindering urinary leakage,
an implant hindering anal incontinence,
an implant controlling the emptying of fecal matter,
an implant monitoring an aneurysm,
an implant for hindering the expansion of an aneurysm,
an implant lubricating a joint,
an implant for affecting the blood flow to an erectile tissue of the patient,
an implant for simulating the engorgement of an erectile tissue,
an implant with a reservoir for holding bodily fluids,
an implant storing and/or emptying a bodily reservoir or a surgically created
reservoir,
an implant communicating with a database outside the body,
an implant able to be programmed from outside the body,
an implant able to be programmed from outside the body with a wireless signal,
an implant treating impotence,
an implant controlling the flow of eggs in the uterine tube,
an implant controlling the flow of sperms in the uterine tube,
an implant controlling the flow of sperms in the vas deferens,
an implant for hindering the transportation of the sperm in the vas deferens,
an implant treating osteoarthritis,
an implant performing a test of parameters inside the body,
an implant controlling specific treatment parameters from inside the body,
an implant controlling bodily parameters from inside the body,
an implant controlling the blood pressure,
an implant controlling the blood pressure by affecting the dilatation of the renal artery,
an implant controlling a drug treatment parameter,
an implant controlling a parameter in the blood,
an implant for adjusting or replacing any bone part of a body of the patient,
an implant replacing an organ of the patient or part of an organ of the patient or the function thereof,
a vascular treatment device,
an implant adapted to move fluid inside the body of the patient,
an implant configured to sense a parameter related to the patient swallowing,
an implant configured to exercise a muscle with electrical or mechanical stimulation,
an implant configured for emptying an intestine portion on command,
an operable implant configured to be invaginated in the stomach of the patient to reduce the volume of the stomach substantially more than the volume of the device,
an implant configured for emptying the urinary bladder from within the patient's body by compressing the bladder,
an implant configured for draining fluid from within the patient's body,
an implant configured for the active lubrication of a joint with an added lubrication fluid,
an implant configured for removing clots and particles from the patient's blood stream,
an implant configured for elongating or straightening a bone in the patient, to reduce scoliosis,
a device to stimulate the brain for a several position to a focused point,
an artificial stomach replacing the function of the natural stomach,
an implant configured for adjusting the position of a female's urinary tract or bladder neck,
an implant configured for stimulating the ampulla vas deference and creating temporary constriction.

According to one embodiment, the system comprises a master private key device configured to allow issuance of a new private key device, wherein the HCP or HCP admin have such master private key device adapted to able to replace and pair a new patient private key device or HCP private key device into the system, through the HCP EID external device.

According to one embodiment, the patient remote external device and the patient EID external device are an integrated unit.

According to one embodiment, the HCP dedicated device and the HCP EID external device are an integrated unit.

According to one embodiment, the system comprises a measurement device or sensor adapted to deliver a measurement to at least one of the DDI, patent EID external device and a patient display device.

According to one embodiment, the system comprises a food sensor, adapted to measure at least if the patient swallows solid food or is drinking fluid, wherein said food sensor is connected to the control unit of a medical device to cause an action to stretch the stomach after a determined amount of food intake.

A patient external device configured for communication with an implantable medical device, when implanted in a patient, is provided. The patient external device comprises a wireless communication unit configured for wireless transmission of control commands to the implantable medical device and configured for wireless communication with a patient display device, and a computing unit configured for running a control software for creating the control commands for the operation of the implantable medical device. The computing unit is configured to transmit a control interface as a remote display portal to a patient display device configured to display the control interface to a user, receive user input from the patient display device, and transform the user input into the control commands for wireless transmission to the implantable medical device.

According to one embodiment, the wireless communication unit comprises a wireless transceiver for wireless transmission of control commands to the implantable medical device, and wireless transmission of the control interface as the remote display portal to the patient display device.

According to one embodiment, the wireless communication unit comprises a first wireless transceiver for wireless transmission of control commands to the implantable medical device, and a second wireless transceiver for wireless transmission of the control interface to the patient display device.

According to one embodiment, the wireless communication unit is configured for wireless communication with the patient display device using a standard network protocol.

According to one embodiment, the wireless communication unit is configured for wireless communication with the implantable medical device using a proprietary network protocol.

According to one embodiment, the wireless communication unit comprises a Bluetooth transceiver.

According to one embodiment, at least one of the first and second wireless transceiver comprises a Bluetooth transceiver.

According to one embodiment, the wireless communication unit comprises a UWB transceiver.

According to one embodiment, at least one of the first and second wireless transceiver comprises a UWB transceiver.

According to one embodiment, the wireless communication unit comprises at least one first wireless transceiver configured for communication with the implantable medical device using a first network protocol, for determining a distance between the patient external device and the implantable medical device, and at least one second wireless transceiver configured for communication with the implantable medical device using a second network protocol, for transferring data between the patient external device and the implantable medical device.

According to one embodiment, the first wireless transceiver is configured for transcutaneous energy transfer for at least one of: powering an energy consuming component of the implantable medical device and charging an implantable energy storage unit.

According to one embodiment, the standard network protocol is one from the list of: Radio Frequency type protocol, RFID type protocol, WLAN type protocol, Bluetooth type protocol, BLE type protocol, NFC type protocol, 3G/4G/5G type protocol, and GSM type protocol.

According to one embodiment, a communication range of the first wireless transceiver is less than a communication range of the second wireless transceiver.

According to one embodiment, at least one of:
the patient external device is configured to authenticate the implantable medical device if a distance between the patient external device and the implantable medical device is less than a predetermined threshold value,
the patient external device is configured to be authenticated by the implantable medical device if a distance between the patient external device and the implantable medical device is less than a predetermined threshold value,
the patient external device is configured to authenticate the patient display device if a distance between the patient external device and the patient display device is less than a predetermined threshold value, and
the patient external device is configured to be authenticated by the implantable medical device if a distance between the patient external device and the patient display device is less than a predetermined threshold value.

According to one embodiment, the patient external device is configured to allow the transfer of data between at least one of: the patient external device and the implantable medical device, and the patient external device and the patient display device, on the basis of the authentication.

According to one embodiment, the computing unit is configured to encrypt at least one of the control interface and the control commands.

According to one embodiment, the implantable medical device comprises at least one of:
an external heart compression device,
an apparatus assisting the pump function of a heart of the patient,
an apparatus assisting the pump function comprising a turbine bump placed within a patient's blood vessel for assisting the pump function of the heart,
an operable artificial heart valve,
an operable artificial heart valve for increasing the blood flow to the coronary arteries.
an implantable drug delivery device,
an implantable drug delivery device for injecting directly into a blood vessel and change the position of the injection site, all from within the patient's body,
an implantable drug delivery device for injecting potency enhancing drugs into an erectile tissue of the patient,
a hydraulic, mechanic, and/or electric constriction implant,
an operable volume filling device,
an operable gastric band,
an operable implant for stretching the stomach wall of the patient for creating satiety,
an implant configured to sense the frequency of the patient ingesting food,
an operable cosmetic implant,
an operable cosmetic implant for adjust the shape and/or size in the breast region of a patient,
an implant controlling medical device for the emptying of a urinary bladder,
an implant hindering urinary leakage,
an implant hindering anal incontinence,
an implant controlling the emptying of fecal matter,
an implant monitoring an aneurysm,
an implant for hindering the expansion of an aneurysm,
an implant lubricating a joint,
an implant for affecting the blood flow to an erectile tissue of the patient,
an implant for simulating the engorgement of an erectile tissue,
an implant with a reservoir for holding bodily fluids,
an implant storing and/or emptying a bodily reservoir or a surgically created
reservoir,
an implant communicating with a database outside the body,
an implant able to be programmed from outside the body,
an implant able to be programmed from outside the body with a wireless signal,
an implant treating impotence,
an implant controlling the flow of eggs in the uterine tube,
an implant controlling the flow of sperms in the uterine tube,
an implant controlling the flow of sperms in the vas deferens,
an implant for hindering the transportation of the sperm in the vas deferens,
an implant treating osteoarthritis,
an implant performing a test of parameters inside the body,
an implant controlling specific treatment parameters from inside the body,
an implant controlling bodily parameters from inside the body,
an implant controlling the blood pressure,
an implant controlling the blood pressure by affecting the dilatation of the renal artery,
an implant controlling a drug treatment parameter,
an implant controlling a parameter in the blood,
an implant for adjusting or replacing any bone part of a body of the patient,
an implant replacing an organ of the patient or part of an organ of the patient or the function thereof,
a vascular treatment device,
an implant adapted to move fluid inside the body of the patient,
an implant configured to sense a parameter related to the patient swallowing,
an implant configured to exercise a muscle with electrical or mechanical stimulation,
an implant configured for emptying an intestine portion on command,
an operable implant configured to be invaginated in the stomach of the patient to reduce the volume of the stomach substantially more than the volume of the device,
an implant configured for emptying the urinary bladder from within the patient's body by compressing the bladder,
an implant configured for draining fluid from within the patient's body,
an implant configured for the active lubrication of a joint with an added lubrication fluid,
an implant configured for removing clots and particles from the patient's blood stream,
an implant configured for elongating or straightening a bone in the patient, to reduce scoliosis,
a device to stimulate the brain for a several position to a focused point,
an artificial stomach replacing the function of the natural stomach,
an implant configured for adjusting the position of a female's urinary tract or bladder neck,
an implant configured for stimulating the ampulla vas deference and creating temporary constriction.

According to one embodiment, the system comprises a master private key device configured to allow issuance of a new private key device, wherein the HCP or HCP admin have such master private key device adapted to able to replace and pair a new patient private key device or HCP private key device into the system, through the HCP EID external device.

According to one embodiment, the patient remote external device and the patient EID external device are an integrated unit.

According to one embodiment, the HCP dedicated device and the HCP EID external device are an integrated unit.

According to one embodiment, the system comprises a measurement device or sensor adapted to deliver a measurement to at least one of the DDI, patent EID external device and a patient display device.

According to one embodiment, the system comprises a food sensor, adapted to measure at least if the patient swallows solid food or is drinking fluid, wherein said food sensor is connected to the control unit of a medical device to cause an action to stretch the stomach after a determined amount of food intake.

A patient display device for communication with a patient remote external device for communication with an implantable medical device is provided. The patient display device comprises a wireless communication unit configured for wirelessly receiving an implant control interface as a remote display portal from the patient remote external device and configured for wirelessly transmitting implant control user input to the patient remote external device, a display for displaying the received implant control interface, and an input device for receiving implant control input from the user.

According to one embodiment, the patient display device further comprises an auxiliary wireless communication unit. The auxiliary wireless communication unit is configured to be disabled to enable at least one of: wirelessly receiving the implant control interface as the remote display portal from the patient remote external device, and wirelessly transmitting implant control user input to the patient remote external device.

According to one embodiment, the wireless communication unit is configured for wireless communication with the patient remote external device using a standard network protocol. The standard network protocol may be one from the list of: Radio Frequency type protocol, RFID type protocol, WLAN type protocol, Bluetooth type protocol, BLE type protocol, NFC type protocol, 3G/4G/5G type protocol, and GSM type protocol.

According to one embodiment, the wireless communication unit is configured for wireless communication with the patient remote external device using a proprietary network protocol.

According to one embodiment, the wireless communication unit comprises a Bluetooth transceiver.

According to one embodiment, the wireless communication unit comprises a UWB transceiver.

According to one embodiment, a communication range of the wireless communication unit is less than a communication range of the auxiliary wireless communication unit.

According to one embodiment, the patient display device is configured to authenticate the patient remote external device if a distance between the patient display device and the patient remote external device is less than a predetermined threshold value, or to be authenticated by the patient remote external device if a distance between the patient display device and the patient remote external device is less than a predetermined threshold value.

According to one embodiment, the patient display device is configured to allow the transfer of data between the patient display device and the patient remote external device on the basis of the authentication.

According to one embodiment, the patient display device is a wearable external device or a handset.

According to one embodiment, the system comprises a master private key device configured to allow issuance of a new private key device, wherein the HCP or HCP admin have such master private key device adapted to able to replace and pair a new patient private key device or HCP private key device into the system, through the HCP EID external device.

According to one embodiment, the patient remote external device and the patient EID external device are an integrated unit.

According to one embodiment, the HCP dedicated device and the HCP EID external device are an integrated unit.

According to one embodiment, the system comprises a measurement device or sensor adapted to deliver a measurement to at least one of the DDI, patent EID external device and a patient display device.

According to one embodiment, the system comprises a food sensor, adapted to measure at least if the patient swallows solid food or is drinking fluid, wherein said food sensor is connected to the control unit of a medical device to cause an action to stretch the stomach after a determined amount of food intake.

A communication system for enabling communication between a patient display device and an implantable medical device, when implanted, is provided. The communication system comprises: a patient display device, a server, and a patient remote external device. The patient display device comprises a wireless communication unit configured for wirelessly receiving an implant control interface as a remote display portal being provided by the patient remote external device. The wireless communication unit is further configured for wirelessly transmitting implant control user input to the server, destined for the patient remote external device. The system further comprises a display for displaying the received remote display portal, and an input device for receiving implant control input from the user, wherein the patient remote external device comprises a wireless communication unit configured for wireless transmission of control commands to the implantable medical device, and a computing unit. The computing unit is configured for running a control software for creating the control commands for the operation of the implantable medical device, transmitting a control interface to the patient display device, receiving implant control user input generated at the patient display device, from the server, and transforming the user input into the control commands for wireless transmission to the implantable medical device.

According to one embodiment, the computing unit is configured to encrypt at least one of the control interface and the control commands.

According to one embodiment, the patient display device is configured to encrypt the user input.

According to one embodiment, the server is configured to encrypt at least one of the user input received from the patient display device and the control interface received from the patient remote external device.

According to one embodiment, the computing unit is configured to encrypt the control interface and the patient display device is configured to decrypt the encrypted control interface.

According to one embodiment, the server is configured to act as a router, transferring the encrypted control interface from the patient remote external device to the patient display device without decryption.

According to one embodiment of the communication system or patient display device the implantable medical device comprises at least one of:
an external heart compression device,
an apparatus assisting the pump function of a heart of the patient,
an apparatus assisting the pump function comprising a turbine bump placed within a patient's blood vessel for assisting the pump function of the heart,
an operable artificial heart valve,
an operable artificial heart valve for increasing the blood flow to the coronary arteries.
an implantable drug delivery device,
an implantable drug delivery device for injecting directly into a blood vessel and change the position of the injection site, all from within the patient's body,
an implantable drug delivery device for injecting potency enhancing drugs into an erectile tissue of the patient,
a hydraulic, mechanic, and/or electric constriction implant,
an operable volume filling device,
an operable gastric band,
an operable implant for stretching the stomach wall of the patient for creating satiety,
an implant configured to sense the frequency of the patient ingesting food,
an operable cosmetic implant,
an operable cosmetic implant for adjust the shape and/or size in the breast region of a patient,
an implant controlling medical device for the emptying of a urinary bladder,
an implant hindering urinary leakage,
an implant hindering anal incontinence,
an implant controlling the emptying of fecal matter,
an implant monitoring an aneurysm,
an implant for hindering the expansion of an aneurysm,
an implant lubricating a joint,
an implant for affecting the blood flow to an erectile tissue of the patient,
an implant for simulating the engorgement of an erectile tissue,
an implant with a reservoir for holding bodily fluids,
an implant storing and/or emptying a bodily reservoir or a surgically created
reservoir,
an implant communicating with a database outside the body,
an implant able to be programmed from outside the body,
an implant able to be programmed from outside the body with a wireless signal,
an implant treating impotence,
an implant controlling the flow of eggs in the uterine tube,
an implant controlling the flow of sperms in the uterine tube,
an implant controlling the flow of sperms in the vas deferens,
an implant for hindering the transportation of the sperm in the vas deferens,
an implant treating osteoarthritis,
an implant performing a test of parameters inside the body,
an implant controlling specific treatment parameters from inside the body,
an implant controlling bodily parameters from inside the body,
an implant controlling the blood pressure,
an implant controlling the blood pressure by affecting the dilatation of the renal artery,
an implant controlling a drug treatment parameter,
an implant controlling a parameter in the blood,
an implant for adjusting or replacing any bone part of a body of the patient,
an implant replacing an organ of the patient or part of an organ of the patient or the function thereof,
a vascular treatment device,
an implant adapted to move fluid inside the body of the patient,
an implant configured to sense a parameter related to the patient swallowing,
an implant configured to exercise a muscle with electrical or mechanical stimulation,
an implant configured for emptying an intestine portion on command,
an operable implant configured to be invaginated in the stomach of the patient to reduce the volume of the stomach substantially more than the volume of the device,
an implant configured for emptying the urinary bladder from within the patient's body by compressing the bladder,
an implant configured for draining fluid from within the patient's body,
an implant configured for the active lubrication of a joint with an added lubrication fluid,
an implant configured for removing clots and particles from the patient's blood stream,
an implant configured for elongating or straightening a bone in the patient, to reduce scoliosis,
a device to stimulate the brain for a several position to a focused point,
an artificial stomach replacing the function of the natural stomach,
an implant configured for adjusting the position of a female's urinary tract or bladder neck,
an implant configured for stimulating the ampulla vas deference and creating temporary constriction.

According to one embodiment, the communication system further comprises a server. The server may comprise a wireless communication unit configured for wirelessly receiving an implant control interface received from the patient remote external device and wirelessly transmitting the implant control interface as a remote display portal to the patient display device. The wireless communication unit is further configured for wirelessly receiving implant control user input from a patient EID external device and wirelessly transmitting the implant control user input to the patient display device.

According to one embodiment, the system comprises a master private key device configured to allow issuance of a new private key device, wherein the HCP or HCP admin have such master private key device adapted to able to replace and pair a new patient private key device or HCP private key device into the system, through the HCP EID external device.

According to one embodiment, the patient remote external device and the patient EID external device are an integrated unit.

According to one embodiment, the HCP dedicated device and the HCP EID external device are an integrated unit.

According to one embodiment, the system comprises a measurement device or sensor adapted to deliver a measurement to at least one of the DDI, patent EID external device and a patient display device.

According to one embodiment, the system comprises a food sensor, adapted to measure at least if the patient swallows solid food or is drinking fluid, wherein said food sensor is connected to the control unit of a medical device to cause an action to stretch the stomach after a determined amount of food intake.

A patient display device for communication with a patient external device for communication with an implantable medical device, when implanted, is provided. The patient display device comprises a wireless communication unit, a display, and an input device for receiving implant control input from the user. The patient display device is configured to run a first application for wireless communication with a server and/or DDI, and run a second application for wireless communication with the patient external device for transmission of the implant control input to a remote display portal of the patient external device for the communication with the implantable medical device, wherein the second application is configured to be accessed through the first application. The patient display device comprises a first log-in function and a second log-in function, wherein the first log-in function gives the user access to the first application and wherein the first and second log-in function in combination gives the user access to the second application. The first log-in function may be configured to use at least one of a password, pin code, fingerprint, voice and face recognition. A second log-in function within the first application may be configured to use a private key from the user to authenticate, for a defined time period, a second hardware key of the patient external device.

According to one embodiment, the first log-in is a PIN-based log-in.

According to one embodiment, at least one of the first and second log-in is a log-in based on a biometric input or a hardware key.

According to one embodiment, the patient display device further comprises an auxiliary wireless communication unit, and wherein the auxiliary wireless communication unit is configured to be disabled to enable wireless communication with the patient external device.

According to one embodiment, the patient display device is configured to wirelessly receive an implant control interface as a remote display portal from the patient external device to be displayed on the display.

According to one embodiment, the wireless communication unit is configured for wireless communication with the patient external device using a standard network protocol.

According to one embodiment, the wireless communication unit is configured for wireless communication with the patient external device using a proprietary network protocol.

According to one embodiment, the wireless communication unit is configured for wireless communication with the patient external device using a first network protocol and with the server using a second network protocol.

According to one embodiment, the wireless communication unit is configured for wireless communication with the patient external device using a first frequency band and with the server using a second frequency band.

According to one embodiment, the wireless communication unit comprises a Bluetooth transceiver.

According to one embodiment, the wireless communication unit comprises a UWB transceiver.

According to one embodiment, the standard network protocol is one from the list of: Radio Frequency type protocol, RFID type protocol, WLAN type protocol, Bluetooth type protocol, BLE type protocol, NFC type protocol, 3G/4G/5G type protocol, and GSM type protocol.

According to one embodiment, a communication range of the wireless communication unit is less than a communication range of the auxiliary wireless communication unit.

According to one embodiment, the wireless communication unit comprises a first wireless transceiver for communication with the patient external device and a second wireless transceiver for communication with the server.

According to one embodiment, the second wireless transceiver is configured to be disabled to enable wireless communication using the first wireless transceiver.

According to one embodiment, the patient display device is configured to authenticate the patient external device if a distance between the patient display device and the patient external device is less than a predetermined threshold value, or to be authenticated by the patient external device if a distance between the patient display device and the patient external device is less than a predetermined threshold value.

According to one embodiment, the patient display device is configured to allow the transfer of data between the patient display device and the patient external device on the basis of the authentication.

According to one embodiment, the patient display device is a wearable external device or a handset.

According to one embodiment, the second application is configured to receive data related to a parameter of the implanted medical device.

According to one embodiment, the second application is configured to receive data related to a sensor value received from the implanted medical device.

According to one embodiment, the second application is configured to receive data related to a parameter related to at least one of: a battery status, a temperature, a time, and an error.

According to one embodiment, the patient display device is configured to encrypt the user input.

According to one embodiment, the display is configured to encrypt the user input for decryption by the implantable medical device.

According to one embodiment, the patient display device is configured to decrypt the control interface received from the patient external device, for displaying the control interface on the display.

According to one embodiment, at least one of the first and second application is configured to receive data from an auxiliary external device and present the received data to the user.

According to one embodiment, at least one of the first and second application is configured to receive data from an auxiliary external device comprising a scale for determining the weight of the user.

According to one embodiment, at least one of the first and second application is configured to receive data related to the weight of the user from an auxiliary external device comprising a scale.

According to one embodiment, the patient display device is configured to: wirelessly transmit the data related to the weight of the user to the patient external device, or wirelessly transmit an instruction derived from the data related to the weight of the user, or wirelessly transmit an instruction derived from a combination of the data related to the weight of the user and the implant control input received from the user.

According to one embodiment, the system comprises a master private key device configured to allow issuance of a new private key device, wherein the HCP or HCP admin have such master private key device adapted to able to replace and pair a new patient private key device or HCP private key device into the system, through the HCP EID external device.

According to one embodiment, the patient remote external device and the patient EID external device are an integrated unit.

According to one embodiment, the HCP dedicated device and the HCP EID external device are an integrated unit.

According to one embodiment, the system comprises a measurement device or sensor adapted to deliver a measurement to at least one of the DDI, patent EID external device and a patient display device.

According to one embodiment, the system comprises a food sensor, adapted to measure at least if the patient swallows solid food or is drinking fluid, wherein said food sensor is connected to the control unit of a medical device to cause an action to stretch the stomach after a determined amount of food intake.

A communication system for enabling communication between a patient display device and an implantable medical device, when implanted, is provided. The communication system comprises a patient display device, a server or DDI, and a patient remote external device. The patient display device comprises a wireless communication unit configured for wirelessly receiving an implant control interface as a remote display portal from the patient remote external device, the wireless communication unit further being configured for wirelessly transmitting implant control user input to the patient remote external device, a display for displaying the received implant control interface as a remote display portal, and an input device for receiving implant control input from the user. The patient display device is configured to run a first application for wireless communication with the server, and to run a second application for wireless communication with the patient remote external device for transmission of the implant control input to the remote display portal of the patient remote external device for the communication with the implantable medical device. The patient remote external device comprises a wireless communication unit configured for wireless transmission of control commands based on the implant control input to the implantable medical device and configured for wireless communication with the patient display device.

According to one embodiment, the patient display device comprises a first log-in function and a second log-in function, and wherein the first log-in function gives the user access to the first application and wherein the first and second log-in function in combination gives the user access to the second application.

According to one embodiment, the second application is configured to receive data related to a parameter of the implanted medical device.

According to one embodiment, the second application is configured to receive data related to a sensor value received from the implanted medical device.

According to one embodiment, the second application is configured to receive data related to a parameter related to at least one of: a battery status,
a temperature, a time, or an error.

According to one embodiment, the patient display device is configured to encrypt the user input.

According to one embodiment, the display is configured to encrypt the user input for decryption by the implantable medical device.

According to one embodiment, the patient remote external device is configured to act as a router, transferring the encrypted user input from the patient display device to the implantable medical device without decryption.

According to one embodiment, the patient remote external device is configured to encrypt at least one of the control interface and the control commands.

According to one embodiment, the patient remote external device is configured to encrypt the control interface and wherein the patient display device is configured to decrypt the encrypted control interface.

A computer program product is provided, configured to run in a patient display device comprising a wireless communication unit, a display for displaying the received implant control interface as a remote display portal, and an input device for receiving implant control input from a user. The computer program product comprises:
a first application for communication with a server or DDI,
a second application for communication with an patient remote external device for transmission of the implant control input via the remote display portal of the patient remote external device for the communication with an implantable medical device, wherein the second application is configured to be accessed through the first application,
a first log-in function using at least one of a password, pincode, fingerprint, or face recognition, and
a second log-in function within the first application, using a private key from the user to authenticate for a defined time period a second hardware key of the patient remote external device. The first log-in function gives the user access to the first application and the first and second log-in function in combination gives the user access to the second application.

According to one embodiment, the second application is configured to receive data related to a parameter of the implanted medical device.

According to one embodiment, the second application is configured to receive data related to a sensor value received from the implanted medical device.

According to one embodiment, the second application is configured to receive data related to a parameter related to at least one of: a battery status,
a temperature, a time, or an error.

According to one embodiment of the communication system, patient display device or computer program product, the implantable medical device comprises at least one of:
an external heart compression device,
an apparatus assisting the pump function of a heart of the patient,
an apparatus assisting the pump function comprising a turbine bump placed within a patient's blood vessel for assisting the pump function of the heart,
an operable artificial heart valve,
an operable artificial heart valve for increasing the blood flow to the coronary arteries.
an implantable drug delivery device,
an implantable drug delivery device for injecting directly into a blood vessel and change the position of the injection site, all from within the patient's body,
an implantable drug delivery device for injecting potency enhancing drugs into an erectile tissue of the patient,
a hydraulic, mechanic, and/or electric constriction implant,
an operable volume filling device,
an operable gastric band,
an operable implant for stretching the stomach wall of the patient for creating satiety,
an implant configured to sense the frequency of the patient ingesting food,
an operable cosmetic implant,
an operable cosmetic implant for adjust the shape and/or size in the breast region of a patient,
an implant controlling medical device for the emptying of a urinary bladder,
an implant hindering urinary leakage,
an implant hindering anal incontinence,
an implant controlling the emptying of fecal matter,
an implant monitoring an aneurysm,
an implant for hindering the expansion of an aneurysm,
an implant lubricating a joint,
an implant for affecting the blood flow to an erectile tissue of the patient,
an implant for simulating the engorgement of an erectile tissue,
an implant with a reservoir for holding bodily fluids,
an implant storing and/or emptying a bodily reservoir or a surgically created
reservoir,
an implant communicating with a database outside the body,
an implant able to be programmed from outside the body,
an implant able to be programmed from outside the body with a wireless signal,
an implant treating impotence,
an implant controlling the flow of eggs in the uterine tube,
an implant controlling the flow of sperms in the uterine tube,
an implant controlling the flow of sperms in the vas deferens,
an implant for hindering the transportation of the sperm in the vas deferens,
an implant treating osteoarthritis,
an implant performing a test of parameters inside the body,
an implant controlling specific treatment parameters from inside the body,
an implant controlling bodily parameters from inside the body,
an implant controlling the blood pressure,
an implant controlling the blood pressure by affecting the dilatation of the renal artery,
an implant controlling a drug treatment parameter,
an implant controlling a parameter in the blood,
an implant for adjusting or replacing any bone part of a body of the patient,
an implant replacing an organ of the patient or part of an organ of the patient or the function thereof,
a vascular treatment device,
an implant adapted to move fluid inside the body of the patient,
an implant configured to sense a parameter related to the patient swallowing,
an implant configured to exercise a muscle with electrical or mechanical stimulation,
an implant configured for emptying an intestine portion on command,
an operable implant configured to be invaginated in the stomach of the patient to reduce the volume of the stomach substantially more than the volume of the device,
an implant configured for emptying the urinary bladder from within the patient's body by compressing the bladder,
an implant configured for draining fluid from within the patient's body,
an implant configured for the active lubrication of a joint with an added lubrication fluid,
an implant configured for removing clots and particles from the patient's blood stream,
an implant configured for elongating or straightening a bone in the patient, to reduce scoliosis,
a device to stimulate the brain for a several position to a focused point,
an artificial stomach replacing the function of the natural stomach,
an implant configured for adjusting the position of a female's urinary tract or bladder neck,
an implant configured for stimulating the ampulla vas deference and creating temporary constriction.

According to one embodiment, the system comprises a master private key device configured to allow issuance of a new private key device, wherein the HCP or HCP admin have such master private key device adapted to able to replace and pair a new patient private key device or HCP private key device into the system, through the HCP EID external device.

According to one embodiment, the patient remote external device and the patient EID external device are an integrated unit.

According to one embodiment, the HCP dedicated device and the HCP EID external device are an integrated unit.

According to one embodiment, the system comprises a measurement device or sensor adapted to deliver a measurement to at least one of the DDI, patent EID external device and a patient display device.

According to one embodiment, the system comprises a food sensor, adapted to measure at least if the patient swallows solid food or is drinking fluid, wherein said food sensor is connected to the control unit of a medical device to cause an action to stretch the stomach after a determined amount of food intake.

A communication system for enabling communication between a patient display device, a patient external device, a server and an implantable medical device, is provided. The communication system comprises a server,
a patient display device, a patient external device, and an implantable medical device. The patient display device comprises a wireless communication unit for wirelessly communicating with at least one of the patient external device and the server, a display, and an input device for receiving input from the user. The patient external device comprises a wireless communication unit configured for wireless transmission of control commands to the implantable medical device and configured for wireless communication with at least one of the patient display device and the server. Further, the server comprises a wireless communication unit configured for wireless communication with at least one of the patient display device and the patient external device, wherein the implantable medical device comprises a wireless communication unit configured for wireless communication with the patient external device. The implantable medical device further comprises an encryption unit and is configured to: encrypt data destined for the server, transmit the data to the server via the patient external device, wherein the patient external device acts as a router transferring the data without full decryption. In an example, the implantable medical device comprises an encryption unit and is configured to: encrypt data destined for the patient display device, transmit the data to the patient display device via the patient external device, wherein the patient external device acts as a router transferring the data without full decryption. In an example, the server comprises an encryption unit and is configured to: encrypt data destined for the implantable medical device, transmit the data to the implantable medical device via the patient external device, wherein the patient external device acts as a router transferring the data without full decryption, In an example, the server comprises an encryption unit and is configured to: encrypt data destined for the implantable medical device, transmit the data to the implantable medical device via the patient display device and the patient external device, wherein the patient display device and the patient external device acts as a router transferring the data without full decryption. In an example, the patient display device comprises an encryption unit and is configured to: encrypt data destined for the implantable medical device, transmit the data to the implantable medical device via the patient external device, wherein the patient external device acts as a router transferring the data without full decryption. In an example, the patient display device comprises an encryption unit and is configured to: encrypt data destined for the implantable medical device, transmit the data to the implantable medical device via the server and the patient external device, wherein the server and the patient external device acts as a router transferring the data without full decryption.

According to one embodiment, the patient display device is configured to wirelessly receive an implant control interface from the patient external device to be displayed on the display.

According to one embodiment, at least two of: the wireless communication unit of the server, the wireless communication unit of the patient display device, the wireless communication unit of the patient external device, and the wireless communication unit of the implantable medical device, are configured for wireless communication using a standard network protocol.

According to one embodiment, wherein at least two of: the wireless communication unit of the server, the wireless communication unit of the patient display device, the wireless communication unit of the patient external device, and the wireless communication unit of the implantable medical device, are configured for wireless communication using a proprietary network protocol.

According to one embodiment, the wireless communication unit of the patient external device is configured to use a first network protocol for communication with the implantable medical device and use a second network protocol for communication with the server, or use a first network protocol for communication with the implantable medical device and use a second network protocol for communication with the patient display device.

According to one embodiment, the wireless communication unit of the patient external device is configured to use a first frequency band for communication with the implantable medical device and use a second frequency band for communication with the server, or use a first frequency band for communication with the implantable medical device and use a second frequency band for communication with the patient display device.

According to one embodiment, the wireless communication unit of the patient display device is configured to use a first network protocol for communication with the patient external device and use a second network protocol for communication with the server.

According to one embodiment, the wireless communication unit of the patient display device is configured to use a first frequency band for communication with the patient external device and use a second frequency band for communication with the server.

According to one embodiment, the wireless communication unit of the server is configured to use a first network protocol for communication with the patient external device and use a second network protocol for communication with the patient display device.

According to one embodiment, the wireless communication unit of the server is configured to use a first frequency band for communication with the patient external device and use a second frequency band for communication with the patient display device.

According to one embodiment, the wireless communication unit of at least one of the server, the patient display device, the patient external device, and the implantable medical device comprises a Bluetooth transceiver.

According to one embodiment, the wireless communication unit of at least one of the server, the patient display device, the patient external device, and the implantable medical device comprises a UWB transceiver.

According to one embodiment, the standard network protocol is one from the list of: Radio Frequency type protocol, RFID type protocol, WLAN type protocol, Bluetooth type protocol, BLE type protocol, NFC type protocol, 3G/4G/5G type protocol, and GSM type protocol.

According to one embodiment, the wireless communication unit of the patient external device comprises a first wireless transceiver for wireless communication with the implantable medical device, and a second wireless transceiver for wireless communication with the server, and wherein the second wireless transceiver has a longer effective range than the first wireless transceiver.

According to one embodiment, the wireless communication unit of the patient external device comprises a first wireless transceiver for wireless communication with the implantable medical device, and a second wireless transceiver for wireless communication with the patient display device, and wherein the second wireless transceiver has a longer effective range than the first wireless transceiver.

According to one embodiment, the wireless communication unit of the patient display device comprises a first wireless transceiver for wireless communication with the patient external device, and a second wireless transceiver for wireless communication with the server, and wherein the second wireless transceiver has a longer effective range than the first wireless transceiver.

According to one embodiment, the second wireless transceiver has an effective range being one of: 2 times, 4 times, 8 times 20 times, 50 times or 100 times longer than the first wireless transceiver.

According to one embodiment, the second wireless transceiver is configured to be disabled to enable wireless communication using the first wireless transceiver.

According to one embodiment, at least one of:
the patient display device is configured to authenticate the patient external device if a distance between the patient display device and the patient external device is less than a predetermined threshold value,
the patient display device is configured to be authenticated by the patient external device if a distance between the patient display device and the patient external device is less than a predetermined threshold value,
the patient display device is configured to authenticate the implantable medical device if a distance between the patient display device and the implantable medical device is less than a predetermined threshold value,
the patient display device is configured to be authenticated by the implantable medical device if a distance between the patient display device and the implantable medical device is less than a predetermined threshold value,
the patient external device is configured to authenticate the patient display device if a distance between the patient external device and the patient display device is less than a predetermined threshold value,
the patient external device is configured to be authenticated by the patient display device if a distance between the patient external device and the patient display device is less than a predetermined threshold value,
the patient external device is configured to authenticate the implantable medical device if a distance between the patient external device and the implantable medical device is less than a predetermined threshold value, and
the patient external device is configured to be authenticated by the implantable medical device if a distance between the patient external device and the implantable medical device is less than a predetermined threshold value.

According to one embodiment, the patient display device is configured to allow the transfer of data between the patient display device and the patient external device on the basis of the authentication.

According to one embodiment, the patient external device is configured to allow the transfer of data between the patient display device and the patient external device on the basis of the authentication.

According to one embodiment, the patient external device is configured to allow the transfer of data between the patient external device and the implantable medical device on the basis of the authentication.

According to one embodiment, the patient display device is a wearable patient external device or a handset.

According to one embodiment, the data encrypted by the implantable medical device is related to at least one of: a battery status, a temperature, a time, or an error.

A server for use in the communication system according to any one of the above embodiments is provided.

A patient display device for use in the communication system according to any one of the above embodiments is provided.

A patient external device for use in the communication system according to any one of the above embodiments is provided.

An implantable medical device for use in the communication system according to any one of the above embodiments is provided.

According to one embodiment, the system comprises a master private key device configured to allow issuance of a new private key device, wherein the HCP or HCP admin have such master private key device adapted to able to replace and pair a new patient private key device or HCP private key device into the system, through the HCP EID external device.

According to one embodiment, the patient remote external device and the patient EID external device are an integrated unit.

According to one embodiment, the HCP dedicated device and the HCP EID external device are an integrated unit.

According to one embodiment, the system comprises a measurement device or sensor adapted to deliver a measurement to at least one of the DDI, patent EID external device and a patient display device.

According to one embodiment, the system comprises a food sensor, adapted to measure at least if the patient swallows solid food or is drinking fluid, wherein said food sensor is connected to the control unit of a medical device to cause an action to stretch the stomach after a determined amount of food intake.

A system configured for changing pre-programmed treatment settings of an implantable medical device, when implanted in a patient, from a distant remote location in relation to the patient, is provided. The system comprises at least one health care provider, HCP, EID external device, and a HCP private key device. HCP EID external device is adapted to receive a command from the HCP to change said pre-programmed treatment settings of an implanted medical device, and further adapted to be activated and authenticated and allowed to perform said command by the HCP providing the HCP private key device, wherein the HCP private key device is adapted to be provided to the HCP EID external device via at least one of: a reading slot or comparable for the HCP private key device, and a RFID communication or other close distance wireless activation communication. The HCP EID external device comprises at least one of: a reading slot or comparable for the HCP private key device, a RFID communication, and other close distance wireless activation communication or electrical direct contact. The HCP EID external device further comprises at least one wireless transceiver configured for communication with a data infrastructure server, DDI, through a first network protocol. Further, the system comprises a data infrastructure server, DDI, adapted to receive command from said HCP EID external device and to relay the received command without modifying said command to a patient EID external device, wherein the DDI comprises one wireless transceiver configured for communication with said patient external device, and a patient EID external device adapted to receive the command relayed by the DDI, further adapted to send this command to the implanted medical device, further adapted to receive a command from the HCP EID external device via the DDI to change said pre-programmed treatment settings of the implanted medical device, and further adapted to be activated and authenticated and allowed to perform said command by the patient providing a patient private key device adapted to be provided to the patient EID external device by the patient via at least one of: a reading slot or comparable for the patient private key device, a RFID communication or other close distance wireless activation communication or electrical direct contact. The patient EID external device comprises at least one of a reading slot or comparable for the HCP private key device, a RFID communication, and other close distance wireless activation communication or electrical direct contact. The patient EID external device further comprises at least one wireless transceiver configured for communication with the implanted medical device through a second network protocol. Further, the implanted medical device is configured to treat the patient or perform a bodily function.

According to one embodiment, at least one of the patient private key device or HCP private key device comprises a hardware key.

According to one embodiment, the private key device is at least one of, a smartcard, a keyring device, a watch an arm or wrist band a neckless or any shaped device.

According to one embodiment of the system, at least two of: the HCP EID external device, the patient EID external device, the HCP private key device, the patient private key device, and the DDI are configured for wireless communication using a standard network protocol.

According to one embodiment, at least two of: the HCP EID external device, the patient EID external device, the HCP private key device, the patient private key device, and the DDI are configured for wireless communication using a proprietary network protocol.

According to one embodiment, the patient EID external device is configured to use a first network protocol for communication with the implantable medical device and use a second network protocol for communication with the DDI.

According to one embodiment, the patient EID external device is configured to use a first frequency band for communication with the implantable medical device and use a second frequency band for communication with the DDI.

According to one embodiment, the DDI is configured to use a first frequency band for communication with the patient EID external device and a second frequency band for communication with the patient private key device.

According to one embodiment, at least one of the HCP EID external device, the patient EID external device, the HCP private key device, the patient private key device and the DDI comprises a Bluetooth transceiver.

According to one embodiment, at least one of the HCP EID external device, the patient EID external device, the HCP private key device, the patient private key device and the DDI comprises a UWB transceiver.

According to one embodiment, the standard network protocol is one from the list of: Radio Frequency type protocol, RFID type protocol, WLAN type protocol, Bluetooth type protocol, BLE type protocol, NFC type protocol, 3G/4G/5G type protocol, and GSM type protocol.

According to one embodiment, the patient EID external device comprises a first wireless transceiver for wireless communication with the implantable medical device, and a second wireless transceiver for wireless communication with the DDI, and wherein the second wireless transceiver has longer effective range than the first wireless transceiver.

According to one embodiment, the patient private key device comprises a first wireless transceiver for wireless communication with the HCP EID external device, and a second wireless transceiver for wireless communication with the DDI, and wherein the second wireless transceiver has longer effective range than the first wireless transceiver.

According to one embodiment, the second wireless transceiver has an effective range being one of: 2 times, 4 times, 8 time, 20 times, 50 times or 100 times longer than the effective range of the first wireless transceiver.

According to one embodiment, the second wireless transceiver is configured to be disabled to enable wireless communication using the first wireless transceiver.

According to one embodiment, the patient EID external device is configured to allow transfer of data between the EID external device and the implantable medical device on the basis of an authentication of the patient EID external device.

According to one embodiment, the patient EID external device is a wearable patient external device or a handset.

According to one embodiment, the data encrypted by the implantable medical device is related to at least one of: a battery status, a temperature, a time, or an error.

According to one embodiment, the system comprises a master private key device configured to allow issuance of a new private key device, wherein the HCP or HCP admin have such master private key device adapted to able to replace and pair a new patient private key device or HCP private key device into the system, through the HCP EID external device.

According to one embodiment, the patient remote external device and the patient EID external device are an integrated unit.

According to one embodiment, the HCP dedicated device and the HCP EID external device are an integrated unit.

According to one embodiment, the system comprises a measurement device or sensor adapted to deliver a measurement to at least one of the DDI, patent EID external device and a patient display device.

According to one embodiment, the system comprises a food sensor, adapted to measure at least if the patient swallows solid food or is drinking fluid, wherein said food sensor is connected to the control unit of a medical device to cause an action to stretch the stomach after a determined amount of food intake.

A system is provided, configured for changing pre-programmed treatment settings of an implantable medical device, when implanted in a patient, by a health care provider, HCP, in the physical presence of the patient. The system comprises at least one HCP EID external device adapted to receive a command from the HCP, directly or indirectly, to change said pre-programmed treatment settings in steps of an implantable medical device, when implanted, wherein the HCP EID external device is further adapted to be activated, authenticated, and allowed to perform said command by the HCP providing an HCP private key device comprising a HCP private key. The HCP private key device comprises at least one of: a smart card, a keyring device, a watch, a arm or wrist band, a necklace, and any shaped device. The HCP EID external device is adapted to be involved in at least one of: receiving information from the implant, receiving information from a patient remote external device, actuating the implanted medical device, changing pre-programmed settings, and updating software of the implantable medical device, when implanted. The HCP EID external device is further adapted to be activated, authenticated, and allowed to perform said command also by the patient. The system further comprises a patient private key device comprising a patient private key, wherein the patient private key device comprising at least one of: a smart card, a keyring device, a watch, a arm or wrist band, a necklace, and any shaped device. The HCP private key and the patient private key are required for performing said actions by the HCP EID external device to at least one of: receive information from the implant, to receive information from a patient remote external device, to actuate the implanted medical device, to change pre-programmed settings, and to update software of the implantable medical device, when the implantable medical device is implanted.

According to one embodiment, the HCP EID external device further comprises a wireless transceiver configured for communication with the implanted medical device through a second network protocol.

According to one embodiment, the HCP private key device is adapted to be provided to the at least one HCP external device via at least one of: a reading slot or comparable for the HCP private key device, a RFID communication, and a close distance wireless activation communication unit, or electrical direct contact.

According to one embodiment, the HCP EID external device comprises at least one of reading slot or comparable for the HCP private key device,
a RFID communication and a close distance wireless activation communication unit, or electrical direct contact.

According to one embodiment, the HCP EID external device is adapted to receive a command from a HCP dedicated device to change said pre-programmed treatment steps of the implantable medical device, when implanted, wherein the HCP dedicated device is further adapted to be activated, authenticated, and allowed to perform said command by the HCP providing their private key.

According to one embodiment, at least two of: the HCP EID external device, the patient EID external device, the HCP private key device, and the patient private key device, are configured for wireless communication using a standard network protocol.

According to one embodiment, at least two of: the HCP EID external device, the patient EID external device, the HCP private key device, and the patient private key device, are configured for wireless communication using a proprietary network protocol.

According to one embodiment, the patient EID external device is configured to use a first network protocol for communication with the implantable medical device and use a second network protocol for communication with the patient private key device.

According to one embodiment, the patient EID external device is configured to use a first frequency band for communication with the implantable medical device and use a second frequency band for communication with the patient private key device.

According to one embodiment, at least one of the HCP EID external device, the patient EID external device, the HCP private key device, and the patient private key device comprises a Bluetooth transceiver.

According to one embodiment, at least one of the HCP EID external device, the patient EID external device, the HCP private key device, and the patient private key device comprises a UWB transceiver.

According to one embodiment, the standard network protocol is one from the list of: Radio Frequency type protocol, RFID type protocol, WLAN type protocol, Bluetooth type protocol, BLE type protocol, NFC type protocol, 3G/4G/5G type protocol, and GSM type protocol.

According to one embodiment, the patient EID external device comprises a first wireless transceiver for wireless communication with the implantable medical device, and a second wireless transceiver for wireless communication with the patient private key device, and wherein the second wireless transceiver has longer effective range than the first wireless transceiver.

According to one embodiment, the second wireless transceiver has an effective range being one of: 2 times, 4 times, 8 time, 20 times, 50 times or 100 times longer than the effective range of the first wireless transceiver.

According to one embodiment, the second wireless transceiver is configured to be disabled to enable wireless communication using the first wireless transceiver.

According to one embodiment, the patient EID external device is configured to allow transfer of data between the EID external device and the implantable medical device on the basis of an authentication of the patient EID external device.

According to one embodiment, the patient EID external device is a wearable patient external device or a handset.

According to one embodiment, the data encrypted by the implantable medical device is related to at least one of: a battery status, a temperature, a time, or an error.

A system is provided, configured to change pre-programmed and pre-selected treatment actions of an implantable medical device, when implanted in a patient, by command from the patient. The system comprises an implantable medical device, a patient remote external device, a wireless transceiver configured for communication with the implantable medical device, when the medical device is implanted, through a second network protocol, and a remote display portal. The remote display portal is configured to receive content delivered from the patient remote external device to expose buttons to express the will to actuate the functions of the implanted medical device by the patient through the patient remote external device, and further configured to present the display portal remotely on a patient display device allowing the patient to actuate the functions of the implanted medical device through the display portal of the patient remote external device visualised on the patient display device.

According to one embodiment, the wireless transceiver, the remote display portal, and the remote display portal are comprised in the patient remote external device.

According to one embodiment, the system further comprises the patient display device, which may comprise a supporting application, a display which hosts the Remote Display Portal, and a patient display device private key.

According to one embodiment, the remote display portal is capable of generating a command to be signed by the patient display device private key.

According to one embodiment, the patient remote external device is adapted to accept input from the patient via said patient display device through its remote display portal.

According to one embodiment, the patient remote external device comprises a graphical user interface arranged on a touch-responsive display exposing buttons to express actuation functions of the implanted medical device.

According to one embodiment, the system is configured to allow the patient to actuate the implant at home through the patient remote external device by means of an authorization granted by a patient private key.

According to one embodiment, the patient private key comprises at least one of: a smart card, a keyring device, a watch, a arm or wrist band, a necklace, and any shaped device.

According to one embodiment, the system is configured to allow the patient to actuate the implantable medical device, when implanted, at home through the patient remote external device, using an authorization granted by the patient private key.

According to one embodiment, system further comprises a patient EID external device comprising at least one of: a reading slot or comparable for the patient private key device, a RFID communication, and a close distance wireless activation communication, or electrical direct contact.

According to one embodiment, the patient EID external device is adapted to be synchronised with the patient remote external device.

According to one embodiment, the patient EID external device further comprises at least one of: a wireless transceiver configured for communication with the patient, a remote external device, and a wired connector for communication with the patient remote external device.

According to one embodiment, the patient EID external device is adapted to generate an authorization to be signed by the patient private key to be installed into at least one of: the patient remote external device through the patient EID external device, and the implantable medical device.

According to one embodiment, the system comprises a patient display device comprising a supporting application capable of displaying the remote display portal with content delivered from the patient remote external device.

According to one embodiment, the remote display portal and patient remote external device are adapted to expose buttons to express the will to actuate the functions of the implanted medical device by the patient through the patient remote external device.

According to one embodiment, the patient display device comprises at least one of: a display which hosts the remote display portal, and a patient display device private key.

According to one embodiment, the remote display portal is capable of generating a command to be signed by the patient private key.

According to one embodiment, the system comprises a master private key device configured to allow issuance of a new private key device, wherein the HCP or HCP admin have such master private key device adapted to able to replace and pair a new patient private key device or HCP private key device into the system, through the HCP EID external device.

According to one embodiment, the patient remote external device and the patient EID external device are an integrated unit.

According to one embodiment, the HCP dedicated device and the HCP EID external device are an integrated unit.

According to one embodiment, the system comprises a measurement device or sensor adapted to deliver a measurement to at least one of the DDI, patent EID external device and a patient display device.

According to one embodiment, the system comprises a food sensor, adapted to measure at least if the patient swallows solid food or is drinking fluid, wherein said food sensor is connected to the control unit of a medical device to cause an action to stretch the stomach after a determined amount of food intake.

A system is provided, configured for providing information from an implantable medical device, when implanted in a patient, from a distant remote location in relation to the patient. The system comprises at least one patient EID external device adapted to receive information from the implant, adapted to send such information further on to a server or dedicated data infrastructure, DDI, further adapted to be activated and authenticated and allowed to receive said information by the implanted medical device by the patient providing a private key. Further, the system comprises a patient private key device comprising the private key adapted to be provided to the patient EID external device via at least one of: a reading slot or comparable for the patient private key device, a RFID communication or other close distance wireless activation communication or direct electrical connection. The patient EID external device comprises at least one of: a reading slot or comparable for the patient private key device, an RFID communication, and other close distance wireless activation communication or direct electrical contact. Further, the patient EID external device comprises at least one wireless transceiver configured for communication with the DDI, through a first network protocol.

According to one embodiment, the at least one patient EID external device is adapted to receive information from the implant, through a second network protocol.

According to one embodiment, the system comprises the DDI, wherein the DD1 is adapted to receive information from said patient EID external device, and wherein the DDI comprises a wireless transceiver configured for communication with said patient EID external device.

According to one embodiment, the patient EID external device is adapted to receive a command relayed by the DDI, to further send the command to the implanted medical device to change said pre-programmed treatment settings of the implanted medical device, and further adapted to be activated and authenticated and allowed to perform said command by the patient providing the patient private key.

According to one embodiment, the patient private key device is adapted to provide the patient private key to the patient EID external device by the patient via at least one of; a reading slot or comparable for the patient private key device, an RFID communication or other close distance wireless activation communication, or electrical direct contact.

According to one embodiment, the patient EID external device comprises at least one of: a reading slot or comparable for the HCP private key device, a RFID communication, and other close distance wireless activation communication, or direct electrical contact.

According to one embodiment, the patient EID external device further comprising at least one wireless transceiver configured for communication with the implanted medical device through a second network protocol.

According to one embodiment, the system comprises the implantable medical device, which may be adapted to, when implanted, treat the patient or perform a bodily function.

According to one embodiment, the patient private key comprises at least one of: a smart card, a keyring device, a watch, an arm band or wrist band, a necklace, and any shaped device.

According to one embodiment, at least two of: the patient EID external device, the IDD, and the patient private key device, are configured for wireless communication using a standard network protocol.

According to one embodiment, at least two of: the patient EID external device, the IDD, and the patient private key device, are configured for wireless communication using a proprietary network protocol.

According to one embodiment, the patient EID external device is configured to use a first network protocol for communication with the implantable medical device and use a second network protocol for communication with the patient private key device.

According to one embodiment, the patient EID external device is configured to use a first frequency band for communication with the implantable medical device and use a second frequency band for communication with the patient private key device.

According to one embodiment, at least one of the patient EID external device, the patient private key device and the IDD comprises a Bluetooth transceiver.

According to one embodiment,
at least one of the patient EID external device, the patient private key device and the IDD comprises a UWB transceiver.

According to one embodiment, the standard network protocol is one from the list of: Radio Frequency type protocol, RFID type protocol, WLAN type protocol, Bluetooth type protocol, BLE type protocol, NFC type protocol, 3G/4G/5G type protocol, and GSM type protocol.

According to one embodiment, the patient EID external device comprises a first wireless transceiver for wireless communication with the implantable medical device, and a second wireless transceiver for wireless communication with the patient private key device, and wherein the second wireless transceiver has longer effective range than the first wireless transceiver.

According to one embodiment, the second wireless transceiver has an effective range being one of: 2 times, 4 times, 8 time, 20 times, 50 times or 100 times longer than the effective range of the first wireless transceiver.

According to one embodiment, the second wireless transceiver is configured to be disabled to enable wireless communication using the first wireless transceiver.

According to one embodiment, the patient EID external device is a wearable patient external device or a handset.

According to one embodiment, the data encrypted by the implantable medical device is related to at least one of: a battery status, a temperature, a time, or an error.

According to one embodiment, the system comprises a master private key device configured to allow issuance of a new private key device, wherein the HCP or HCP admin have such master private key device adapted to able to replace and pair a new patient private key device or HCP private key device into the system, through the HCP EID external device.

According to one embodiment, the patient remote external device and the patient EID external device are an integrated unit.

According to one embodiment, the HCP dedicated device and the HCP EID external device are an integrated unit.

According to one embodiment, the system comprises a measurement device or sensor adapted to deliver a measurement to at least one of the DDI, patent EID external device and a patient display device.

According to one embodiment, the system comprises a food sensor, adapted to measure at least if the patient swallows solid food or is drinking fluid, wherein said food sensor is connected to the control unit of a medical device to cause an action to stretch the stomach after a determined amount of food intake.

A system is provided, comprising, an implantable medical device adapted to, when implanted in a patient, to communicate with an external device, the external device comprising at least one of a patient remote external device or a patient EID external device. The system further comprises the patient EID external device adapted to communicate with and send commands to the implantable medical device when implanted, to change pre-programmed settings, and a patient private key device comprising a patient private key, adapted to activate and authenticate and allow to perform said command by the patient EID external device, wherein said private key is adapted to be provided to the external device via at least one of: a reading slot or comparable for the HCP private key device, an RFID communication or other close distance wireless activation communication, or direct electrical contact. Further the system comprises a data infrastructure server, DDI, adapted to send commands to the patient EID external device for further transport to the implanted medical device, to inactivate the authority and authenticating function of the patient private key.

According to one embodiment, the at least one patient remote external device comprises a patient remote external device private key, wherein the DDI via the patient EID external device is able to inactivate the authority and authenticating function of the patient remote external device, thereby inactivating the patient remote external device.

According to one embodiment, the patient EID external device comprises at least one wireless transceiver configured for communication with the DD1 via a first network protocol.

According to one embodiment, the system comprises the DDI, wherein the DDI is adapted to receive command from a HCP EID external device, and to send the received command to the patient EID external device, wherein the DDI comprises a wireless transceiver configured for communication with said patient external device.

According to one embodiment, the patient EID external device is adapted to receive the command from the DDI, wherein the command originates from a health care provider, HCP, and wherein the patient EID is adapted to inactivate the patient private key and to send the command to the implanted medical device.

According to one embodiment, the patient EID external device is adapted to receive the command from the DDI, wherein the command originates from a health care provider, HCP, wherein the patient EID external device is adapted to receive the command from the HCP via the DDI to inactivate the patient remote external device comprising a patient remote external device private key, and wherein the patient EID external device is further adapted to send this command to the implanted medical device.

According to one embodiment, the patient EID external device further comprises at least one wireless transceiver configured for communication with the implanted medical device through a second network protocol.

According to one embodiment, at least one of the patient private key and a patient remote external device private key comprises a hardware key.

According to one embodiment, the private key device is at least one of, a smartcard, a keyring device, a watch an arm or wrist band a neckless or any shaped device.

According to one embodiment, at least two of: the patient remote external device, the patient EID external device, the patient private key device, and the DDI, are configured for wireless communication using a standard network protocol.

According to one embodiment, wherein at least two of: the patient remote external device, the patient EID external device, the patient private key device, and the DDI, are configured for wireless communication using a proprietary network protocol.

According to one embodiment, the patient EID external device is configured to use a first network protocol for communication with the implantable medical device and use a second network protocol for communication with the patient private key device.

According to one embodiment, the patient EID external device is configured to use a first frequency band for communication with the implantable medical device and use a second frequency band for communication with the patient private key device.

According to one embodiment, at least one of the patient remote external device, the patient EID external device, the patient private key device, and the DDI, comprise a Bluetooth transceiver.

According to one embodiment, at least one of the patient remote external device, the patient EID external device, the patient private key device, and the DDI, comprise an UWB transceiver.

According to one embodiment, the standard network protocol is one from the list of: Radio Frequency type protocol, RFID type protocol, WLAN type protocol, Bluetooth type protocol, BLE type protocol, NFC type protocol, 3G/4G/5G type protocol, and GSM type protocol.

According to one embodiment, the patient EID external device comprises a first wireless transceiver for wireless communication with the implantable medical device, and a second wireless transceiver for wireless communication with the patient private key device, and wherein the second wireless transceiver has longer effective range than the first wireless transceiver.

According to one embodiment, the second wireless transceiver has an effective range being one of: 2 times, 4 times, 8 time, 20 times, 50 times or 100 times longer than the effective range of the first wireless transceiver.

According to one embodiment, the second wireless transceiver is configured to be disabled to enable wireless communication using the first wireless transceiver.

According to one embodiment, the patient EID external device is a wearable patient external device or a handset.

According to one embodiment, the data encrypted by the implantable medical device is related to at least one of: a battery status, a temperature, a time, or an error.

According to one embodiment, the system comprises a master private key device configured to allow issuance of new private key device, wherein the HCP or HCP admin have such master private key device adapted to be able to replace and pair a new patient private key device or HCP private key device into the system, through the HCP EID external device.

According to one embodiment, the patient remote external device and the patient EID external device are an integrated unit.

According to one embodiment, the HCP dedicated device and the HCP EID external device are an integrated unit.

According to one embodiment, the system comprises a measurement device or sensor adapted to deliver a measurement to at least one of the DDI, patent EID external device and a patient display device.

According to one embodiment, the system comprises a food sensor, adapted to measure at least if the patient swallows solid food or is drinking fluid, wherein said food sensor is connected to the control unit of a medical device to cause an action to stretch the stomach after a determined amount of food intake.

A system is provided, configured for changing pre-programmed treatment settings in steps of an implantable medical device, when implanted in a patient, by a health care provider, HCP, either in the physical presence of the patient or remotely with the patient on distance. The system comprises at least one HCP EID external device adapted to receive a command directly or indirectly from the HCP to change said pre-programmed treatment settings in steps of the implantable medical device, when implanted. The HCP EID external device is further adapted to be activated, authenticated, and allowed to perform said command by the HCP providing a HCP private key device comprising a HCP private key. The HCP private key comprises at least one of: a smart card, a keyring device, a watch, an arm or wrist band, a necklace, and any shaped device. The system further comprises a patient private key device comprising a patient private key, comprising at least one of: a smart card, a keyring device, a watch, an arm or wrist band, a necklace, and any shaped device. Both the HCP and patient private key is required for performing said action by the HCP EID external device to change the pre-programmed settings in the implant and to update software of the implantable medical device, when the implantable medical device is implanted. The patient private key is adapted to activate, be authenticated, and allowed to perform said command provided by the HCP, either via the HCP EID external device or when the action is performed remotely via a patient EID external device.

According to one embodiment, the system comprises a master private key device that allow issuance of new private key device wherein the HCP or HCP admin have such master private key device adapted to be able to replace and pair a new patient private key device or HCP private key device into the system, through the HCP EID external device.

According to one embodiment, the patient remote external device and the patient EID external device are an integrated unit.

According to one embodiment, the HCP dedicated device and the HCP EID external device are an integrated unit.

According to one embodiment, the system further comprises a measurement device or sensor adapted to deliver a measurement to at least one of the DDI, patent EID external device and a patient display device.

According to one embodiment, the system further comprises a food sensor adapted to measure at least if the patient swallows solid food or is drinking fluid, wherein said food sensor is configured to be connected to the control unit of a medical device to cause an action to stretch the stomach after a determined amount of food intake.

According to one embodiment, the HCP EID external device further comprises a wireless transceiver configured for communication with the implanted medical device through a second network protocol.

According to one embodiment, the HCP private key device is adapted to be provided to the at least one HCP external device via at least one of: a reading slot or comparable for the HCP private key device, a RFID communication, and a close distance wireless activation communication unit, or electrical direct contact.

According to one embodiment, the HCP EID external device comprises at least one of: reading slot or comparable for the HCP private key device, a RFID communication, and a close distance wireless activation communication unit, or electrical direct contact.

According to one embodiment, the HCP EID external device is adapted to receive a command from an HCP dedicated device to change said pre-programmed treatment steps of the implantable medical device, when implanted, wherein the HCP dedicated device is further adapted to be activated, authenticated, and allowed to perform said command by the HCP providing their private key.

According to one embodiment, the HCP EID external device and the HCP private key device are configured for wireless communication using a standard network protocol.

According to one embodiment, the HCP EID external device and the HCP private key device are configured for wireless communication using a proprietary network protocol.

According to one embodiment, the HCP EID external device is configured to use a first network protocol for communication with the implantable medical device and use a second network protocol for communication with the HCP private key device.

According to one embodiment, the HPC EID external device is configured to use a first frequency band for communication with the implantable medical device and use a second frequency band for communication with the HCP private key device.

According to one embodiment, at least one of the HCP EID external device and the HCP private key device comprises a Bluetooth transceiver.

According to one embodiment, at least one of the HCP EID external device and the HCP private key device comprises a UWB transceiver.

A system is provided, configured for changing pre-programmed treatment settings in steps of an implantable medical device, when implanted in a patient, by a health care provider, HCP, with the patient on remote on distance. The system comprises at least one HCP EID external device adapted to receive a command from the HCP direct or indirect, to change said pre-programmed treatment settings in steps of an implantable medical device, when implanted, wherein the HCP EID external device is further adapted to be activated, authenticated, and allowed to perform said command by the HCP. The action by the HCP EID external device to change pre-programmed settings in the implant and to update software of the implantable medical device, when the implantable medical device is implanted, is adapted to be authenticated by a HCP private key device and a patient private key device.

According to one embodiment, the HCP private key device comprising a HCP private key, comprising at least one of: a smart card, a keyring device, a watch, an arm or wrist band, a necklace, and any shaped device.

According to one embodiment, the patient private key device comprises a patient private key, comprising at least one of: a smart card, a keyring device, a watch, an arm or wrist band, a necklace, and any shaped device.

According to one embodiment, the patient private key is adapted to activate, be authenticated, and allowed to perform said command provided by the HCP, either via the HCP EID external device or when the action is performed remotely via a patient EID external device.

According to one embodiment, the system further comprises a dedicated data infrastructure, DDI, the patient EID external device, and the HCP EID external device, wherein the communication between the patient EID external device and the HCP EID external device is performed via the DDI.

According to one embodiment, the system comprises a master private key device that allows issuance of new private key device wherein the HCP or HCP admin have such master private key device adapted to be able to replace and pair a new patient private key device or HCP private key device into the system.

According to one embodiment, the patient remote external device and the patient EID external device are an integrated unit.

According to one embodiment, the HCP dedicated device and the HCP EID external device are an integrated unit.

According to one embodiment, the system further comprises a measurement device or sensor adapted to deliver a measurement to at least one of the DDI, patent EID external device and patient display device.

According to one embodiment, the system comprises a food sensor, adapted to measure at least if the patient swallow solid food or is drinking fluid, wherein said food sensor is connected to the control unit of a medical device to cause an action to stretch the stomach after a determined amount of food intake.

According to one embodiment, the HCP EID external device further comprises a wireless transceiver configured for communication with the implanted medical device through a second network protocol.

According to one embodiment, the HCP private key device is adapted to be provided to the at least one HCP external device via at least one of: a reading slot or comparable for the HCP private key device, a RFID communication, and a close distance wireless activation communication unit, or electrical direct contact.

According to one embodiment, the HCP EID external device comprises at least one of: reading slot or comparable for the HCP private key device, a RFID communication, and a close distance wireless activation communication unit, or electrical direct contact.

According to one embodiment, the HCP EID external device is adapted to receive a command from an HCP dedicated device to change said pre-programmed treatment steps of the implantable medical device, when implanted, wherein the HCP dedicated device is further adapted to be activated, authenticated, and allowed to perform said command by the HCP providing their private key.

According to one embodiment, the HCP EID external device and the HCP private key device are configured for wireless communication using a standard network protocol.

According to one embodiment, the HCP EID external device and the HCP private key device are configured for wireless communication using a proprietary network protocol.

According to one embodiment, the HCP EID external device is configured to use a first network protocol for communication with the implantable medical device and use a second network protocol for communication with the HCP private key device.

According to one embodiment, the HPC EID external device is configured to use a first frequency band for communication with the implantable medical device and use a second frequency band for communication with the HCP private key device.

According to one embodiment, at least one of the HCP EID external device and the HCP private key device comprises a Bluetooth transceiver.

According to one embodiment, at least one of the HCP EID external device and the HCP private key device comprises a UWB transceiver.

A system is provided, which is configured for changing pre-programmed treatment settings of an implantable medical device, when implanted in a patient, from a distant remote location in relation to the patient. The system comprises at least one health care provider, HCP, external device adapted to receive a command from the HCP to change said pre-programmed treatment settings of an implanted medical device. The HCP external device is further adapted to be activated and authenticated and allowed to perform said command by the HCP providing a HCP private key device adapted to be provided to an HCP EID external device via at least one of; a reading slot or comparable for the HCP private key device, a RFID communication or other close distance wireless activation communication. The HCP EID external device comprises at least one of: a reading slot or comparable for the HCP private key device, a RFID communication, and other close distance wireless activation communication or electrical direct contact. The HCP EID external device further comprises at least one wireless transceiver configured for communication with a patient EID external device, through a first network protocol. The system comprises the patient EID external device, the patient EID external device being adapted to receive command from said HCP external device, and to relay the received command without modifying said command to the implanted medical device. The patient EID external device comprises one wireless transceiver configured for communication with said patient external device, wherein the patient EID is adapted to send the command to the implanted medical device, to receive a command from the HCP to change said pre-programmed treatment settings of the implanted medical device, and further to be activated and authenticated and allowed to perform said command by the patient providing a patient private key device comprising a patient private key.

According to one embodiment, at least one of the patient private key device or HCP private key device comprises a hardware key.

According to one embodiment, the private key device is at least one of, a smartcard, a keyring device, a watch an arm or wrist band a neckless or any shaped device.

According to one embodiment, the system comprises a master private key device that allow issuance of new private key device wherein the HCP or HCP admin have such master private key device adapted to be able to replace and pair a new patient private key device or HCP private key device into the system, through the HCP EID external device.

According to one embodiment, the patient remote external device and the patient EID external device is an integrated unit.

According to one embodiment, the HCP dedicated device and the HCP EID external device are an integrated unit.

According to one embodiment, the system comprises a measurement device or sensor adapted to deliver a measurement to at least one of the DDI, patent EID external device and a patient display device.

According to one embodiment, the system comprises a food sensor, adapted to measure at least if the patient swallow solid food or is drinking fluid, wherein said food sensor is connected to the control unit of a medical device to cause an action to stretch the stomach after a determined amount of food intake.

According to one embodiment, the HCP EID external device further comprises a wireless transceiver configured for communication with the implanted medical device through a second network protocol.

According to one embodiment, the HCP private key device is adapted to be provided to the at least one HCP external device via at least one of; a reading slot or comparable for the HCP private key device, a RFID communication, and a close distance wireless activation communication unit, or electrical direct contact.

According to one embodiment, the HCP EID external device comprises at least one of: reading slot or comparable for the HCP private key device, a RFID communication, and a close distance wireless activation communication unit, or electrical direct contact.

According to one embodiment, the HCP EID external device is adapted to receive a command from an HCP dedicated device to change said pre-programmed treatment steps of the implantable medical device, when implanted, wherein the HCP dedicated device is further adapted to be activated, authenticated, and allowed to perform said command by the HCP providing their private key.

According to one embodiment, the HCP EID external device and the HCP private key device are configured for wireless communication using a standard network protocol.

According to one embodiment, the HCP EID external device and the HCP private key device are configured for wireless communication using a proprietary network protocol.

According to one embodiment, the HCP EID external device is configured to use a first network protocol for communication with the implantable medical device and use a second network protocol for communication with the HCP private key device.

According to one embodiment, the HPC EID external device is configured to use a first frequency band for communication with the implantable medical device and use a second frequency band for communication with the HCP private key device.

According to one embodiment, at least one of the HCP EID external device and the HCP private key device comprises a Bluetooth transceiver.

According to one embodiment, at least one of the HCP EID external device and the HCP private key device comprises a UWB transceiver.

An implantable energized medical device configured to be held in position by a tissue portion of a patient is provided, the medical device comprising: a first portion configured to be placed on a first side of the tissue portion, the first portion having a first cross-sectional area in a first plane and comprising a first surface configured to face a first tissue surface of the first side of the tissue portion, a second portion configured to be placed on a second side of the tissue portion, the second side opposing the first side, the second portion having a second cross-sectional area in a second plane and comprising a second surface configured to engage a second tissue surface of the second side of the tissue portion, and a connecting portion configured to be placed through a hole in the tissue portion extending between the first and second sides of the tissue portion, the connecting portion having a third cross-sectional area in a third plane and being configured to connect the first portion to the second portion, wherein: the first, second, and third planes are parallel to each other, the third cross-sectional area is smaller than the first and second cross-sectional areas, such that the first portion and second portion are prevented from travelling through the hole in the tissue portion in a direction perpendicular to the first, second and third planes, the connecting portion and second portion are configured to form a connecting interface between the connecting portion and the second portion, and the second portion extends along a first direction being parallel to the second plane, wherein the second portion has a lengthwise cross-sectional area along the first direction, wherein a second lengthwise cross-sectional area is smaller than a first lengthwise cross-sectional area and wherein the first lengthwise cross-sectional area is located closer to said connecting interface with regard to the first direction.

In some embodiments, the second portion has a first end and a second end opposing the first end along the first direction, wherein the second portion has a length between the first and second end, and wherein the second portion has an intermediate region and a distal region, wherein the intermediate region is defined by the connecting interface between the connecting portion and the second portion, and the distal region extends from the connecting interface between the connecting portion and the second portion to the second end.

In some embodiments, the lengthwise cross-sectional area of the second portion decreases continuously from an end of the intermediate region towards the second end.

In some embodiments, the lengthwise cross-sectional area of the second portion decreases linearly from an end of the intermediate region towards the second end.

In some embodiments, the lengthwise cross-sectional area of the second portion decreases stepwise from an end of the intermediate region towards the second end.

In some embodiments, the distal region of the second portion is conically shaped.

In some embodiments, the second portion has rotational symmetry along the first direction.

In some embodiments, the second surface of the second portion is substantially perpendicular to a central extension of the connecting portion.

In some embodiments, the second surface of the second portion is substantially parallel to the second plane.

In some embodiments, the second surface of the second portion is substantially flat and configured to form a contact area to the second tissue surface, and wherein the second portion further comprises a lower surface facing away from the first portion configured to taper towards the second end.

In some embodiments, the second portion has a proximal region, wherein the proximal region extends from the first end to the connecting interface between the connecting portion and the second portion.

In some embodiments, the lengthwise cross-sectional area of the second portion decreases continuously from an end of the intermediate region towards the first end.

In some embodiments, the lengthwise cross-sectional area of the second portion decreases linearly from an end of the intermediate region towards the first end.

In some embodiments, the lengthwise cross-sectional area of the second portion decreases stepwise from an end of the intermediate region towards the first end.

In some embodiments, the proximal region of the second portion is conically shaped.

In some embodiments, the first and second ends comprise an elliptical point respectively.

In some embodiments, the first and second ends comprise a hemispherical end cap respectively.

In some embodiments, the second portion has at least one circular cross-section along the length between the first and second end.

In some embodiments, the second portion has at least one oval cross-section along the length between the first and second end.

In some embodiments, the second portion has at least one elliptical cross-section along the length between the first and second end.

In some embodiments, the second portion has said length in a direction being different to a central extension of the connecting portion.

In some embodiments, the connecting interface between the connecting portion and the second portion is excentric with respect to the second portion.

In some embodiments, the connecting interface between the connecting portion and the second portion is excentric, with respect to the second portion, in the first direction, but not in a second direction being perpendicular to the first direction.

In some embodiments, the connecting interface between the connecting portion and the second portion is excentric, with respect to the second portion, in the first direction and in a second direction being perpendicular to the first direction.

In some embodiments, the second direction is parallel to the second plane.

In some embodiments, the proximal region and the distal region comprises the second surface configured to engage the second surface of the second side of the tissue portion.

In some embodiments, the second portion is tapered from the first end to the second end.

In some embodiments, the second portion is tapered from the intermediate region of the second portion to each of the first end and second end.

In some embodiments, the first portion has a maximum dimension being in the range of 10 to 40 mm, such as in the range of 10 to 30 mm, such as in the range of 15 to 25 mm.

In some embodiments, the first portion has a diameter being in the range of 10 to 40 mm, such as in the range of 10 to 30 mm, such as in the range of 15 to 25 mm.

In some embodiments, the connecting portion has a maximum dimension in the third plane in the range of 2 to 20 mm, such as in the range of 2 to 15 mm, such as in the range of 5 to 10 mm.

In some embodiments, the second portion has a maximum dimension being in the range of 30 to 90 mm, such as in the range of 30 to 70 mm, such as in the range of 35 to 60 mm.

In some embodiments, the first portion has one or more of a spherical shape, an ellipsoidal shape, a polyhedral shape, an elongated shape, and a flat disk shape.

In some embodiments, the connecting portion has one of an oval cross-section, an elongated cross-section, and a circular cross-section, in a plane parallel to the third plane.

In some embodiments, the distal region is configured to be directed downwards in a standing patient.

In some embodiments, the first portion has a first height, and the second portion has a second height, both heights being in a direction perpendicular to the first and second planes, wherein the first height is smaller than the second height.

In some embodiments, the first height is less than 2/3 of the second height, such as less than 1/2 of the second height, such as less than 1/3 of the second height.

In some embodiments, the second end of the second portion comprises connections for connecting to an implant being located in a caudal direction from a location of the implantable energized medical device in the patient.

**In** some embodiments, the first end of the second portion comprises connections for connecting to an implant being located in a cranial direction from a location of the implantable energized medical device in the patient.

In some embodiments, the connecting portion further comprises a fourth cross-sectional area in a fourth plane, wherein the fourth plane is parallel to the first, second and third planes, and wherein the third cross-sectional area is smaller than the fourth cross-sectional area.

In some embodiments, the connecting portion comprises a protruding element comprising the fourth cross-sectional area.

In some embodiments, the first surface is configured to engage the first tissue surface of the first side of the tissue portion.

In some embodiments, the first portion comprises a first wireless energy receiver configured to receive energy transmitted wirelessly from an external wireless energy transmitter.

In some embodiments, the first portion comprises an internal wireless energy transmitter.

In some embodiments, the second portion comprises a second wireless energy receiver.

In some embodiments, the first portion comprises a first energy storage unit.

In some embodiments, the second portion comprises a second energy storage unit.

In some embodiments, at least one of the first and second energy storage unit is a solid-state battery.

In some embodiments, the solid-state battery is a thionyl-chloride battery.

In some embodiments, the first wireless energy receiver is configured to receive energy transmitted wirelessly by the external wireless energy transmitter, and store the received energy in the first energy storage unit, the internal wireless energy transmitter is configured to wirelessly transmit energy stored in the first energy storage unit to the second wireless energy receiver, and the second wireless energy receiver is configured to receive energy transmitted wirelessly by the internal wireless energy transmitter and store the received energy in the second energy storage unit.

**In** some embodiments, the first portion comprises a first controller comprising at least one processing unit.

**In** some embodiments, the second portion comprises a second controller comprising at least one processing unit.

**In** some embodiments, at least one of the first and second controller is connected to a wireless transceiver for communicating wirelessly with an external device.

**In** some embodiments, the first controller is connected to a first wireless communication receiver in the first portion for receiving wireless communication from an external device, the first controller is connected to a first wireless communication transmitter in the first portion for transmitting wireless communication to a second wireless communication receiver in the second portion.

In some embodiments, the second controller is connected to the second wireless communication receiver for receiving wireless communication from the first portion.

In some embodiments, the first wireless energy receiver comprises a first coil and the internal wireless energy transmitter comprises a second coil.

In some embodiments, the first portion comprises a combined coil, wherein the combined coil is configured to receive energy wirelessly from an external wireless energy transmitter, and transmit energy wirelessly to the second wireless receiver of the second portion.

In some embodiments, at least one of the coils are embedded in a ceramic material.

In some embodiments, the implantable energized medical device further comprises a housing configured to enclose at least the first portion, and wherein a first portion of the housing is made from titanium and a second portion of the housing is made from a ceramic material.

In some embodiments, the portion of the housing made from a ceramic material comprises at least one coil embedded in the ceramic material.

In some embodiments, the implantable energized medical device further comprises a housing configured to enclose at least the second portion, and wherein a first portion of the housing is made from titanium and a second portion of the housing is made from a ceramic material.

In some embodiments, the portion of the housing made from a ceramic material comprises at least one coil embedded in the ceramic material.

In some embodiments, the implantable energized medical device further comprises at least one sensor for providing input to at least one of the first and second controller.

In some embodiments, the sensor is a sensor configured to sense a physical parameter of the implantable energized medical device.

In some embodiments, the sensor is a sensor configured to sense at least one of: a temperature of the implantable energized medical device or of a body engaging portion, a parameter related to the power consumption of the implantable energized medical device or of a body engaging portion, a parameter related to a status of at least one of the first and second energy storage unit, a parameter related to the wireless transfer of energy from a source external to the body of the patient, and a hydraulic pressure.

In some embodiments, the sensor is a sensor configured to sense a physiological parameter of the patient.

In some embodiments, the sensor is a sensor configured to sense at least one of: a parameter related to the patient swallowing, a local temperature, a systemic temperature, blood saturation, blood oxygenation, blood pressure, a parameter related to an ischemia marker, and pH.

In some embodiments, the sensor configured to sense a parameter related to the patient swallowing comprises at least one of: a motility sensor, a sonic sensor, an optical sensor, and a strain sensor.

In some embodiments, the sensor configured to sense pH is configured to sense the acidity in the stomach.

In some embodiments, the controller is configured to transmit information based on sensor input to a device external to the body of the patient.

In some embodiments, the second portion comprises at least a portion of an operation device for operating an implantable body engaging portion.

In some embodiments, the second portion comprises at least one electrical motor.

In some embodiments, the second portion comprises a transmission configured to reduce the velocity and increase the force of the movement generated by the electrical motor.

In some embodiments, the transmission is configured to transfer a week force with a high velocity into a stronger force with lower velocity.

In some embodiments, the transmission is configured to transfer a rotating force into a linear force.

In some embodiments, the transmission comprises a gear system.

In some embodiments, the second portion comprises a magnetic coupling for transferring mechanical work from the electrical motor through one of: a barrier separating a first chamber of the second portion from a second chamber of the second portion, a housing enclosing at least the second portion.

In some embodiments, the second portion comprises at least one hydraulic pump.

In some embodiments, the hydraulic pump comprises a pump comprising at least one compressible hydraulic reservoir.

In some embodiments, the implantable energized medical device further comprises a capacitor connected to at least one of the first and second energy storage unit and connected to the electrical motor, wherein the capacitor is configured to: be charged by at least one of the first and second energy storage units, and provide the electrical motor with electrical power.

In some embodiments, at least one of the first and second portion comprises a sensation generator adapted to generate a sensation detectable by a sense of the patient.

In some embodiments, the second portion comprises a force transferring element configured to mechanically transfer force from the second portion to an implanted body engaging portion.

In some embodiments, the second portion comprises a force transferring element configured to hydraulically transfer force from the second portion to an implanted body engaging portion.

In some embodiments, the second portion comprises at least one lead for transferring electrical energy and/or information from the second portion to an implanted body engaging portion.

In some embodiments, the first portion comprises an injection port for injecting fluid into the first portion.

In some embodiments, the connecting portion comprises a conduit for transferring a fluid from the first portion to the second portion.

In some embodiments, the conduit is arranged to extend through the hollow portion of the connecting portion.

In some embodiments, the second portion comprises a first and a second chamber separated from each other, wherein the first chamber comprises a first liquid and the second chamber comprises a second liquid, and wherein the second liquid is a hydraulic liquid configured to transfer force to an implantable element configured to exert force on the body portion of the patient.

In some embodiments, a wall portion of the first chamber is resilient to allow an expansion of the first chamber.

In some embodiments, the second portion comprises a first hydraulic system in fluid connection with a first hydraulically operable implantable element configured to exert force on the body portion of the patient, and a second hydraulic system in fluid connection with a second hydraulically operable implantable element configured to exert force on the body portion of the patient, wherein the first and second hydraulically operable implantable elements are adjustable independently from each other.

In some embodiments, the first hydraulic system comprises a first hydraulic pump and the second hydraulic systems comprises a second hydraulic pump.

In some embodiments, each of the first and second hydraulic systems comprises a reservoir for holding hydraulic fluid.

In some embodiments, the implantable energized medical device further comprises a first pressure sensor configured to sense a pressure in the first hydraulic system, and a second pressure sensor configured to sense a pressure in the second hydraulic system.

In some embodiments, the first surface is configured to engage the first tissue surface of the first side of the tissue portion.

In some embodiments, the first, second and third planes are parallel to a major extension plane of the tissue.

In some embodiments, the fourth plane is parallel to a major extension plane of the tissue.

According to an embodiment of the present inventive concept, an implantable device for exerting a force on a body portion of a patient is provided, the implantable device comprising: an implantable energized medical device and an implantable element configured to exert a force on a body portion of the patient.

In some embodiments, the implantable element configured to exert a force on a body portion of the patient is an implantable hydraulic constriction device.

In some embodiments, the implantable hydraulic constriction device is configured for constricting a luminary organ of the patient.

In some embodiments, the implantable hydraulic constriction device comprises an implantable hydraulic constriction device for constricting an intestine of the patient.

In some embodiments, the implantable hydraulic constriction device comprises an implantable hydraulic constriction device for constricting a colon or rectum of the patient.

In some embodiments, the implantable hydraulic constriction device comprises an implantable hydraulic constriction device for constricting the intestine at a region of a stoma of the patient.

In some embodiments, the implantable hydraulic constriction device comprises an implantable hydraulic constriction device for constricting a blood vessel of the patient.

In some embodiments, the implantable hydraulic constriction device for constricting a blood vessel of the patient is configured to constrict the venous blood flow leading from an erectile tissue for promoting the engorgement of the erectile tissue.

In some embodiments, the implantable hydraulic constriction device comprises an implantable hydraulic constriction device for constricting a vas deference of the patient.

In some embodiments, the implantable element configured to exert a force on a body portion of the patient is an implantable element for actively emptying the urinary bladder of the patient.

In some embodiments, the implantable element for actively emptying the urinary bladder of the patient is configured to empty the bladder of the patient by compressing the urinary bladder from the outside thereof.

In some embodiments, the implantable element configured to exert a force on a body portion of the patient is an implantable element for actively stretching a stomach wall of the patient to create a feeling of satiety.

An implantable energized medical device configured to be held in position by a tissue portion of a patient is provided, the medical device comprising: a first portion configured to be placed on a first side of the tissue portion, the first portion having a first cross-sectional area in a first plane and comprising a first surface configured to face a first tissue surface of the first side of the tissue portion, a second portion configured to be placed on a second side of the tissue portion, the second side opposing the first side, the second portion having a second cross-sectional area in a second plane and comprising a second surface configured to engage a second tissue surface of the second side of the tissue portion, and a connecting portion configured to be placed through a hole in the tissue portion extending between the first and second sides of the tissue portion, the connecting portion having a third cross-sectional area in a third plane and a third surface configured to engage the first tissue surface of the first side of the tissue portion, wherein the connecting portion is configured to connect the first portion to the second portion, wherein: the first, second, and third planes are parallel to each other, the third cross-sectional area is smaller than the first and second cross-sectional area, such that the first portion, second portion and connecting portion are prevented from travelling through the hole in the tissue portion in a direction perpendicular to the first, second and third planes, and the first portion is configured to be movable in relation to the connecting portion, and/or comprises a first element and a second element, the first element being configured to be moved in relation to the second element to increase an area of the first surface.

In some embodiments, the connecting portion is configured to extend along a central extension between the first portion and the second portion, and wherein the first portion is configured to be moveable to assume several positions along a direction perpendicular to the central extension.

In some embodiments, the first portion is configured to be fixed in the several positions by a locking mechanism arranged on either or both of the first portion and connecting portion.

In some embodiments, the first element is configured to assume a first state, wherein the first element is arranged on top of the second element or within the second element, and a second state, wherein the first element is arranged adjacent to the second element.

In some embodiments, the first element is hingedly connected to the second element.

In some embodiments, the first element and the second element are integrally formed, and wherein the first portion is flexible to allow the first element to fold over the second element to assume the first state.

In some embodiments, the second element comprises a slot, and wherein the first element is configured to be partially or fully housed within the slot in the first state, and wherein the first element is configured to protrude from the slot in the second state.

In some embodiments, the first element comprises a slot, and wherein the second element is configured to be partially or fully housed within the slot in a first state of the second element, and wherein the second element is configured to protrude from the slot in a second state of the second element.

In some embodiments, the first element is configured to rotate about an axis being parallel to said central extension.

In some embodiments, the first element is configured to rotate up to a maximum of 180 degrees about the axis.

In some embodiments, the first element is configured to rotate up to a maximum of 90 degrees about the axis.

In some embodiments, the second element is configured to be connected to the connecting portion.

In some embodiments, the first element is configured to be moved in relation to the second element to protrude or to further protrude beyond an edge of the second element to increase an area of the first surface.

In some embodiments, the second element is movable in relation to the first element to increase an area of the first surface.

In some embodiments, the first element and the second element are configured to be moved from a first state, wherein ends of the first and second elements respectively point in a direction substantially perpendicular to the first plane, to a second state, wherein said ends of the first and second ends point in one or more directions being substantially parallel to the first plane.

In some embodiments, the first element and the second element are configured to assume an upright position extending away from the connecting portion, and to be moved towards a sideways position being substantially perpendicular to the upright position.

In some embodiments, the connecting portion comprises a protruding element and the first portion comprises a slot, wherein the protruding element is configured to slide within the slot along a predetermined path.

In some embodiments, the protruding element is configured to be interlocked within the slot such that the protruding element can only be removed from the slot in a preconfigured position.

In some embodiments, the protruding element is configured to be interlocked within the slot such that the protruding element is permanently enclosed within the slot, or wherein the protruding element is configured to be interlocked within the slot such that the protruding element is permanently enclosed within the slot.

In some embodiments, the connecting portion further comprises a fourth cross-sectional area in a fourth plane, wherein the fourth plane is parallel to the first, second and third planes, and wherein the third cross-sectional area is smaller than the fourth cross-sectional area.

In some embodiments, the connecting portion comprises a protruding element comprising the fourth cross-sectional area.

In some embodiments, the connecting portion comprises a flange comprising the fourth cross-sectional area, such that the flange is prevented from travelling through the hole in the tissue portion in a direction perpendicular to the first, second and third planes.

In some embodiments, the flange protrudes in a direction parallel to the first, second, third and fourth planes, and perpendicular to a central extension of the connecting portion.

In some embodiments, the flange comprises the third surface configured to engage the first tissue surface of the first side of the tissue portion.

In some embodiments, the first portion comprises a first wireless energy receiver configured to receive energy transmitted wirelessly from an external wireless energy transmitter.

In some embodiments, the first portion comprises an internal wireless energy transmitter.

In some embodiments, the second portion comprises a second wireless energy receiver.

In some embodiments, the first portion comprises a first energy storage unit.

In some embodiments, the second portion comprises a second energy storage unit.

In some embodiments, at least one of the first and second energy storage unit is a solid-state battery.

In some embodiments, the solid-state battery is a thionyl-chloride battery.

In some embodiments, the first wireless energy receiver is configured to receive energy transmitted wirelessly by the external wireless energy transmitter, and store the received energy in the first energy storage unit, the internal wireless energy transmitter is configured to wirelessly transmit energy stored in the first energy storage unit to the second wireless energy receiver, and the second wireless energy receiver is configured to receive energy transmitted wirelessly by the internal wireless energy transmitter and store the received energy in the second energy storage unit.

In some embodiments, the first portion comprises a first controller comprising at least one processing unit.

In some embodiments, the second portion comprises a second controller comprising at least one processing unit.

In some embodiments, at least one of the first and second controller is connected to a wireless transceiver for communicating wirelessly with an external device.

In some embodiments, the first controller is connected to a first wireless communication receiver in the first portion for receiving wireless communication from an external device, and the first controller is connected to a first wireless communication transmitter in the first portion for transmitting wireless communication to a second wireless communication receiver in the second portion.

In some embodiments, the second controller is connected to the second wireless communication receiver for receiving wireless communication from the first portion.

In some embodiments, the first wireless energy receiver comprises a first coil and the internal wireless energy transmitter comprises a second coil.

In some embodiments, the first portion comprises a combined coil, wherein the combined coil is configured to receive energy wirelessly from an external wireless energy transmitter, and transmit energy wirelessly to the second wireless receiver of the second portion.

In some embodiments, at least one of the coils are embedded in a ceramic material.

In some embodiments, the implantable energized medical device further comprises a housing configured to enclose at least the first portion, and wherein a first portion of the housing is made from titanium and a second portion of the housing is made from a ceramic material.

In some embodiments, the portion of the housing made from a ceramic material comprises at least one coil embedded in the ceramic material.

In some embodiments, the implantable energized medical device further comprises a housing configured to enclose at least the second portion, and wherein a first portion of the housing is made from titanium and a second portion of the housing is made from a ceramic material.

In some embodiments, the portion of the housing made from a ceramic material comprises at least one coil embedded in the ceramic material.

In some embodiments, the implantable energized medical device further comprises at least one sensor for providing input to at least one of the first and second controller.

In some embodiments, the sensor is a sensor configured to sense a physical parameter of the implantable energized medical device.

In some embodiments, the sensor is a sensor configured to sense at least one of: a temperature of the implantable energized medical device or of a body engaging portion, a parameter related to the power consumption of the implantable energized medical device or of a body engaging portion, a parameter related to a status of at least one of the first and second energy storage unit, a parameter related to the wireless transfer of energy from a source external to the body of the patient, and a hydraulic pressure.

In some embodiments, the sensor is a sensor configured to sense a physiological parameter of the patient.

In some embodiments, the sensor is a sensor configured to sense at least one of: a parameter related to the patient swallowing, a local temperature, a systemic temperature, blood saturation, blood oxygenation, blood pressure, a parameter related to an ischemia marker, and pH.

In some embodiments, the sensor configured to sense a parameter related to the patient swallowing comprises at least one of: a motility sensor, a sonic sensor, an optical sensor, and a strain sensor.

In some embodiments, the sensor configured to sense pH is configured to sense the acidity in the stomach.

In some embodiments, the controller is configured to transmit information based on sensor input to a device external to the body of the patient.

In some embodiments, the second portion comprises at least a portion of an operation device for operating an implantable body engaging portion.

In some embodiments, the second portion comprises at least one electrical motor.

In some embodiments, the second portion comprises a transmission configured to reduce the velocity and increase the force of the movement generated by the electrical motor.

In some embodiments, the transmission is configured to transfer a week force with a high velocity into a stronger force with lower velocity.

In some embodiments, the transmission is configured to transfer a rotating force into a linear force.

In some embodiments, the transmission comprises a gear system.

In some embodiments, the second portion comprises a magnetic coupling for transferring mechanical work from the electrical motor through one of: a barrier separating a first chamber of the second portion from a second chamber of the second portion, a housing enclosing at least the second portion.

In some embodiments, the second portion comprises at least one hydraulic pump.

In some embodiments, the hydraulic pump comprises a pump comprising at least one compressible hydraulic reservoir.

In some embodiments, the implantable energized medical device further comprises a capacitor connected to at least one of the first and second energy storage unit and connected to the electrical motor, wherein the capacitor is configured to:
be charged by at least one of the first and second energy storage units, and
provide the electrical motor with electrical power.

In some embodiments, at least one of the first and second portion comprises a sensation generator adapted to generate a sensation detectable by a sense of the patient.

In some embodiments, the second portion comprises a force transferring element configured to mechanically transfer force from the second portion to an implanted body engaging portion.

In some embodiments, the second portion comprises a force transferring element configured to hydraulically transfer force from the second portion to an implanted body engaging portion.

In some embodiments, the second portion comprises at least one lead for transferring electrical energy and/or information from the second portion to an implanted body engaging portion.

In some embodiments, the first portion comprises an injection port for injecting fluid into the first portion.

In some embodiments, the connecting portion comprises a conduit for transferring a fluid from the first portion to the second portion.

In some embodiments, the conduit is arranged to extend through the hollow portion of the connecting portion.

In some embodiments, the second portion comprises a first and a second chamber separated from each other, wherein the first chamber comprises a first liquid and the second chamber comprises a second liquid, and wherein the second liquid is a hydraulic liquid configured to transfer force to an implantable element configured to exert force on the body portion of the patient.

In some embodiments, a wall portion of the first chamber is resilient to allow an expansion of the first chamber.

In some embodiments, the second portion comprises a first hydraulic system in fluid connection with a first hydraulically operable implantable element configured to exert force on the body portion of the patient, and a second hydraulic system in fluid connection with a second hydraulically operable implantable element configured to exert force on the body portion of the patient, wherein the first and second hydraulically operable implantable elements are adjustable independently from each other.

In some embodiments, the first hydraulic system comprises a first hydraulic pump and the second hydraulic systems comprises a second hydraulic pump.

In some embodiments, each of the first and second hydraulic systems comprises a reservoir for holding hydraulic fluid.

In some embodiments, the implantable energized medical device further comprises a first pressure sensor configured to sense a pressure in the first hydraulic system, and a second pressure sensor configured to sense a pressure in the second hydraulic system.

In some embodiments, the first surface is configured to engage the first tissue surface of the first side of the tissue portion.

In some embodiments, the first, second and third planes are parallel to a major extension plane of the tissue.

In some embodiments, the fourth plane is parallel to a major extension plane of the tissue.

In some embodiments, the third cross-sectional area is smaller than the first cross-sectional area.

In some embodiments, the third cross-sectional area is equal to or larger than the first cross-sectional area.

An implantable device for exerting a force on a body portion of a patient is provided, the device comprising: an implantable energized medical device, and an implantable element configured to exert a force on a body portion of the patient.

In some embodiments, the implantable element configured to exert a force on a body portion of the patient is an implantable hydraulic constriction device.

In some embodiments, the implantable hydraulic constriction device is configured for constricting a luminary organ of the patient.

In some embodiments, the implantable hydraulic constriction device comprises an implantable hydraulic constriction device for constricting an intestine of the patient.

In some embodiments, the implantable hydraulic constriction device comprises an implantable hydraulic constriction device for constricting a colon or rectum of the patient.

In some embodiments, the implantable hydraulic constriction device comprises an implantable hydraulic constriction device for constricting the intestine at a region of a stoma of the patient.

In some embodiments, the implantable hydraulic constriction device comprises an implantable hydraulic constriction device for constricting a blood vessel of the patient.

In some embodiments, the implantable hydraulic constriction device for constricting a blood vessel of the patient is configured to constrict the venous blood flow leading from an erectile tissue for promoting the engorgement of the erectile tissue.

In some embodiments, the implantable hydraulic constriction device comprises an implantable hydraulic constriction device for constricting a vas deference of the patient.

In some embodiments, the implantable element configured to exert a force on a body portion of the patient is an implantable element for actively emptying the urinary bladder of the patient.

In some embodiments, the implantable element for actively emptying the urinary bladder of the patient is configured to empty the bladder of the patient by compressing the urinary bladder from the outside thereof.

In some embodiments, the implantable element configured to exert a force on a body portion of the patient is an implantable element for actively stretching a stomach wall of the patient to create a feeling of satiety.

An implantable energized medical device configured to be held in position by a tissue portion of a patient is provided, the medical device comprising: a first portion configured to be placed on a first side of the tissue portion, the first portion having a first cross-sectional area in a first plane and comprising a first surface configured to face a first tissue surface of the first side of the tissue portion, a second portion configured to be placed on a second side of the tissue portion, the second side opposing the first side, the second portion having a second cross-sectional area in a second plane and comprising a second surface configured to engage a second tissue surface of the second side of the tissue portion, and a connecting portion configured to be placed through a hole in the tissue portion extending between the first and second sides of the tissue portion, the connecting portion having a third cross-sectional area in a third plane and a third surface configured to engage the first tissue surface of the first side of the tissue portion, wherein the connecting portion is configured to connect the first portion to the second portion, wherein: the first, second, and third planes are parallel to each other, the third cross-sectional area is smaller than the second cross-sectional area, such that the first portion, second portion and connecting portion are prevented from travelling through the hole in the tissue portion in a direction perpendicular to the first, second and third planes, the first portion is configured to receive electromagnetic waves at a frequency above a frequency level, and/or to transmit electromagnetic waves at a frequency below the frequency level, wherein the second portion is configured to receive and/or transmit electromagnetic waves at a frequency below the frequency level, and wherein the frequency level is 100 kHz.

In some embodiments, wherein the first portion is configured to transmit electromagnetic waves at the frequency below the frequency level to the second portion.

In some embodiments, the first portion is configured to transmit electromagnetic waves at the frequency above the frequency level to an external device.

In some embodiments, the frequency level is 40 kHz or 20 kHz.

In some embodiments, the electromagnetic waves comprise wireless energy and/or wireless communication.

In some embodiments, the first portion comprises a first wireless energy receiver for receiving energy transmitted wirelessly by an external wireless energy transmitter above the frequency level, and an internal wireless energy transmitter configured to transmit energy wirelessly to the second portion below the frequency level, and the second portion comprises a second wireless energy receiver configured to receive energy transmitted wirelessly by the internal wireless energy transmitter below the frequency level.

In some embodiments, the first portion comprises a first controller comprising at least one processing unit.

In some embodiments, the second portion comprises a second controller comprising at least one processing unit.

In some embodiments, the first controller is connected to a first wireless communication receiver in the first portion for receiving wireless communication from an external device above the frequency level, the first controller is connected to a first wireless communication transmitter in the first portion for transmitting wireless communication to a second wireless communication receiver in the second portion below the frequency level.

In some embodiments, the second controller is connected to the second wireless communication receiver for receiving wireless communication from the first portion below the frequency level.

In some embodiments, the first portion comprises an outer casing made from a polymer material.

In some embodiments, the outer casing forms a complete enclosure, such that electromagnetic waves received and transmitted by the first portion must travel through the casing.

In some embodiments, the second portion comprises an outer casing made from titanium.

In some embodiments, the outer casing forms a complete enclosure, such that electromagnetic waves received and transmitted by the second portion must travel through the casing.

An implantable energized medical device configured to be held in position by a tissue portion of a patient is provided, the medical device comprising: a first portion configured to be placed on a first side of the tissue portion, the first portion having a first cross-sectional area in a first plane and comprising a first surface configured to face a first tissue surface of the first side of the tissue portion, a second portion configured to be placed on a second side of the tissue portion, the second side opposing the first side, the second portion having a second cross-sectional area in a second plane and comprising a second surface configured to engage a second tissue surface of the second side of the tissue portion, and a connecting portion configured to be placed through a hole in the tissue portion extending between the first and second sides of the tissue portion, the connecting portion having a third cross-sectional area in a third plane and a third surface configured to engage the first tissue surface of the first side of the tissue portion, wherein the connecting portion is configured to connect the first portion to the second portion, wherein: the first, second, and third planes are parallel to each other, the third cross-sectional area is smaller than the second cross-sectional area, such that the first portion, second portion and connecting portion are prevented from travelling through the hole in the tissue portion in a direction perpendicular to the first, second and third planes, the first portion is configured to receive and/or transmit electromagnetic waves at a frequency below the frequency level, and wherein the frequency level is 100 kHz.

In some embodiments, the second portion is configured to receive and/or transmit electromagnetic waves at a frequency below the frequency level.

In some embodiments, the first portion is configured to transmit electromagnetic waves at the frequency below the frequency level to the second portion.

In some embodiments, the first portion is configured to transmit electromagnetic waves at the frequency below the frequency level to an external device.

In some embodiments, the frequency level is 40 kHz or 20 kHz.

In some embodiments, the electromagnetic waves comprise wireless energy and/or wireless communication.

In some embodiments, the first portion comprises a first wireless energy receiver for receiving energy transmitted wirelessly by an external wireless energy transmitter below the frequency level, and an internal wireless energy transmitter configured to transmit energy wirelessly to the second portion below the frequency level, and the second portion comprises a second wireless energy receiver configured to receive energy transmitted wirelessly by the internal wireless energy transmitter below the frequency level.

In some embodiments, the first portion comprises a first controller comprising at least one processing unit.

In some embodiments, the second portion comprises a second controller comprising at least one processing unit.

In some embodiments, the first controller is connected to a first wireless communication receiver in the first portion for receiving wireless communication from an external device below the frequency level, the first controller is connected to a first wireless communication transmitter in the first portion for transmitting wireless communication to a second wireless communication receiver in the second portion below the frequency level.

In some embodiments, the second controller is connected to the second wireless communication receiver for receiving wireless communication from the first portion below the frequency level.

In some embodiments, the first portion comprises an outer casing made from a polymer material.

In some embodiments, the first portion comprises an outer casing made from titanium.

In some embodiments, the outer casing forms a complete enclosure, such that electromagnetic waves received and transmitted by the first portion must travel through the casing.

In some embodiments, the second portion comprises an outer casing made from titanium.

In some embodiments, the outer casing forms a complete enclosure, such that electromagnetic waves received and transmitted by the second portion must travel through the casing.

An implantable energized medical device configured to be held in position by a tissue portion of a patient is provided, the medical device comprising: a first portion configured to be placed on a first side of the tissue portion, the first portion having a first cross-sectional area in a first plane and comprising a first surface configured to face a first tissue surface of the first side of the tissue portion, a second portion configured to be placed on a second side of the tissue portion, the second side opposing the first side, the second portion having a second cross-sectional area in a second plane and comprising a second surface configured to engage a second tissue surface of the second side of the tissue portion, and a connecting portion configured to be placed through a hole in the tissue portion extending between the first and second sides of the tissue portion, the connecting portion having a third cross-sectional area in a third plane and a third surface configured to engage the first tissue surface of the first side of the tissue portion, wherein the connecting portion is configured to connect the first portion to the second portion, wherein: the first, second, and third planes are parallel to each other, the third cross-sectional area is smaller than the second cross-sectional area, such that the first portion, second portion and connecting portion are prevented from travelling through the hole in the tissue portion in a direction perpendicular to the first, second and third planes, the first portion is made from a polymer material, the second portion comprises a casing made from titanium, wherein the casing forms a complete enclosure.

In some embodiments, the casing of the second portion forms a complete enclosure such that the entirety of the outer surface of the second portion is covered by the casing, when the second portion is connected to the connecting portion.

In some embodiments, the first portion comprises a casing made from the polymer material.

In some embodiments, the casing of the first portion forms a complete enclosure such that the entirety of the outer surface of the first portion is covered by the casing.

In some embodiments, the connecting portion comprises a connection arranged to connect to the first and second portion respectively and carry electrical signals and/or energy.

In some embodiments, the connection is arranged in a core of the connecting portion such that it is encapsulated by outer material of the connecting portion.

In some embodiments, the connecting portion comprises a ceramic material.

In some embodiments, the connection is encapsulated within the ceramic material.

In some embodiments, the first portion comprises a first connection configured to connect to the connection of the connecting portion.

In some embodiments, the second portion comprises a second connection configured to connect to the connection of the connection portion.

In some embodiments, the casing of the second portion is hermetically sealed.

In some embodiments, the second connection is arranged such that the hermetical seal of the second portion is kept intact.

In some embodiments, the casing of the first portion is hermetically sealed.

An implantable energized medical device configured to be held in position by a tissue portion of a patient is provided, the medical device comprising: a first portion configured to be placed on a first side of the tissue portion, the first portion having a first cross-sectional area in a first plane and comprising a first surface configured to face a first tissue surface of the first side of the tissue portion, a second portion configured to be placed on a second side of the tissue portion, the second side opposing the first side, the second portion having a second cross-sectional area in a second plane and comprising a second surface configured to engage a second tissue surface of the second side of the tissue portion, and a connecting portion configured to be placed through a hole in the tissue portion extending between the first and second sides of the tissue portion, the connecting portion having a third cross-sectional area in a third plane and a third surface configured to engage the first tissue surface of the first side of the tissue portion, wherein the connecting portion is configured to connect the first portion to the second portion, wherein: the first, second, and third planes are parallel to each other, the third cross-sectional area is smaller than the second cross-sectional area, such that the first portion, second portion and connecting portion are prevented from travelling through the hole in the tissue portion in a direction perpendicular to the first, second and third planes, and wherein the connecting portion is configured to extend between the first portion and the second portion along a central extension axis, and wherein the second portion is configured to extend in a length direction being divergent with the central extension axis, and wherein the connecting portion has a substantially constant cross-sectional area along the central extension axis, or wherein the connecting portion has a decreasing cross-sectional area in a direction from the first portion towards the second portion along the central extension axis, and/or wherein the second portion has a substantially constant cross-sectional area along the length direction, or wherein the second portion has a decreasing cross-sectional area in the length direction.

In some embodiments, the third cross-sectional area is smaller than the first cross-sectional area.

In some embodiments, the connecting portion is tapered in the direction from the first portion towards the second portion along the central extension axis.

In some embodiments, the connecting portion has a circular or oval cross-section along the central extension axis with a decreasing diameter in the direction from the first portion towards the second portion.

In some embodiments, the second portion is tapered in the length direction.

In some embodiments, the connecting portion has a circular or oval cross-section in the length direction with a decreasing diameter in the length direction.

In some embodiments, the length direction extends from an interface between the connecting portion and the second portion towards an end of the second portion.

In some embodiments, the length direction extends in a direction substantially perpendicular to the central extension axis.

In some embodiments, the connecting portion comprises a protruding element and the first portion comprises a slot, wherein the protruding element is configured to slide within the slot along a predetermined path.

In some embodiments, the protruding element is configured to be interlocked within the slot such that the protruding element can only be removed from the slot in a preconfigured position.

In some embodiments, the protruding element is configured to be interlocked within the slot such that the protruding element is permanently enclosed within the slot, or wherein the protruding element is configured to be interlocked within the slot such that the protruding element is permanently enclosed within the slot.

In some embodiments, the connecting portion further comprises a fourth cross-sectional area in a fourth plane, wherein the fourth plane is parallel to the first, second and third planes, and wherein the third cross-sectional area is smaller than the fourth cross-sectional area.

In some embodiments, the connecting portion comprises a protruding element comprising the fourth cross-sectional area.

In some embodiments, the connecting portion comprises a flange comprising the fourth cross-sectional area, such that the flange is prevented from travelling through the hole in the tissue portion in a direction perpendicular to the first, second and third planes.

In some embodiments, the flange protrudes in a direction parallel to the first, second, third and fourth planes, and perpendicular to a central extension of the connecting portion.

In some embodiments, the flange comprises the third surface configured to engage the first tissue surface of the first side of the tissue portion.

In some embodiments, the first portion comprises a first wireless energy receiver configured to receive energy transmitted wirelessly from an external wireless energy transmitter.

In some embodiments, the first portion comprises an internal wireless energy transmitter.

In some embodiments, the second portion comprises a second wireless energy receiver.

In some embodiments, the first portion comprises a first energy storage unit.

In some embodiments, the second portion comprises a second energy storage unit.

In some embodiments, at least one of the first and second energy storage unit is a solid-state battery.

In some embodiments, the solid-state battery is a thionyl-chloride battery.

In some embodiments, the first wireless energy receiver is configured to receive energy transmitted wirelessly by the external wireless energy transmitter, and store the received energy in the first energy storage unit, the internal wireless energy transmitter is configured to wirelessly transmit energy stored in the first energy storage unit to the second wireless energy receiver, and the second wireless energy receiver is configured to receive energy transmitted wirelessly by the internal wireless energy transmitter and store the received energy in the second energy storage unit.

In some embodiments, the first portion comprises a first controller comprising at least one processing unit.

In some embodiments, the second portion comprises a second controller comprising at least one processing unit.

In some embodiments, at least one of the first and second controller is connected to a wireless transceiver for communicating wirelessly with an external device.

In some embodiments, the first controller is connected to a first wireless communication receiver in the first portion for receiving wireless communication from an external device, the first controller is connected to a first wireless communication transmitter in the first portion for transmitting wireless communication to a second wireless communication receiver in the second portion.

In some embodiments, the second controller is connected to the second wireless communication receiver for receiving wireless communication from the first portion.

In some embodiments, the first wireless energy receiver comprises a first coil and the internal wireless energy transmitter comprises a second coil.

In some embodiments, the first portion comprises a combined coil, wherein the combined coil is configured to receive energy wirelessly from an external wireless energy transmitter, and transmit energy wirelessly to the second wireless receiver of the second portion.

In some embodiments, at least one of the coils are embedded in a ceramic material.

In some embodiments, the implantable energized medical device further comprises a housing configured to enclose at least the first portion, and wherein a first portion of the housing is made from titanium and a second portion of the housing is made from a ceramic material.

In some embodiments, the portion of the housing made from a ceramic material comprises at least one coil embedded in the ceramic material.

In some embodiments, the implantable energized medical device further comprises a housing configured to enclose at least the second portion, and wherein a first portion of the housing is made from titanium and a second portion of the housing is made from a ceramic material.

In some embodiments, the portion of the housing made from a ceramic material comprises at least one coil embedded in the ceramic material.

In some embodiments, the implantable energized medical device further comprises at least one sensor for providing input to at least one of the first and second controller.

In some embodiments, the sensor is a sensor configured to sense a physical parameter of the implantable energized medical device.

In some embodiments, the sensor is a sensor configured to sense at least one of: a temperature of the implantable energized medical device or of a body engaging portion, a parameter related to the power consumption of the implantable energized medical device or of a body engaging portion, a parameter related to a status of at least one of the first and second energy storage unit, a parameter related to the wireless transfer of energy from a source external to the body of the patient, and a hydraulic pressure.

In some embodiments, the sensor is a sensor configured to sense a physiological parameter of the patient.

In some embodiments, sensor is a sensor configured to sense at least one of: a parameter related to the patient swallowing, a local temperature, a systemic temperature, blood saturation, blood oxygenation, blood pressure, a parameter related to an ischemia marker, and pH.

In some embodiments, the sensor configured to sense a parameter related to the patient swallowing comprises at least one of: a motility sensor, a sonic sensor, an optical sensor, and a strain sensor.

In some embodiments, the sensor configured to sense pH is configured to sense the acidity in the stomach.

In some embodiments, the controller is configured to transmit information based on sensor input to a device external to the body of the patient.

In some embodiments, the second portion comprises at least a portion of an operation device for operating an implantable body engaging portion.

In some embodiments, the second portion comprises at least one electrical motor.

In some embodiments, the second portion comprises a transmission configured to reduce the velocity and increase the force of the movement generated by the electrical motor.

In some embodiments, the transmission is configured to transfer a week force with a high velocity into a stronger force with lower velocity.

In some embodiments, the transmission is configured to transfer a rotating force into a linear force.

In some embodiments, the transmission comprises a gear system.

In some embodiments, the second portion comprises a magnetic coupling for transferring mechanical work from the electrical motor through one of: a barrier separating a first chamber of the second portion from a second chamber of the second portion, a housing enclosing at least the second portion.

In some embodiments, the second portion comprises at least one hydraulic pump.

In some embodiments, the hydraulic pump comprises a pump comprising at least one compressible hydraulic reservoir.

In some embodiments, the implantable energized medical device further comprises a capacitor connected to at least one of the first and second energy storage unit and connected to the electrical motor, wherein the capacitor is configured to: be charged by at least one of the first and second energy storage units, and provide the electrical motor with electrical power.

In some embodiments, at least one of the first and second portion comprises a sensation generator adapted to generate a sensation detectable by a sense of the patient.

In some embodiments, the second portion comprises a force transferring element configured to mechanically transfer force from the second portion to an implanted body engaging portion.

In some embodiments, the second portion comprises a force transferring element configured to hydraulically transfer force from the second portion to an implanted body engaging portion.

In some embodiments, the second portion comprises at least one lead for transferring electrical energy and/or information from the second portion to an implanted body engaging portion.

In some embodiments, the first portion comprises an injection port for injecting fluid into the first portion.

In some embodiments, the connecting portion comprises a conduit for transferring a fluid from the first portion to the second portion.

In some embodiments, the conduit is arranged to extend through the hollow portion of the connecting portion.

In some embodiments, the second portion comprises a first and a second chamber separated from each other, wherein the first chamber comprises a first liquid and the second chamber comprises a second liquid, and wherein the second liquid is a hydraulic liquid configured to transfer force to an implantable element configured to exert force on the body portion of the patient.

In some embodiments, a wall portion of the first chamber is resilient to allow an expansion of the first chamber.

In some embodiments, the second portion comprises a first hydraulic system in fluid connection with a first hydraulically operable implantable element configured to exert force on the body portion of the patient, and a second hydraulic system in fluid connection with a second hydraulically operable implantable element configured to exert force on the body portion of the patient, wherein the first and second hydraulically operable implantable elements are adjustable independently from each other.

In some embodiments, the first hydraulic system comprises a first hydraulic pump and the second hydraulic systems comprises a second hydraulic pump.

In some embodiments, each of the first and second hydraulic systems comprises a reservoir for holding hydraulic fluid.

In some embodiments, the implantable energized medical device further comprises a first pressure sensor configured to sense a pressure in the first hydraulic system, and a second pressure sensor configured to sense a pressure in the second hydraulic system.

In some embodiments, the first surface is configured to engage the first tissue surface of the first side of the tissue portion.

In some embodiments, the first, second and third planes are parallel to a major extension plane of the tissue.

In some embodiments, the fourth plane is parallel to a major extension plane of the tissue.

In some embodiments, the third cross-sectional area is smaller than the first cross-sectional area.

In some embodiments, the third cross-sectional area is equal to or larger than the first cross-sectional area.

An implantable device for exerting a force on a body portion of a patient is provided, the device comprising: an implantable energized medical device, an implantable element configured to exert a force on a body portion of the patient.

In some embodiments, the implantable element configured to exert a force on a body portion of the patient is an implantable hydraulic constriction device.

In some embodiments, the implantable hydraulic constriction device is configured for constricting a luminary organ of the patient.

In some embodiments, the implantable hydraulic constriction device comprises an implantable hydraulic constriction device for constricting an intestine of the patient.

In some embodiments, the implantable hydraulic constriction device comprises an implantable hydraulic constriction device for constricting a colon or rectum of the patient.

In some embodiments, the implantable hydraulic constriction device comprises an implantable hydraulic constriction device for constricting the intestine at a region of a stoma of the patient.

In some embodiments, the implantable hydraulic constriction device comprises an implantable hydraulic constriction device for constricting a blood vessel of the patient.

In some embodiments, the implantable hydraulic constriction device for constricting a blood vessel of the patient is configured to constrict the venous blood flow leading from an erectile tissue for promoting the engorgement of the erectile tissue.

In some embodiments, the implantable hydraulic constriction device comprises an implantable hydraulic constriction device for constricting a vas deference of the patient.

In some embodiments, the implantable element configured to exert a force on a body portion of the patient is an implantable element for actively emptying the urinary bladder of the patient.

In some embodiments, the implantable element for actively emptying the urinary bladder of the patient is configured to empty the bladder of the patient by compressing the urinary bladder from the outside thereof.

In some embodiments, the implantable element configured to exert a force on a body portion of the patient is an implantable element for actively stretching a stomach wall of the patient to create a feeling of satiety.

### Brief description of the drawings

The above, as well as additional objects, features and advantages of the present inventive concept, will be better understood through the following illustrative and non-limiting detailed description of the present inventive concept, with reference to the appended drawings, wherein:
Fig. 1 shows a cross-sectional view of an implantable energized medical device for powering an implantable medical device with hydraulic force.
Fig. 2 shows an exploded cross-sectional view of an implantable energized medical device for powering an implantable medical device with hydraulic force.
Fig. 3a shows a detailed cross-sectional view of a first unit of an implantable energized medical device for powering an implantable medical device with hydraulic force.
Fig. 3b shows a detailed cross-sectional view of a first unit of an implantable energized medical device for powering an implantable medical device with hydraulic force.
Fig. 3c shows a detailed cross-sectional view of a first unit of an implantable energized medical device for powering an implantable medical device with hydraulic force.
Fig. 3d shows a detailed cross-sectional view of a first unit of an implantable energized medical device for powering an implantable medical device with hydraulic force.
Figs. 4a-4b,5a-5b, 6a-6b show alternative embodiments of connecting portions for an implantable energized medical device.
Fig. 7 shows, schematically, a kit of components forming an implantable energized medical device.
Fig. 8 shows a detailed cross-sectional view of an embodiment of an implantable energized medical device for powering an implantable medical device.
Fig. 9 shows a perspective elevated view from the right of an embodiment of an implantable energized medical device for powering an implantable medical device.
Fig. 10 shows a perspective elevated view from the right of a portion of an embodiment of an implantable energized medical device for powering an implantable medical device.
Fig. 11 shows a perspective elevated view from the right of a portion of an embodiment of an implantable energized medical device for powering an implantable medical device.
Fig. 12a shows a cross-sectional plain side view of an embodiment of an implantable energized medical device for powering an implantable medical device.
Fig. 12b shows a cross-sectional plain side view of an embodiment of an implantable energized medical device for powering an implantable medical device.
Fig. 12c shows a cross-sectional plain side view of an embodiment of an implantable energized medical device for powering an implantable medical device.
Fig. 12d shows a cross-sectional plain side view of an embodiment of an implantable energized medical device for powering an implantable medical device.
Fig. 13a-k, 13m, 13n, 13p and 13q show perspective elevated views from the right of embodiments of an implantable energized medical device for powering an implantable medical device.
Fig. 14 shows a perspective elevated view from the right of an embodiment of an implantable energized medical device for powering an implantable medical device.
Fig. 15 shows a plain top view of an embodiment of an implantable energized medical device for powering an implantable medical device.
Figs. 16 and 17 show, schematically, plain top views of two embodiments of implantable energized medical devices for powering implantable medical devices.
Figs. 18a - 18c illustrate three stages of insertion and fixation of an embodiment of an implantable energized medical device for powering an implantable medical device.
Fig. 19 shows a detailed cross-sectional view of an embodiment of an implantable energized medical device for powering an implantable medical device.
Fig. 20a shows, schematically, a portion of an implantable energized medical device for powering an implantable medical device.
Fig. 20b shows, schematically, a portion of an implantable energized medical device for powering an implantable medical device.
Fig. 20c shows, schematically, a portion of an implantable energized medical device for powering an implantable medical device.
Fig. 21a shows a frontal view of a human patient in cross section when a remotely powered medical device for actively stretching a stomach wall of a patient has been implanted.
Fig. 21b shows a frontal view of a human patient in cross section when a remotely powered medical device for affecting the flow of urine of a patient has been implanted.
Fig. 21c shows a sectional side view of an embodiment of a hydraulic pump for an implantable medical device.
Fig. 21d shows a sectional side view of an embodiment of a hydraulic pump for an implantable medical device.
Fig. 21e shows a partially sectional perspective view from the left of an embodiment of a hydraulic pump for an implantable medical device.
Fig. 21f shows a partially sectional perspective view from the left of an embodiment of a hydraulic pump for an implantable medical device.
Fig. 21g shows a partially sectional perspective view from the left of an embodiment of a hydraulic pump for an implantable medical device.
Fig. 21h shows a partially sectional perspective view from the left of an embodiment of a hydraulic pump for an implantable medical device.
Fig. 21i shows a partially sectional perspective view from the left of an embodiment of a hydraulic pump for an implantable medical device.
Fig. 21k shows a partially sectional perspective view from the left of an embodiment of a hydraulic pump for an implantable medical device.
Fig. 21m shows a partially sectional perspective view from the left of an embodiment of a hydraulic pump for an implantable medical device.
Fig. 21n shows an elevated perspective view from the left of an embodiment of a hydraulic pump for an implantable medical device.
Figs. 22a - 22f show an embodiment and describes various functions of an implantable controller for controlling the implantable constriction device.
Figs. 22g - 22i, 22k, and 22m - 22p show embodiments and describes various functions of an implantable controller for controlling the implantable medical device / implant and a system for communication between different external devices.
Figs. 22q shows an elevated perspective view from the left of a housing unit.
Fig. 22r shows a plain view from the left of a housing unit.
Fig. 22s shows an elevated perspective view from the left of a housing unit.
Fig. 22t shows a plain view from the left of a housing unit.
Fig. 22u shows a system overview of an external device comprising a housing unit and a display device in wireless communication with an implanted medical device.
Fig. 23a shows a perspective elevated view from the right of an embodiment of an implantable energized medical device for powering an implantable medical device.
Figs. 23b and 23c show lengthwise cross-sectional areas of the implantable medical device along the line A-A in Fig. 23a.
Figs. 24-26 show cross-sectional plain side views of embodiments of an implantable energized medical device for powering an implantable medical device.
Fig. 27a shows a perspective elevated view from the right of an embodiment of an implantable energized medical device for powering an implantable medical device
Figs. 27b and 27c show lengthwise cross-sectional areas of the implantable medical device along the line A-A in Fig. 27a.

The figures are not necessarily to scale, and generally only show parts that are necessary in order to elucidate the inventive concept, wherein other parts may be omitted or merely suggested.

### Detailed description

In the following a detailed description of embodiments of the invention will be given with reference to the accompanying drawings. It will be appreciated that the drawings are for illustration only and are not in any way restricting the scope of the invention. Thus, any references to directions, such as "up" or "down", are only referring to the directions shown in the figures. It should be noted that the features having the same reference numerals have the same function, a feature in one embodiment could thus be exchanged for a feature from another embodiment having the same reference numeral unless clearly contradictory. The descriptions of the features having the same reference numerals should thus be seen as complementing each other in describing the fundamental idea of the feature and thereby showing the features' versatility.

The different aspects or any part of an aspect or different embodiments or any part of an embodiment may all be combined in any possible way. Any method or any step of method may be seen also as an apparatus description, as well as, any apparatus embodiment, aspect or part of aspect or part of embodiment may be seen as a method description and all may be combined in any possible way down to the smallest detail. Any detailed description should be interpreted in its broadest outline as a general summary description, and please note that any embodiment or part of embodiment as well as any method or part of method could be combined in any way. All examples herein should be seen as part of the general description and therefore possible to combine in any way in general terms.

It is important to note that although the implantable energized medical device is disclosed herein as having a third cross-sectional area being smaller than a first cross-sectional area, this feature is not essential. The third cross-sectional area may be equal to or larger than the first cross-sectional area.

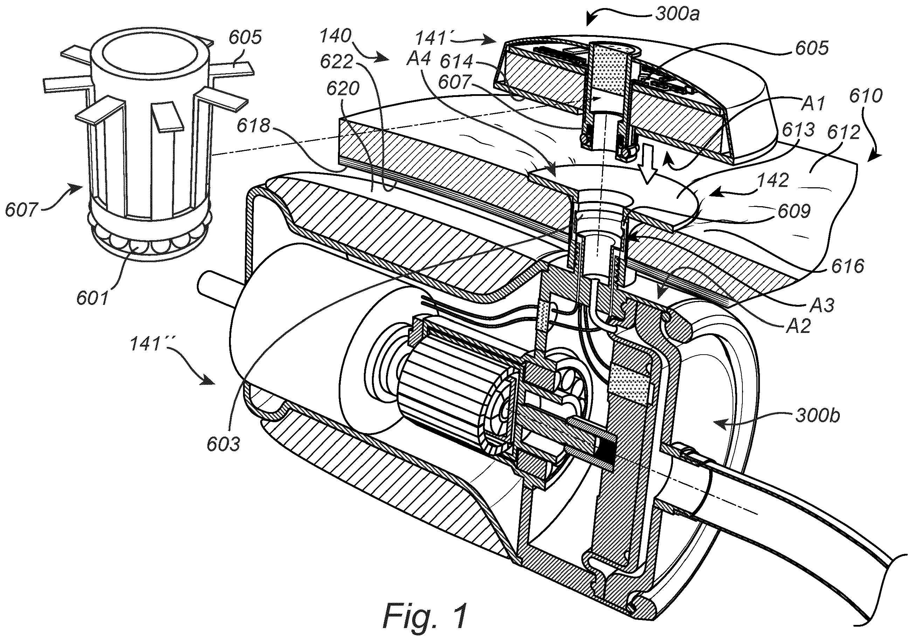

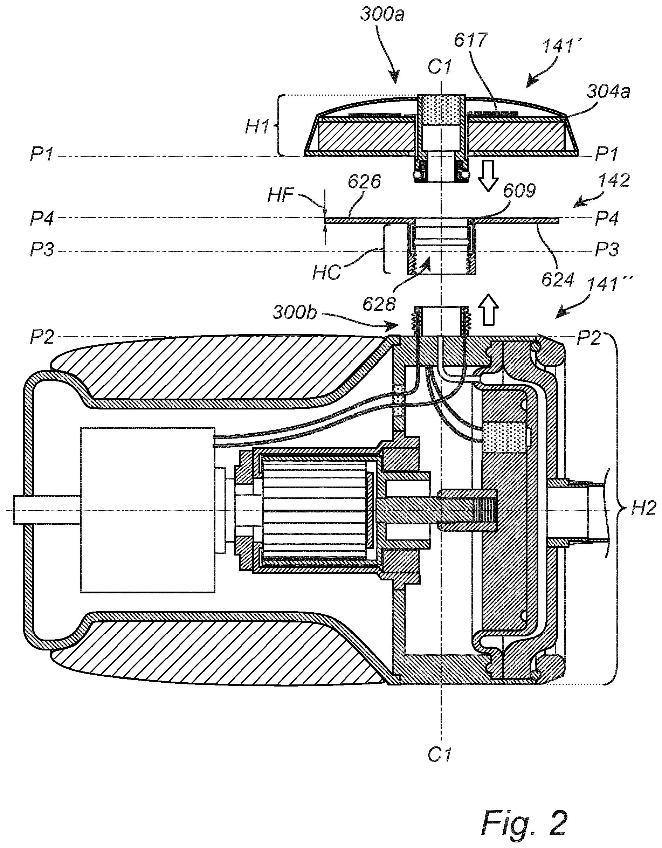

Figs. 1 and 2 show an embodiment of an implantable energized medical device 140, which may be referred to as a remote unit in other parts of the present disclosure. The device 140 is configured to be held in position by a tissue portion 610 of a patient. The device 140 comprises a first portion 141' configured to be placed on a first side 612 of the tissue portion 610, the first portion 141' having a first cross-sectional area A1 in a first plane P1 and comprising a first surface 614 configured to face a first tissue surface 616 of the first side 612 of the tissue portion 610. The device 140 further comprises a second portion 141" configured to be placed on a second side 618 of the tissue portion 610, the second side 618 opposing the first side 612, the second portion 141" having a second cross-sectional area A2 in a second plane P2 and comprising a second surface 620 configured to engage a second tissue surface 622 of the second side 618 of the tissue portion 610. The device 140 further comprises a connecting portion 142 configured to be placed through a hole in the tissue portion 610 extending between the first and second sides 612, 618 of the tissue portion 610. The connecting portion 142 here has a third cross-sectional area A3 in a third plane P3 and a fourth cross-sectional area A4 in a fourth plane P4 and a third surface 624 configured to engage the first tissue surface 616 of the first side 612 of the tissue portion 610. The connecting portion 142 is configured to connect the first portion 141' to the second portion 141".

The connecting portion 142 thus has a portion being sized and shaped to fit through the hole in the tissue portion 610, such portion having the third cross-sectional area A3. Furthermore, the connecting portion 142 may have another portion being sized and shaped to not fit through the hole in the tissue portion 610, such portion having the fourth cross-sectional area A4. Likewise, the second portion 141" may have a portion being sized and shaped to not fit through the hole in the tissue portion 610, such portion having the second cross-sectional area A2. Thus, the connecting portion 142 may cooperate with the second portion 141" to keep the device in place in the hole of the tissue portion 610.

In the embodiment illustrated in Fig. 1, the first portion 141' is configured to detachably connect, i.e. reversibly connect to the connecting portion 142 by a mechanical and/or magnetic mechanism. In the illustrated embodiment, a mechanic mechanism is used, wherein one or several spring-loaded spherical elements 601 lock in place in a groove 603 of the connecting portion 142 when the first portion 141' is inserted into the connecting portion 142. Other locking mechanisms are envisioned, including corresponding threads and grooves, self-locking elements, and twist and lock fittings.

The device 140 is configured such that, when implanted, the first portion 141' will be placed closer to an outside of the patient than the second portion 141". Furthermore, in some implantation procedures the device 140 may be implanted such that space will be available beyond the second portion, i.e. beyond the second side 618 of the tissue portion 610, whereas there may not be as much space on the first side 612 of the tissue portion. Furthermore, tissue and/or skin may exert a force on the first portion 141" towards the tissue portion 610, and provide for that the second portion 141" does not travel through the hole in the tissue portion towards the first side 612 of the tissue portion. Thus, it is preferably if the device 140 is primarily configured to prevent the first portion 141" from travelling through the hole in the tissue portion 612 towards the second side 618 of the tissue portion 610.

The first portion 141' may further comprise one or several connections 605 for transferring energy and/or communication signals to the second portion 141" via the connecting portion 142. The connections 605 in the illustrated embodiment are symmetrically arranged around a circumference of a protrusion 607 of the first portion 141' and are arranged to engage with a corresponding connection 609 arranged at an inner surface of the connecting portion 142. The protrusion 607 may extend in a central extension C1 of the central portion 142. The second portion 141" may also comprise one or several connections 611, which may be similarly arranged and configured as the connections 605 of the first portion 141'. For example, the one or several connections 611 may engage with the connection 609 of the connecting portion 142 to receive energy and/or communication signals from the first portion 141'. Although the protrusion 607 is illustrated separately in Fig. 1, it is to be understood that the protrusion 607 may be formed as one integral unit with the first portion 141'.

Other arrangements of connections are envisioned, such as asymmetrically arranged connections around the circumference of the protrusion 607. It is also envisioned that one or several connections may be arranged on the first surface 614 of the first portion 141', wherein the connections are arranged to engage with corresponding connections arranged on the opposing surface 613 of the connecting portion. Such connections on the opposing surface 613 may cover a relatively large area as compared to the connection 609, thus allowing a larger area of contact and a higher rate and/or signal strength of energy and/or communication signal transfer. Furthermore, it is envisioned that a physical connection between the first portion 141', connecting portion 142 and second portion 141" may be replaced or accompanied by a wireless arrangement, as described further in other parts of the present disclosure.

Any of the first surface 614 of the first portion 141', the second surface 620 of the second portion 141', the third surface 624 of the connecting portion 142, and an opposing surface 613 of the connecting portion 142, may be provided with at least one of ribs, barbs, hooks, a friction enhancing surface treatment, and a friction enhancing material, to facilitate the device 140 being held in position by the tissue portion, and/or to facilitate that the different parts of the device are held in mutual position.

The opposing surface 613 of the connecting portion 142 and the first surface 614 of the first portion 141' may provide, fully or partly, a connection mechanism to detachably connect the first portion 141' to the connecting portion 142. Such connection mechanisms have been described previously in the presented disclosure, and can be arranged on one or both of the opposing surface 613 and the first surface 614, and will not be further described here.

The opposing surface 613 may be provided with a recess configured to house at least part of the first portion 141'. In particular, such recess may be configured to receive at least a portion of the first portion 141', including the first surface 614. Similarly, the first surface 614 may be provided with a recess configured to house at least part of the connecting portion 142. In particular, such recess may be configured to receive at least a portion of the connecting portion 142, and in some embodiments such recess may be configured to receive at least one protruding element to at least partially enclose at least one protruding element or flange.

In the illustrated embodiment, the first portion 141' comprises a first energy storage unit 304a and a controller 300a comprising one or several processing units connected to the first energy storage unit 304a. The first energy storage unit 304a may be rechargeable by wireless transfer of energy. In some embodiments, the first energy storage unit 304a may be non-rechargeable. Upon reaching the life-time end of such first energy storage, a replacement first portion comprising a new first energy storage unit may simply be swapped in place for the first portion having the depleted first energy storage unit. The second portion 141" may further comprise a controller 300b comprising one or several processing units.

As will be described in other parts of the present disclosure, the first portion 141' and the second portion 141" may comprise one or several functional parts, such as receivers, transmitters, transceivers, control units, processing units, sensors, energy storage units, sensors, etc.

The device 140 may be non-inflatable.

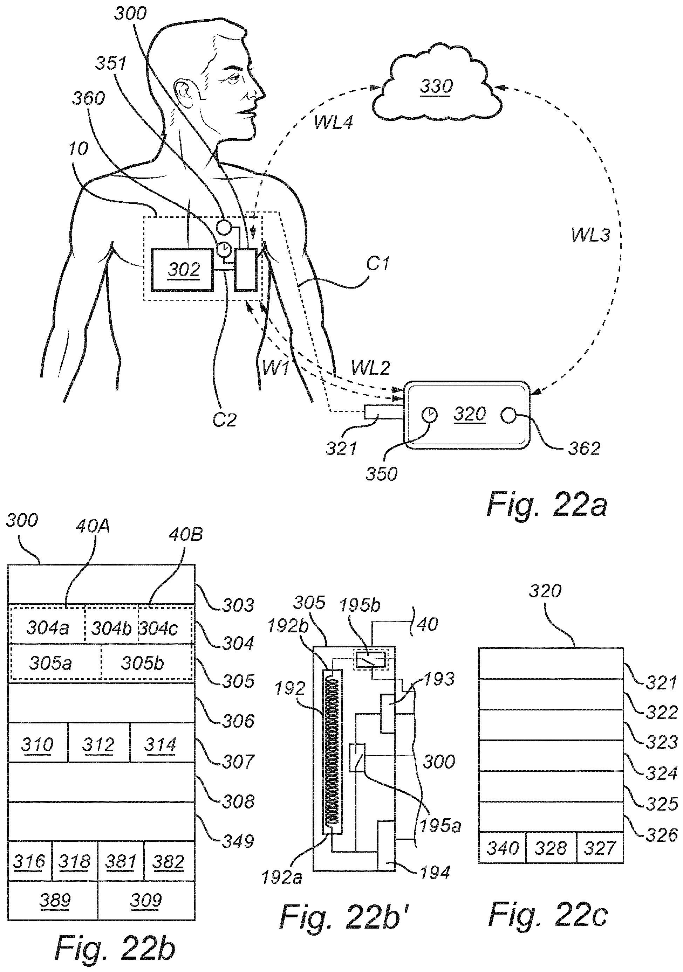

The second portion 141" in the illustrated embodiment comprises a pump as described in conjunction with Figs. 22c-22n However, it is to be understood that other embodiments of the second portion 141" are able to be connected to the first portion 141' via the connecting portion 142, such as second portions 141" comprising a motor for providing mechanical work without the use of fluids. Furthermore, although the connecting portion 142 is illustrated in Fig. 1 as a separate unit, the connecting portion 142 may form part of the second portion 141".

The first portion 141' may be detachably connected to at least one of the connecting portion 142 and the second portion 141".

As can be seen in Fig. 2, the first, second, third and fourth planes P1, P2, P3 and P4, are parallel to each other. Furthermore, in the illustrated embodiment, the third cross-sectional area A3 is smaller than the first, second and fourth cross-sectional areas A1, A2 and A4, such that the first portion 141', second portion 141" and connecting portion 142 are prevented from travelling through the hole in the tissue portion 610 in a direction perpendicular to the first, second and third planes P1, P2 and P3. Hereby, the second portion 141" and the connecting portion 142 can be held in position by the tissue portion 610 of the patient also when the first portion 141' is disconnected from the connecting portion 142.

It is to be understood that the illustrated planes P1, P2, P3 and P4 are merely an example of how such planes may intersect the device 140. Other arrangements of planes are possible, as long as the conditions above are fulfilled, i.e. that the portions have cross-sectional areas, wherein the third cross-sectional area in the third plane P3 is smaller than the first, second and fourth cross-sectional areas, and that the planes P1, P2, P3 and P4 are parallel to each other.

The connecting portion 142 illustrated in Fig. 2 may be defined as a connecting portion 142 comprising a flange 626. The flange 626 thus comprises the fourth cross-sectional area A4 such that the flange 626 is prevented from travelling through the hole in the tissue portion 610 in a direction perpendicular to the first, second and third planes P1, P2 and P3. The flange 626 may protrude in a direction parallel to the first, second, third and fourth planes P1, P2, P3 and P4. This direction is perpendicular to a central extension C1 of the connecting portion 142.

The connecting portion 142 is not restricted to flanges, however. Other protruding elements may additionally or alternatively be incorporated into the connecting portion 142. As such, the connecting portion 142 may comprise at least one protruding element comprising the fourth cross-sectional area A4, such that the at least one protruding element is prevented from travelling through the hole in the tissue portion 610, such that the second portion 141" and the connecting portion 142 can be held in position by the tissue portion 610 of the patient also when the first portion 141' is disconnected from the connecting portion 142. The at least one protruding element may protrude in a direction parallel to the first, second, third and fourth planes P1, P2, P3 and P4. This direction is perpendicular to a central extension C1 of the connecting portion 142. As such, the at least one protruding element will also comprise the third surface configured to engage the first tissue surface 616 of the first side 612 of the tissue portion 610.

The connecting portion 142 may comprise a hollow portion 628. The hollow portion 628 may provide a passage between the first and second portions 141', 141". In particular, the hollow portion 628 may house a conduit for transferring fluid from the first portion 141' to the second portion 141". The hollow portion 628 may also comprise or house one or several connections or electrical leads for transferring energy and/or communication signals between the first portion 141' and the second portion 141".

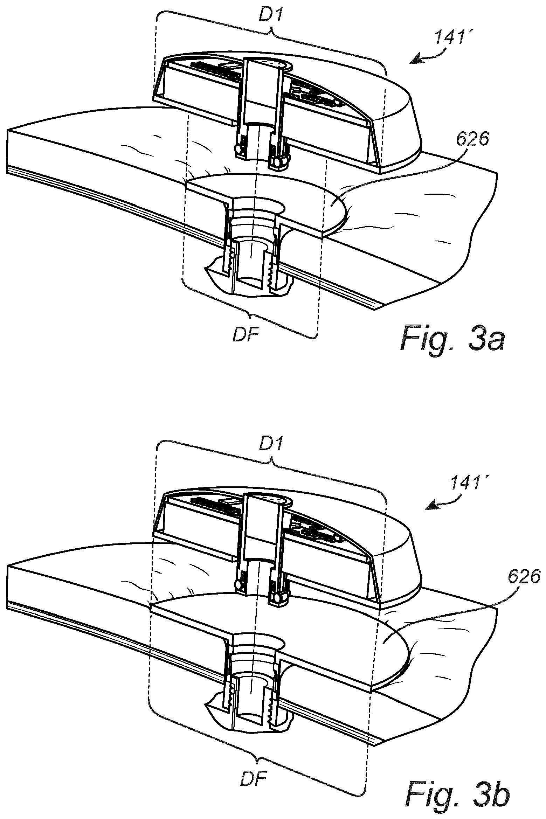

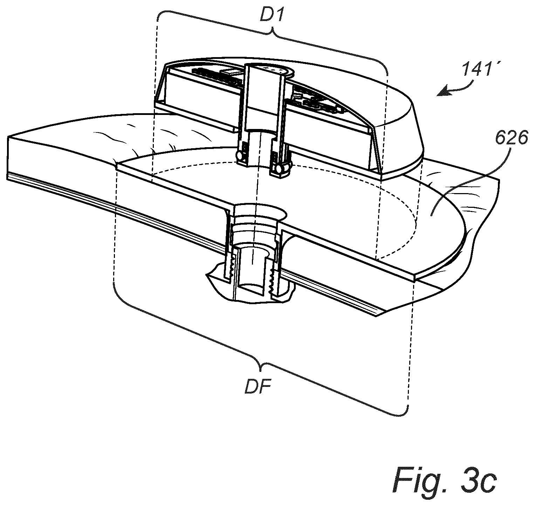

Some relative dimensions of the device 140 will now be described with reference to Figs. 2 and 3A-3D, however it is to be understood that these dimensions may also apply to other embodiments of the device 140. The at least one protruding element 626 may have a height HF in a direction perpendicular to the fourth plane being less than a height H1 of the first portion 141' in said direction. The height HF may alternatively be less than half of said height H1 of the first portion 141' in said direction, less than a quarter of said height H1 of the first portion 141' in said direction, or less than a tenth of said height H1 of the first portion 141' in said direction.

The height H1 of the first portion 141' in a direction perpendicular to the first plane may be less than a height H2 of the second portion 141" in said direction, such as less than half of said height H2 of the second portion 141"in said direction, less than a quarter of said height H2 of the second portion 141"in said direction, or less than a tenth of said height H2 of the second portion 141" in said direction.

The at least one protruding element 626 may have a diameter DF in the fourth plane being one of less than a diameter D1 of the first portion 141' in the first plane, equal to a diameter D1 of the first portion 141' in the first plane, and larger than a diameter D1 of the first portion 141' in the first plane. Similarly, the cross-sectional area of the at least one protruding element 626 in the fourth plane may be less, equal to, or larger than a cross-sectional area of the first portion in the first plane.

The at least one protruding element 626 may have a height HF in a direction perpendicular to the fourth plane being less than a height HC of the connecting portion 142 in said direction. Here, the height HC of the connecting portion 142 is defined as the height excluding the at least one protruding element, which forms part of the connecting portion 142. The height HF may alternatively be less than half of said height HC of the connecting portion 142 in said direction, less than a quarter of said height HC of the connecting portion 142 in said direction, or less than a tenth of said height HC of connecting portion 142 in said direction.

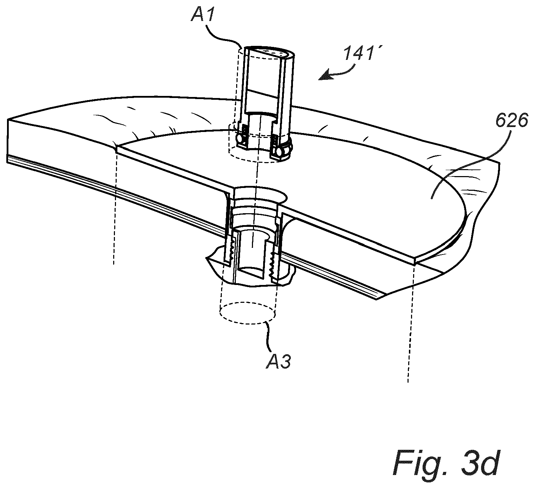

As shown in Fig. 3D, the first portion 141' may have a first cross-sectional area A1 being equal to or smaller than the third cross-sectional area A3 of the connecting portion 142. In particular, the first portion 141' does not necessarily need to provide a cross-sectional area being larger than the third cross-sectional area of connecting portion 142, intended to pass through a hole in the tissue, if the connecting portion 142 provides an additional cross-sectional area being larger than the third cross-sectional area of the connecting portion 142. The first portion 141' as illustrated in Fig. 3D may comprise the components discussed elsewhere in the present disclosure, although not shown, such as an energy storage unit, receiver, transmitter, etc.

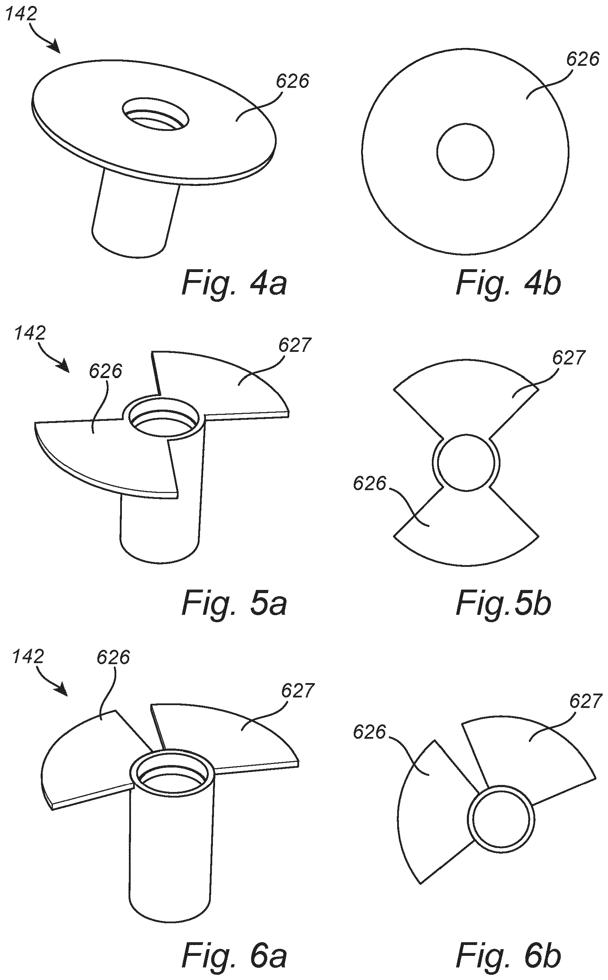

As shown in Figs. 4A-4B, the at least one protruding element 626 may have an annular shape, such as a disk shape. However, elliptical, elongated and/or other polyhedral or irregular shapes are also possible. In the illustrated embodiment, the at least one protruding element 626 extends a full revolution around the center axis of the connecting portion 142. However, other arrangements are possible, wherein the at least one protruding element 626 constitute a partial circle sector. In the case of a plurality of protruding elements, such plurality of protruding elements may constitute several partial circle sectors.

As shown in Figs. 5A-5B, 6A-6B, the connecting portion 142 may comprise at least two protruding elements 626, 627. For example, the connecting portion 142 may comprise at least three, four, five, fix, seven, eight, nine, ten protruding elements, and so on. In such embodiments, the at least two protruding elements 626, 627 may together comprise the fourth cross-sectional area, thus providing a necessary cross-sectional area to prevent the first portion and second portion from travelling through the hole in the tissue portion.

The at least two protruding elements 626, 627 may be symmetrically arranged about the central axis of the connecting portion, as shown in Figs. 5A-5B, or asymmetrically arranged about the central axis of the connecting portion, as shown in Figs. 6A-6B. In particular, the at least two protruding elements 626, 627 may be asymmetrically arranged so as to be located towards one side of the connecting portion 142, as shown in Figs. 6A-6B. The arrangement of protruding element(s) may allow the device 140, and in particular the connecting portion 142, to be placed in areas of the patient where space is limited in one or more directions.

The first portion 141' may comprise a first energy storage unit for supplying the device 140 with energy.

Although one type or embodiment of the implantable energized medical device 140, which may be referred to as a remote unit in other parts of the present disclosure, may fit most patients, it may be necessary to provide a selection of implantable energized medical devices 140 or portions to be assembled into implantable energized medical devices 140. For example, some patients may require different lengths, shapes, sizes, widths or heights depending on individual anatomy. Furthermore, some parts or portions of the implantable energized medical device 140 may be common among several different types or embodiments of implantable energized medical devices, while other parts or portions may be replaceable or interchangeable. Such parts or portions may include energy storage devices, communication devices, fluid connections, mechanical connections, electrical connections, and so on.

To provide flexibility and increase user friendliness, a kit of parts may be provided. The kit preferably comprises a group of one or more first portions, a group of one or more second portions, and a group of one or more connecting portions, the first portions, second portions and connecting portions being embodied as described throughout the present disclosure. At least one of the groups comprises at least two different types of said respective portions. By the term "type", it is hereby meant a variety, class or embodiment of said respective portion.

In some embodiments of the kit, the group of one or more first portions, the group of one or more second portions, and the group of one or more connecting portions, comprise separate parts which may be assembled into a complete implantable energized medical device. The implantable energized medical device may thus be said to be modular, in that the first portion, the second portion, and/or the connecting portion may be interchanged for another type of the respective portion.

In some embodiments, the connecting portion form part of the first portion or the second portion.

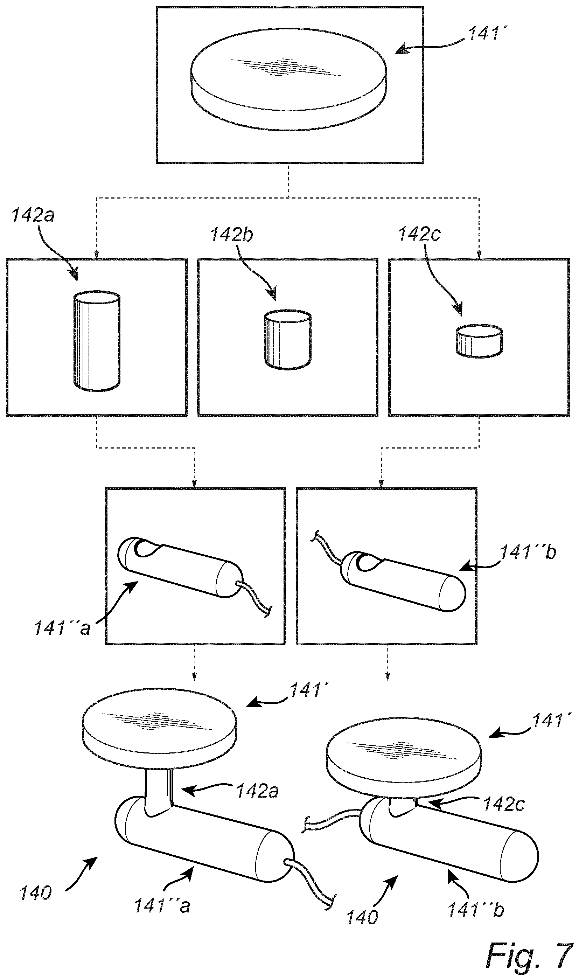

With reference to Fig. 7, the kit for assembling the implantable energized medical device comprises a group 650 of one or more first portions 141', in the illustrated example a group of one first portion 141', a group 652 of one or more connecting portions 142, in the illustrated example a group of three connecting portions 142, and a group 654 of one or more second portions 141", in the illustrated example a group of two second portions 141". For simplicity, all types and combinations of first portions, second portions and connecting portions will not be illustrated or described in detail.

Accordingly, the group 652 of one or more connecting portions 142 comprise three different types of connecting portions 142. Here, the different types of connecting portions 142 comprise connecting portions 142a, 142b, 142c having different heights. Furthermore, the group 654 of one or more second portions 141" comprise two different types of second portions 141".

Here, the different types of second portions 141" comprise a second portion 141"a being configured to excentrically connect to a connecting portion, having a first end and a second end as described in other parts of the present disclosure, wherein the second end of the second portion 141"a comprises or is configured for at least one connection for connecting to an implant being located in a caudal direction from a location of the implantable energized medical device in the patient, when the device is assembled. In the illustrated figure, the at least one connection is visualized as a lead or wire. However, other embodiments are possible, including the second end comprising a port, connector or other type of connective element for transmission of power, fluid, and/or signals.

Furthermore, the different types of second portions 141" comprise a second portion 141"b being configured to excentrically connect to a connecting portion, having a first end and a second end as described in other parts of the present disclosure, wherein the first end of the second portion 141"b comprises or is configured for at least one connection for connecting to an implant being located in a cranial direction from a location of the implantable energized medical device in the patient, when the device is assembled. In the illustrated figure, the at least one connection is visualized as a lead or wire. However, other embodiments are possible, including the first end comprising a port, connector or other type of connective element for transmission of power, fluid, and/or signals.

Thus, the implantable energized medical device may be modular, and different types of devices can be achieved by selecting and combining a first portion 141', a connecting portion 142, and a second portion 141", from each of the groups 652, 654, 656.

In the illustrated example, a first implantable energized medical device 140a is achieved by a selection of the first portion 141', the connecting portion 142a, and the second portion 141"a. Such device 140a may be particularly advantageous in that the connecting portion 142a may be able to extend through a thick layer of tissue to connect the first portion 141' and the second portion 141"a. Another implantable energized medical device 140b is achieved by a selection of the first portion 141', the connecting portion 142c, and the second portion 141"b. Such device may be particularly advantageous in that the connecting portion 142c has a smaller footprint than the connecting portion 142a, i.e. occupying less space in the patient. Owing to the modular property of the devices 140a and 140b, a practician or surgeon may select a suitable connecting portion as needed upon having assessed the anatomy of a patient. Furthermore, since devices 140a and 140b share a common type of first portions 141', it will not be necessary for a practician or surgeon to maintain a stock of different first portions (or a stock of complete, assembled devices) merely for the sake of achieving a device having different connections located in the first end or second end of the second portion respectively, as in the case of second portions 141"a, 141"b.

The example illustrated in Fig. 7 is merely exemplifying to display the idea of a modular implantable energized medical device 140. The group 650 of one or more first portions 141' may comprise a variety of different features, such as first portions with or without a first energy storage unit, with or without a first wireless energy receiver unit for receiving energy transmitted wirelessly by an external wireless energy transmitter, with or without an internal wireless energy transmitter, and/or other features as described throughout the present disclosure. Other features include different height, width, or length of the first portion. It is to be understood that first portions having one or more such features may be combined with a particular shape or dimensions to achieve a variety of first portions. The same applies to connecting portions and second portions.

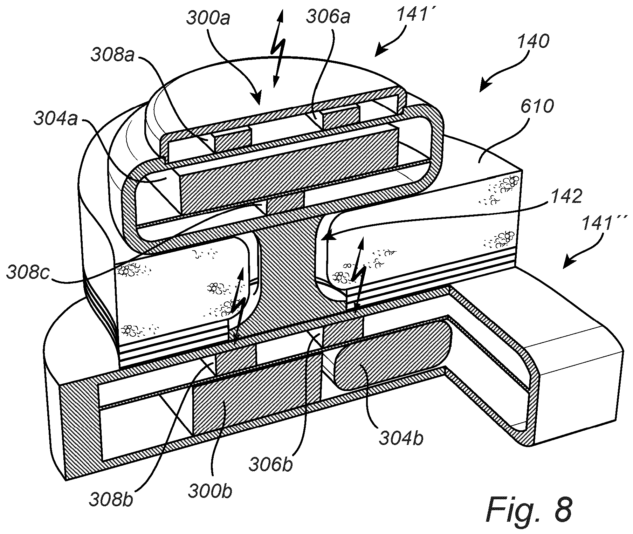

With reference to Fig. 8, an embodiment of an implantable energized medical device 140, which may be referred to as a remote unit in other parts of the present disclosure, will be described. The device 140 is configured to be held in position by a tissue portion 610 of a patient. The device 140 comprises a first portion 141' configured to be placed on a first side of the tissue portion 610, the first portion 141' having a first cross-sectional area in a first plane and comprising a first surface configured to face and/or engage a first tissue surface of the first side of the tissue portion 610. The device 140 further comprises a second portion 141" configured to be placed on a second side of the tissue portion 610, the second side opposing the first side, the second portion 141" having a second cross-sectional area in a second plane and comprising a second surface configured to engage a second tissue surface of the second side of the tissue portion 610. The device 140 further comprises a connecting portion 142 configured to be placed through a hole in the tissue portion 610 extending between the first and second sides of the tissue portion 610. The connecting portion 142 here has a third cross-sectional area in a third plane. The connecting portion 142 is configured to connect the first portion 141' to the second portion 141". Here, the first portion 141' comprises a first wireless energy receiver 308a for receiving energy transmitted wirelessly by an external wireless energy transmitter, and an internal wireless energy transmitter 308a configured to transmit energy wirelessly to the second portion. Furthermore, the second portion here comprises a second wireless energy receiver 308b configured to receive energy transmitted wirelessly by the internal wireless energy transmitter 308a.

Although receivers and transmitters may be discussed and illustrated separately in the present disclosure, it is to be understood that the receivers and/or transmitters may be comprised in a transceiver. Furthermore, the receivers and/or transmitters in the first portion 141' and second portion 141" respectively may form part of a single receiving or transmitting unit configured for receiving or transmitting energy and/or communication signals, including data. Furthermore, the internal wireless energy transmitter and/or a first wireless communication receiver/transmitter may be a separate unit 308c located in a lower portion of the first portion 141', referred to as a proximal end of the first portion 141' in other parts of the present disclosure, close to the connecting portion 142 and the second portion 141". Such placement may provide for that energy and/or communication signals transmitted by the unit 308c will not be attenuated by internal components of the first portion 141' when being transmitted to the second portion 141". Such internal components may include a first energy storage unit 304a.

The first portion 141' here comprises a first energy storage unit 304a connected to the first wireless energy receiver 308a. The second portion comprises a second energy storage unit 304b connected to the second wireless energy receiver 308b. Such an energy storage unit may be a solid-state battery, such as a thionyl-chloride battery.

In some embodiments, the first wireless energy receiver 308a is configured to receive energy transmitted wirelessly by the external wireless energy transmitter and store the received energy in the first energy storage unit 304a. Furthermore, the internal wireless energy transmitter 308a is configured to wirelessly transmit energy stored in the first energy storage unit 304a to the second wireless energy receiver 308b, and the second wireless energy receiver 308b is configured to receive energy transmitted wirelessly by the internal wireless energy transmitter 308a and store the received energy in the second energy storage unit 305b.

The first energy storage unit 304a may be configured to store less energy than the second energy storage unit 304b, and/or configured to be charged faster than the second energy storage unit 304b. Hereby, charging of the first energy storage unit 304a may be relatively quick, whereas transfer of energy from the first energy storage unit 304a to the second energy storage unit 304b may be relatively slow. Thus, a user can quickly charge the first energy storage unit 304a, and will not during such charging be restricted for a long period of time by being connected to an external wireless energy transmitter, e.g. at a particular location. After having charged the first energy storage unit 304a, the user may move freely while energy slowly transfers from the first energy storage unit 304a to the second energy storage unit 304b, via the first wireless energy transmitter 308a,c and the second wireless energy receiver 308b.

The first portion may comprise a first controller comprising at least one processing unit 306a. The second portion may comprise a second controller comprising at least one processing unit 306b. At least one of the first and second processing unit 306a, 306b may be connected to a wireless transceiver 308a,b,c for communicating wirelessly with an external device.

The first controller may be connected to a first wireless communication receiver 308a,c in the first portion 141' for receiving wireless communication from an external device and/or from a wireless communication transmitter 308b in the second portion 141". Furthermore, the first controller may be connected to a first wireless communication transmitter 308a,c in the first portion 141' for transmitting wireless communication to a second wireless communication receiver 308b in the second portion 141". The second controller may be connected to the second wireless communication receiver 308b for receiving wireless communication from the first portion 141'. The second controller may further be connected to a second wireless communication transmitter 308b for transmitting wireless communication to the first portion 141'.

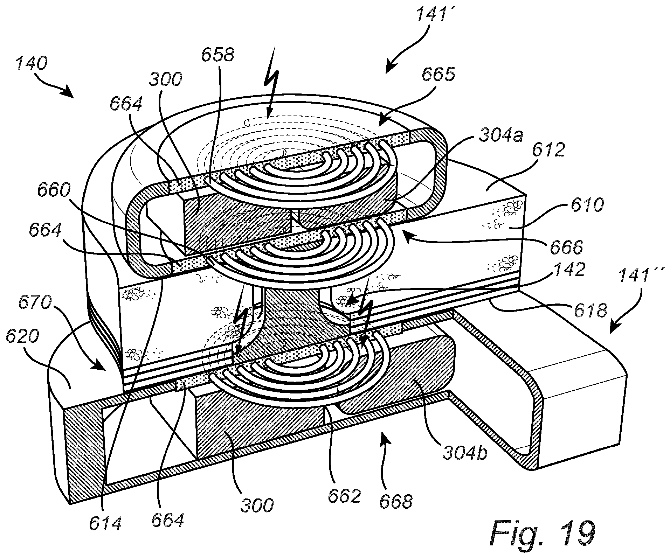

In some embodiments, the first wireless energy receiver 308a comprises a first coil, and the wireless energy transmitter 308a,c comprises a second coil, as shown in Fig. 19.

The device may further comprising at least one sensor (not shown) for providing input to at least one of the first and second controller. Such sensor data may be transmitted to an external device via the first wireless communication transmitter 308a and/or the second wireless communication transmitter 308b. The sensor may be or comprise a sensor configured to sense a physical parameter of the device 140. The sensor may also be or comprise a sensor configured to sense at least one of a temperature of the device 140, a temperature of a body engaging portion, a parameter related to the power consumption of the device, a parameter related to the power consumption of a body engaging portion, a parameter related to a status of at least one of the first and second energy storage units 304a, 304b, such as a health status of at least one of the first and second energy storage units 304a, 304b, a parameter related to the wireless transfer of energy from a source external to the body of the patient, and a hydraulic pressure. By the term "health status" it is hereby meant a status indicating the current total capacity of the energy storage unit as compared to the total capacity of an unused energy storage unit. The sensor may also be or comprise a sensor configured to sense a physiological parameter of the patient, such as at least one of a parameter related to the patient swallowing, a local temperature, a systemic temperature, a blood saturation, a blood oxygenation, a blood pressure, a parameter related to an ischemia marker, or pH. The sensor configured to sense a parameter related to the patient swallowing may comprise at least one of a motility sensor, a sonic sensor, an optical sensor, and a strain sensor. The sensor configured to sense pH may be configured to sense the acidity in the stomach.

The sensor may be configured to sense a temperature of the device 140, to avoid excessive heating of tissue connected to the device during operation of the device, or during operation of an external implant using the device, or charging of an energy storage unit in the device 140. Excessive heating may also damage the device and/or the energy storage unit. Excessive heating may also be an indicator that something is wrong with the device and may be used for triggering an alarm function for alerting the patient or physician. The sensor may also be configured to sense a parameter related to the power consumption of the device 140 or the power consumption of an external implant being powered by the device 140, to avoid excessive power consumption which may drain and/or damage the energy storage unit of the device 140. Excessive power consumption may also be an indicator that something is wrong with the device 140 and may be used for triggering an alarm function for alerting the patient or physician.

Wireless energy receivers and/or communication receivers and/or transmitters in the first portion 141' may be configured to receive energy from and/or communicate wirelessly with an external device outside the body using electromagnetic waves at a frequency below 100 kHz, or more specifically below 40 kHz, or more specifically below 20 kHz. The wireless energy receivers and/or communication receivers and/or transmitters in the first portion 141' may thus be configured to communicate with the external device using "Very Low Frequency" communication (VLF). VLF signals have the ability to penetrate a titanium housing of the implantable energized medical device, such that the electronics of the implantable medical device can be completely encapsulated in a titanium housing. In addition, or alternatively, communication and energy transfer between the first portion 141' and second portion 141" may be made using VLF signals. In such embodiments, receivers and transmitters (for energy and/or communication) of the first portion 141' and second portion 141" are configured accordingly.

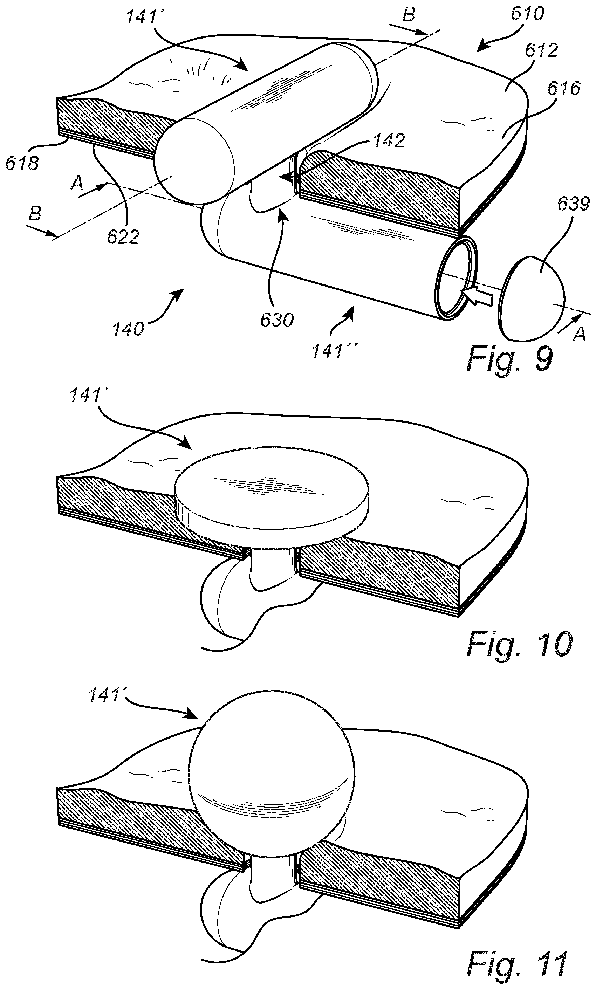

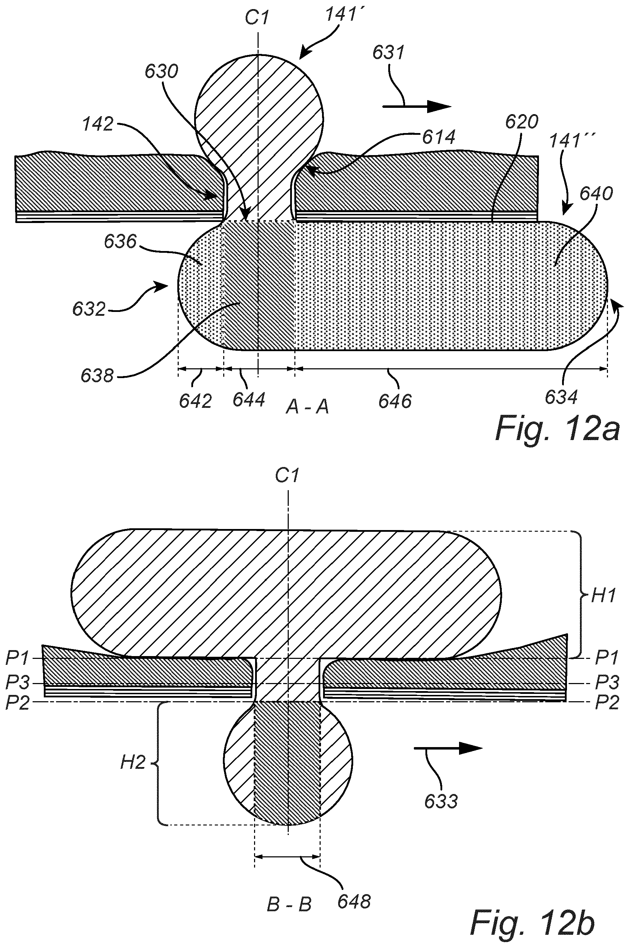

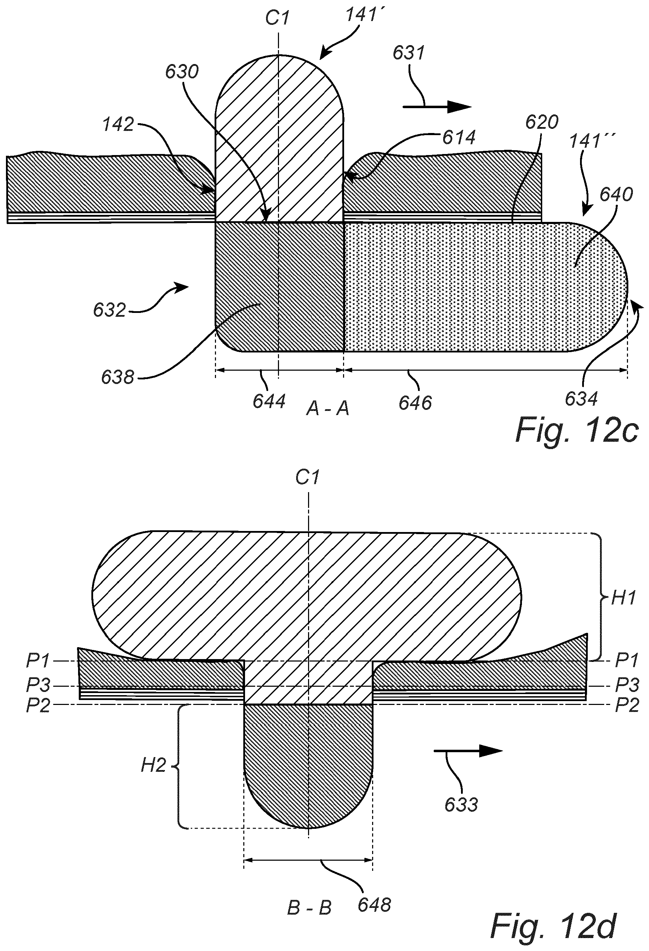

With reference to Figs. 9, 12A and 12B, an embodiment of an implantable energized medical device 140, which may be referred to as a remote unit in other parts of the present disclosure, will be described. The device 140 is configured to be held in position by a tissue portion 610 of a patient. The device 140 comprises a first portion 141' configured to be placed on a first side 612 of the tissue portion 610, the first portion 141' having a first cross-sectional area A1 in a first plane P1 and comprising a first surface 614 configured to face and/or engage a first tissue surface 616 of the first side 612 of the tissue portion 610. The device 140 further comprises a second portion 141" configured to be placed on a second side 618 of the tissue portion 610, the second side 618 opposing the first side 612, the second portion 141" having a second cross-sectional area A2 in a second plane P2 and comprising a second surface 620 configured to engage a second tissue surface 622 of the second side 618 of the tissue portion 610. The device 140 further comprises a connecting portion 142 configured to be placed through a hole in the tissue portion 610 extending between the first and second sides 612, 618 of the tissue portion 610. The connecting portion 142 here has a third cross-sectional area A3 in a third plane P3. The connecting portion 142 is configured to connect the first portion 141' to the second portion 141". In the illustrated embodiment, a connecting interface 630 between the connecting portion 142 and the second portion 141" is excentric with respect to the second portion 141".

The first portion 141' has an elongated shape in the illustrated embodiment of Fig. 1. Similarly, the second portion 141" has an elongated shape. However, the first portion 141' and/or second portion 141" may assume other shapes, such as a flat disk e.g. having a width and length being larger than the height, a sphere, an ellipsoid, or any other polyhedral or irregular shape, some of these being exemplified in Figs. 9-11.

As illustrated in Figs. 12A and 12B, the connecting interface 630 between the connecting portion 142 and the second portion 141" may be excentric, with respect to the second portion 141" in a first direction 631, but not in a second direction 633 being perpendicular to the first direction. The first direction 631 is here parallel to the line A-A, to the second plane P2, and to a length of the second portion 141". The second direction 633 is here parallel to the line B-B, to the second plane P2, and to a width of the second portion 141". It is also possible that the connecting interface between the connecting portion 142 and the second portion 141" is excentric, with respect to the second portion 141", in the first direction 631 as well as in the second direction 633 being perpendicular to the first direction 631.

Similarly, a connecting interface between the connecting portion 142 and the first portion 141' may be excentric with respect to the first portion 141' in the first direction 631, and/or in the second direction 633.

The first portion 141', connecting portion 142 and second portion 141" may structurally form one integral unit. It is however also possible that the first portion 141' and the connecting portion 142 structurally form one integral unit, while the second portion 141" form a separate unit, or, that the second portion 141" and the connecting portion 142 structurally form one integral unit, while the first portion 141' form a separate unit.

Additionally, or alternatively, the second portion 141" may comprise a removable and/or interchangeable portion 639. In some embodiments, the removable portion 639 may form part of a distal region which will be further described in other parts of the present disclosure. A removable portion may also form part of a proximal region. Thus, the second portion 141" may comprise at least two removable portions, each being arranged at a respective end of the second portion 141". The removable portion 639 may house, hold or comprise one or several functional parts of the device 140, such as gears, motors, connections, reservoirs, and the like as described in other parts of the present disclosure. An embodiment having such removable portion 639 will be able to be modified as necessary to circumstances of a particular patient.

In the case of the first portion 141', connecting portion 142 and second portion 141" structurally forming one integral unit, the excentric connecting interface between the connecting portion 142 and the second portion 141", with respect to the second portion 141", will provide for that the device 140 will be able to be inserted into the hole in the tissue portion. The device 140 may for example be inserted into the hole at an angle, similar to how a foot is inserted into a shoe, to allow most or all of the second portion 141" to pass through the hole, before it is angled, rotated, and/or pivoted to allow any remaining portion of the second portion 141" to pass through the hole and allow the device 140 to assume its intended position.

As illustrated in Figs. 9, 10 and 11, the first portion 141' may assume a variety of shapes, such as an oblong shape, a flat disk shape, a spherical shape, or any other polyhedral or irregular shape. Similarly, the second portion 141" may assume a variety of shapes, such as an oblong shape, a flat disk shape, a spherical shape, or any other polyhedral or irregular shape. The proposed shapes of the first and second portions 141', 141" may be mixed and combined to form embodiments not exemplified in the illustrated embodiments. For example, one or both of the first and second portions 141', 141" may have a flat oblong shape. In this context, the term "flat" is related to the height of the first or second portion 141', 141", i.e. in a direction parallel to a central extension C1 of the connecting portion 142. The term "oblong" is related to a length of the first or second portion 141', 141". A definition of such length is further discussed in other parts of the present disclosure.

With reference to Figs. 12A-12B, the second portion 141" has a first end 632 and a second end 634 opposing the first end 632. The length of the second portion 141" is defined as the length between the first end 632 and the second end 634. The length of the second portion 141" is furthermore extending in a direction being different to the central extension C1 of the connecting portion 142. The first end 632 and second end 634 are separated in a direction parallel to the second plane P2. Similarly, the first portion 141' has a length between a first and a second end, the length extending in a direction being different to the central extension C1 of the connecting portion 142.

The second portion 141" may be curved along its length. For example, one or both ends of the second portion 141" may point in a direction being substantially different from the second plane P2, i.e. curving away from or towards the tissue portion when implanted. In some embodiments, the second portion 141" curves within the second plane P2, exclusively or in combination with curving in other planes. The second portion 141" may also be curved in more than one direction, i.e. along its length and along its width, the width extending in a direction perpendicular to the length.

The first and second ends 632, 634 of the second portion 141" may comprise an elliptical point respectively. For example, the first and second ends 632, 634 may comprise a hemispherical end cap respectively. It is to be understood that also the first and second ends of the first portion 141' may have such features.

The second portion 141" may have at least one circular cross-section along the length between the first end 632 and second end 634, as illustrated in Fig. 9 . It is however possible for the second portion 141" to have at least one oval cross-section or at least one elliptical cross-section along the length between the first end 632 and the second end 634. Such cross-sectional shapes may also exist between ends in a width direction of the second portion 141". Similarly, such cross-sectional shapes may also exist between ends in a length and/or width direction in the first portion 141'.

In the following paragraphs, some features and properties of the second portion 141" will be described. It is however to be understood that these features and properties may also apply to the first portion 141'.

The second portion 141" has a proximal region 636, an intermediate region 638, and a distal region 640. The proximal region 636 extends from the first end 632 to an interface between the connecting portion 142 and the second portion 141", the intermediate region 638 is defined by the connecting interface 630 between the connecting portion 142 and the second portion 141", and the distal region 640 extends from the connecting interface 630 between the connecting portion 142 and the second portion 141" to the second end 634. The proximal region 636 is shorter than the distal region 640 with respect to the length of the second portion, i.e. with respect to the length direction 631. Thus, a heel (the proximal region) and a toe (the distal region) is present in the second portion 141".

The second surface 620, configured to engage with the second tissue surface 622 of the second side 618 of the tissue portion 610, is part of the proximal region 636 and the distal region 640. If a length of the second portion 141" is defined as x, and the width of the second portion 141" is defined as y along respective length and width directions 631, 633 being perpendicular to each other and substantially parallel to the second plane P2, the connecting interface between the connecting portion 142 and the second portion 141" is contained within a region extending from x>0 to x<x/2 and/or y>0 to y<y/2, x and y and 0 being respective end points of the second portion 141" along said length and width directions. In other words, the connecting interface between the connecting portion 142 and the second portion 141" is excentric in at least one direction with respect to the second portion 141", such that a heel and a toe is formed in the second portion 141".

The first surface 614 configured to face and/or engage the first tissue surface 616 of the first side 612 of the tissue portion 610 may be substantially flat. In other words, the first portion 141' may comprise a substantially flat side facing towards the tissue portion 610. Furthermore, an opposing surface of the first portion 141', facing away from the tissue portion 610, may be substantially flat. Similarly, the second surface 620 configured to engage the second tissue surface 622 of the second side 618 of the tissue portion 610 may be substantially flat. In other words, the second portion 141" may comprise a substantially flat side facing towards the tissue portion 610. Furthermore, an opposing surface of the second portion 141", facing away from the tissue portion 610, may be substantially flat.

The second portion 141" may be tapered from the first end 632 to the second end 634, thus giving the second portion 141" different heights and/or widths along the length of the second portion 141". The second portion may also be tapered from each of the first end 632 and second end 634 towards the intermediate region 638 of the second portion 141".

Some dimensions of the first portion 141', the second portion 141" and the connecting portion 142 will now be disclosed. Any of the following disclosures of numerical intervals may include or exclude the end points of said intervals.

The first portion 141' may have a maximum dimension being in the range of 10 to 60 mm, such as in the range of 10 to 40 mm such as in the range of 10 to 30 mm, such as in the range of 10 to 25 mm, such as in the range of 15 to 40 mm, such as in the range of 15 to 35 mm, such as in the range of 15 to 30 mm, such as in the range of 15 to 25 mm. By the term "maximum dimension" it is hereby meant the largest dimension in any direction.

The first portion 141' may have a diameter being in the range of 10 to 60 mm, such as in the range of 10 to 40 mm such as in the range of 10 to 30 mm, such as in the range of 10 to 25 mm, such as in the range of 15 to 40 mm, such as in the range of 15 to 35 mm, such as in the range of 15 to 30 mm, such as in the range of 15 to 25 mm.

The connecting portion 142 may have a maximum dimension in the third plane P3 in the range of 2 to 20 mm, such as in the range of 2 to 15 mm, such as in the range of 2 to 10 mm, such as in the range of 5 to 10 mm, such as in the range of 8 to 20 mm, such as in the range of 8 to 15 mm, such as in the range of 8 to 10 mm.

The second portion 141" may have a maximum dimension being in the range of 30 to 90 mm, such as in the range of 30 to 70 mm, such as in the range of 30 to 60 mm, such as in the range of 30 to 40 mm, such as in the range of 35 to 90 mm, such as in the range of 35 to 70 mm, such as in the range of 35 to 60 mm, such as in the range of 35 to 40 mm.

The first portion has a first height H1, and the second portion has a second height H2, both heights being in a direction perpendicular to the first and second planes P1, P2. The first height may be smaller than the second height. However, in the embodiments illustrated in Figs. 12A-12B, the first height H1 is substantially equal to the second height H2. Other height ratios are possible, for example the first height H1 may be less than 2/3 of the second height H2, such as less than 1/2 of the second height H2, such as less than 1/3 of the second height H2, such as less than 1/4 of the second height H2, such as less than 1/5 of the second height H2, such as less than 1/10 of the second height H2.

As illustrated in Figs. 12A-12B, the proximal region 636 has a length 642 being shorter than a length 646 of the distal region 640. The intermediate region 638 has a length 644, and a width 648. In some embodiments, the length 644 of the intermediate region 638 is longer than the width 648. In other words, the connecting interface between the connecting portion 142 and the second portion 141" may be elongated, having a longer dimension (in the exemplified case, the length) and a shorter dimension (in the exemplified case, the width). It is also possible that the length 644 of the intermediate region 638 is shorter than the width 648 of the intermediate region 638.

The length 646 of the distal region 640 is preferably longer than the length 644 of the intermediate region 638, however, an equally long distal region 640 and intermediate region 638, or a shorter distal region 640 than the intermediate region 638, is also possible. The length 642 of the proximal region 636 may be shorter than, equal to, or longer than the length 644 of the intermediate region 638.

The length 644 of the intermediate region 638 is preferably less than half of the length of the second portion 141", i.e. less than half of the combined length of the proximal region 636, the intermediate region 638, and the distal region 630. In some embodiments, the length 644 of the intermediate region 638 is less than a third of the length of the second portion 141", such as less than a fourth, less than a fifth, or less than a tenth of the length of the second portion 141".

The connecting portion may have one of an oval cross-section, an elongated cross-section, and a circular cross-section, in a plane parallel to the third plane P3. In particular, the connecting portion may have several different cross-sectional shapes along its length in the central extension C1.

Figs. 12c-12d illustrate an embodiment similar to the one described in conjunction with Figs. 12a-12b. However, the embodiment of Figs. 12c-12d lacks a proximal portion, i.e. the second portion 141" does not comprise a "heel". Furthermore, such embodiment may have a connecting portion 142 having a length and width, in directions 631 and 633 respectively, being equal to a height of the second portion in a direction parallel to the central extension C1, as illustrated. Thus, the connecting portion 142 and the second portion 141" may be constituted by a substantially uniformly wide body.

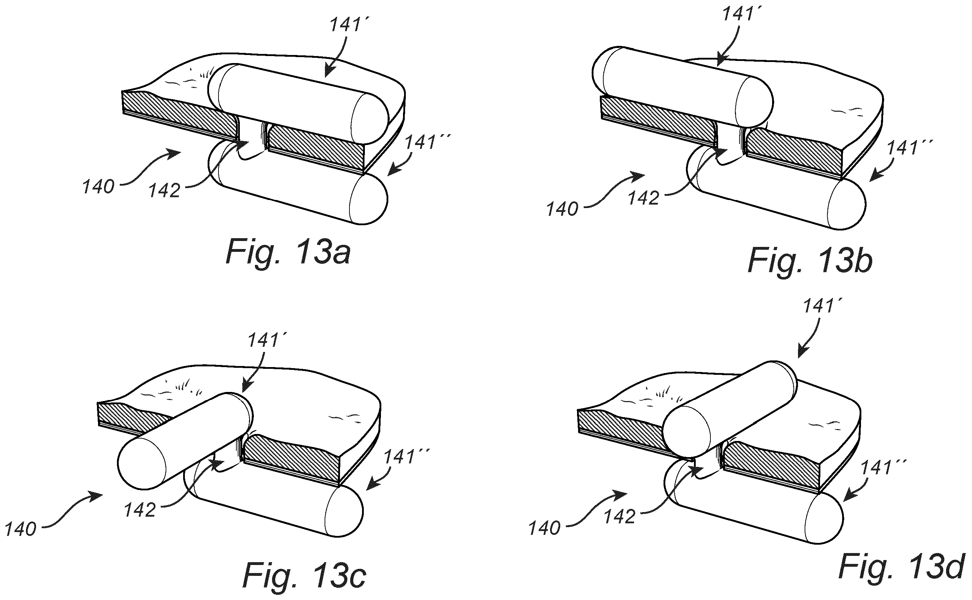

In some embodiments the distal region 640 is configured to be directed downwards in a standing patient, i.e. in a caudal direction when the device 140 is implanted. As illustrated in Figs. 13A-13D, different orientations of the second portion 141" relative the first portion 141' are possible. In some embodiments, a connection between either the first portion 141' and the connecting portion 142, or between the second portion 141" and the connecting portion 142, may allow for a plurality of different connecting orientations. For example, a connection mechanism between the first portion 141' and the connecting portion 142 (or between the second portion 141" and the connecting portion 142) may posses a 90 degree rotational symmetry to allow the second portion 141' to be set in four different positions with respect to the first portion 141, each differing from the other by 90 degrees. Other degrees of rotational symmetry are of course possible, such as 30 degrees, 45 degrees, 60 degrees, 120 degrees, 180 degrees and so on. In other embodiments there are no connective mechanism between any of the first portion 141', the connecting portion 142, and the second portion 141" (i.e. the portions are made as one integral unit), and in such cases different variants of the device 140 can be achieved during manufacturing. In other embodiments, the connective mechanism between the first portion 141' and the connecting portion 142 (or between the second portion 141" and the connecting portion 142) is non-reversible, i.e. the first portion 141' and the second portion 141" may initially be handled as separate parts, but the orientation of the second portion 141" relative the first portion 141' cannot be changed once it has been selected and the parts have been connected via the connecting portion 142.

The different orientations of the second portion 141" relative the first portion 141' may be defined as the length direction of the second portion 141" having a relation or angle with respect to a length direction of the first portion 141'. Such angle may be 15 degrees, 30, 45, 60, 75 90, 105, 120, 135, 150, 165, 180, 195, 210, 225, 240, 255, 270, 285, 300, 315, 330, 345 or 360 degrees. In particular, the angle between the first portion 141' and the second portion 141" may be defined as an angle in the planes P1 and P2, or as an angle in a plane parallel to the tissue portion 610, when the device 140 is implanted. In the embodiment illustrated in Figs. 13A-13D, the length direction of the second portion 141" is angled by 0, 90, 180, and 270 degrees with respect to the length direction of the first portion 141'.

The second end 634 of the second portion 141" may comprise one or several connections for connecting to an implant being located in a caudal direction from a location of the implantable energized medical device in the patient. Hereby, when the device 140 is implanted in a patient, preferably with the distal region 640 and second end 634 pointing downwards in a standing patient, the connections will be closer to the implant as the second end 634 will be pointing in the caudal direction whereas the first end 632 will be pointing in the cranial direction. It is also possible that the second end 634 of the second portion 141" is configured for connecting to an implant, i.e. the second end 634 may comprise a port, connector or other type of connective element for transmission of power, fluid, and/or signals.

Likewise, the first end 632 of the second portion 141" may comprise one or several connections for connecting to an implant being located in a cranial direction from a location of the implantable energized medical device in the patient. Hereby, when the device 140 is implanted in a patient, preferably with the distal region 640 and second end 634 pointing downwards in a standing patient, the connections will be closer to the implant as the first end 632 will be pointing in the cranial direction whereas the second end 634 will be pointing in the caudal direction. It is also possible that the first end 632 of the second portion 141" is configured for connecting to an implant, i.e. the first end 632 may comprise a port, connector or other type of connective element for transmission of power, fluid, and/or signals.

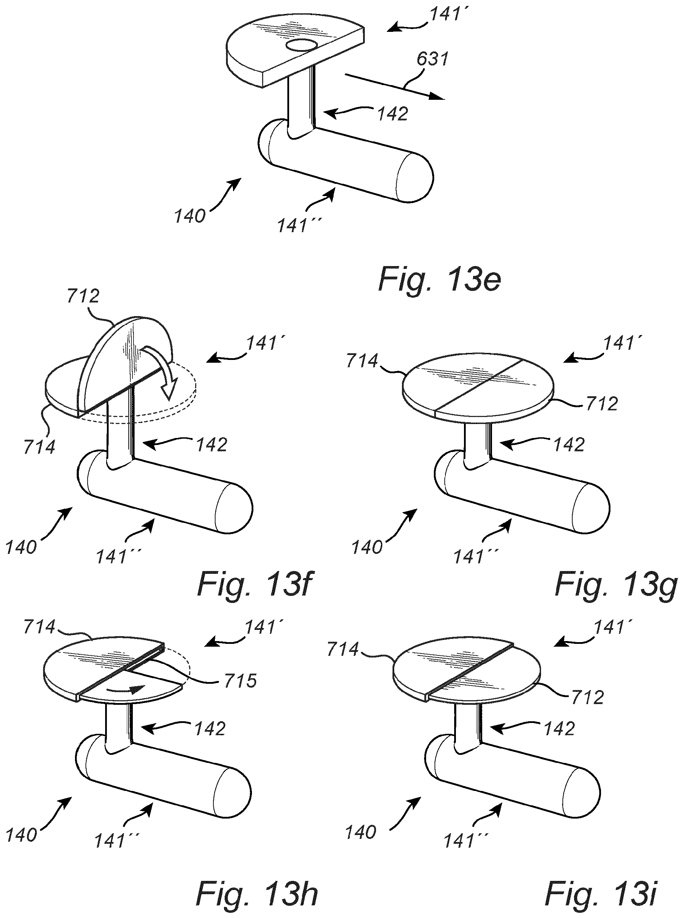

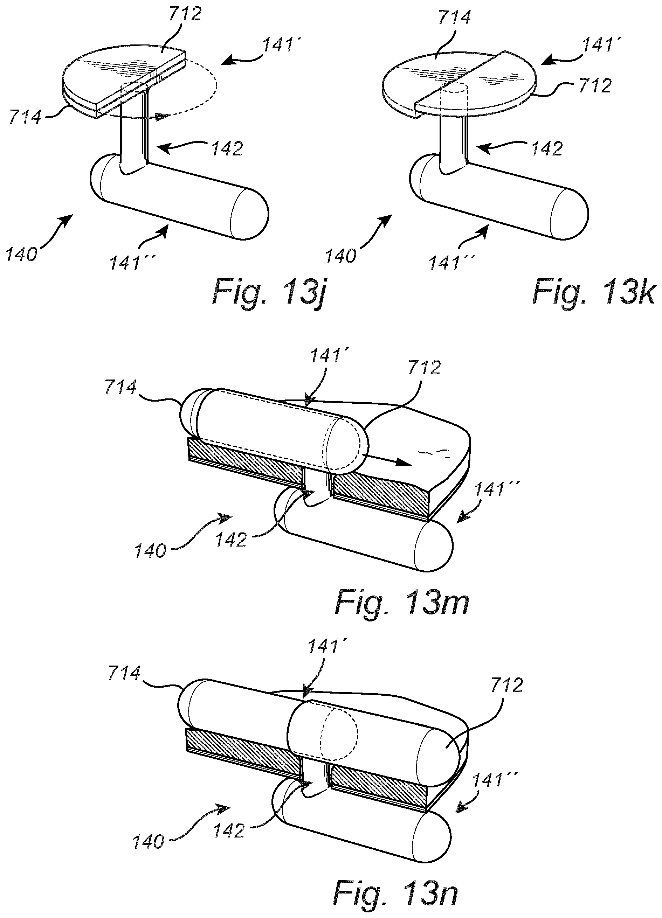

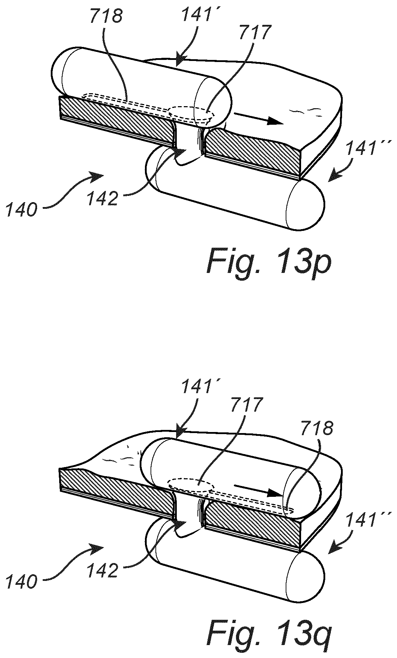

Referring now to Figs. 13e-k, 13m, 13n, 13p and 13q. The following will discuss some features of the first portion 141', and in some cases additionally or alternatively of the connecting portion 142, which enable the first portion 141' to increase its cross-sectional area in the first plane (i.e. to increase an area of the first surface configured to face the first tissue surface), and/or which enable the first portion 141' to be rotated, translated, or otherwise moved in relation to the connecting portion 142. In some embodiments, the first portion 141' will be configured to extend further away from the connecting portion 142 in or within the first plane. It is to be understood that these features can be combined with other features of the implantable energized medical device. In particular, the specific shape of the first portion, connecting portion and/or second portion in the illustrated embodiments are merely exemplary. Other shapes are possible, as discussed in the present disclosure. Accordingly, the elongated second portion 141" does not necessarily need to be elongated as shown for example in Fig. 13e, and furthermore, the first portion 141' does not necessarily need to have a semicircular shape.

With reference to Fig. 13e, an implantable energized medical device 140 is shown, wherein the first portion 141' is configured and shaped such that an edge 710 of the first portion 141' is substantially aligned with the connecting portion 142 with regard to the first direction 631. In other words, no part of the first portion 141' protrudes forward of the connecting portion 142 with regard to the first direction 631. Hereby, insertion of the implantable energized medical device 140 may be facilitated, in particular when angled downwards, since the first portion 141' will not abut the tissue until most or all of the second portion 141" has been inserted through the hole in the tissue. Although the edge 710, as well as other edges of the first portion 141', are hereby shown as having no radius, radiused edges are possible. Thus, the edge 710 may have a radius, and/or the first portion 141', and/or the second portion 141", and/or the connecting portion 142, may comprise radiused edges.

With reference to Figs. 13f and 13g, a first portion 141' is shown being configured to have its surface area increased. Here, the first cross-sectional area is increased, thereby increasing an area of the first surface configured to face (and in some embodiments also configured to contact) the first tissue surface. In the illustrated embodiment, the first portion 141' comprises a first element 712 and a second element 714 being hingedly interconnected to allow the first element 712 to assume a first state (not shown) wherein the first element 712 is arranged on top of the second element 714, and a second state wherein the first element 712 is folded to be located adjacent or next to the second element 714. A similar configuration may be achieved by other means of interconnection between the first element 712 and second element 714, i.e. the configuration is not limited to a hinge-type connection. For example, the first element 712 and second element 714 may be constructed of a single piece of material being flexible enough to be able to fold over itself to assume the first and second state respectively.

Preferably, the first and second element 712, 714 are interconnected and formed such that a transition between the first and second element 712, 714 along the first direction 631 is flush. Furthermore, while in the first state, the first portion 141' may possess the same feature as discussed in conjunction with Fig. 13e, i.e. the first portion 141' may be substantially aligned with the connecting portion 142.

With reference to Figs. 13h and 13i, a first portion 141' is shown being configured to have its surface area increased. Here, the first cross-sectional area is increased, thereby increasing an area of the first surface configured to face (and in some embodiments also configured to contact) the first tissue surface. In the illustrated embodiment, the first portion 141' comprises a first element 712 and a second element 714. The second element 714 here comprises a slot 715 configured to partially or fully house the first element 712. The first element 712 is configured to rotate about an axis to assume a first state, wherein the first element 712 is partially or completely housed in within the slot 715, and a second state wherein the first element 712 protrudes from the slot 715 to increase the first cross-sectional area. The first element 712 may be configured to rotate 180 degrees about the axis. In the illustrated example, the first and second elements 712, 714 are shaped as semi-circles and form a shape conforming to a full circle in the second state. However, it is also possible that the first element 712 only rotate about the axis up to 90 degrees, thus forming a shape conforming to three quarters of a circle in the second state. Other shapes are also possible, e.g. polygons.

With reference to Figs. 13j and 13k, a similar configuration as described with reference to Figs. 13h and 13i is shown. However, here the second element 714 does not comprise a slot, and the first element is thus not housed in a slot. Instead, the first element 712 is arranged on top of the second element 714 (similar to the embodiment of Figs. 13f and 13g). The first portion 141' is here configured to have its surface area increased, in particular the first cross-sectional area is increased, thereby increasing an area of the first surface configured to face (and in some embodiments also configured to contact) the first tissue surface. The first element 712 is configured to rotate about an axis to assume a first state, wherein the first element 712 is partially or completely arranged on top of the second element 714. Here, "completely arranged on top of" means that the first element 712 is confined within the borders of the second element 714. By rotation of the first element 712 about the axis, the first element 712 can assume a second state wherein the first element 712 protrudes over an edge or border of the second element 714 to increase the first cross-sectional area. The first element 712 may be configured to rotate 180 degrees about the axis. However, it is also possible that the first element 712 only rotate about the axis up to 90 degrees. Other shapes of the first and second element 712, 714 are also possible, e.g. polygons.

With reference to Figs. 13m and 13n, a first portion 141' is shown being configured to have its surface area increased. Here, the first cross-sectional area is increased, thereby increasing an area of the first surface configured to face (and in some embodiments also configured to contact) the first tissue surface. In the illustrated embodiment, the first portion 141' comprises a first element 712 and a second element 714. The first element 712 here comprises a slot configured to partially or completely house the second element 714. The first element 712 is configured to assume a first state, as shown in Fig. 13m, wherein the second element 714 is arranged partially or fully within the slot of the first element 712, and a second state, as shown in Fig. 13n, wherein the first element 712 has been moved in a first direction to cause the second element 714 to protrude from the slot of the first element 712, and to cause the first element 712 to extend further away from the connecting portion 142 in the first plane. As will be understood, other variations are possible, e.g. the second element 714 may comprise the slot, and the first element 712 may be partially or fully housed within such slot, and subsequently the first element 712 or the second element 714 may be moved to protrude from such slot.

With reference to Figs. 13p and 13q, a first portion 141' is shown being configured to be moved in relation to the connecting portion 142. The expression "configured to be moved" may in this context be interpreted as the first portion 141' being configured to assume at least two different positions with regard to the connecting portion 142 while still remaining in direct contact with the connecting portion. Here, the connecting portion 142 comprises a protruding element 717 and the first portion 141' comprises a slot 718, wherein the protruding element 717 is configured to slide within the slot 718 along a predetermined path, e.g. in a first direction and a direction opposite said first direction. The protruding element 717 may be configured to be interlocked within the slot 718 such that the protruding element 717 can only be removed from the slot 718 in a preconfigured position. In other embodiments, the protruding element 717 may be permanently enclosed within the slot 718. By sliding the first portion 141' in the first direction, an extension of the first portion 141' in the first plane with respect to the connecting portion 142 will be able to be adjusted. Any position between the endpoints of the slot 718 may be able to be assumed by the first portion 141'. In particular, first portion 141' and/or the connecting portion 142 may comprise a locking mechanism configured to secure a position of the first portion 141' in relation to the connecting portion 142. Such locking mechanism may rely on flexible parts being biased towards each other to maintain the first portion 141' and connecting portion 142 in a fixed position in relation to each other. Other possible locking mechanisms include the use of friction, snap-locking means, etc.

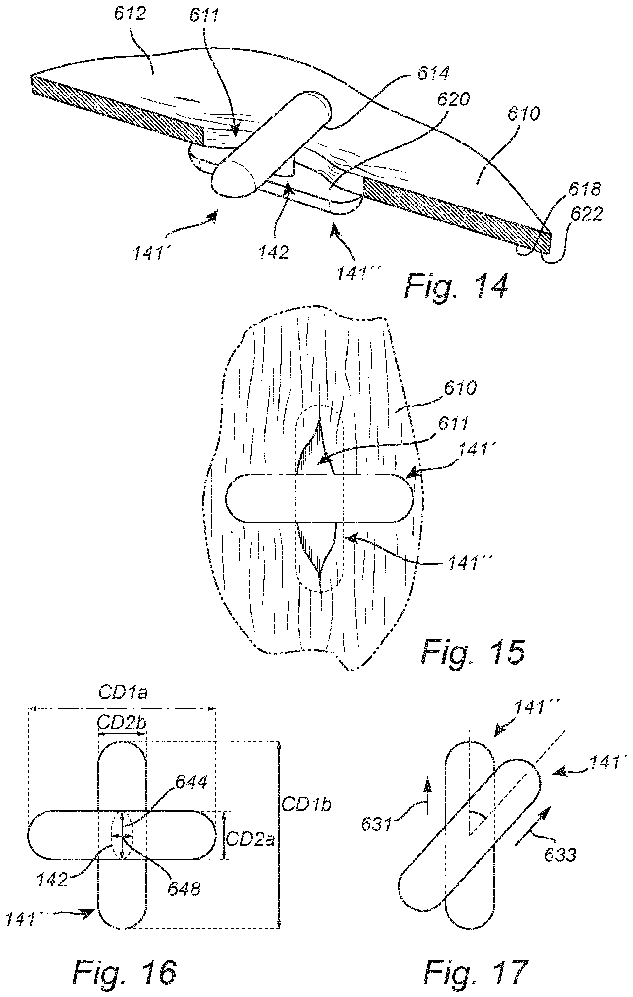

With reference to Figs. 14 and 15, an embodiment of an implantable energized medical device 140, which may be referred to as a remote unit in other parts of the present disclosure, will be described. The device 140 is configured to be held in position by a tissue portion 610 of a patient. The device 140 comprises a first portion 141' configured to be placed on a first side 612 of the tissue portion 610, the first portion 141' having a first cross-sectional area in a first plane and comprising a first surface 614 configured to face and/or engage a first tissue surface 616 of the first side 612 of the tissue portion 610. The device 140 further comprises a second portion 141" configured to be placed on a second side 618 of the tissue portion 610, the second side 618 opposing the first side 612, the second portion 141" having a second cross-sectional area in a second plane and comprising a second surface 620 configured to engage a second tissue surface 622 of the second side 618 of the tissue portion 610. The device 140 further comprises a connecting portion 142 configured to be placed through a hole in the tissue portion 610 extending between the first and second sides 612, 618 of the tissue portion 610. The connecting portion 142 here has a third cross-sectional area in a third plane. The connecting portion 142 is configured to connect the first portion 141' to the second portion 141".

With reference to Fig. 16, the first cross-sectional area has a first cross-sectional distance CD1a and a second cross-sectional distance CD2a, the first and second cross-sectional distances CD1a, CD2a being perpendicular to each other and the first cross-sectional distance CD1a being longer than the second cross-sectional distance CD2a. Furthermore, the second cross-sectional area has a first cross-sectional distance CD1b and a second cross-sectional distance CD2b, the first and second cross-sectional distances CD2a, CD2b being perpendicular to each other and the first cross-sectional distance CD1b being longer than the second cross-sectional distance CD2b. The first cross-sectional distance CD1a of the first cross-sectional area and the first cross-sectional distance CD1b of the second cross-sectional area are rotationally displaced in relation to each other with an angle exceeding 45° to facilitate insertion of the second portion 141" through the hole in the tissue portion. In the embodiment illustrated in Fig. 16, the rotational displacement is 90°.

The rotational displacement of the first portion 141' and the second portion 141" forms a cross-like structure, being particularly advantageous in that insertion through the hole in the tissue portion 610 may be facilitated, and once positioned in the hole in the tissue portion 610 a secure position may be achieved. In particular, if the device 140 is positioned such that the second portion 141" has its first cross-sectional distance CD1b extending along a length extension of the hole 611 in the tissue portion 610, insertion of the second potion 141" through the hole 611 may be facilitated. Furthermore, if the first portion 141' is then displaced in relation to the second portion 141" such that the first cross-sectional distance CD1a of the first portion 141' is displaced in relation to a length extension of the hole 611, the first portion 141' may be prevented from travelling through the hole 611 in the tissue portion. In these cases, it is particularly advantageous if the hole 611 in the tissue portion is oblong, ellipsoidal, or at least has one dimension in one direction being longer than a dimension in another direction. Such oblong holes in a tissue portion may be formed for example in tissue having a fiber direction, where the longest dimension of the hole may be aligned with the fiber direction.

In the embodiment illustrated in Fig. 14, the first surface 614 of the first portion 141' is flat, thus providing a larger contact surface to the first tissue surface 616 and consequently less pressure on the tissue portion. A more stable position may also be achieved by the flat surface. Also the second surface 620 of the second portion 141" may be flat. However, other shapes, such as those described in other parts of the present disclosure, are possible.

As shown in Fig. 16, the connecting portion 142 may have an elongated cross-section in the third plane. It may be particularly advantageous if the connecting portion 142 has a longer length 644 than width 648, said length 644 extending in the same direction as a length direction of the second portion 141", i.e. in the same direction as an elongation of the second portion 141". Hereby, the elongation of the connecting portion 142 may run in the same direction as an elongation of the hole in the tissue portion.

With reference to Fig. 17, the rotational displacement of first cross-sectional distance of the first cross-sectional area and the first cross-sectional distance of the second cross-sectional area is shown, here at an angle about 45°. Accordingly, there is a rotational displacement, in the first, second and third planes, between a length direction 633 of the first portion 141' and a length direction 631 of the second portion 141". Other angles of rotational displacement are possible, such as 60°, 75, 90°, 105°, 120°, 135°, etc.

One and the same device 140 may be capable of assuming several different arrangements with regards to rotational displacement of the first portion 141' and the second portion 141". In particular, this is possible when the first portion 141' and/or the second portion 141" is configured to detachably connect to the interconnecting portion 142. For example, a connection mechanism between the first portion 141' and the connecting portion 142, or between the second portion 141" and the connecting portion 142, may posses a rotational symmetry to allow the first portion 141' to be set in different positions in relation to the connecting portion 142 and in extension also in relation to the second portion 141". Likewise, such rotational symmetry may allow the second portion 142" to be set in different positions in relation to the connecting portion 142 and in extension also in relation to the first portion 141'.

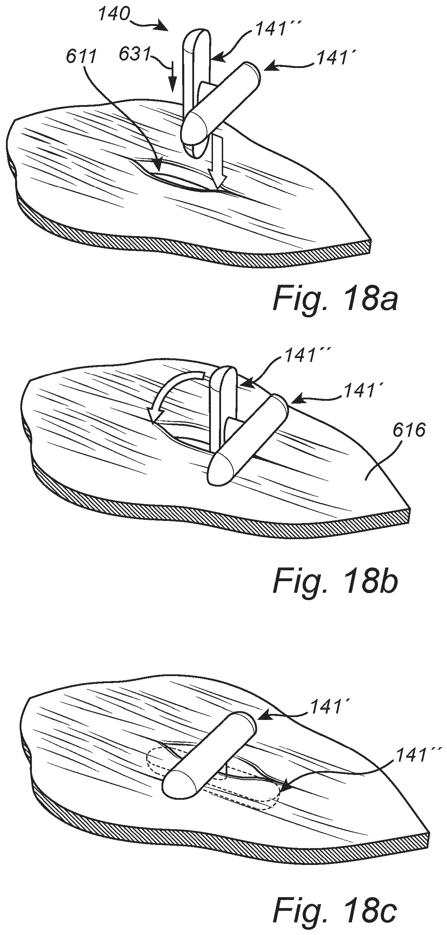

With reference to Figs. 18a-18c, a procedure of insertion of the device 140 in a tissue portion 610 will be described. The device 140 may be oriented such that a length direction 631 of the second portion 141" points downwards into the hole 611. Preferably, the second portion 141" is positioned such that it is inserted close to an edge of the hole 611. The second portion 141" may then be inserted partially through the hole 611, until the point where the first portion 141' abuts the first tissue surface 616. Here, a 90° rotational displacement between the first portion 141' and the second portion 141", as described above, will allow a relatively large portion of the second portion 141" to be inserted before the first portion 141' abuts the first tissue surface 616. Subsequently, the device 140 may be pivoted to slide or insert the remaining portion of the second portion 141" through the hole 611. While inserting the remaining portion of the second portion 141", the tissue may naturally flex and move to give way for the second portion 141". Upon having fully inserted the second portion 141" through the hole 611, such that the second portion 141" is completely located on the other side of the tissue portion 610, the tissue may naturally flex back.

With reference to Fig. 19, an embodiment of an implantable energized medical device 140, which may be referred to as a remote unit in other parts of the present disclosure, will be described. The device 140 is configured to be held in position by a tissue portion 610 of a patient. The device 140 comprises a first portion 141' configured to be placed on a first side 612 of the tissue portion 610, the first portion 141' having a first cross-sectional area in a first plane and comprising a first surface 614 configured to face and/or engage a first tissue surface of the first side 612 of the tissue portion 610. The device 140 further comprises a second portion 141" configured to be placed on a second side 618 of the tissue portion 610, the second side 618 opposing the first side 612, the second portion 141" having a second cross-sectional area in a second plane and comprising a second surface 620 configured to engage a second tissue surface of the second side 618 of the tissue portion 610. The device 140 further comprises a connecting portion 142 configured to be placed through a hole in the tissue portion 610 extending between the first and second sides 612, 618 of the tissue portion 610. The connecting portion 142 here has a third cross-sectional area in a third plane. The connecting portion 142 is configured to connect the first portion 141' to the second portion 141".

At least one of the first portion and the second portion comprises at least one coil embedded in a ceramic material, the at least one coil being configured for at least one of: receiving energy transmitted wirelessly, transmitting energy wirelessly, receiving wireless communication, and transmitting wireless communication. In the illustrated embodiment, the first portion 141' comprises a first coil 658 and a second coil 660, and the second portion 141" comprises a third coil 662. The coils are embedded in a ceramic material 664

As discussed in other part of the present disclosure, the first portion 141' may comprise a first wireless energy receiver configured to receive energy transmitted wirelessly from an external wireless energy transmitter, and further the first portion 141' may comprise a first wireless communication receiver. The first wireless energy receiver and the first wireless communication receiver may comprise the first coil. Accordingly, the first coil may be configured to receive energy wirelessly, and/or to receive communication wirelessly.

By the expression "the receiver/transmitter comprising the coil" it is to be understood that said coil may form part of the receiver/transmitter.

The first portion 141' comprises a distal end 665 and a proximal end 666, here defined with respect to the connecting portion 142. In particular, the proximal end 665 is arranged closer to the connecting portion 142 and closer to the second portion 141" when the device 140 is assembled. In the illustrated embodiment, the first coil 658 is arranged at the distal end 665.

The first portion 141' may comprise an internal wireless energy transmitter, and further a first wireless communication transmitter. In some embodiments, the internal wireless energy transmitter and/or the first wireless communication transmitter comprises the first coil 658. However, in some embodiments the internal wireless energy transmitter and/or the first wireless communication transmitter comprises the second coil 660. The second coil 660 is here arranged at the proximal end 665 of the first portion 141'. Such placement of the second coil 660 may provide for that energy and/or communication signals transmitted by the second coil 660 will not be attenuated by internal components of the first portion 141' when being transmitted to the second portion 141".

In some embodiments, the first wireless energy receiver and the internal wireless energy transmitter comprises a single coil embedded in a ceramic material. Accordingly, a single coil may be configured for receiving energy wirelessly and for transmitting energy wirelessly. Similarly, the first wireless communication receiver and the first wireless communication transmitter may comprise a single coil embedded in a ceramic material. Even further, in some embodiments a single coil may be configured for receiving and transmitting energy wirelessly, and for receiving and transmitting communication signals wirelessly.

The coils discussed herein are preferably arranged in a plane extending substantially parallel to the tissue portion 610.

When utilizing one or several coils for receiving and/or transmitting communication signals or energy, it may be preferable to design the coils such that the transmitting coil have a diameter being larger than a diameter of the receiving coil. For example, a transmitting coil in an external device may have a diameter being larger than a receiving coil in the first portion 141'. Furthermore, a transmitting coil in the first portion 141' may have a larger diameter than a receiving coil in the second portion 141". The diameter of the transmitting coil may be at least 30% larger, such as at least 50% larger, such as at least 100% larger, than the receiving coil.

The second portion 141" may comprise a second wireless energy receiver, and/or a second wireless communication receiver. In some embodiments, the third coil 662 in the second portion 141" comprises the second wireless energy receiver and/or the second wireless communication receiver.

The second portion 141" comprises a distal end 668 and a proximal end 670, here defined with respect to the connecting portion 142. In particular, the proximal end 668 is arranged closer to the connecting portion 142 and closer to the first portion 141' when the device 140 is assembled. In the illustrated embodiment, the third coil 662 is arranged at the proximal end 668 of the second portion 141". Such placement of the third coil 662 may provide for that energy and/or communication signals received by the third coil 662 will not be attenuated by internal components of the second portion 141" when being received from the first portion 141'.

The first portion 141' may comprise a first controller 300a connected to the first coil 658, second coil 660, and/or third coil 662. The second portion 141" may comprise a second controller 300b connected to the first coil, 658, second coil 660, and/or third coil 662.

In the illustrated embodiment , the first portion 141' comprises a first energy storage unit 304a connected to the first wireless energy receiver 308a, i.e. the first coil 658. The second portion comprises a second energy storage unit 304b connected to the second wireless energy receiver 308b, i.e. the third coil 662. Such an energy storage unit may be a solid-state battery, such as a thionyl-chloride battery.

In some embodiments, the first coil 658 is configured to receive energy transmitted wirelessly by the external wireless energy transmitter and store the received energy in the first energy storage unit 304a. Furthermore, the first coil 658 and/or the second coil 660 may be configured to wirelessly transmit energy stored in the first energy storage unit 304a to the third coil 662, and the third coil 662 may be configured to receive energy transmitted wirelessly by the first coil 658 and/or the second coil 660 and store the received energy in the second energy storage unit 305b.

The first energy storage unit 304a may be configured to store less energy than the second energy storage unit 304b, and/or configured to be charged faster than the second energy storage unit 304b. Hereby, charging of the first energy storage unit 304a may be relatively quick, whereas transfer of energy from the first energy storage unit 304a to the second energy storage unit 304b may be relatively slow. Thus, a user can quickly charge the first energy storage unit 304a, and will not during such charging be restricted for a long period of time by being connected to an external wireless energy transmitter, e.g. at a particular location. After having charged the first energy storage unit 304a, the user may move freely while energy slowly transfers from the first energy storage unit 304a to the second energy storage unit 304b, via the first and/or second coil and the third coil.

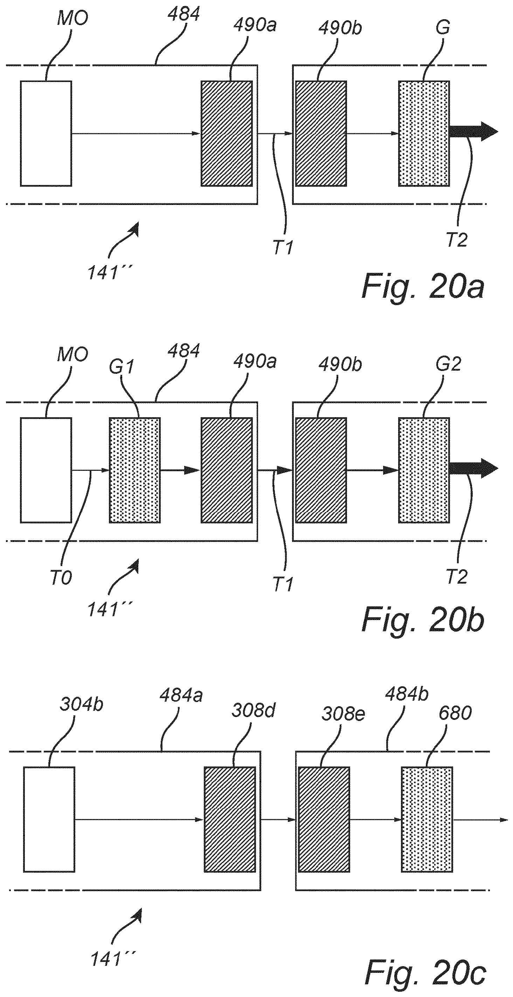

Figs. 20a and 20b illustrate a gear arrangement and magnetic coupling for coupling the implantable energized medical device to an implant (or element) exerting force on a body part, and in particular a gear arrangement for transferring mechanical movement through an outer housing of the device or an outer housing of the second portion 141".

The housing 484 of the device or second portion 141" may be present in some embodiments of the device. In such embodiments, the housing 484 is configured to enclose, at least, the controller (not shown), motor M, any receivers and transmitters if present (not shown), and any gear arrangements G, G1, G2 if present. Hereby, such features are protected from bodily fluids. The housing 484 may be an enclosure made from one of or a combination of: a carbon based material (such as graphite, silicon carbide, or a carbon fiber material), a boron material, a polymer material (such as silicone, Peek^{®}, polyurethane, UHWPE or PTFE,), a metallic material (such as titanium, stainless steel, tantalum, platinum, niobium or aluminum), a ceramic material (such as zirconium dioxide, aluminum oxide or tungsten carbide) or glass. In any instance the enclosure should be made from a material with low permeability, such that migration of fluid through the walls of the enclosure is prevented.

The implantable energized medical device may comprise at least part of a magnetic coupling, such as a magnetic coupling part 490a. A complementary part of the magnetic coupling, such as magnetic coupling part 490b, may be arranged adjacent to the device 140, so as to magnetically couple to the magnetic coupling part 490a and form the magnetic coupling. The magnetic coupling part 490b may form part of an entity not forming part of the device 140. However, in some embodiments the second portion 141" comprises several chambers being hermetically sealed from each other. Such chambers may be coupled via a magnetic coupling as discussed herein. The magnetic coupling 490a, 490b provide for that mechanical work output by the device 140 via e.g. an electric motor can be transferred from the device to e.g. an implant (or element) configured to exert force on a body part of a patient. In other words, the magnetic coupling 490a, 490b provides for that mechanical force can be transferred through the housing 484.

The coupling between components, such as between a motor and gear arrangement, or between a gear arrangement and a magnetic coupling, may be achieved by e.g. a shaft or the like.

In some embodiments, for example as illustrated in Fig. 20a, a force output of a motor MO in the second portion 141" is connected to the magnetic coupling part 490a. The magnetic coupling part 490a transfers the force output from the motor MO to the magnetic coupling part 490b, i.e. via the magnetic coupling 490a, 490b. The force output transferred via the magnetic coupling 490a, 490b here has a torque T1, which is substantially the same torque as delivered by the motor MO. The magnetic coupling part 490b is connected to a gear arrangement G, located external to the device, for example in a medical implant configured to exert force on a body part, or intermediate to a medical implant configured to exert force on a body part. The gear arrangement G is configured to increase the torque of the force delivered via the magnetic coupling 490a, 490b to deliver a force with torque T2 being higher than torque T1 to a medical implant. Consequently, low torque may be provided by the motor MO, i.e. a relatively small force with high angular velocity, which is transferred via the magnetic coupling 490a, 490b before the torque is increased via gear arrangement G to achieve a relatively large force with low angular velocity. Hereby, the magnetic coupling 490a, 490b may utilize relatively weak magnetic forces to transfer the mechanical work through the housing 484 of the device without the risk of slipping between the magnetic coupling parts 490a, 490b.

In some embodiments, for example as illustrated in Fig. 20b, a force output of a motor MO in the second portion 141" is connected to a first gear arrangement G1, which in turn is coupled to the magnetic coupling part 490a. The motor MO here provides a mechanical force with torque T0. The magnetic coupling part 490a transfers the force output from the motor MO to the first gear arrangement G1. The first gear arrangement G1 is configured to increase the torque of the force delivered from the motor MO to deliver a force with a higher torque T1 to the magnetic coupling 490a, 490b. The magnetic coupling part 490a transfers the force with torque T1 to the magnetic coupling part 490b. The magnetic coupling part 490b is connected to a second gear arrangement G2, located external to the device, for example in a medical implant configured to exert force on a body part, or intermediate to a medical implant configured to exert force on a body part. The second gear arrangement G2 is configured to increase the torque of the force delivered via the magnetic coupling 490a, 490b to deliver a force with torque T2 being higher than torque T1, and thus higher than torque T0, to a medical implant. Consequently, low torque may be provided by the motor MO, i.e. a relatively small force with high angular velocity. The torque of the force provided by the motor MO is then increased by the first gear arrangement G1, before the force is transferred via the magnetic coupling 490a, 490b. The torque of the force transferred via the magnetic coupling 490a, 490b is then yet again increased via the second gear arrangement G2 to achieve a relatively large force with low angular velocity. Hereby, the magnetic coupling 490a, 490b may utilize relatively weak magnetic forces to transfer the mechanical work through the housing 484 of the device without the risk of slipping between the magnetic coupling parts 490a, 490b. Furthermore, since some of the torque increase is made within the second portion 141", and a remaining portion of the torque increase is made external to the device and the second portion 141", the gear arrangements G1, G2 may be sized and configured appropriately to share the work of increasing the torque.

Fig. 20c schematically illustrates an energy storage 304b connected to a wireless energy transmitter 308. The energy storage 304b and the wireless energy transmitter 308 are arranged in one portion or chamber of the second portion 141". Furthermore, a wireless energy receiver 308e is arranged in another portion or chamber of the second portion 141". The portions or chambers may be separated or defined by respective housings, external walls and/or internal walls 484a, 484b. The wireless energy transmitter 308d is configured to wirelessly transmit energy to the wireless energy receiver 308e. Hereby, an internal energy transfer is achieved within the second portion 141". The wireless energy transmitter 308d and wireless energy receiver 308e may comprise one or more coils, respectively. The wireless energy receiver 308e may be connected to a further energy storage 680 arranged within the second portion 141". Such energy storage 680 may be connected to a medical implant, such that the energy storage 680 can deliver energy to the medical implant. In some embodiments however, the wireless energy receiver 308e is directly connected to a medical implant to deliver energy directly to the medical implant, thus omitting the energy storage 680.

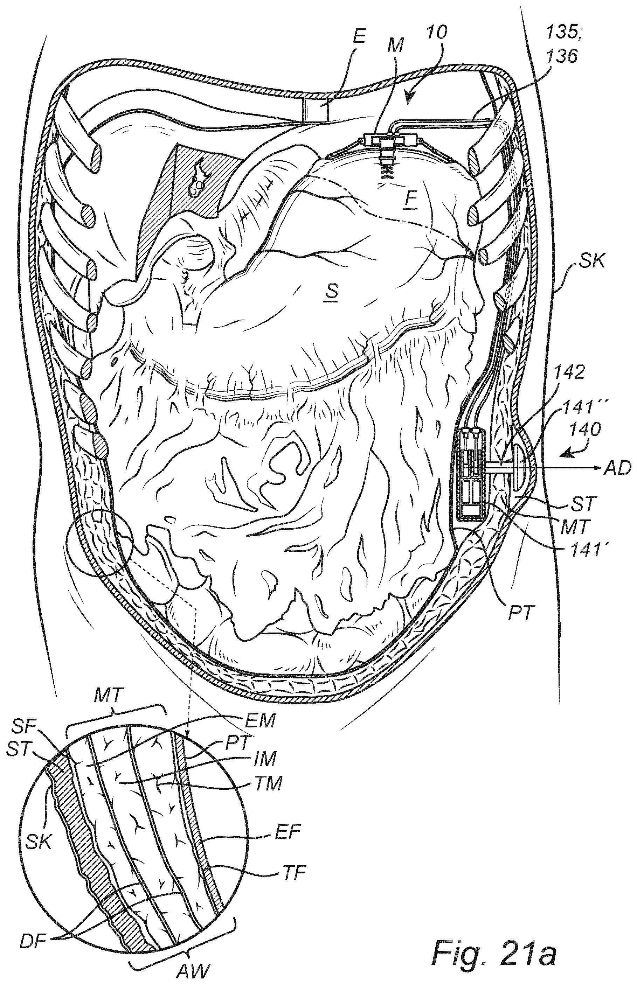

Fig. 21a shows a frontal view of the abdomen of a patient when a medical device 10 configured to exert force on a body portion of the patient has been implanted. Here, the medical device 10 is configured to exert a force on the stomach of the patient. The medical device 10 is in the embodiment shown in fig. 21a operated by a remote unit 140. This is however only an example of a remote unit for operation of the medical device 10 and it is clear that any of the embodiments of remote units disclosed herein can be implanted and connected in the manner described with reference to fig. 21a. The remote unit 140 comprises a first portion 141', a second portion 141", and a connecting portion 142, mechanically connecting the first and second portions 141',141". The first and second portions will hereinafter be interchangeably used with the terms "first unit" and "second unit" respectively. The second unit 141" is in the embodiment shown in fig. 21a placed on the inside of muscular tissue MT of the abdominal wall AW of the patient, whereas the first unit 141' is placed on the outside of the muscular tissue MT of the abdominal wall AW, in the subcutaneous tissue ST. As such, the connecting portion 142 travels through a created hole in, or natural orifice between, the muscles of the muscular tissue MT. A cross-sectional area of the connecting portion 142, in a plane in the extension of the muscular tissue MT is smaller than a cross-sectional area of the first and second units 141',141", parallel to the cross-sectional area of the connecting portion 142. The cross-sectional areas of the first and second units 141',141" are also larger than the created hole or natural orifice though which the connecting portion 142 is placed. As such, the first and second units 141',141" are unable to pass through the created hole or natural orifice and is as such fixated to the muscular tissue MT of the abdominal wall. This enables the remote unit 140 to be suspended and fixated to the muscle tissue MT of the abdominal wall AW.

In the embodiment shown in fig. 21a, the connecting portion 142, is a connecting portion 142 having a circular cross-section and an axial direction AD extending from the first unit 141' to the second unit 141". The plane in the extension of the muscular tissue MT, is in the embodiment of fig. 21a perpendicular to the axial direction AD of the connecting portion 142 extending from the first unit 141' to the second unit 141".

In the embodiment of fig. 21a, a controller is placed in the second unit 141", and an implantable energy storage unit is placed in the second unit 141". The controller and the implantable energy storage unit are electrically connected to each other by means of a lead running in the connecting portion 142, such that electrical energy and communication can be transferred from the first portion 141' to the second portion 141", and vice versa. In the embodiment of fig. 21a, the first portion 141' further comprises a wireless energy receiver for receiving wireless energy for charging the implantable energy storage unit and/or for powering the medical device 10, and a transceiver for receiving and/or transmitting wireless signals to/from the outside the body. Further features and functions of the controller and the implantable energy storage unit are further described with reference to figs. 22a-22f.

The abdominal wall AW is most locations generally formed by a set of layers of skin, fat/fascia, muscles and the peritoneum. The deepest layer in the abdominal wall AW is the peritoneum PT, which covers many of the abdominal organs, for example the large and small intestines. The peritoneum PT is a serous membrane composed of a layer of mesothelium supported by a thin layer of connective tissue and serves as a conduit for abdominal organ's blood vessels, lymphatic vessels, and nerves. The area of the abdomen enclosed by the peritoneum PT is called the intraperitoneal space. The tissue and organs within the intraperitoneal space are called "intraperitoneal" (e.g., the stomach and intestines). The tissue and organs in the abdominal cavity that are located behind the intraperitoneal space are called "retroperitoneal" (e.g., the kidneys), and tissue and organs located below the intraperitoneal space are called "subperitoneal" or "infraperitoneal" (e.g., the bladder).

The peritoneum PT is connected to a layer of extraperitoneal fat EF which is connected to a layer or transversalis fascia TF. Connected to the transversalis fascia TF, at the area of the abdominal wall AW at which the section is extracted, is muscle tissue MT separated by layers of deep fascia DF. The deep fascia DF between the layers of muscle is thinner than the transversalis fascia TF and the Scarpa's fascia SF placed on the outside of the muscle tissue MT. Both the transversalis fascia TF and the Scarpa's fascia SF are relatively firm membranous sheets. At the area of the abdominal wall AW at which the section is extracted, the muscle tissue MT is composed of the transverse abdominal muscle TM (transversus abdominis), the internal oblique muscle IM (obliquus internus) and the external oblique muscle EM (obliquus externus). In other areas of the abdominal wall AW, the muscle tissue could also be composed of the rectus abdominis and the pyramidalis muscle.

The layer outside of the muscle tissue MT, beneath the skin SK of the patient is called subcutaneous tissue ST, also called the hypodermis, hypoderm, subcutis or superficial fascia. The main portion of the subcutaneous tissue ST is made up of Camper's fascia which consists primarily of loose connective tissue and fat. Generally, the subcutaneous tissue ST contains larger blood vessels and nerves than those found in the skin.

Placing the remote unit 140 at an area of the abdomen is advantageous as the intestines are easily displaced for making sufficient room for the remote unit 140, without the remote unit 140 affecting the patient too much in a sensational or visual way. Also, the placement of the remote unit 140 in the area of the abdomen makes it possible to fixate the remote unit 140 to the muscle tissue MT of the abdomen for creating an attachment keeping the remote unit 140 firmly in place. In the embodiment shown in fig. 21a, the second portion 141" of the remote unit 140 is placed on the left side of the patient in between the peritoneum PT and the muscle tissue MT. The first portion 141' is placed in the subcutaneous tissue ST between the muscle tissue MT and the skin SK of the patient. Placing the first portion 141' subcutaneously enables easy access to the first portion 141' for e.g. wireless communication using a wireless transceiver placed in the first portion 141', wireless charging of an implantable storage unit using a wireless energy receiver placed in the first portion 141', injection of a hydraulic fluid (relevant when the operation device is a hydraulic operation device), into an injection port placed in the first portion 141', manual manipulation of for example a push button placed in the first portion 141', or maintenance or replacement of the first portion 141' via a small incision in the skin SK at the first portion 141'.

In the embodiment shown in fig. 21a, the flexible wires 135 running inside of protective a cover 136 transports linear mechanical force from the remote unit 140 to the main portion M of the medical device 10. The flexible wires 135 run between the peritoneum PT and the muscle tissue MT vertically until the flexible wires 135 reaches the height of the main portion M of the medical device 10. At this height, the wires 135 enters the peritoneum PT and travels substantially horizontally to the main portion M of the medical device 10. As such, the flexible wire 135 is placed inside of the intraperitoneal space for as short distance as possible which reduces the risk that implanted, foreign body, elements disturbs the intraperitoneal organs, reducing the risk of damage to organs, and reducing the risk that foreign body elements cause ileus.

In the embodiment shown in fig. 21a, the connecting portion 142 connects the first and second portions 141', 141" through three layers of muscle tissue MT, namely tissue of the transverse abdominal muscle TM, the internal oblique muscle IM and the external oblique muscle EM. In alternative embodiments, it is however conceivable that the second portion 141" is placed in between layers of muscle, such as between tissue of the transverse abdominal muscle TM, the internal oblique muscle IM, or between the internal oblique muscle IM and the external oblique muscle EM. As such, it is conceivable that in alternative embodiments, the connecting portion 142 connects the first and second portions 141', 141" through two layers of muscle tissue MT, or through one layer of muscle tissue MT.

In alternative embodiments, it is furthermore conceivable that the first portion 141' is placed in between layers of muscle, such as between tissue of external oblique muscle EM and the internal oblique muscle IM, or between the internal oblique muscle IM and the transverse abdominal muscle TM.

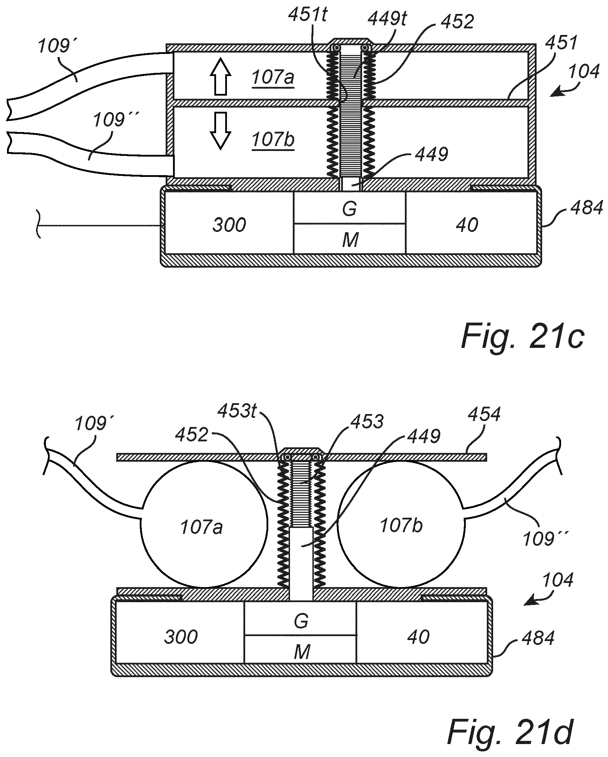

In embodiments in which the medical device exerting a force on a body part is hydraulically remotely operable (such as via a remote unit comprising a pump as further described with reference to fig. 21c), the flexible wires 135 running inside of protective a cover 136 for transporting linear mechanical force from the remote unit 140 to the main portion M shown in fig. 21a is replaced by conduits (109 in fig. 22c) for conducting hydraulic fluid for transferring force from a portion of the hydraulic operation device placed in the remote unit 140 to a portion of the operation device placed in the main portion M of the medical device 10 hydraulically.

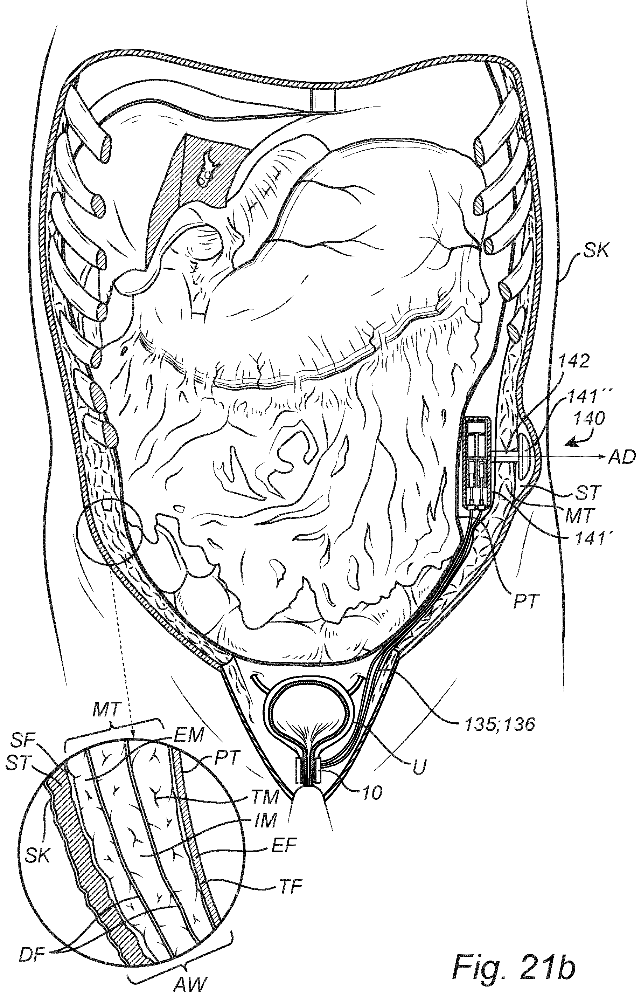

Fig. 21b shows a frontal view of the abdomen of the patient when a medical device 10 for exerting a force on a body part has been implanted. Here, the medical device 10 is configured to affect the flow of urine of the patient. The medical device 10 is in the embodiment shown in fig. 21b operated by a remote unit 140 and it is clear that any of the embodiments of remote units disclosed herein can be implanted and connected in the manner described with reference to fig. 21b. The remote unit 140 comprises a first portion 141', a second portion 141", and a connecting portion 142, mechanically connecting the first and second portion 141',141". The second portion 141" is in the embodiment shown in fig. 21b placed on the inside of muscular tissue MT of the abdominal wall AW of the patient, whereas the first portion 141' is placed on the outside of the muscular tissue MT of the abdominal wall AW, in the subcutaneous tissue ST. As such, the connecting portion 142 travels through a created hole in, or natural orifice between, the muscles of the muscular tissue MT. A cross-sectional area of the connecting portion 142, in a plane in the extension of the muscular tissue MT is smaller than a cross-sectional area of the first and second portions 141',141", parallel to the cross-sectional area of the connecting portion 142. The cross-sectional areas of the first and second portions 141',141" are also larger than the created hole or natural orifice though which the connecting portion 142 is placed. As such, the first and second portions 141',141" are unable to pass through the created hole or natural orifice and is as such fixated to the muscular tissue MT of the abdominal wall. This enables the remote unit 140 to be suspended and fixated to the muscle tissue MT of the abdominal wall AW.

In the embodiment shown in fig. 21b, the second portion 141" is configured to connect to the medical implant 10 in a cadial direction, i.e. a distal end of the second portion 141" comprises a connecting interface for delivering mechanical force, fluid, energy and/or for transmitting or receiving communication signals, to and from the medical implant 10.

In the embodiment shown in fig. 21b, the flexible wires 135 running inside of protective a cover 136 transports linear mechanical force from the remote unit 140 to the medical device 10. The flexible wires 135 run between the peritoneum PT and the muscle tissue MT vertically until the flexible wires 135 reaches the area of the urinary bladder U in the subperitoneal space below the intraperitoneal space. As such, the flexible wire 135 never needs to enter the intraperitoneal space which reduces the risk that implanted, foreign body, elements disturbs the intraperitoneal organs, reducing the risk of damage to organs, and reducing the risk that foreign body elements cause ileus.

In the embodiment shown in fig. 21b, the connecting portion 142 connects the first and second portions 141',141" though three layers of muscle tissue MT, namely tissue of the transverse abdominal muscle TM, the internal oblique muscle IM and the external oblique muscle EM. In alternative embodiments, it is however conceivable that the second portion 141" is placed in between layers of muscle, such as between tissue of the transverse abdominal muscle TM, the internal oblique muscle IM, or between the internal oblique muscle IM and the external oblique muscle EM. As such, it is conceivable that in alternative embodiments, the connecting portion 142 connects the first and second portions 141',141" through two layers of muscle tissue MT, or through one layer of muscle tissue MT.

In alternative embodiments, it is furthermore conceivable that the first portion 141' is placed in between layers of muscle, such as between tissue of external oblique muscle EM and the internal oblique muscle IM, or between the internal oblique muscle IM and the transverse abdominal muscle TM.

With reference to Figs. 21c-21n, hydraulic pumps will be described. Such hydraulic pumps may be placed in a second portion of an implantable energized medical device as described herein. However, it is also possible to place an electrical motor in the second portion of such a device for providing mechanical work to an external implant, without the use of hydraulic pumps or any hydraulic means.

Fig. 21c shows a cross-sectional view of an electrical motor **M** in combination with a gear system **G** for propulsion of a hydraulic pump 104. The hydraulic pump 104 may be placed in a remote unit, such as an implantable energized medical device, and in particular in a second portion of such a device. The electrical motor **M** is connected to the controller 300 which in turn is connected to an energy storage unit 40. The energy storage unit 40 may be a battery, a chargeable battery or a capacitor by means of which energy can be stored in the body of the patient. The energy storage unit 40 may comprise an energy storage unit and wireless charging components, such as wireless receiver and transmitters for receiving and transmitting energy wirelessly.

The controller 300, the energy storage unit 40 and the motor **M** and gear system **G** may be enclosed by a housing 484 such that the controller 300 is protected from bodily fluids. The housing 484 may be an enclosure made from one of or a combination of: a carbon based material (such as graphite, silicon carbide, or a carbon fiber material), a boron material, a polymer material (such as silicone, Peek^{®}, polyurethane, UHWPE or PTFE,), a metallic material (such as titanium, stainless steel, tantalum, platinum, niobium or aluminum), a ceramic material (such as zirconium dioxide, aluminum oxide or tungsten carbide) or glass. In any instance the enclosure should be made from a material with low permeability, such that migration of fluid through the walls of the enclosure is prevented.

Turning now to the hydraulic pump 104 shown in fig. 21c. In the embodiment shown in fig. 21c, the force output 449 of the gear system G is threaded 449t and engages a correspondingly threaded portion 451t of the movable wall 451 such that the rotating force created by the motor **M** and gear system **G** is transferred to a linear force moving the movable wall 451. The threaded force output 449 is enclosed by pleated bellows portions 452 both above and below the movable wall 451 such that the threaded force output 449 is protected from the fluid in the lumens of the reservoirs 107a, 107b. The reservoirs 107a, 107b has a common moveable wall 451 for changing the volume of the implantable fluid reservoirs 107a, 107b and thereby increasing fluid in the first fluid reservoir 107a simultaneously with decreasing fluid in the second fluid reservoir 107b and vice versa. The peristaltic pump is a sealed pump which means that fluid will not leak through the pump even at standstill. As the peristaltic pump is a sealed pump no additional valve is needed to keep the fluid through the fluid conduits 109',109" closed. The movable wall pump 104 of fig. 21c is a sealed pump which means that fluid will not leak through the pump even at standstill. As the movable wall pump 104 is a sealed pump, no additional valve is needed to keep the fluid through the fluid conduits 109',109" closed.

Fig. 21d shows a cross-sectional view of a hydraulic pump comprising two expandible reservoirs 107a,107b. The hydraulic pump 104 may be placed in a remote unit. The hydraulic pump 104 of fig. 21d comprises an encapsulated motor **M,** gear system **G,** controller 300 and energy storage unit 40 being identical to that described with reference to fig. 21c. Turning to the hydraulic pump 104, the force output 449 is, in the embodiment described in fig. 21d a hollow shaft equipped with inner threads (not shown) adapted to engage outer threads 453t of a threaded member 453, such that the interaction between the hollow shaft 449 and the threaded member 453 transforms the radially rotating force generated by the motor **M** and the gear system **G,** to a linear force. The threaded member 453 is connected to a radially extending engaging member 454 adapted to engage the first and second reservoirs 107a,107b containing a hydraulic fluid. The reservoirs 107a, 107b may be fixated to the radially extending engaging members 454, for example by means of an adhesive, such that the reservoirs 107a,107b are forced to expand when the radially extending engaging member 454 is moved upwards in the expanding direction of the reservoirs 107a, 107b. The first reservoir 107a is connected to a first fluid conduit and the second reservoir 107b is connected to a second fluid conduit 109". The embodiment shown in fig. 21d further comprises a pleated bellows portions 452 for encapsulating and protecting the force output 449 and the threaded member 453 from bodily fluids. The reservoirs 107a, 107b are preferably made from medical grade implantable silicone or Parylene^{®} coated medical grade implantable silicone, but may in alternative embodiments be made from another resilient material such as NBR, Hypalon, Viton, PVC, EPDM, Polyurethane or Natural Rubber. When the reservoirs 107a, 107b are compressed and expanded they function as hydraulic pumps for moving hydraulic fluid any of the hydraulic embodiments herein.

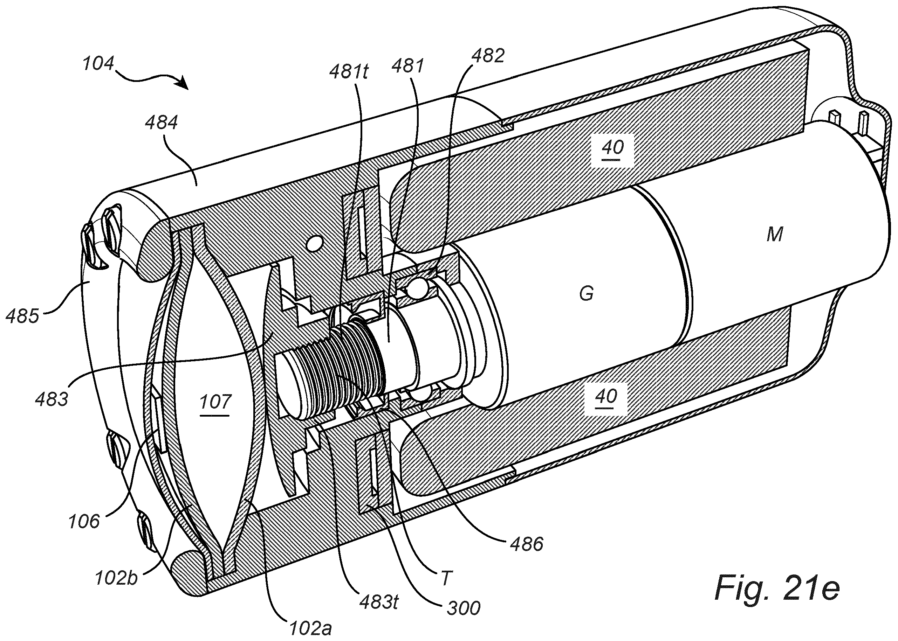

Fig. 21e shows a cross-sectional view of a hydraulic pump 104 similar to the hydraulic pump or the embodiment of fig. 21d. In the embodiment of fig. 21e, the hydraulic pump 104 comprises one expandible reservoir 107. The hydraulic pump 104 comprises an encapsulated motor **M,** gear system **G,** controller 300 and energy storage unit 40. The motor **M** is configured to generate force in a radial direction by rotation of the force output in the form of a shaft 481. The shaft 481 is equipped with outer threads 481t adapted to engage inner threads 483t of a compression member 483, such that the interaction between the threaded shaft 481, 481t and the threaded portion 483t of the compression member 483 transforms the radially rotating force generated by the motor **M** and the gear system **G,** to a linear force acting in the axial direction of the shaft 481, and thus makes up a transmission **T.** The axial force acts on the compression member 483 which engages a first resilient wall 102a of the compressible reservoir 107 for compressing the compressible reservoir 107 and thus increasing the pressure on a hydraulic fluid in the compressible reservoir 107. The compression member 483 may be fixated to the first resilient wall portion 102a by means of an adhesive, such that the reservoir 107 is forced to expand when the compression member 483 moves in the expanding direction of the reservoir 107. The reservoir 107 is connected to a fluid conduit (not shown) for conducting hydraulic fluid from the compressible reservoir to the and from the reservoir 107. The reservoir 107 is preferably made from medical grade implantable silicone or Parylene^{®} coated medical grade implantable silicone, but may in alternative embodiments be made from another resilient material such as NBR, Hypalon, Viton, PVC, EPDM, Polyurethane or Natural Rubber. When the reservoir 107 is compressed and expanded it functions as hydraulic pump for moving hydraulic fluid to and from a medical implant configured to exert a force on a body part.

The hydraulic pump 104 further comprises at least one bearing 482 for the shaft 481 placed between the gear system **G** and the compressible reservoir 107. The bearing 482 is configured to withhold at least half of the force in the axial direction, for reducing the axial load on the motor **M** and the gear system **G** which is caused by the compression of the reservoir 107. In the embodiment shown in fig. 21e, the bearing 482 is a ball bearing, but in other embodiments the bearing may comprise a roller bearing or a plain bearing preferably including a self-lubricating material such as PTFE or HDPE.

The gear system **G** is connected to the motor **M,** and placed between the motor **M** and transmission **T** and adapted to receive mechanical work via the shaft 481 having a force and a velocity, and output mechanical work having a stronger force and a lower velocity. The compressible reservoir 107 comprises a first resilient wall portion 102a and a second resilient wall portion 102b, wherein the first resilient wall portion 102a is more resilient than the second resilient wall portion 102b.

In alternative embodiments, the compression member 483 may be directly connected to the first resilient wall portion 102a, and in such embodiments, the threaded portion 483t may be integrated in the first resilient wall portion 102a.

In the embodiment shown in fig. 21e, the hydraulic pump 104 further comprises a pressure sensor 106 connected to the compressible reservoir 107 and configured to sense the pressure in the compressible reservoir 107. The pressure sensor 106 is integrated in, and placed on the outside of, the second resilient wall portion 102b of the compressible reservoir 107. It may be important to measure strain or pressure in or exerted by the medical device, as too high strain or pressure risks hampering the blood flow to the tissue of a stomach wall, which in the long term could lead to damage of the tissue and in the worst-case lead to necrosis.

The compressible reservoir 107 in the embodiment shown in fig. 21e comprises a first and second resilient wall portion 102a, 102b in the form of a first and second circular diaphragm 102a, 102b. The first resilient wall portion 102a has a convex shape facing the compression member 483, and the second resilient wall portion 102b has a convex shape facing away from the compression member 483 and a lumen is formed between the two diaphragms 102a, 102b, and being enclosed by the concave surfaces of the diaphragms 102a, 102b. The first resilient wall portion 102a is configured to be compressed and thus inverted, such that the part of the first resilient wall portion 102a facing the compression member 483 assumes a concave shape facing the compression member 483, and as such, a convex shape is formed towards the lumen of the compressible reservoir 107. The inverted, convex, portion of the first resilient wall portion 102a thus enters the concave shape of the second resilient wall portion 102b. The portion of the compression member 483 configured to engage the first resilient wall portion 102a comprises a convex portion for facilitating the inversion of the convex portion of the first resilient wall portion 102a. In the embodiment shown in fig. 21e, the first resilient wall portion 102a is more resilient than the second resilient wall portion 102b such that the compressible reservoir 107 can create a suction when the compression member 483 moves in the direction away from the compressible reservoir 107 thus enabling the compressible reservoir 107 to expand. In the embodiment shown in fig. 21e, a major portion of the first resilient wall portion is made from a material having a modulus of elasticity (E) which is less than 70% or the modulus of elasticity (E) of the material of a major portion of the second resilient wall portion 102b. In alternative embodiments, it is conceivable that the first and second resilient wall portions 102a, 102b are made from the same material, but with the second resilient wall portion 102b being more than 1,5 times as thick as the first resilient wall portion 102a. In the embodiment shown in fig. 21e, the two diaphragms 102a, 102b are pressed against each other, for creating the sealed lumen between the first and second diaphragm, by means of a fixation ring 485, which is screwed into the housing 484.

In the embodiment shown in fig. 21e, the hydraulic pump further comprises a shaft sealing 486, which is a sealing engaging the shaft and thus creating a seal between the portion of the pump housing 484 comprising the motor **M,** gear system **G,** energy storage unit 40 and controller 300, and the portion of the pump housing 484 comprising the compressible reservoir 107. The seal reduces the risk that hydraulic fluid that may leak from the compressible reservoir 107 will come in contact with any of the motor **M,** gear system **G,** energy storage unit 40 and/or controller 300. In the embodiment shown in fig. 21e, the shaft sealing comprises a spring-loaded PTFE sealing 486. A spring engages the housing 484 of the hydraulic pump 104 and the PTFE sealing for creating a constant elastic pressure between the sealing and the shaft 481 which ensures a self-lubricating tight seal. In alternative embodiments, the spring may be replaced by a different type of elastic element, such as an elastic element made from an elastomer. In alternative embodiment, the shaft sealing 486 could be a shaft sealing made from another self-lubricating material such as HDPE.

The hydraulic pump 104 of fig. 21e is enclosed by a pump housing 484, which in the embodiment shown in fig. 21e is a titanium housing 484. In alternative embodiments, the housing could be made from a another medical grade metal alloy, such as medical grade stainless steel or could comprise a ceramic material such as zirconium carbide, or a stiff medical grade polymer material such as Ultra-high-molecular-weight polyethylene (UHMWPE) or Polytetrafluoroethylene (PTFE) or a thermoplastic polyester such as polylactide (PLA). The housing could also comprise at least one composite material, such as any combination of metallic/ceramic and polymer materials or a polymer material reinforced with organic or inorganic fibers, such as carbon or mineral fibers.

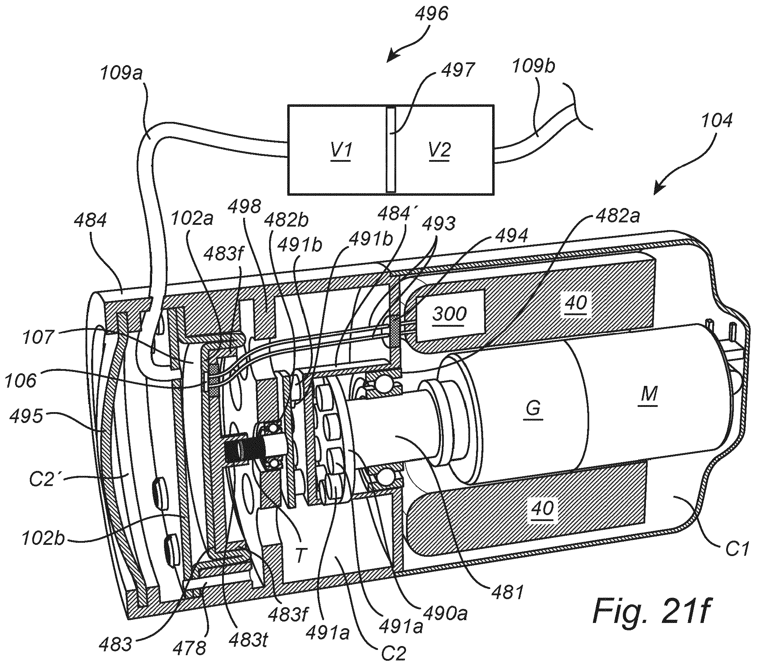

Fig. 21f shows a cross-sectional view of a hydraulic pump 104 similar to the hydraulic pump of the embodiment of fig. 21e. In the embodiment of fig. 21f, the hydraulic pump comprises one expandible reservoir 107. The hydraulic pump 104 comprises a housing 484 comprising a first and a second chamber C1, C2 separated from each other by a barrier 484'. Just as in the embodiment of fig. 21e, the first chamber C1 comprises the motor **M** configured for transforming electrical energy to mechanical work and the gear system gear system **G** adapted to receive mechanical work having a first force and first velocity, and output mechanical work having a different second force and a different second velocity, such that the high velocity movement supplied by the electrical motor **M** is transformed to low velocity movement with increased force. The output mechanical work having the different second force and different second velocity acts on a shaft 481 which transfers the force to a magnetic coupling 490a, 490b for transferring mechanical work from the motor **M** to an actuator in the form of a compression member 483 for compressing the expandible reservoir 107 for pressing a hydraulic fluid through the conduit 109a. The magnetic coupling 490a, 490b comprises a first disc shaped member 490a mounted to the shaft 481 such that the first disc shaped member 490a rotates along with the shaft 481. The shaft 481 is supported by ball bearings 482 assisting in the centering of the shaft 481.

The first disc shaped member 490a comprises magnets (or a material susceptible to magnetic fields) 491 evenly distributed axially in a circular formation on the distal surface of the first disc shaped member 490a.

The barrier 484' separates the first chamber C1 of the housing 484 from the second chamber C2 of the housing. In the embodiment shown in fig. 21f, the barrier 484' is made from the same material as the outer wall of the housing 484, i.e. medical grade titanium. In the embodiment shown in fig. 21f the barrier is materially integrated with the portion of the outer wall of the housing 484 enclosing the second chamber C2. However, in other embodiments it is equally conceivable that the barrier is materially integrated with the portion of the outer wall of the housing 484 enclosing the first chamber C1. In any event, the purpose is the both the first and second chambers C2 should be hermetically enclosed and separated from each other.

The second part of the magnetic coupling comprises a second disc shaped member 490b positioned in the second chamber C2 and held in place by a ball bearing 482b being fixated to the inside of the wall of the housing 484 enclosing the second chamber C2 by means of an internal wall portion 498. The second disc shaped member 490b comprises magnets (or a material susceptible to magnetic fields) 491b evenly distributed in a circular formation axially on the distal surface of the first disc shaped member 490b. The magnets 490b of the second disc shaped member 490b are configured to be magnetically connected to the magnets 491a of the first disc shaped member 490a such that the second disc shaped member 490b is dragged by the first disc shaped member 490a by means of the magnetic connection. As such, force from the motor **M** is transferred from the first hermetically enclosed chamber C1 to the second hermetically enclosed chamber C2.

The second disc shaped member 490b comprises a threaded shaft which is configured to be placed in and engage with a sleeve of a compression member 483. The sleeve of the compression member 483 comprises inside threads 483t for creating a transmission **T** that transforms the radially rotating force generated by the motor **M** and the gear system **G,** to a linear force acting in the axial direction of the shaft 481, and thus makes up a transmission **T.**

The compression member 483 is a disc shaped element having a distal surface engaging a first resilient wall portion 102a of the reservoir 107 for moving the first resilient wall portion 102a and thereby compressing the reservoir 107. The periphery of the compression member 483 comprises a flange 483f extending towards the first chamber C1 in the proximal direction creating a lateral surface area towards the housing 484. The lateral surface of the flange 483f is configured to engage the first resilient wall portion 102a for creating a rolling crease of the first resilient wall portion 102a. The disc shaped compression member 483 is rigid and made from titanium, just as the rest of the housing 484. That the compression member 483 is rigid makes the reservoir 107 stiff which ensures that the fluid amount in the medical implant connected to the reservoir 107 remains the same even as the pressure exerted on the medical implant increases.

The reservoir 107 is further enclosed by a second wall portion 102b which is a rigid titanium wall portion through which the conduit 109a enters the reservoir 107. Compression of the reservoir 107 thus forces the fluid from the reservoir through the conduit 109a. The housing 484 further comprises a transfer channel 478 creating a fluid connection between the second chamber C2 and a portion of the second chamber C2' placed more distally. The transfer channel ensures that the pressure is the same in the second chamber C2 and distal portion of the second chamber C2'. The distal portion C2' of the second chamber C2 comprises an expansion portion comprising a resilient membrane 495 configured to move to alter the volume of the distal portion C2' of the second chamber C2 for compensating for the changes to the volume of the reservoir 107 which is created by the movement of the first resilient wall portion 102a of the reservoir 107. As such, the pressure in the second chamber C2 will be substantially constant. The resilient membrane 495 is in the embodiment shown in fig. 21f made from a medical grade elastic silicone material but may in alternative embodiments be made from another biocompatible polymer material, such as polyurethane.

The hydraulic pump of fig. 21f further comprises a pressure sensor 106 placed on the first resilient wall portion 102a of the chamber 107 for sensing the pressure in the chamber 107. The sensor 106, is connected to electrical conduits 493 for transferring an electrical sensor signal from the pressure sensor 106 to the controller 300. The electrical conduits 493 passes from the second chamber C2 to the first chamber C1 through an electrically insulating ceramic grommet 494 integrated in the barrier 484' wall such that the conduits 493 can pass the barrier 484' without being further insulated which enables the conduits 493 to pass through the barrier 484' whilst the barrier hermetically separates the first chamber C1 from the second chamber C2. It may be important to measure strain or pressure in or exerted by the medical device, as too high strain or pressure risks hampering the blood flow to the tissue of the stomach wall, which in the long term could lead to damage of the tissue and in the worst case lead to necrosis.

A first portion 109a of the fluid conduit is connected to an implantable hydraulic force transfer device 496 comprising a first chamber V1 configured to house a first fluid, and as such the first portion 109a of the fluid conduit forms a fluid inlet into the first chamber V1. The first chamber V1 is in connection with a movable wall portion 497 for varying the size of the first chamber V1. The movable wall portion 497 is in turn connected to a second chamber V2 configured to house a second fluid. The second chamber comprises an outlet formed by a second portion 109b of the fluid conduit. The second portion 109b of the fluid conduit fluidly connects the second chamber C2 to a conduit (139) in any of the hydraulic embodiments described herein. As such, the implantable hydraulic force transfer device 496 transfers hydraulic force from a remote unit to the main portion of a medical device configured to exert a force on a body part without mixing the first and second fluids.

In the embodiment shown in fig. 21f, the implantable hydraulic force transfer device 496 comprises a cylinder-shaped housing in which the piston-like movable wall portion 497 moves linearly. The piston-like movable wall portion 497 seals against the inner side of the wall of the cylinder-shaped housing such that the first and second chambers V1, V2 remains separated. The implantable hydraulic force transfer device 496 enables the system to have a first fluid in the compressible reservoir 107 and in the first chamber V1 of the implantable hydraulic force transfer device 496. This part of the system may be hermetically sealed in such a way that leakage is highly improbable, which enables this part of the system to use a fluid which cannot be allowed to escape into the body, such as an oil based fluid, such as a silicone oil. The second part of the system, comprising the second chamber C2 of the implantable hydraulic force transfer device 496, the second portion 109b of the fluid conduit, and the rest of the hydraulic operation device of the medical device (not shown) will have a second fluid which must be a biocompatible fluid as some level of leakage or diffusion may be hard to avoid. In the second part of the system the fluid could for example be an isotone aqueous fluid, such as a saline solution.

The housing 484 and the housing of the implantable hydraulic force transfer device 496 may be a titanium housing. However, it is equally conceivable that the housing is made from another biocompatible material such as a medical grade metal alloy, such as medical grade stainless steel or a ceramic material such as zirconium carbide, or a stiff medical grade polymer material such as Ultra-high-molecular-weight polyethylene (UHMWPE) or Polytetrafluoroethylene (PTFE) or a thermoplastic polyester such as polylactide (PLA).

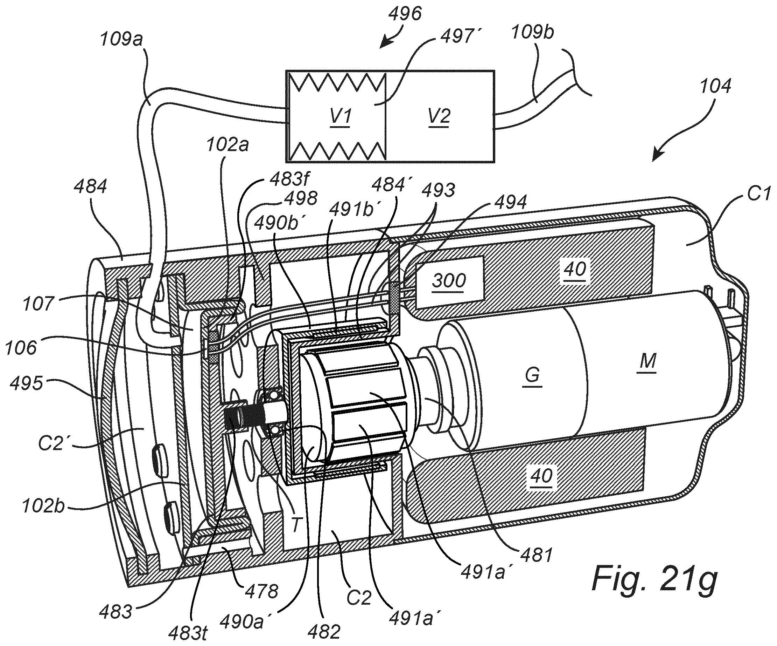

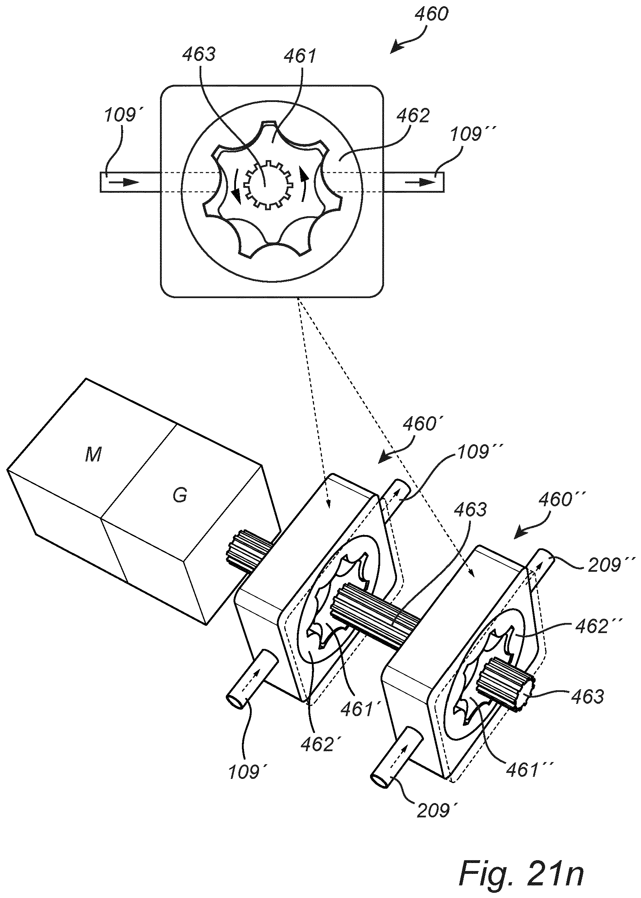

In alternative embodiments, the magnetic coupling described with reference to figs. 21f and 21g could be used in connection with another type of pumps, such as the pumps described with reference to figs. 21c and 21n. In the alternative, the magnetic coupling could be used in connection with a gear pump. It is also conceivable that the magnetic coupling could be used in connection with a mechanical actuator configured to transfer mechanical force from the magnetic coupling to a medical device to exert a force on a body portion of a patient. The mechanical actuator could be an actuator configured to transfer a rotating force into a linear force, such as the transmission (T) described with reference to figs. 21c - 21i.

Fig. 21g shows a hydraulic pump in an embodiment similar to the embodiment shown in fig. 21f. One difference with the embodiment of fig. 21g in comparison to the embodiment of fig. 21f is that the first coupling part 490a' comprises magnets 491a' or material susceptible to magnetic fields which are placed radially along an outer periphery, on the lateral surface, of the cylinder-like first coupling part 490a'. The magnets 491a' of the first coupling part 490a' are magnetically connected to magnets 491b' placed radially on the inner letteral surface of the cylinder-shaped second coupling part 490b'. The magnets 491a',491b' of the first and second coupling parts 490a', 490b' are separated from each other by the barrier 484'. The second coupling part 490b' is connected to a rotatable shaft which is supported by ball bearings 482b being fixated to the inside of the wall of the housing 484 enclosing the second chamber C2 by means of an internal wall portion 498. The rotatable shaft comprises a threaded portion which is configured to be placed in and engage with a sleeve of a compression member 483. The sleeve of the compression member 483 comprises inside threads 483t for creating a transmission T that transforms the radially rotating force generated by the motor **M** and the gear system **G,** to a linear force acting in the axial direction of the shaft 481, and thus makes up a transmission **T.**

Another difference between the embodiment shown in fig. 21f and the embodiment shown in fig. 21g is in the implantable hydraulic force transfer device 496. In the embodiment shown in fig. 21g, the implantable hydraulic force transfer device 496 comprises a movable wall portion 497' in the form of a bellows with a pleated flexible wall portion which can be compressed and expanded. The material of the flexible wall portion could be an elastic material, such as an elastic polymer material or a substantially inelastic material such as a metal material forming a metal bellows which is mainly flexible due to its shape. In an alternative embodiment, the flexible wall portion can be purely elastic and thus be without the pleats, which means that the expansion and contraction of the reservoir is done purely based on the elasticity of the material in the flexible wall. The flexible movable wall portion 497' encloses the first chamber V1 and keeps the chamber V1 completely separated from the chamber V2. The implantable hydraulic force transfer device 496 enables the system to have a first fluid in the compressible reservoir 107 and in the first chamber V1 of the implantable hydraulic force transfer device 496. This part of the system may be hermetically sealed in such a way that leakage is highly improbable, which enables this part of the system to use a fluid which cannot be allowed to escape into the body, such as an oil based fluid, such as a silicone oil. The second part of the system, comprising the second chamber C2 of the implantable hydraulic force transfer device 496, the second portion 109b of the fluid conduit, and the implantable hydraulic constriction element (not shown) will have a second fluid which must be a biocompatible fluid as some level of leakage or diffusion may be hard to avoid. In the second part of the system the fluid could for example be an isotone aqueous fluid, such as a saline solution.

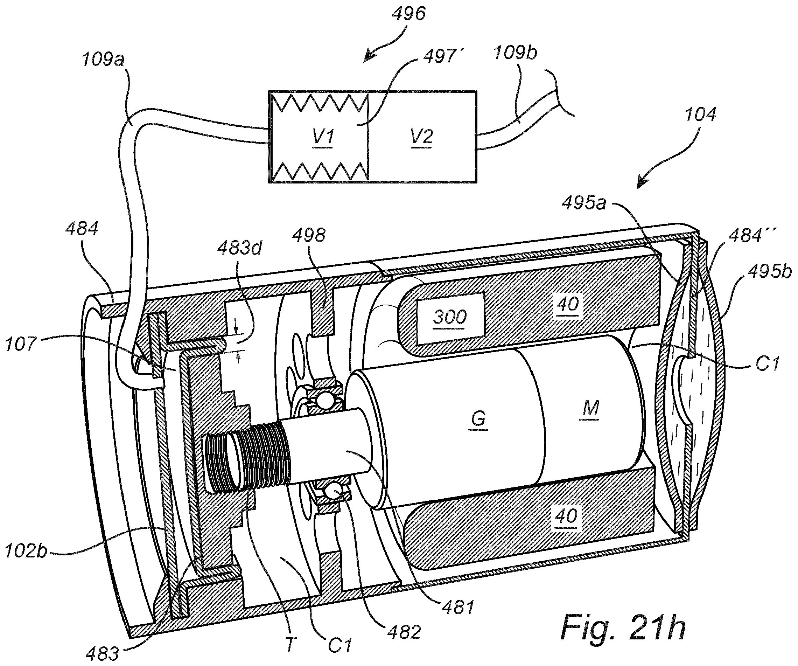

Fig. 21h shows an embodiment of a hydraulic pump 104 which is similar to the embodiment shown in fig. 21e. One difference in comparison to the embodiment of fig. 21e is that the compression member 483 has a flat circular surface engaging the first resilient wall portion 102a of the reservoir 107. The flat surface is bonded to the first resilient wall portion 102a such that the first resilient wall portion 102a moves along with the compression member 483. The compression member 483 has a diameter such that a distance 483d is created between the compression member 483 and the portion of the housing facing the compression member 483. The distance is slightly more than two times the thickness of the first resilient wall portion 102a, such that the first resilient wall portion 102a can be folded such that a rolling crease of the first resilient wall portion 102a is created which moves along with the compression member 483. The distance 483d is smaller than the radius (or half cross-sectional distance) of the compression member 483. The distance is 483d is also smaller than half the radius of the compression member 483. The first resilient wall portion 102a, towards the second chamber C2, being either folded or supported by the compression member means that ensures that the reservoir 107 will be substantially stiff which enables the fluid amount in an hydraulically operable medical device connected to the reservoir 107 to remain the same even as the pressure exerted on the hydraulically operable medical device increases.

The embodiment of fig. 21h differs from the embodiment of fig. 21f and 21g in that it only comprises a single chamber C1. The housing 484 of the hydraulic pump 104 of fig. 21h comprises an expansion portion placed in the proximal portion of the hydraulic pump 104 (on the right side of the hydraulic pump of fig. 21h). The expansion portion comprises a first and second resilient membrane 495a, 495b with a silicone oil filling the space formed between the first and second resilient membranes 495a, 495b. The oil between the first and second resilient membrane 495a, 495b reduces the risk of diffusion of fluids through the expansion portion. The first and second resilient membranes 495a, 495b are placed on two sides of a portion 484" of the housing comprising a hole through which the fluid can travel as the expansion portion compensates for the changes to the volume of the reservoir 107 which is created by the movement of the first resilient wall portion 102a of the reservoir 107. As such, the pressure in the first chamber C1 will be substantially constant. The first and second resilient membranes 495a, 495b are in the embodiment shown in fig. 21h made from a medical grade elastic silicone material but may in alternative embodiments be made from another biocompatible polymer material, such as polyurethane.

Another aspect of having the housings of any of the embodiments herein, is that the atmospheric pressure that the patient exists in may vary. At sea level, the air pressure is about 101 kPa, in a commercial airplane at cruising altitude, the air pressure is about 80 kPa which is about the same as in Mexico city, whereas in La Paz, the highest situated city, air pressure is only 62 kPa. This difference in air pressure affects any gaseous fluid, such as the air present in the chamber C1 in the embodiment of fig. 21h. The reduced atmospheric air pressure means that the gaseous fluid inside of the housing needs to be able to expand if the pressure in the housing should remain the same. If the pressure in the housing would increase 20% - 40%, the motor would have to operate the hydraulic medical device against that pressure which would mean that the motor would have to be more powerful which would require more energy. As the expansion portion comprises a resilient membrane, the expansion portion allows the gaseous fluid in the housing to expand which at least reduces the pressure increase in the housing in response to a reduced atmospheric pressure.

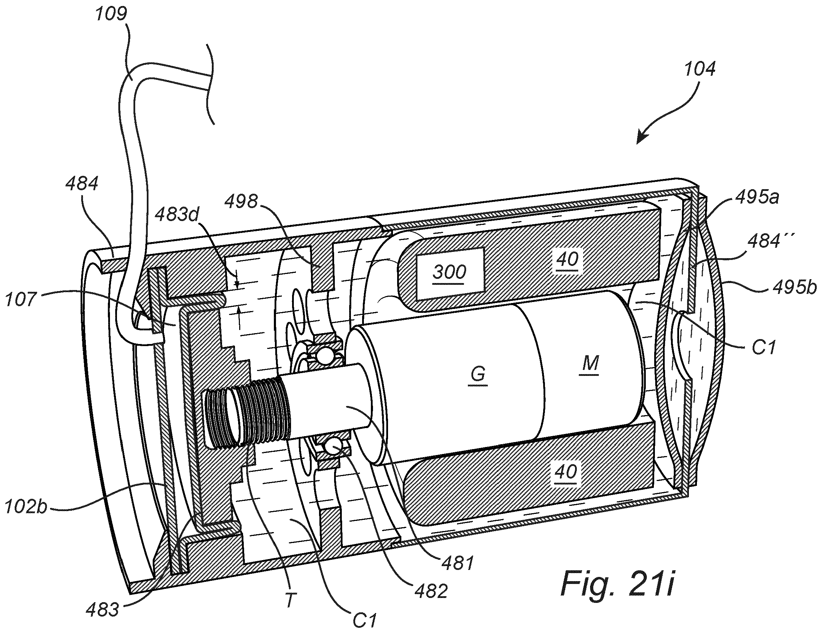

Fig. 21i differs from the embodiment of fig. 21h only in that the chamber C1 is completely filled with a liquid dielectric silicone oil. The liquid fluid could in the alternative be a synthetic single-phase liquid dielectric fluid, such as ElectroCool EC-100, from Engineered Fluids, or a 2-phase coolant such as Fluorinert or Novec from 3M. The fluid in the chamber C1 is non-conductive and as such does not risk damaging the electrical components placed in the chamber C1, such as the energy storage unit 40. In the embodiment shown in fig. 21i, the expandible reservoir 107, the conduit 109 and the medical device configured to exert force on the body portion of the patient forms the second chamber and second hydraulic system configured to comprise a second liquid which is a hydraulic liquid configured to transfer force. The second liquid may be an isotone aqueous liquid, such as a saline solution.

In the embodiment shown in fig. 21i, the first chamber comprises the motor **M,** the gear system **G** and the transmission **T** for transforming the rotating force generated by the motor **M** to a linear force for pressing on the expandible reservoir 107. Advantages with having the housing and the first chamber C1 entirely filled with a liquid fluid includes the liquid acting as a cooling agent for components that may produce heat, such as the controller 300, the energy storage unit 40, the motor **M,** gear system **G,** bearing 482 and transmission **T,** and as a lubricant for components that may require lubrication, such as the motor **M,** gear system **G,** bearing 482 and transmission **T.**

Just as in fig. 21h, the housing 484 of the hydraulic pump 104 comprises an expansion portion 495a, 484", 495b placed in the proximal portion of the hydraulic pump 104 (on the right side of the hydraulic pump of fig. 21i), such that the housing can expand when the expandable reservoir 107 expands.

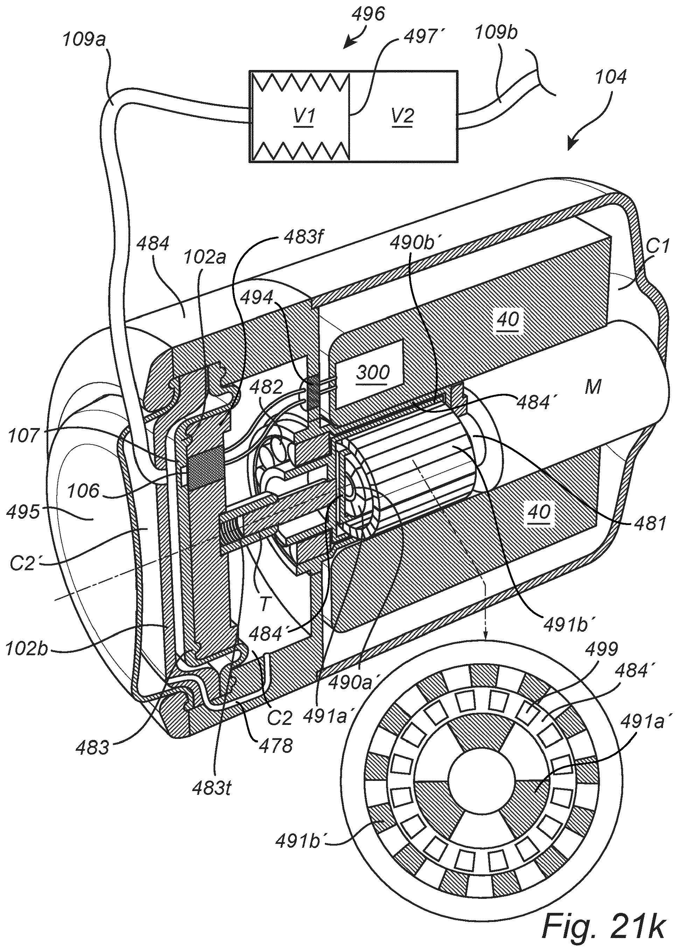

In alternative embodiments, the liquid filled first chamber C1 could be used in connection with another type of pump, i.e. the shaft 481 could be connected to another type of pump, such as the pumps described with reference to figs. 21c and 21k, or a gear pump.

Fig. 21k shows an embodiment of a hydraulic pump 104 which is similar to the embodiment shown in fig. 21g. The main difference with the embodiment shown in fig. 21k is that it made more compact as the gear system is integrated in the magnetic coupling. The magnetic coupling thus comprises a magnetic gear which transfers a week force with a high velocity into a stronger force with lower velocity. The magnetic coupling/gear comprises a first coupling part 490a' fixated to the shaft 481 connected to the electrical motor **M** such that the first coupling part 490a' rotates along with the electrical motor **M.** The first coupling part 490a' comprises a first number of magnets 491a', which in the embodiment shown in fig. 21k is 6 magnets, 3 with each polarity (3 pole pairs). The magnets are placed radially along an outer periphery, on the lateral surface, of the cylinder-like first coupling part 490a'. The second coupling part 490b' comprises a second number of magnets 491b', placed radially on the inner letteral surface of the cylinder-shaped second coupling part 490b'. In the embodiment shown in fig. 21k the second coupling part 490b' comprises 26 (twenty six) magnets, 13 (thirteen) with each polarity. Between the first coupling part 490a' and the second coupling part 490b' there is a stationary part, which is a portion of the barrier 484'. The stationary part comprises a plurality of intermediate ferromagnetic elements 499 thus placed between the first and second coupling parts 490a', 490b'. The intermediate ferromagnetic elements 499 directs the concentration of the magnetic lines between the magnets 491a', 491b' of the first coupling part 490a' and the second coupling part 490b'. The gear ratio between the first coupling part 490a' and the second coupling part 490b' is the number of magnetic pole pairs on the second coupling part 490a' divided by the number of magnetic pole pairs on the second coupling part 490b'. In the embodiment shown in fig. 21k, the gear ratio is 13/3. The number of intermediate ferromagnetic elements 499 is equal to the sum of pole pairs on the first and second coupling parts 490a', 490b'. In the embodiment shown in fig. 21k this means that the number of intermediate ferromagnetic elements 499 is 16 (13+3). In operation, this set up of magnetic gear changes the direction of rotation of the coupling, which means that that in operation the second coupling part 490b' will rotate in the opposite direction and 4,33 times slower than the first coupling part 490a'. The embodiment having a magnetic gear have a number of advantages, for example, the magnetic gear is quiet, does not wear and does not need to be lubricated.

The second coupling part 490b' is connected to a rotatable shaft which is supported by roller bearings 482 being fixated to the inside of the wall of the housing 484. The rotatable shaft comprises a threaded portion which is configured to be placed in and engage with a sleeve of a compression member 483. The sleeve of the compression member 483 comprises inside threads 483t for creating a transmission **T** that transforms the radially rotating force generated by the motor **M** and the gear system **G,** to a linear force acting in the axial direction of the shaft 481, and thus makes up a transmission **T.**

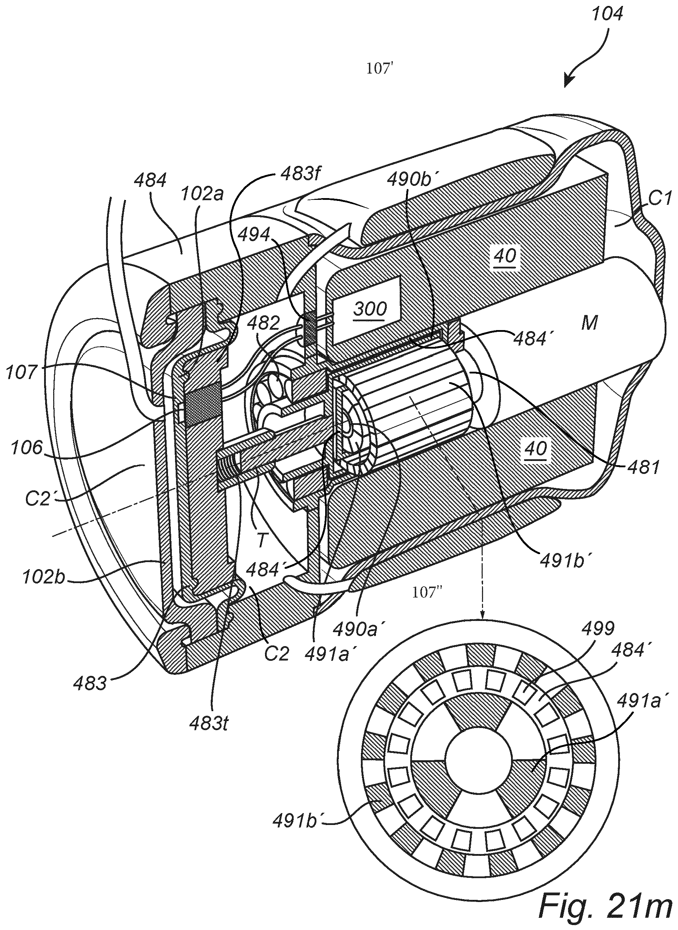

Fig. 21m shows an embodiment of a hydraulic pump 104 which is similar to the embodiment shown in fig. 21k. The main difference with the embodiment shown in fig. 21m is that the expansion portion is replaced with two resilient reservoirs 107',107" which are placed in indentations in the housing, on respective two opposite sides of the housing. The two resilient reservoirs 107',107" are configured to expand and contract to compensate for the changes to the volume of the reservoir 107 which is created by the movement of the first resilient wall portion 102a of the reservoir 107. As such, the pressure in the second chamber C2 will be substantially constant. The two resilient reservoirs 107',107" are made from a medical grade elastic silicone material but may in alternative embodiments be made from another biocompatible polymer material, such as polyurethane.

Fig. 21n shows and embodiment of a system comprising a motor **M,** gear system **G** and two implantable pumps 460',460". In the embodiment shown in fig. 21n, the force output of the motor **M** is connected to a force input of the gear system **G.** The gear system **G** is configured to reduce the velocity and increase the force of the movement generated by the motor **M,** such that the movement exiting the gear system **G** at the force output of the gear system **G** is a mechanical force with a lower velocity and a greater force than the movement entering the force input of the gear system **G.** Typically, an implantable brushless DC motor, such as the motors provided by Maxon group or Dr. Fritz Faulhaber, typically produces a rotational velocity exceeding 10 000 rpm. For such a motor to be able to mechanically operate any of the hydraulic pumps described herein, a gear system **G** is needed. In the embodiment shown with reference to fig. 21n, the gear system **G** reduces the rotational velocity 100 times, to about 100 rpm. The force output of the gear system **G** is mechanically connected to a common rotating shaft 463. The first hydraulic pump comprises a first gerotor pump 460' and the second hydraulic pump comprises a second gerotor pump 460". The common rotating shaft 463 is mechanically connected to an inner rotor 461' of the first gerotor pump 460' and an inner rotor 461" of the second gerotor pump, such that the motor **M** propels the first and second gerotor pump 460'460". A gerotor is a positive displacement pump comprising consists of an inner rotor 461 and an outer rotor 462. The inner rotor 461 has 6 teeth, while the outer rotor has 7 teeth (the importance being that the outer rotor 462 has one tooth more than the inner rotor 461. The axis of the inner rotor 461, which is the rotational center of the common rotating shaft 463, is offset from the rotational center or axis of the outer rotor 462. Both the inner and outer rotors 461, 462 rotate on their respective axes. The geometry of the two rotors 461, 462 partitions the volume between them into 6 different dynamically changing volumes. During the rotation cycle, each of these volumes changes continuously, so any given volume first increases, and then decreases. An increase creates a vacuum. This vacuum creates suction, and hence, this part of the cycle is where the inlet 109' is located. As a volume decreases compression occurs which pumps the fluid though the outlet 109".

In the embodiment shown in fig. 21n, the first gerotor pump 460' is configured to be in fluid connection with a first operable hydraulic constriction element for pumping hydraulic fluid into the first operable hydraulic constriction element for inflating the first operable hydraulic constriction element to exert a pressure on the luminary organ and thereby restrict the flow or fluid therethrough. The second gerotor pump 460" is configured to be in fluid connection with a second operable hydraulic constriction element for pumping hydraulic fluid into the second operable hydraulic constriction element for inflating the second operable hydraulic constriction element to exert a pressure on the luminary organ and thereby restrict the flow or fluid therethrough. The inlets 109',109" of the first and second gerotor pumps 460',460" are configured to be connected to a reservoir for holding hydraulic fluid, or in the alternative, the first inlet 109' is configured to be connected to a first implantable reservoir and the second inlet 109" is configured to be connected to a second implantable reservoir.

In alternative embodiments, the first and second hydraulic pump mechanically connected to a common rotating shaft could be pump comprising at least one compressible hydraulic reservoir (such as the pump described with reference to fig. 21d), a pump comprising a displaceable wall (such as the pump described with reference to fig. 21c), or a peristaltic pump.

The function and features of the controller (such as comprised in a remote unit) for will now described with reference to figures 22a - 22f. The features of the controller described with reference to figs. 22a - 22f may be implemented in any remote unit (also referred to as implantable energized medical device) disclosed herein. Any controller 300 may comprise an internal computing unit, also called a processor or processing unit, and it may comprise a communication unit and implement methods for communication, including verification, authentication and encryption of data, as described in the following.

The controller may comprise a collection of communication related sub-units such as a wired transceiver, a wireless transceiver, energy storage unit, an energy receiver, a computing unit, a memory, or a feedback unit. The sub-units of the controller may cooperate with each other or operate independently with different purposes. The sub-units of the controller may inherit the prefix "internal". This is to distinguish these sub-units from the sub-units of the external devices as similar sub-units may be present for both the implanted controller and the external devices. The sub-units of the external devices may similarly inherit the prefix "external".

A wireless transceiver may comprise both a wireless transmitter and a wireless receiver. The wireless transceiver may also comprise a first wireless transceiver and a second wireless transceiver. **In** this case, the wireless transceiver may be part of a first communication system (using the first wireless transceiver) and a second communication system (using the second wireless transceiver).

In some embodiments, two communication systems may be implemented using a single wireless transceiver in e.g. the implant and a single wireless transceiver in e.g. an external device (i.e. one antenna at the implant and one antenna at the external device), but where for example the network protocol used for data transmission from the external device to the implant is different from the network protocol used for data transmission from the implant to the external device, thus achieving two separate communication systems.

Alternatively, the wireless transceiver may be referred to as either a wireless transmitter or a wireless receiver as not all embodiments of secure wireless communication discussed herein require two-way communication capability of the wireless transceiver. The wireless transceiver may transmit or receive wireless communication via wireless connections. The wireless transceiver may connect to both the implant and to external devices, i.e. devices not implanted in the patient.

The wireless connections may be based on radio frequency identification (RFID), near field charge (NFC), Bluetooth, Bluetooth low energy (BLE), or wireless local area network (WLAN). The wireless connections may further be based on mobile telecommunication regimes such as 1G, 2G, 3G, 4G, or 5G. The wireless connections may further be based on modulation techniques such as amplitude modulation (AM), frequency modulation (FM), phase modulation (PM), or quadrature amplitude modulation (QAM). The wireless connection may further feature technologies such as time-division multiple access (TDMA), frequency-division multiple access (FDMA), or code-division multiple access (CDMA). The wireless connection may also be based on infra-red (IR) communication. The wireless connection may feature radio frequencies in the high frequency band (HF), very-high frequency band (VHF), and the ultra-high frequency band (UHF) as well as essentially any other applicable band for electromagnetic wave communication. The wireless connection may also be based on ultrasound communication to name at least one example that does not rely on electromagnetic waves.

A wired transceiver may comprise both a wired transmitter and a wired receiver. The wording wired transceiver aims to distinguish from a wireless transceiver. It may generally be considered a conductive transceiver. The wired transceiver may transmit or receive conductive communication via conductive connections. Conductive connections may alternatively be referred to as electrical connections or as wired connections. The wording wired however, does not imply there needs to be a physical wire for conducting the communication. The body tissue of the patient may be considered as the wire. Conductive connection may use the body of the patient as a conductor. Conductive connections may still use ohmic conductors such as metals to at least some extent, and more specifically at the interface between the wired transceiver and the chosen conductor.

Communication, conductive or wireless may be understood as digital or analogue. In analogue communication, the message signal is in analogue form i.e., a continuous time signal. In digital communication, usually digital data i.e., discrete time signals containing information is transmitted.

The controller may comprise a sensation generator. A sensation generator is a device or unit that generates a sensation. The sensation generated may be configured to be experienceable by the patient such that the patient may take actions to authenticate a device, connection or communication. The sensation generator may be configured to generate a single sensation or a plurality of sensation components. The sensation or sensation components may comprise a vibration (e.g. a fixed frequency mechanical vibration), a sound (e.g. a superposition of fixed frequency mechanical vibrations), a photonic signal (e.g. a non-visible light pulse such as an infra-red pulse), a light signal (e.g. a visual light pulse), an electric signal (e.g. an electrical current pulse) or a heat signal (e.g. a thermal pulse). The sensation generator may be implanted, configured to be worn in contact with the skin of the patient or capable of creating sensation without being in physical contact with the patient, such as a beeping alarm.

The sensations generated by the sensation generator may be configured to be experienceable by a sensory function or a sense of the patient from the list of tactile, pressure, pain, heat, cold, taste, smell, sight, and hearing. Sensations may be generated of varying power or force as to adapt to sensory variations in the patient. Power or force may be increased gradually until the patient is able to experience the sensation. Variations in power or force may be controlled via feedback. Sensation strength or force may be configured to stay within safety margins. The sensation generator may be connected to the implant. The sensation generator may be comprised within the implant or be a separate unit.

A motor, e.g. of the remote unit or implant for exerting a force on a body part, for controlling a physical function in the body of the patient, may provide a secondary function as a sensation generator, generating a vibration or sound. Generation of vibrations or sounds of the motor MO may be achieved by operating the motor at specific frequencies. When functioning as to generate a sensation the motor MO may operate outside of its normal ranges for frequency controlling a physical function in the body. The power or force of the motor when operating to generate a sensation may also vary from its normal ranges for controlling a physical function in the body. The motor for use as an active device and a sensation generator could for example be an implantable brushless DC motor with integrated gear box, such as the motors provided by Maxon group or Dr. Fritz Faulhaber.

An external device is a device which is external to the patient in which the implant is implanted in. The external device may be also be enumerated (first, second, third, etc.) to separate different external devices from each other. Two or more external devices may be connected by means of a wired or wireless communication as described above, for example through IP (internet protocol), or a local area network (LAN). The wired or wireless communication may take place using a standard network protocol such as any suitable IP protocol (IPv4, IPv6) or Wireless Local Area Network (IEEE 802.11), Bluetooth, NFC, RFID etc. The wired or wireless communication may take place using a proprietary network protocol. Any external device may also be in communication with the implant using wired or wireless communication according to the above. Communication with implanted devices may be thus accomplished with a wired connection or with wireless radiofrequency (RF) telemetry. Other methods of wireless communication may be used to communicate with implants, including optical and ultrasound. Alternatively, the concept of intrabody communication may be used for wireless communication, which uses the conductive properties of the body to transmit signals, i.e. conductive (capacitive or galvanic) communication with the implant. Means for conductive communication between an external device and an implant may also be called "electrical connection" between an external device and an implant. The conductive communication may be achieved by placing a conductive member of the external device in contact with the skin of the patient. By doing this, the external device and/or the implant may assure that it is in direct electrical connection with the other device. The concept relies on using the inherent conductive or electrical properties of a human body. Signals may preferably be configured to affect the body or body functions minimally. For conductive communication this may mean using low currents. A current may flow from an external device to an implant or vice versa. Also, for conductive communication, each device may have a transceiver portion for transmitting or receiving the current. These may comprise amplifiers for amplifying at least the received current. The current may contain or carry a signal which may carry e.g. an authentication input, implant operation instructions, or information pertaining to the operation of the implant.

Alternatively, conductive communication may be referred to as electrical or ohmic or resistive communication.

The conductive member may be an integrated part of the external device (e.g. in the surface of a smartwatch that is intended to be in contact with the wrist of the person wearing it), or it may be a separate device which can be connected to the external device using a conductive interrace such as the charging port or the headphone port of a smartphone.

A conductive member may be considered any device or structure set up for data communication with the implant via electric conductive body tissue. The data communication to the implant may be achieved by e.g. current pulses transmitted from the conductive member through the body of the patient to be received by a receiver at the implant. Any suitable coding scheme known in the art may be employed. The conductive member may comprise an energy storage unit such as a battery or receive energy from e.g. a connected external device.

The term conductive interface is representing any suitable interface configured for data exchange between the conductive member and the external device. The conductive member may in an alternative configuration receive and transmit data to the external device through a radio interface, NFC, and the like.

An external device may act as a relay for communication between an implant and a remote device, such as e.g. second, third, or other external devices. Generally, the methods of relaying communication via an external device may be preferable for a large number of reasons. The transmission capabilities of the implant may be reduced, reducing its technical complexity, physical dimensions, and medical effects on the patient in which the implant is implanted. Communication may also be more efficient as direct communication, i.e. without a relaying device, with an implant from a remote device may require higher energy transmissions to account for different mediums and different rates of attenuation for different communication means. Remote communication with lower transmission energy may also increase the security of the communication as the spatial area or volume where the communication may be at all noticeable may be made smaller. Utilizing such a relay system further enables the use of different communication means for communication with the implant and communication with remote devices that are more optimized for their respective mediums.

An external device may be any device having processing power or a processor to perform the methods and functions needed to provide safe operation of the implant and provide the patient or other stakeholders (caregiver, spouse, employer etc.) with information and feedback from the implant. Feedback parameters could include battery status, energy level at the controller, the fluid level of the hydraulic restriction device, number of operations that the restriction device has performed, properties, version number etc. relating to functionality of the implantable medical device. The external device may for example be a handset such as a smartphone, smartwatch, tablet etc. handled by the patient or other stakeholders. The external device may be a server or personal computer handled by the patient or other stakeholders. The external device may be cloud based or a virtual machine. In the drawings, the external device handled by the patient is often shown as a smart watch, or a device adapted to be worn by the patient at the wrist of the patient. This is merely by way of example and any other type of external device, depending on the context, is equally applicable.

Several external devices may exist such as a second external device, a third external device, or another external device. The above listed external devices may e.g. be available to and controllable by a patient, in which an implant is implanted, a caregiver of the patient, a healthcare professional of the patient, a trusted relative of the patient, an employer or professional superior of the patient, a supplier or producer of the implant or its related features. By controlling the external devices may provide options for e.g. controlling or safeguarding a function of the implant, monitoring the function of the implant, monitoring parameters of the patient, updating or amending software of the implant etc.

An external device under control by a supplier or producer of the implant may be connected to a database comprising data pertaining to control program updates and/or instructions. Such database may be regularly updated to provide new or improved functionality of the implant, or to mitigate for previously undetected flaws of the implant. When an update of a control program of an implant is scheduled, the updated control program may be transmitted from the database in a push mode and optionally routed via one or more further external devices before received by the implanted controller. In another embodiment, the update is received from the database by request from e.g. an external device under control by the patient having the implant implanted in his/her body, a pull mode.

The external device may require authentication to be operated in communication with other external devices or the implant. Passwords, multi-factor authentication, biometric identification (fingerprint, iris scanner, facial recognition, etc.) or any other way of authentication may be employed.

The external device may have a user interface (UI) for receiving input and displaying information/feedback from/to a user. The UI may be a graphical UI (GUI), a voice command interface, speaker, vibrators, lamps, etc.

The communication between external devices, or between an external device and the implant may be encrypted. Any suitable type of encryption may be employed such as symmetric or asymmetric encryption. The encryption may be a single key encryption or a multi-key encryption. In multi-key encryption, several keys are required to decrypt encrypted data. The several keys may be called first key, second key, third key, etc. or first part of a key, second part of the key, third part of the key, etc. The several keys are then combined in any suitable way (depending on the encryption method and use case) to derive a combined key which may be used for decryption. In some cases, deriving a combined key is intended to mean that each key is used one by one to decrypt data, and that the decrypted data is achieved when using the final key.

In other cases, the combination of the several key result in one "master key" which will decrypt the data. In other words, it is a form of secret sharing, where a secret is divided into parts, giving each participant (external device(s), internal device) its own unique part. To reconstruct the original message (decrypt), a minimum number of parts (keys) is required. In a threshold scheme this number is less than the total number of parts (e.g. the key at the implant and the key from one of the two external device are needed to decrypt the data). In other embodiments, all keys are needed to reconstruct the original secret, to achieve the combined key which may decrypt the data.

In should be noted that it is not necessary that the generator of a key for decryption is the unit that in the end sends the key to another unit to be used at that unit. In some cases, the generator of a key is merely a facilitator of encryption/decryption, and the working in behalf of another device/user.

A verification unit may comprise any suitable means for verifying or authenticating the use (i.e. user authentication) of a unit comprising or connected to the verification unit, e.g. the external device. For example, a verification unit may comprise or be connected to an interface (UI, GUI) for receiving authentication input from a user. The verification unit may comprise a communication interface for receiving authentication data from a device (separate from the external device) connected to the device comprising the verification unit. Authentication input/data may comprise a code, a key, biometric data based on any suitable techniques such as fingerprint, a palm vein structure, image recognition, face recognition, iris recognition, a retinal scan, a hand geometry, and genome comparison, etc. The verification/authentication may be provided using third party applications, installed at or in connection with the verification unit.

The verification unit may be used as one part of a two-part authentication procedure. The other part may e.g. comprise conductive communication authentication, sensation authentication, or parameter authentication.

The verification unit may comprise a card reader for reading a smart card. A smart card is a secure microcontroller that is typically used for generating, storing and operating on cryptographic keys. Smart card authentication provides users with smart card devices for the purpose of authentication. Users connect their smart card to the verification unit. Software on the verification unit interacts with the keys material and other secrets stored on the smart card to authenticate the user. In order for the smart card to operate, a user may need to unlock it with a user-PIN. Smart cards are considered a very strong form of authentication because cryptographic keys and other secrets stored on the card are very well protected both physically and logically, and are therefore hard to steal.

The verification unit may comprise a personal e-ID that is comparable to, for example, passport and driving license. The e-ID system comprises is a security software installed at the verification unit, and a e-ID which is downloaded from a web site of a trusted provided or provided via a smart card from the trusted provider.

The verification unit may comprise software for SMS-based two-factor authentication. Any other two-factor authentication systems may be used. Two-factor authentication requires two things to get authorized: something you know (your password, code, etc.) and something you have (an additional security code from your mobile device (e.g. a SMS, or a e-ID) or a physical token such as a smart card).

Other types of verification/user authentication may be employed. For example, a verification unit which communicate with an external device using visible light instead of wired communication or wireless communication using radio. A light source of the verification unit may transmit (e.g. by flashing in different patterns) secret keys or similar to the external device which uses the received data to verify the user, decrypt data or by any other means perform authentication. Light is easier to block and hide from an eavesdropping adversary than radio waves, which thus provides an advantage in this context. In similar embodiments, electromagnetic radiation is used instead of visible light for transmitting verification data to the external device.

Parameters relating to functionality of the implant may comprise for example a status indicator of the implant such as battery level, version of control program, properties of the implant, status of a motor of the implant, etc.

Data comprising operating instructions sent to the implant may comprise a new or updated control program, parameters relating to specific configurations of the implant, etc. Such data may for example comprise instructions how to operate the body engaging portion of the implantable medical device, instructions to collect patient data, instructions to transmit feedback, etc.

The expressions "confirming the electrical connection between an implant and an external device" or "authenticating a connection between an implant and an external device", or similar expressions, are intended to encompass methods and processes for ensuring or be reasonably sure that the connection has not been compromised. Due to weaknesses in the wireless communication protocols, it is a simple task for a device to "listen" to the data and grab sensitive information, e.g. personal data regarding the patient sent from the implant, or even to try to compromise (hack) the implant by sending malicious commands or data to the implant. Encryption may not always be enough as a security measure (encryption schemes may be predictable), and other means of confirming or authenticating the external device being connected to the implant may be needed.

The expression "network protocol" is intended to encompass communication protocols used in computer networks. a communication protocol is a system of rules that allow two or more entities of a communications system to transmit information via any kind of variation of a physical quantity. The protocol defines the rules, syntax, semantics and synchronization of communication and possible error recovery methods. Protocols may be implemented by hardware, software, or a combination of both. Communication protocols have to be agreed upon by the parties involved. In this field, the term "standard" and "proprietary" is well defined. A communication protocol may be developed into a protocol standard by getting the approval of a standards organization. To get the approval the paper draft needs to enter and successfully complete the standardization process. When this is done, the network protocol can be referred to a "standard network protocol" or a "standard communication protocol". Standard protocols are agreed and accepted by whole industry. Standard protocols are not vendor specific. Standard protocols are often, as mentioned above, developed by collaborative effort of experts from different organizations.

Proprietary network protocols, on the other hand, are usually developed by a single company for the devices (or Operating System) which they manufacture. A proprietary network protocol is a communications protocol owned by a single organization or individual. Specifications for proprietary protocols may or may not be published, and implementations are not freely distributed. Consequently, any device may not communicate with another device using a proprietary network protocol, without having the license to use the proprietary network protocol, and knowledge of the specifications for proprietary protocol. Ownership by a single organization thus gives the owner the ability to place restrictions on the use of the protocol and to change the protocol unilaterally.

A control program is intended to define any software used for controlling the implant. Such software may comprise an operating system of the implant, of parts of an operating system or an application running on the implant such as software controlling a specific functionality of the implant (e.g. the active unit of the implant, feedback functionality of the implant, a transceiver of the implant, encoding/decoding functionality of the implant, etc.). The control program may thus control the medical function of the implant, for example the pressure applied by a member or the power of the electrical stimulation device. Alternatively or additionally, the control program may control internal hardware functionality of the implant such as energy usage, transceiver functionality, etc.

The systems and methods disclosed hereinabove may be implemented as software, firmware, hardware or a combination thereof. In a hardware implementation, the division of tasks between functional units referred to in the above description does not necessarily correspond to the division into physical units; to the contrary, one physical component may have multiple functionalities, and one task may be carried out by several physical components in cooperation. Certain components or all components may be implemented as software executed by a digital signal processor or microprocessor or be implemented as hardware or as an application-specific integrated circuit. Such software may be distributed on computer readable media, which may comprise computer storage media (or non-transitory media) and communication media (or transitory media). As is well known to a person skilled in the art, the term computer storage media includes both volatile and non-volatile, removable and non-removable media implemented in any method or technology for storage of information such as computer readable instructions, data structures, program modules or other data. Computer storage media includes, but is not limited to, RAM, ROM, EEPROM, flash memory or other memory technology, CD-ROM, digital versatile disks (DVD) or other optical disk storage, magnetic cassettes, magnetic tape, magnetic disk storage or other magnetic storage devices, or any other medium which can be used to store the desired information, and which can be accessed by a computer. Further, it is well known to the skilled person that communication media typically embodies computer readable instructions, data structures, program modules or other data in a modulated data signal such as a carrier wave or other transport mechanism and includes any information delivery media.

A controller 300 for controlling the medical device according to any of the embodiments herein and for communicating with devices external to the body of the patient and/or implantable sensors will now be described with reference to figures 22a - 22f. Figure 22a shows a patient when a medical device 10 comprising a controller 300 has been implanted. The medical device 10 may comprise an active unit 302, which is the part of the medical device for exerting a force on a body part, which may comprise one or more members and operation device for operating the members etc.. The medical device 10 may further comprise a remote unit which comprises the controller 300. The active unit is directly or indirectly connected to the stomach wall of the patient for stretching the stomach wall for creating a sensation of satiety. The active unit 302 and/or remote unit (not shown) is connected to the controller 300 via an electrical connection C2. The controller 300 (further described with reference to figure 22b) is configured to communicate with an external device 320 (further described with reference to figure 22c). The controller 300 can communicate wirelessly with the external device 320 through a wireless connection WL1, and/or through an electrical connection C1.

Referring now to figure 22b, one embodiment of the controller 300 will be describe in more detail. The controller 300 comprises an internal computing unit 306 configured to control the function performed by the implantable medical device 10. The computing unit 306 comprises an internal memory 307 configured to store programs thereon. In the embodiment described in fig. 22b, the internal memory 307 comprises a first control program 310 which can control the function of the medical device 10. The first control program 310 may be seen as a program with minimum functionality to be run at the medical device only during updating of the second control program 312. When the medical device is running with the first control program 310, the medical device may be seen as running in safe mode, with reduced functionality. For example, the first control program 310 may result in that no sensor data is stored in the medical device while being run, or that no feedback is transmitted from the medical device while the first control program 310 is running. By having a low complexity first control program, memory at the medical device is saved, and the risk of failure of the medical device during updating of the second control program 312 is reduced.

The second control program 312 is the program controlling the medical device in normal circumstances, providing the medical device with full functionality and features.

The memory 307 can further comprise a second, updatable, control program 312. The term updatable is to be interpreted as the program being configured to receive incremental or iterative updates to its code, or be replaced by a new version of the code. Updates may provide new and/or improved functionality to the implant as well as fixing previous deficiencies in the code. The computing unit 306 can receive updates to the second control program 312 via the controller 300. The updates can be received wirelessly WL1 or via the electrical connection C1. As shown in figure 22b, the internal memory 307 of the controller 300 can possibly store a third program 314. The third program 314 can control the function of the implantable medical device 10 and the computing unit 306 may be configured to update the second program 312 to the third program 314. The third program 314 can be utilized when rebooting an original state of the second program 312. The third program 314 may thus be seen as providing a factory reset of the controller 300, e.g. restore it back to factory settings. The third program 314 may thus be included in the implant 300 in a secure part of the memory 307 to be used for resetting the software (second control program 312) found in the controller 300 to original manufacturer settings.

The controller 300 may comprise a reset function 316 connected to or part of the internal computing unit 306 or transmitted to said internal computing unit 306. The reset function 316 is configured to make the internal computing unit 306 switch from running the second control program 312 to the first control program 310. The reset function 316 could be configured to make the internal computing unit 306 delete the second control program 312 from the memory 307. The reset function 316 can be operated by palpating or pushing/put pressure on the skin of the patient. This could be performed by having a button on the implant. Alternatively, the reset function 316 can be invoked via a timer or a reset module. Temperature sensors and/ or pressure sensors can be utilized for sensing the palpating. The reset function 316 could also be operated by penetrating the skin of the patient. It is further plausible that the reset function 316 can be operated by magnetic means. This could be performed by utilizing a magnetic sensor and applying a magnetic force from outside the body. The reset function 316 could be configured such that it only responds to magnetic forces applied for a duration of time exceeding a limit, such as 2 seconds. The time limit could equally plausible be 5 or 10 seconds, or longer. In these cases, the implant could comprise a timer. The reset function 316 may thus include or be connected to a sensor for sensing such magnetic force.

In addition to or as an alternative to the reset function described above, the implant may comprise an internal computing unit 306 (comprising an internal processor) comprising the second control program 312 for controlling a function of the implantable medical device, and a reset function 318. The reset function 318 may be configured to restart or reset said second control program 312 in response to: i. a timer of the reset function 318 has not been reset, or ii. a malfunction in the first control program 310.

The reset function 318 may comprise a first reset function, such as, for example, comprise a computer operating properly, COP, function connected to the internal computing unit 306. The first reset function may be configured to restart or reset the first or the second control program 312 using a second reset function. The first reset function comprises a timer, and the first or the second control program is configured to periodically reset the timer.

The reset function 318 may further comprise a third reset function connected to the internal computing unit and to the second reset function. The third reset function may in an example be configured to trigger a corrective function for correcting the first 310 or second control program 312, and the second reset function is configured to restart the first 310 or second control program 312 sometime after the corrective function has been triggered. The corrective function may be a soft reset or a hard reset.

The second or third reset function may, for example, configured to invoke a hardware reset by triggering a hardware reset by activating an internal or external pulse generator which is configured to create a reset pulse. Alternatively, the second or third reset function may be implemented by software.

The controller 300 may further comprise an internal wireless transceiver 308. The transceiver 308 communicates wirelessly with the external device 320 through the wireless connection W1. The transceiver may further communicate with an external device 320, 300 via wireless connection WL2 or WL4. The transceiver may both transmit and receive data via either of the connections C1, WL1, WL2 and WL4. Optionally, the external devices 320 and 300, when present, may communicate with each other, for example via a wireless connection WL3.

The controller 300 can further be electrically connected C1 to the external device 320 and communicate by using the patient's body as a conductor. The controller 300 may thus comprise a wired transceiver 303 or an internal transceiver 303 for the electrical connection C1.

The confirmation/authentication of the electrical connection can be performed as described herein in the section for confirmation and/or authentication. In these cases, the implanted medical device and/or external device(s) 320 comprises the necessary features and functionality (described in the respective sections of this document) for performing such confirmation/authentication. By authenticating according to these aspects, security of the authentication may be increased as it may require a malicious third party to know or gain access to either the transient physiological parameter of the patient or detect randomized sensations generated at or within the patient.

In figures 22a - 22f the patient is a human, but other mammals are equally plausible. It is also plausible that the communication is performed by inductive means. It is also plausible that the communication is direct.

The controller 300 of the implantable medical device 10 according to figure 22b further comprises a feedback unit 349. The feedback unit 349 provides feedback related to the switching from the second control program 312 to the first control program 310. The feedback could for example represent the information on when the update of the software, i.e. the second control program 312, has started, and when the update has finished. This feedback can be visually communicated to the patient, via for example a display on the external device 320. This display could be located on a watch, or a phone, or any other external device 320 coupled to the controller 300. Preferably, the feedback unit 349 provides this feedback signal wirelessly WL1 to the external device 320. Potentially, the words "Update started", or "Update finished", could be displayed to the patient, or similar terms with the same meaning. Another option could be to display different colors, where green for example could mean that the update has finished, and red or yellow that the update is ongoing. Obviously, any color is equally plausible, and the user could choose these depending on personal preference. Another possibility would be to flash a light on the external device 320. In this case the external device 320 comprises the light emitting device(s) needed. Such light could for example be a LED. Different colors could, again, represent the status of the program update. One way of representing that the update is ongoing and not yet finished could be to flash the light, i.e. turning the light on and off. Once the light stops flashing, the patient would be aware of that the update is finished. The feedback could also be audible, and provided by the implantable medical device 300 directly, or by the external device 320. In such cases, the implantable medical device 10 and external device 320 comprises means for providing audio. The feedback could also be tactile, for example in the form of a vibration that the user can sense. In such case, either the implantable medical device 10 or external device 320 comprises means for providing a tactile sensation, such as a vibration and/or a vibrator.

As seen in figure 22b, the controller 300 can further comprise a first energy storage unit 40A. The first energy storage unit 40A runs the first control program 310. The controller 300 further comprises a second energy storage unit 40B which runs the second control program 312. This may further increase security during update, since the first control program 310 has its own separate energy storage unit 40A. The energy storage unit 40A can comprise a first energy storage 304a and/or a first energy receiver 305a. The second energy storage unit 40B can comprise a second energy storage 304b and/or a second energy receiver 305b. The energy can be received wirelessly by inductive or conductive means. An external energy storage unit can for example transfer an amount of wireless energy to the energy receiver 305a, 305b inside the patient's body by utilizing an external coil which induces a voltage in an internal coil (not shown in figures). It is plausible that the first energy receiver 305a receives energy via a RFID pulse. The feedback unit 349 can provide feedback pertaining to the amount of energy received via the RFID pulse. The amount of RFID pulse energy that is being received can be adjusted based on the feedback, such that the pulse frequency is successively raised until a satisfying level is reached.

The controller 300 of the medical device 10 according to figure 22b further comprises a feedback unit an electrical switch 309. The electrical switch 309 could be mechanically connected to a member of the medical device configured to exert a force on the stomach wall of the patient and being configured to be switched as a result of the force exerted on the stomach wall of the patient exceeding a threshold value. The switch 309 could for example be bonded to one of the members being connected to the stomach wall, in any of the embodiments herein, or to a portion of a fluid conduit, reservoir or hydraulic operation device, such as a pump, being in fluid connection with the member and be switched by the expansion, movement or bending of the member. The switch 309 could alternatively be electrically connected to the operation device and being configured to be switched as a result of the current supplied to the operation device exceeding a threshold value. The switch 309 could for example be connected to the motor and be configured to be switched if the current to the motor exceeds a threshold value. Such a switch could for example be a switch 309 configured to switch if exposed to a temperature exceeding a threshold value, such as a bimetal switch which is switched by the heat created by the flow of current to e.g. the motor. In the alternative, the switch 309 configured to switch if exposed to a temperature exceeding a threshold value could be placed at a different location on the medical device 10 to switch in case of exceeding temperatures, thereby hindering the medical device from overheating which may cause tissue damage.

The switch 309 could either be configured to cut the power to the operation device or to generate a control signal to the processor 306 of the implantable controller 300, such that the controller 300 can take appropriate action, such as reducing power or turning off the operation device.

The external device 320 is represented in figure 22c. The external device 320 can be placed anywhere on the patient's body, preferably on a convenient and comfortable place. The external device 320 could be a wristband, and/or have the shape of a watch. It is also plausible that the external device is a mobile phone or other device not attached directly to the patient. The external device as shown in figure 22c comprises a wired transceiver 323, and an energy storage 324. It also comprises a wireless transceiver 328 and an energy transmitter 325. It further comprises a computing unit 326 and a memory 327. The feedback unit 322 in the external device 320 is configured to provide feedback related to the computing unit 326. The feedback provided by the feedback unit 322 could be visual. The external device 320 could have a display showing such visual feedback to the patient. It is equally plausible that the feedback is audible, and that the external device 320 comprises means for providing audio. The feedback given by the feedback unit 322 could also be tactile, such as vibrating. The feedback could also be provided in the form of a wireless signal WL1, WL2, WL3, WL4.

The second, third or fourth communication methods WL2, WL3, WL4 may be a wireless form of communication. The second, third or fourth communication method WL2, WL3, WL4 may preferably be a form of electromagnetic or radio-based communication. The second, third and fourth communication method WL2, WL3, WL4 may be based on telecommunication methods. The second, third or fourth communication method WL2, WL3, WL4 may comprise or be related to the items of the following list: Wireless Local Area Network (WLAN), Bluetooth, Bluetooth 5, BLE, GSM or 2G (2nd generation cellular technology), 3G, 4G or 5G.

The external device 320 may be adapted to be in electrical connection C1 with the medical device 10, using the body as a conductor. The electrical connection C1 is in this case used for conductive communication between the external device 320 and the medical device 10.

In one embodiment, the communication between controller 300 and the external device 320 over either of the communication methods WL2, WL3, WL4, C1 may be encrypted and/or decrypted with public and/or private keys, now described with reference to Figs. 22a - 22f. For example, the controller 300 may comprise a private key and a corresponding public key, and the external device 320 may comprise a private and a corresponding public key.

The controller 320 and the external device 320 may exchange public keys and the communication may thus be performed using public key encryption. The person skilled in the art may utilize any known method for exchanging the keys.

The controller may encrypt data to be sent to the external device 320 using a public key corresponding to the external device 320. The encrypted data may be transmitted over a wired, wireless or electrical communication channel C1, WL1, WL2, WL3 to the external device. The external device 320 may receive the encrypted data and decode it using the private key comprised in the external device 320, the private key corresponding to the public key with which the data has been encrypted. The external device 320 may transmit encrypted data to the controller 300. The external device 320 may encrypt the data to be sent using a public key corresponding to the private key of the controller 300. The external device 320 may transmit the encrypted data over a wired, wireless or electrical connection C1, WL1, WL2, WL3, WL4, directly or indirectly, to the controller of the implant. The controller may receive the data and decode it using the private key comprised in the controller 300.

In an alternative to the public key encryption, described with reference to figs. 22a - 22f, the data to be sent between the controller 300 of the implantable medical device 10 and an external device 320, 330 or between an external device 320, 330 and the controller 300 may be signed. In a method for sending data from the controller 300 to the external device 320, 330, the data to be sent from the controller 300 may be signed using the private key of the controller 300. The data may be transmitted over a communication channel or connection C1, WL1, WL2, WL3, WL4. The external device 320, 330 may receive the message and verify the authenticity of the data using the public key corresponding to the private key of the controller 300. In this way, the external device 320, 330 may determine that the sender of the data was sent from the controller 300 and not from another device or source.

A method for communication between external devices and the controller 300 using a combined key is now described with reference to figs. 22a - 22f. A first step of the method comprises receiving, at the implant, by a wireless transmission WL1, WL2, WL3, WL4 or otherwise, a first key from an external device 320, 330. The method further comprises receiving, at the implant, by a wireless transmission WL1, WL2, WL3, a second key. The second key may be generated by a second external device, separate from the external device 320, 330 or by another external device being a generator of the second key on behalf of the second external device 320, 330. The second key may be received at the medical device from anyone of, the external device 320, the second external device 330, and the generator of the second key. The second external device may be controlled by a caretaker, or any other stakeholder. Said another external device may be controlled by a manufacturer of the implant, or medical staff, caretaker, etc.

In case the controller 300 is receiving the second key from the external device 320, this means that the second key is routed through the external device from the second external device 330 or from another external device (generator). The routing may be performed as described herein under the tenth aspect. In these cases, the implanted medical device and/or external device(s) comprises the necessary features and functionality (described in the respective sections of this document) for performing such routing. Using the external device 320 as a relay, with or without verification from the patient, may provide an extra layer of security as the external device 320 may not need to store or otherwise handle decrypted information. As such, the external device 320 may be lost without losing decrypted information. The controller 300 a computing unit 306 configured for deriving a combined key by combining the first key and the second key with a third key held by the controller 300, for example in memory 307 of the controller 300. The third key could for example be a license number of the implant or a chip number of the implantable medical device. The combined key may be used for decrypting, by the computing unit 306, encrypted data transmitted by a wireless transmission WL1 from the external device 320 to the controller 300. Optionally, the decrypted data may be used for altering, by the computing unit 306 an operation of the implantable medical device. The altering an operation of the implantable medical device may comprise controlling or switching an active unit 302 of the implantable medical device. In some embodiments, the method further comprises at least one of the steps of, based on the decrypted data, updating a control program running in the controller 300, and operating the implantable medical device 10 using operation instructions in the decrypted data.

Methods for encrypted communication between an external device 320 and the controller 300 are provided. These methods comprise:
receiving, at the external device 320, by a wireless transceiver 328, a first key, the first key being generated by a second external device 330, separate from the external device 320 or by another external device being a generator of the second key on behalf of the second external device 330, the first key being received from anyone of the second external device 330 and the generator of the second key,
receiving, at the external device 320 by the wireless transceiver 328, a second key from the controller 300,
deriving a combined key, by a computing unit 326 of the external device 320, by combining the first key and the second key with a third key held by the external device 320 (e.g. in memory 307),
transmitting encrypted data from the implant to the external device and receiving the encrypted data at the external device by the wireless transceiver 328, and
decrypting, by the computing unit 326, the encrypted data, in the external device 320, using the combined key.

As described above, further keys may be necessary to decrypt the data. Consequently, the wireless transceiver 328 is configured for:
receiving a fourth key from a third external device,
wherein the computing unit 326 is configured for:
deriving a combined key by combining the first, second and fourth key with the third key held by the external device, and
decrypting the encrypted data using the combined key.

These embodiments further increase the security in the communication. The computing unit 326 may be configured to confirm the communication between the implantable medical device and the external device, wherein the confirmation comprises:
measuring a parameter of the patient, by the external device 320,
receiving a measured parameter of the patient, from the implantable medical device 10,
comparing the parameter measured by the implantable medical device 10 to the parameter measured by the external device 320,
performing confirmation of the connection based on the comparison, and
as a result of the confirmation, decrypting the encrypted data, in the external device, using the combined key.

The keys described in this section may in some embodiments be generated based on data sensed by sensors described herein under the twelfth or thirteenth aspect, e.g. using the sensed data as seed for the generated keys. A seed is an initial value that is fed into a pseudo random number generator to start the process of random number generation. The seed may thus be made hard to predict without access or knowledge of the physiological parameters of the patient which it is based on, providing an extra level of security to the generated keys.

Further, increased security for communication between an external device(s) and the implantable medical device is provided.

A method of communication between an external device 320 and a remote unit is now described with reference to Figs. 22a - 22f, when the implantable medical device 10 is implanted in a patient and the external device 320 is positioned external to the body of the patient. The external device 320 is adapted to be in electrical connection C1 with the controller 300, using the body as a conductor. The electrical connection C1 is used for conductive communication between the external device 320 and the implantable medical device 10. The implantable medical device 10 comprises the controller 300. Both the controller 300 and the external device 320 comprises a wireless transceiver 308, 208 for wireless communication C1 between the controller 300 and the external device 320. The wireless transceiver 308 (included in the controller 300) may in some embodiments comprise sub-transceivers for receiving data from the external device 320 and other external devices, e.g. using different frequency bands, modulation schemes etc.

In a first step of the method, the electrical connection C1 between the controller 300 and the external device 320 is confirmed and thus authenticated. The confirmation and authentication of the electrical connection may be performed as described herein under the fifth, thirteenth and fifteenth aspect. In these cases, the implant and/or external device(s) comprises the necessary features and functionality (described in the respective sections of this document) for performing such authentication. By authenticating according to these aspects, security of the authentication may be increased as it may require a malicious third party to know or gain access to either the transient physiological parameter of the patient or detect randomized sensations generated at or within the patient.

The implant may comprise a first transceiver 303 configured to be in electrical connection C1 with the external device, using the body as a conductor. The implantable medical device may comprise a first external transmitter 203 configured to be in electrical connection C1 with the implantable medical device, using the body as a conductor, and the wireless transmitter 208 configured to transmit wireless communication W1 to the controller 300. The first transmitter 323 of the external device 320 may be wired or wireless. The first transmitter 323 and the wireless transmitter 208 may be the same or separate transmitters. The first transceiver 303 of the controller 300 may be wired or wireless. The first transceiver 303 and the wireless transceiver 102 may be the same or separate transceivers.

The controller 300 may comprise a computing unit 306 configured to confirm the electrical connection between the external device 320 and the internal transceiver 303 and accept wireless communication WL1 (of the data) from the external device 320 on the basis of the confirmation.

Data is transmitted from the external device 320 to the controller 300 wirelessly, e.g. using the respective wireless transceiver 308, 208 of the controller 300 and the external device 320. Data may alternatively be transmitted through the electrical connection C1. As a result of the confirmation, the received data may be used for instructing the implantable medical device 10. For example, a control program 310 running in the controller 300 may be updated, the controller 300 may be operated using operation instructions in the received data. This may be handled by the computing unit 306.

The method may comprise transmitting data from the external device 320 to the controller 300 wirelessly comprises transmitting encrypted data wirelessly. To decrypt the encrypted data (for example using the computing unit 306), several methods may be used.

In one embodiment, a key is transmitted using the confirmed conductive communication channel C1 (i.e. the electrical connection) from the external device 320 to the controller 300. The key is received at the controller (by the first internal transceiver 303). The key is then used for decrypting the encrypted data.

In some embodiments the key is enough to decrypt the encrypted data. In other embodiments, further keys are necessary to decrypt the data. In one embodiment, a key is transmitted using the confirmed conductive communication channel C1 (i.e. the electrical connection) from the external device 320 to the controller 300. The key is received at the controller 300 (by the first internal transceiver 303). A second key is transmitted (by the wireless transceiver 208) from the external device 320 using the wireless communication WL1 and received at the controller 300 by the wireless transceiver 308. The computing unit 306 is then deriving a combined key from the key and second key and uses this for decrypting the encrypted data.

In yet other embodiments, a key is transmitted using the confirmed conductive communication channel C1 (i.e. the electrical connection) from the external device 320 to the controller 300. The key is received at the controller (by the first internal transceiver 303). A third key is transmitted from a second external device 330, separate from the external device 320, to the implant wirelessly WL2. The third key may be received by a second wireless receiver (part of the wireless transceiver 308) of the controller 300 configured for receiving wireless communication WL2 from second external device 330.

The first and third key may be used to derive a combined key by the computing unit 306, which then decrypts the encrypted data. The decrypted data is then used for instructing the implantable medical device 10 as described above.

The second external device 330 may be controlled by for example a caregiver, to further increase security and validity of data sent and decrypted by the controller 300.

It should be noted that in some embodiments, the external device is further configured to receive WL2 secondary wireless communication from the second external device 330, and transmit data received from the secondary wireless communication WL2 to the implantable medical device. This routing of data may be achieved using the wireless transceivers 308, 208 (i.e. the wireless connection WL1, or by using a further wireless connection WL4 between the controller 300 and the external device 320. In these cases, the medical device and/or external device(s) comprises the necessary features and functionality for performing such routing. Consequently, in some embodiments, the third key is generated by the second external device 330 and transmitted WL2 to the external device 320 which routes the third key to the controller 300 to be used for decryption of the encrypted data. In other words, the step of transmitting a third key from a second external device, separate from the external device, to the implant wirelessly, comprises routing the third key through the external device 320. Using the external device 320 as a relay, with or without verification from the patient, may provide an extra layer of security as the external device 320 may not need to store or otherwise handle decrypted information. As such, the external device 320 may be lost without losing decrypted information.

In yet other embodiments, a key is transmitted using the confirmed conductive communication channel C1 (i.e. the electrical connection) from the external device 320 to the controller 300. The key is received at the implant (by the first internal transceiver 303). A second key is transmitted from the external device 320 to the controller 300 wirelessly WL1, received at the at the controller 300. A third key is transmitted from the second external device, separate from the external device 320, to the controller 300 wirelessly WL4. Encrypted data transmitted from the external device 320 to the controller 300 is then decrypted using a derived combined key from the key, the second key and the third key. The external device may be a wearable external device.

The external device 320 may be a handset. The second external device 330 may be a handset. The second external device 330 may be a server. The second external device 330 may be cloud based.

In some embodiments, the electrical connection C1 between the external device 320 and the controller 300 is achieved by placing a conductive member 201, configured to be in connection with the external device 320, in electrical connection with a skin of the patient for conductive communication C1 with the medical device. In these cases, the medical device and/or external device(s) comprises the necessary features and functionality (described in the respective sections of this document) for performing such conductive communication. The communication may thus be provided with an extra layer of security in addition to the encryption by being electrically confined to the conducting path e.g. external device 320, conductive member 201, conductive connection C1, controller 300, meaning the communication will be excessively difficult to be intercepted by a third party not in physical contact with, or at least proximal to, the patient.

The keys described in this section may in some embodiments be generated based on data sensed by sensors described herein, e.g. using the sensed data as seed for the generated keys. A seed is an initial value that is fed into a pseudo random number generator to start the process of random number generation. The seed may thus be made hard to predict without access or knowledge of the physiological parameters of the patient which it is based on, providing an extra level of security to the generated keys.

Increased security for communication between an external device(s) and an implanted medical device is provided, now described with reference to figs. 22a - 22f.

In these embodiments, a method for communication between an external device 320 and the implantable controller 300 is provided. The wireless transceiver 308 (included in the controller 300) may in some embodiments comprise sub-transceivers for receiving data from the external device 320 and other external devices 330, e.g. using different frequency bands, modulation schemes etc.

A first step of the method comprises receiving, at the implanted medical device, by a wireless transmission WL1 or otherwise, a first key from an external device 320. The method further comprises receiving, at the implanted medical device, by a wireless transmission WL1, WL2, WL3, a second key. The second key may be generated by a second external device 330, separate from the external device 320 or by another external device being a generator of the second key on behalf of the second external device 330. The second key may be received at the implanted medical device from anyone of, the external device 320, the second external device 330, and a generator of the second key. The second external device 330 may be controlled by a caretaker, or any other stakeholder. Said another external device may be controlled by a manufacturer of the medical device, or medical staff, caretaker, etc.

In case the medical device is receiving the second key from the external device 320, this means that the second key is routed through the external device from the second external device 330 or from the another external device (generator). In these cases, the medical device and/or external device(s) comprises the necessary features and functionality (described in the respective sections of this document) for performing such routing. Using the external device 320 as a relay, with or without verification from the patient, may provide an extra layer of security as the external device 320 may not need to store or otherwise handle decrypted information. As such, the external device 320 may be lost without losing decrypted information.

The controller 300 comprises a computing unit 306 configured for deriving a combined key by combining the first key and the second key with a third key held by the controller 300, for example in memory 307 of the controller. The combined key may be used for decrypting, by the computing unit 306, encrypted data transmitted by a wireless transmission WL1 from the external device 320 to the controller 300. Optionally, the decrypted data may be used for altering, by the computing unit 306 an operation of the implantable medical device 10. The altering an operation of the implantable medical device may comprise controlling or switching an active unit 302 of the medical device. In some embodiments, the method further comprises at least one of the steps of, based on the decrypted data, updating a control program running in the implant, and operating the implantable medical device 10 using operation instructions in the decrypted data.

In some embodiments, further keys are necessary to derive a combined key for decrypting the encrypted data received at the controller 300. In these embodiments, the first and second key are received as described above. Further, the method comprises receiving, at the implanted medical device, a fourth key from a third external device, the third external device being separate from the external device, deriving a combined key by combining the first, second and fourth key with the third key held by the controller 300, and decrypting the encrypted data, in the controller 300, using the combined key. Optionally, the decrypted data may be used for altering, by the computing unit 306, an operation of the implanted medical device as described above. In some embodiments, the fourth key is routed through the external device from the third external device.

In some embodiments, further security measures are needed before using the decrypted data for altering, by the computing unit 306, an operation of the implantable medical device. For example, an electrical connection C1 between the implantable medical device and the external device 320, using the body as a conductor, may be used for further verification of validity of the decrypted data. The electrical connection C1 may be achieved by placing a conductive member 201, configured to be in connection with the external device, in electrical connection with a skin of the patient for conductive communication C1 with the implantable medical device. The communication may thus be provided with an extra layer of security in addition to the encryption by being electrically confined to the conducting path e.g. external device 320, conductive member 201, conductive connection C1, controller 300, meaning the communication will be excessively difficult to be intercepted by a third party not in physical contact with, or at least proximal to, the patient.

Accordingly, in some embodiments, the method comprising confirming the electrical connection between the controller 300 and the external device 320, and as a result of the confirmation, altering an operation of the implantable medical device based on the decrypted data. The confirmation and authentication of the electrical connection may be performed as described herein under the general features section. In these cases, the implantable medical device and/or external device(s) 320 comprises the necessary features and functionality (described in the respective sections of this document) for performing such authentication. By authenticating according to these aspects, security of the authentication may be increased as it may require a malicious third party to know or gain access to either the transient physiological parameter of the patient or detect randomized sensations generated at or within the patient.

In some embodiments, the confirmation of the electrical connection comprises: measuring a parameter of the patient, by e.g. a sensor of the implantable medical device 10, measuring the parameter of the patient, by the external device 320, comparing the parameter measured by the implantable medical device to the parameter measured by the external device 320, and authenticating the connection based on the comparison. As mentioned above, as a result of the confirmation, an operation of the implantable medical device may be altered based on the decrypted data.

Further methods for encrypted communication between an external device 320 and an implantable medical device 10 are provided. These methods comprise:
receiving, at the external device 320 by a wireless transceiver 328, a first key, the first key being generated by a second external device 330, separate from the external device 320 or by another external device being a generator of the second key on behalf of the second external device 320, the first key being received from anyone of the second external device 330 and the generator of the second key,
receiving, at the external device 320 by the wireless transceiver 328, a second key from the controller 300,
deriving a combined key, by a computing unit 326 of the external device 320, by combining the first key and the second key with a third key held by the external device 320 (e.g. in memory 327),
transmitting encrypted data from the implant to the external device and receiving the encrypted data at the external device by the wireless transceiver 328, and
decrypting, by the computing unit 326, the encrypted data, in the external device 320, using the combined key.

As described above, further keys may be necessary to decrypt the data. Consequently, the wireless transceiver 328 is configured for:
receiving a fourth key from a third external device,
wherein the computing unit 326 is configured for:
deriving a combined key by combining the first, second and fourth key with the third key held by the external device, and
decrypting the encrypted data using the combined key.

In some embodiments, the communication between the controller 300 and the external device 320 needs to be confirmed (authenticated) before decrypting the data. In these cases, the implantable medical device and/or external device(s) comprises the necessary features and functionality (described in the respective sections of this document) for performing such authentication.

These embodiments further increase the security in the communication. **In** these embodiments the computing unit 326 is configured to confirm the communication between the implantable medical device and the external device, wherein the confirmation comprises:
measuring a parameter of the patient, by the external device 320,
receiving a measured parameter of the patient, from the implantable medical device 10,
comparing the parameter measured by the implantable medical device 320 to the parameter measured by the external device 320,
performing confirmation of the connection based on the comparison, and
as a result of the confirmation, decrypting the encrypted data, in the external device, using the combined key.

One or more of the first, second and third key may comprise a biometric key.

The keys described in this section may in some embodiments be generated based on data sensed by sensors, e.g. using the sensed data as seed for the generated keys. A seed is an initial value that is fed into a pseudo random number generator to start the process of random number generation. The seed may thus be made hard to predict without access or knowledge of the physiological parameters of the patient which it is based on, providing an extra level of security to the generated keys.

Further, increased security for communication between an external device(s) 320, 330 and an implantable medical device is provided, described with reference to Figs. 22a - 22f. The system being configured for enabling communication between an external device 320 and the controller 300 implanted in a patient. The system comprises a conductive member 321 configured to be in connection (electrical/conductive or wireless or otherwise) with the external device, the conductive member 321 being configured to be placed in electrical connection with a skin of the patient for conductive communication C1 with the implantable medical device 10. By using a conductive member 321 as defined herein, an increased security for communication between the external device and the implantable medical device may be achieved. For example, when a sensitive update of a control program of the controller 300 is to be made, or if sensitive data regarding physical parameters of the patient is to be sent to the external device 320 (or otherwise), the conductive member 321 may ensure that the patient is aware of such communication and actively participate in validating that the communication may take place. The conductive member may, by being placed in connection with the skin of the patient, open the conductive communication channel C1 between the external device and the controller to be used for data transmission.

Electrical or conductive communication, such as this or as described under the other embodiments, may be very hard to detect remotely, or at least relatively so, in relation to wireless communications such as radio transmissions. Direct electrical communication may further safeguard the connection between the implantable medical device 10 and the external device 320 from electromagnetic jamming i.e. high-power transmissions other a broad range of radio frequencies aimed at drowning other communications within the frequency range. Electrical or conductive communication will be excessively difficult to be intercepted by a third party not in physical contact with, or at least proximal to, the patient, providing an extra level of security to the communication.

In some embodiments, the conductive member comprises a conductive interface for connecting the conductive member to the external device.

In some embodiments, the conductive member 201 is a device which is plugged into the external device 320, and easily visible and identifiable for simplified usage by the patient. In other embodiments, the conductive member 321 is to a higher degree integrated with the external device 320, for example in the form of a case of the external device 320 comprising a capacitive area configured to be in electrical connection with a skin of the patient. In one example, the case is a mobile phone case (smartphone case) for a mobile phone, but the case may in other embodiments be a case for a personal computer, or a body worn camera or any other suitable type of external device as described herein. The case may for example be connected to the phone using a wire from the case and connected to the headphone port or charging port of the mobile phone.

The conductive communication C1 may be used both for communication between the controller 300 and the external device 320 in any or both directions. Consequently, according to some embodiments, the external device 320 is configured to transmit a conductive communication (conductive data) to the controller 300 via the conductive member 321.

According to some embodiments, the controller 300 is configured to transmit a conductive communication to the external device 320. These embodiments start by placing the conductive member 321, configured to be in connection with the external device 320, in electrical connection with a skin of the patient for conductive communication C1 with the controller 300. The conductive communication between the external device 320 and the controller 300 may follow an electrically/conductively confined path comprising e.g. the external device 320, conductive member 321, conductive connection C1, controller 300.

For the embodiments when the external device 320 transmits data to the controller, the communication may comprise transmitting a conductive communication to the controller 300 by the external device 320.

The transmitted data may comprise instructions for operating the implantable medical device 10. Consequently, some embodiments comprise operating the implantable medical device 10 using operation instructions, by an internal computing unit 306 of the controller 300, wherein the conductive communication C1 comprises instructions for operating the implantable medical device 10. The operation instruction may for example involve adjusting or setting up (e.g. properties or functionality of) the active unit 302 of the implantable medical device 10.

The transmitted data may comprise instructions for updating a control program 310 stored in memory 307 of the controller 300. Consequently, some embodiments comprise updating the control program 310 running in the controller 300, by the internal computing unit 306 of the implantable medical device, wherein the conductive communication comprises instructions for updating the control program 310.

For the embodiments when the controller 300 transmits data to the external device 320, the communication may comprise transmitting conductive communication C1 to the external device 320 by the controller 300. The conductive communication may comprise feedback parameters. Feedback parameters could include battery status, energy level at the controller, the fluid level of the hydraulic restriction device, number of operations that the restriction device has performed, properties, version number etc. relating to functionality of the implantable medical device 10. In other embodiments, the conductive communication C1 comprises data pertaining to least one physiological parameter of the patient, such as blood pressure etc. The physiological parameter(s) may be stored in memory 307 of the controller 300 or sensed in prior (in real time or with delay) to transmitting the conductive communication C1. Consequently, in some embodiments, the implantable medical device 10 comprises a sensor 150 for sensing at least one physiological parameter of the patient, wherein the conductive communication comprises said at least one physiological parameter of the patient.

To further increase security of the communication between the controller 300 and the external device 320, different types of authentication, verification and/or encryption may be employed. In some embodiments, the external device 320 comprises a verification unit 340. The verification unit 340 may be any type of unit suitable for verification of a user, i.e. configured to receive authentication input from a user, for authenticating the conductive communication between the implantable medical device and the external device. In some embodiments, the verification unit and the external device comprises means for collecting authentication input from the user (which may or may not be the patient). Such means may comprise a fingerprint reader, a retina scanner, a camera, a GUI for inputting a code, a microphone, device configured to draw blood, etc. The authentication input may thus comprise a code or any be based on a biometric technique selected from the list of: a fingerprint, a palm vein structure, image recognition, face recognition, iris recognition, a retinal scan, a hand geometry, and genome comparison. The means for collecting the authentication input may alternatively be part of the conductive member which comprise any of the above examples of functionality, such as a fingerprint reader or other type of biometric reader.

In some embodiments, the security may thus be increased by receiving an authentication input from a user by the verification unit 340 of the external device 320, and authenticating the conductive communication between the controller 300 and the external device using the authentication input. Upon a positive authentication, the conductive communication channel C1 may be employed for comprising transmitting a conductive communication to the controller 300 by external device 320 and/or transmitting a conductive communication to the external device 320 by the controller 300. In other embodiments, a positive authentication is needed prior to operating the implantable medical device 10 based on received conductive communication, and/or updating a control program running in the controller 300 as described above.

Figs. 22a - 22f further shows an implantable medical device 10 implanted in a patient and being connected to a sensation generator 381.

The sensation generator 381 may be configured to generate a sensation. The sensation generator 381 may be contained within the implantable medical device 10 or be a separate unit. The sensation generator 381 may be implanted. The sensation generator 381 may also be located so that it is not implanted as such but still is in connection with a patient so that only the patient may experience sensations generated. The controller 300 is configured for storing authentication data, related to the sensation generated by the sensation generator 381.

The controller 300 is further configured for receiving input authentication data from the external device 320. Authentication data related to the sensation generated may by stored by a memory 307 of the controller 300. The authentication data may include information about the generated sensation such that it may be analyzed, e.g. compared, to input authentication data to authenticate the connection, communication or device. Input authentication data relates to information generated by a patient input to the external device 320. The input authentication data may be the actual patient input or an encoded version of the patient input, encoded by the external device 320. Authentication data and input authentication data may comprise a number of sensations or sensation components.

The authentication data may comprise a timestamp. The input authentication data may comprise a timestamp of the input from the patient. The timestamps may be a time of the event such as the generation of a sensation by the sensation generator 381 or the creation of input authentication data by the patient. The timestamps may be encoded. The timestamps may feature arbitrary time units, i.e. not the actual time. Timestamps may be provided by an internal clock 360 of the controller 300 and an external clock 362 of the external device 320. The clocks 360, 362 may be synchronized with each other. The clocks 360, 362 may be synchronized by using a conductive connection C1 or a wireless connection WL1 for communicating synchronization data from the external device 320, and its respective clock 362, to the controller 300, and its respective clock 360, and vice versa. Synchronization of the clocks 360, 362 may be performed continuously and may not be reliant on secure communication.

Authentication of the connection may comprise calculating a time difference between the timestamp of the sensation and the timestamp of the input from the patient, and upon determining that the time difference is less than a threshold, authenticating the connection. An example of a threshold may be 1s. The analysis may also comprise a low threshold as to filter away input from the patient that is faster than normal human response times. The low threshold may e.g. be 50ms.

Authentication data may comprise a number of times that the sensation is generated by the sensation generator, and wherein the input authentication data comprises an input from the patient relating to a number of times the patient detected the sensation. Authenticating the connection may then comprise: upon determining that the number of times that the authentication data and the input authentication data are equal, authenticating the connection.

A method of authenticating the connection between an implantable medical device 10 implanted in a patient, and an external device 320 according includes the following steps.

Generating, by a sensation generator 381, a sensation detectable by a sense of the patient. The sensation may comprise a plurality of sensation components. The sensation or sensation components may comprise a vibration (e.g. a fixed frequency mechanical vibration), a sound (e.g. a superposition of fixed frequency mechanical vibrations), a photonic signal (e.g. a non-visible light pulse such as an infra-red pulse), a light signal (e.g. a visual light pulse), an electric signal (e.g. an electrical current pulse) or a heat signal (e.g. a thermal pulse). The sensation generator may be implanted, configured to be worn in contact with the skin of the patient or capable of creating sensation without being in physical contact with the patient, such as a beeping alarm.

Sensations may be configured to be consistently felt by a sense of the patient while not risking harm to or affecting internal biological processes of the patient.

The sensation generator 381, may be contained within the controller 300 or be a separate entity connected to the controller 300. The sensation may be generated by a motor (denoted as MO in several embodiments shown herein) of the implantable medical device 10, wherein the motor being the sensation generator 381. The sensation may be a vibration, or a sound created by running the motor. The sensation generator 381 may be located close to a skin of the patient and thus also the sensory receptors of the skin. Thereby the strength of some signal types may be reduced.

Storing, by the controller 300, authentication data, related to the generated sensation.

Providing, by the patient input to the external device, resulting in input authentication data. Providing the input may e.g. comprise an engaging an electrical switch, using a biometric input sensor or entry into digital interface running on the external device 320 to name just a few examples.

Transmitting the input authentication data from the external device to the controller 300. If the step was performed, the analysis may be performed by the controller 300.

Transmitting the authentication data from the implantable medical device 10 to the external device 320. If the step was performed, the analysis may be performed by the external device 320. The wireless connection WL1 or the conductive connection C1 may be used to transmit the authentication data or the input authentication data.

Authenticating the connection based on an analysis of the input authentication data and the authentication data e.g. by comparing a number of sensations generated and experienced or comparing timestamps of the authentication data and the input authentication data. If step was performed, the analysis may be performed by the implantable medical device 10.

Communicating further data between the controller 300 and the external device 320 following positive authentication. The wireless connection WL1 or the conductive connection C1 may be used to communicate the further data. The further data may comprise data for updating a control program 310 running in the controller 300 or operation instructions for operating the implantable medical device 10. The further data may also comprise data sensed by a sensor 150 connected to the controller 300.

If the analysis was performed by the controller 300, the external device 320 may continuously request or receive, information of an authentication status of the connection between the controller 300 and the external device 320, and upon determining, at the external device 320, that the connection is authenticated, transmitting further data from the external device 320 to the controller 300.

If the analysis was performed by the external device 320, the controller 300 may continuously request or receive, information of an authentication status of the connection between the controller 300 and the external device 320, and upon determining, at the controller 300, that the connection is authenticated, transmitting further data from the controller 300 to the external device 320.

A main advantage of authenticating a connection according to this method is that only the patient may be able to experience the sensation. Thus, only the patient may be able to authenticate the connection by providing authentication input corresponding to the sensation generation.

The sensation generator 381, sensation, sensation components, authentication data, input authentication data, and further data may be further described herein. In these cases, the implantable medical device 10 and/or external device(s) comprises the necessary features and functionality (described in the respective sections of this document). Further information and definitions can be found in this document in conjunction with the other aspects.

The method may further comprise transmitting further data between the controller 300 and the external device, wherein the further data is used or acted upon, only after authentication of the connection is performed.

The analysis or step of analyzing may be understood as a comparison or a step of comparing.

In one method, increased security for communication between an external device(s) and an implanted controller is provided. Figs. 22a - 22f show an implantable medical device 10 comprising a controller 300 and an external device 320 which may form a system.

The controller 300 comprises a transceiver 308, 303 configured to establish a connection with an external device 320, i.e. with a corresponding transceiver 328, 323. The connection may be an electrical connection C1 using the transceivers 303, 323, or a wireless connection WL1 using the transceivers 308, 328. The controller 300 further comprises a computing unit 306 configured to verify the authenticity of instructions received at the transceiver 308, 303 from the external device 320. In this aspect, the concept of using previously transmitted instructions for verifying a currently transmitted instructions are employed. Consequently, the transmitting node (in this case the external device) need to be aware of previously instructions transmitted to the implantable medical device, which reduces the risk of a malicious device instructing the implant without having the authority to do so.

In an embodiment, the computing unit 306 is configured to verify the authenticity of instructions received at the transceiver 308, 303 by extracting a previously transmitted set of instructions from a first combined set of instructions received by the transceiver. The external device 320 may thus comprise an external device comprising a computing unit 326 configured for: combining a first set of instructions with a previously transmitted set of instructions, forming a combined set of instructions, and transmitting the combined set of instructions to the implantable medical device. The previously transmitted set of instructions, or a representation thereof, may be stored in memory 327 of the external device 320.

The combined set of instructions may have a data format which facilitates such extraction, for example including metadata identifying data relating to the previously transmitted set of instructions in the combined set of instructions. In some embodiments, the combined set of instructions comprises the first set of instructions and a cryptographic hash of the previously transmitted set of instructions. Consequently, the method comprises combining, at the external device, a first set of instructions with a previously transmitted set of instructions, forming a first combined set of instructions. A cryptographic hash function is a special class of hash function that has certain properties which make it suitable for use in cryptography. It is a mathematical algorithm that maps data of arbitrary size to a bit string of a fixed size (a hash) and is designed to be a one-way function, that is, a function which is infeasible to invert. Examples include MD5, SHA1, SHA 256, etc. Increased security is thus achieved.

The first combined set of instructions is then transmitted to the implanted controller 300, where it is received by e.g. the transceiver 303, 308. The first combined set of instructions may be transmitted to the implantable medical device using a proprietary network protocol. The first combined set of instructions may be transmitted to the controller 300 using a standard network protocol. In these cases, the controller 300 and/or external device(s) comprises the necessary features and functionality (described in the respective sections of this document) for performing transmission of data. By using different communication protocols, at the external device 320, for communication with the controller 300 and with a second external device 330, an extra layer of security is added as the communication between controller 300 and the external device 320 may be made less directly accessible to remote third parties.

At the controller 300, the computing unit 306 verifies the authenticity of the received first combined set of instructions, by: extracting the previously transmitted set of instructions from the first combined set of instructions, and comparing the extracted previously transmitted set of instructions with previously received instructions stored in the implantable medical device.

Upon determining that the extracted previously transmitted set of instructions equals the previously received instructions stored in the controller 300, the authenticity of the received first combined set of instructions may be determined as valid, and consequently, the first set of instructions may be safely run at the controller 300, and the first combined set of instructions may be stored in memory 307 of the controller 300, to be used for verifying a subsequent received set of instructions.

**In** some embodiments, upon determining by the internal computing unit 306 that the extracted previously transmitted set of instructions differs from the previously received instructions stored in the controller 300, feedback related to an unauthorized attempt to instruct the implantable medical device 10 may be provided. For example, the transceiver 308, 303 may send out a distress signal to e.g. the external device 320 or to any other connected devices. The controller 300 may otherwise inform the patient that something is wrong by e.g. vibration or audio. The implantable medical device 10 may be run in safe mode, using a preconfigured control program which is stored in memory 307 of the controller 300 and specifically set up for these situations, e.g. by requiring specific encoding to instruct the implantable medical device 10, or only allow a predetermined device (e.g. provided by the manufacturer) to instruct the implantable medical device 10. **In** some embodiments, when receiving such feedback at the external device 320, the external device 320 retransmits the first combined set of instructions again, since the unauthorized attempt may in reality be an error in transmission (where bits of the combined set of instructions are lost in transmission), and where the attempt to instruct the implantable medical device 10 is indeed authorized.

The step of comparing the extracted previously transmitted set of instructions with previously received instructions stored in the controller 300 may be done in different ways. For example, the step of comparing the extracted previously transmitted set of instructions with previously received instructions stored in the controller 300 comprises calculating a difference between the extracted previously transmitted set of instructions with previously received instructions stored in the controller 300, and comparing the difference with a threshold value, wherein the extracted previously transmitted set of instructions is determined to equal the previously received instructions stored in the controller 300 in the case of the difference value not exceeding the threshold value. This embodiment may be used when received instructions is stored in clear text, or a representation thereof, in the controller 300, and where the combined set of instructions, transmitted from the external device also includes such a representation of the previously transmitted instructions. This embodiment may be robust against error in transmission where bits of information are lost or otherwise scrambled.

In other embodiments, the combined set of instructions comprises the first set of instructions and a cryptographic hash of the previously transmitted set of instructions, wherein the method further comprises, at the controller 300, calculating a cryptographic hash of the previously received instructions stored in the controller 300 and comparing the calculated cryptographic hash to the cryptographic hash included in the first combined set of instructions. This embodiment provides increased security since the cryptographic hash is difficult to decode or forge.

The above way of verifying the authenticity of received instructions at the controller 300 may be iteratively employed for further sets if instructions.

To further increase security, the transmission of a first set of instructions, to be stored at the controller 300 for verifying subsequent sets of combined instructions, where each set of received combined instructions will comprise data which in some form will represent, or be based on, the first set of instruction, may be performed.

In one example, the external device 320 may be adapted to communicate with the controller 300 using two separate communication methods. A communication range of a first communication method WL1 may be less than a communication range of a second communication method WL2. A method may comprise the steps of: sending a first part of a key from the external device 320 to the controller 300, using the first communication method WL1 and sending a second part of the key from the external device 320 to the controller 300, using the second communication method WL2. The method may further comprise deriving, in the controller 300, a combined key from the first part of the key and the second part of the key and decrypting the encrypted data, in the controller 300, using the combined key. The encrypted data may also be sent from the external device 320 to the controller 300 using the second communication method WL2. The method may then further comprise confirming an electrical connection C1 between the controller 300 and the external device 320 and as a result of the confirmation, decrypting the encrypted data in the controller 300 and using the decrypted data for instructing the controller 300.

The method may also comprise placing a conductive member 321, configured to be in connection with the external device 320, in electrical connection with a skin of the patient for conductive communication with the controller 300. By means of the electrical connection an extra layer of security is added as a potential hacker would have to be in contact with the patient to access or affect the operation of the implantable medical device 10.

Using a plurality of communication methods, may increase the security of the authentication and the communication with the implantable medical device 10 as more than one channel for communication may need to be hacked or hijacked by an unauthorized entity to gain access to the implantable medical device 10 or the communication.

The electrical connection C1 the conductive member 321 and conductive communication may be further described herein in the general definitions section. In these cases, the controller 300 and/or external device 320 comprise the necessary features and functionality (described in the respective sections of this document).

It should also be noted that any one of the first and second communication methods WL1, WL2 may be needed to be confirmed in order to decrypt the encrypted data in the controller 300 and using the decrypted data for instructing the implantable medical device 10.

The method may further comprise the step of wirelessly receiving, at the controller 300, a third part of the key from the second external device 330. In this case, the combined key may be derived from the first part of the key, the second part of the key and the third part of the key.

The first communication method WL1 may be a wireless form of communication. The first communication method WL1 may preferably be a form of electromagnetic or radio-based communication however, other forms of communication are not excluded. The first communication method WL1 may comprise or be related to the items of the following list: Radio-frequency identification (RFID), Bluetooth, Bluetooth 5, Bluetooth Low Energy (BLE), Near Field Communication (NFC), NFC-V, Infrared (IR) based communication, Ultrasound based communication.

RFID communication may enable the use of a passive receiver circuit such as those in a RFID access/key or payment card. IR based communication may comprise fiber optical communication and IR diodes. IR diodes may alternatively be used directly, without a fiber, such as in television remote control devices. Ultrasound based communication may be based on the non-invasive, ultrasound imaging found in use for medical purposes such as monitoring the development of mammal fetuses.

The first communication method WL1 may use a specific frequency band. The frequency band of the first communication method WL1 may have a center frequency of 13.56 MHz or 27.12 MHz. These bands may be referred to as industrial, scientific and medical (ISM) radio bands. Other ISM bands not mentioned here may also be utilized for the communication methods WL1, WL2. A bandwidth of the 13.56 MHz centered band may be 14 kHz and a bandwidth of the 27.12 MHz centered band may be 326 kHz.

The communication range of the first communication method WL1 may be less than 10 meters, preferably less than 2 meters, more preferably less than 1 meter and most preferably less than 20 centimeters. The communication range of the first communication method WL1 may be limited by adjusting a frequency and/or a phase of the communication. Different frequencies may have different rates of attenuation. By implementing a short communication range of the first communication method, security may be increased since it may be ensured or made probable that the external device is under control of the patient (holding the external device close to the implant)

The communication range of the first communication method WL1 should be evaluated by assuming that a patient's body, tissue, and bones present the propagation medium. Such a propagation medium may present different attenuation rates as compared to a free space of an air-filled atmosphere or a vacuum.

By restricting the communication range, it may be established that the external device communicating with the implanted controller 300 is in fact on, or at least proximal to, the patient. This may add extra security to the communication.

The second communication method WL2 may be a wireless form of communication. The second communication method WL2 may preferably be a form of electromagnetic or radio-based communication. The second communication method WL2 may be based on telecommunication methods. The second communication method WL2 may comprise or be related to the items of the following list: Wireless Local Area Network (WLAN), Bluetooth, Bluetooth 5, BLE, GSM or 2G (2nd generation cellular technology), 3G, 4G, 5G.

The second communication method WL2 may utilize the ISM bands as mentioned in the above for the first communication method WL1.

A communication range of the second communication method WL2 may be longer than the communication range of the first communication method WL1. The communication range of the second communication method WL2 may preferably be longer than 10 meters, more preferably longer than 50 meters, and most preferably longer than 100 meters.

Encrypted data may comprise instructions for updating a control program 310 running in the implantable medical device 10. Encrypted data may further comprise instructions for operating the implantable medical device 10.

In one embodiment, the implantable medical device 10 may transmit data to an external device 320 which may add an additional layer of encryption and transmit the data to a second external device 330, described with reference to figs. 22a - 22f. By having the external device add an additional layer of encryption, less computing resources may be needed in the implanted controller 300, as the controller 300 may transmit unencrypted data or data encrypted using a less secure or less computing resource requiring encryption. In this way, data can still be relatively securely transmitted to a third device. The transmission of data can be performed using any of the method described herein in addition to the method or in the system described below.

Thus, in an embodiment, a system is provided. The system comprises an implantable medical device 10 comprising a controller 300 configured to transmit data from the body of the patient to an external device 320, and an encryption unit 382 for encrypting the data to be transmitted. The system further comprises an external device 320 configured to receive the data transmitted by the controller 300, encrypt the received data using a first key and transmit the encrypted received data to a third external device 330. The encryption can be performed using any of the keys described above or below. In some embodiments, the external device 320 is configured to decrypt the data received from the controller 300 before encrypting and transmitting the data. Alternatively, the external device 320 may encrypt and transmit the data received from the controller 300 without decrypting it first.

In one example, the encryption unit 382 is configured to encrypt the data to be transmitted using a second key. The first key or the second key may, for example, information specific to the implantable medical device 10, a secret key associated with the external device 320, an identifier of the implantable medical device 10 or an identifier of the controller 300. The second key could be a key transmitted by the external device 320 to the controller 300. In some examples, the second key is a combined key comprising a third key received by the controller 300 from the external device 320.

The first key may be a combined key comprising a fourth key, wherein the fourth key is received by the external device 320 from a fourth device. The fourth device may be a verification unit, either comprised in the external device, or external to the external device and connected to it. The verification unit may have a sensor 350 for verification, such as a fingerprint sensor. More details in regard to this will be described below. Alternatively, the verification unit may be a generator, as described above.

The system may be configured to perform a method for transmitting data using a sensed parameter. The method may comprise transmitting a parameter measured by the external device 320 from the external device 320 to the controller 300. In this case, the comparison of the parameter of the patient measured by the external device 320 and the parameter of the patient measured by the controller 300 may be performed by the controller 300. The implantable medical device 10 may comprise a first sensor 150 for measuring the parameter of the patient at the implantable medical device 10. The external device 320 may comprise an external sensor 350 for measuring the parameter of the patient at the external device 320.

Authentication of the connection between the controller 300 and the external device 320 may be performed automatically without input, authentication, or verification from a user or patient. This is because the comparison of parameters measured internally and externally, by the internal and external sensors 351, 350 respectively may be enough to authenticate the connection. This may typically be the case when the parameter of the patient is related to an automatically occurring physiological function of the patient such as e.g. a pulse of the patient. Certain types of authentication may however require actions from the patient, e.g. having the patient perform specific movements.

In the embodiments described herein, the controller 300 may comprise or be connected to a sensation generator 381 as described above. In response to an event in the implantable medical device, such as a reset, a restart, receipt of new instructions, receipt of a new configuration or update, installation or activation of new instructions or configuration or update, the controller 300 may be configured to cause the sensation generator 381 to generate a sensation detectable by the patient in which the implantable medical device 10 is implanted. In some examples, the user may after the sensation verify an action, for example via a user interface of an external device 320.

The implantable medical device 10 may further implement a method for improving the security of the data transmitted from the controller 300. The method, for encrypted communication between a controller 300, when implanted in a patient's body, and an external device 320, comprises encoding or encrypting, by the controller 300 or a processor 306 comprised in or connected to the controller 300, data relating to the implantable medical device 10 or the operation thereof; transmitting, by the controller 300, the data; receiving, by a second communication unit comprised the external device 320, the data; encrypting, by the external device 320, the data using an encryption key to obtain encrypted data; and transmitting the encrypted data to a third external device 330. In this way, the external device 320 may add or exchange the encryption, or add an extra layer of encryption, to the data transmitted by the controller 300. When the controller 300 encodes the data to be transmitted it may be configured to not encrypt the data before transmitting, or only using a light-weight encryption, thus not needing as much processing power as if the controller were to fully encrypt the data before the transmission.

The encrypting, by the controller 300, may comprise encrypting the data using a second key. The encryption using the second key may be a more light-weight encryption than the encryption performed by the external device using the second key, i.e. an encryption that does not require as much computing resources as the encryption performed by the external device 320.

The first or the second key may comprise a private key exchanged as described above with reference to encryption and authentication, or the first or the second key may comprise an information specific to the implantable medical device 10, a secret key associated with the external device, an identifier of the implantable medical device 10 or an identifier of the controller 300. They may be combined keys as described in this description, and the content of the keys, any combination of keys, and the exchange of a key or keys is described in the encryption and/or authentication section.

In an embodiment, the implantable medical device 10 comprises at least one sensor for sensing at least one physiological parameter of the patient or a functional parameter of the implantable medical device 10, now described with reference to figs. 22a - 22f. The sensor 351 may, for example, be a pressure sensor, an electrical sensor, a clock, a temperature sensor, a motion sensor, an optical sensor, a sonic sensor, an ultrasonic sensor. The sensor 351 is configured to periodically sense the parameter and the controller 300 is configured to, in response to the sensed parameter being above a predetermined threshold, wirelessly broadcast information relating to the sensed parameter. The controller 300 may be configured to broadcast the information using a short to mid-range transmitting protocol, such as a Radio Frequency type protocol, a RFID type protocol, a WLAN type protocol, a Bluetooth type protocol, a BLE type protocol, a NFC type protocol, a 3G/4G/5G type protocol, or a GSM type protocol.

The controller of the implant may be connected to the sensor 351 and be configured to anonymize the information before it is transmitted. The transmission of data may also be called broadcasting of data.

In addition to or as an alternative to transmitting the data when the sensed parameter is above a predetermined threshold, the controller 300 may be configured to broadcast the information periodically. The controller 300 may be configured to broadcast the information in response to a second parameter being above a predetermined threshold. The second parameter may, for example, be related to the controller 300 itself, such as a free memory or free storage space parameter, or a battery status parameter. When the implantable medical device 10 comprises an implantable energy storage unit and an energy storage unit indicator, the energy storage unit indicator is configured to indicate a functional status of the implantable energy storage unit and the indication may be comprised in the transmitted data. The functional status may indicate at least one of charge level and temperature of the implantable energy storage unit.

In some embodiments the external device 320 is configured to receive the broadcasted information, encrypt the received information using an encryption key and transmit the encrypted received information. In this way, the external device 320 may add an additional layer of encryption or exchange the encryption performed by the controller 300.

In an embodiment, the controller 300 is configured to transmit the data using the body of the patient as a conductor C1, and the external device 320 is configured to receive the data via the body. Alternatively, or in combination, the controller 300 of the implant is configured to transmit the data wirelessly to the external device WL2.

Thus, the controller 300 may implement a method for transmitting data from the controller 300 comprising a processor 306, comprising: obtaining sensor measurement data via a sensor 150 connected to or comprised in the controller 300, the sensor measurement relating to at least one physiological parameter of the patient or a functional parameter of the implantable medical device 10, and transmitting by the controller 300 the sensor measurement data in response to the sensor measurement being above a predetermined threshold, wherein the sensor 150 is configured to periodically sense the parameter. The method may further comprise broadcasting the sensor measurement data, to be received by an external device 320. The transmitting or broadcasting may comprise using at least one of a Radio Frequency type protocol, RFID type protocol, WLAN type protocol, Bluetooth type protocol, BLE type protocol, NFC type protocol, 3G/4G/5G type protocol, or a GSM type protocol.

The method may further comprise, at the processor 306, anonymizing, by the processor, the sensor measurement data before it is transmitted, or encrypting the sensor measurement data, using an encryptor 382 comprised in the processing unit 306, before it is transmitted. The transmitting of the data may further comprise to encode the data before the transmitting. The type of encoding may be dependent on the communication channel or the protocol used for the transmission.

The transmitting may be performed periodically, or in response to a signal received by the processor, for example, by an internal part of the implantable medical device 10 such as a sensor 150, or by an external device 320.

The parameter may, for example, be at least one of a functional parameter of the implantable medical device 10 (such as a battery parameter, a free memory parameter, a temperature, a pressure, an error count, a status of any of the control programs, or any other functional parameter mentioned in this description) or a parameter relating to the patient (such as a temperature, a blood pressure, or any other parameter mentioned in this description). In one example, the implantable medical device 10 comprises an implantable energy storage unit 40 and an energy storage unit indicator 304c, and the energy storage unit indicator 304c is configured to indicate a functional status of the implantable energy storage unit 40, and the sensor measurement comprises data related to the energy storage unit indicator.

In one example, the transmitting comprises transmitting the sensor measurement to an internal processor 306 configured to cause a sensation generator 381 to cause a sensation detectable by the patient in which the implantable energized medical device 10 is implanted.

The method may be implemented in a system comprising the implantable energized medical device 10 and an external device 320, and further comprise receiving the sensor measurement data at the external device 320, and, at the external device 320, encrypting the sensor measurement data using a key to obtain encrypted data, and, transmitting the encrypted data. The transmitting may, for example, be performed wirelessly WL3 or conductively C1.

In the examples or embodiments transmitting data from or to the implantable medical device 10, the following method may be implanted in order to verify the integrity of the data, described with reference to figs. 22a - 22b. By verifying the integrity of the data, an external device 320 or a processor 306 comprised in the controller 300 may verify that the data has not been corrupted or tampered with during the transmission. In some examples, data integrity for data communicated between a controller 300 and an external device 320 or between an external device 320 and the controller 300 may be performed using a cyclic redundancy check.

Thus, in a first example, a method for evaluating a parameter of a controller 300 implanted in a patient is described. The controller 300 comprises a processor 306 and a sensor 150 for measuring the parameter. The method comprises measuring, using the sensor 150, the functional parameter to obtain measurement data; establishing a connection between the internal controller 300 and an external device 320 configured to receive data from the implant; determining, by the processor 306, a cryptographic hash or a metadata relating to the measurement data and adapted to be used by the external device 320 to verify the integrity of the received data; transmitting the cryptographic hash or metadata; and transmitting, from the controller 300, the measurement data.

The parameter may, for example, be a parameter of the controller 300, such as a temperature, a pressure, a battery status indicator, a time period length, a pressure at a restriction device, a pressure at a sphincter, or a physiological parameter of the patient, such as a pulse, a blood pressure, or a temperature. In some examples, multiple parameters may be used.

The method may further comprise evaluating the measurement data relating to the functional parameter. By evaluating it may be meant to determine if the parameter is exceeding or less than a predetermined value, to extract another parameter from the measurement data, compare the another parameter to a predetermined value, or displaying the another parameter to a user. For example, the method may further comprise, at the external device 320, to determining, based on the evaluating, that the implantable medical device 10 is functioning correctly, or determining based on the evaluating that the implantable medical device 10 is not functioning correctly.

If it is determined that the implantable medical device 10 is not functioning correctly, the method may further comprise sending, from the external device 320, a corrective command to the controller 300, receiving the corrective command at the controller 300, and by running the corrective command correcting the functioning of the implantable medical device 10 according to the corrective command.

The method may further comprise, at the external device 320, receiving the transmitted cryptographic hash or metadata, receiving the measurement data, and verifying the integrity of the measurement data using the cryptographic hash or metadata. The cryptographic hash algorithm be any type of hash algorithm, i.e. an algorithm comprising a one-way function configured to have an input data of any length as input and produce a fixed-length hash value. For example, the cryptographic hash algorithm may be MD5, SHA1, SHA 256, etc.

**In** some examples, the cryptographic hash is a signature obtained by using a private key of the controller 300, and wherein the verifying, by the external device 320, comprises verifying the signature using a public key corresponding to the private key.

When using a cryptographic hash, the method may further comprise calculating a second cryptographic hash for the received measurement data using a same cryptographic hash algorithm as the processor, and determining that the measurement data has been correctly received based on that the cryptographic hash and the second cryptographic hash are equal (i.e. have the same value).

When using a metadata the verifying the integrity of the data may comprises obtaining a second metadata for the received measurement data relating to the functional parameter, and determining that the data has been correctly received based on that metadata and the second metadata are equal. The metadata may, for example, be a length of the data or a timestamp. **In** some examples the measurement data is transmitted in a plurality of data packets. **In** those examples, the cryptographic hash or metadata comprises a plurality of cryptographic hashes or metadata each corresponding to a respective data packet, and the transmitting of each the cryptographic hashes or metadata is performed for each of the corresponding data packets.

A similar method may be utilized for communicating instructions from an external device 320 to a controller 300 implanted in a patient. The method comprises establishing a first connection between the external device 320 and the controller 300, establishing a second connection between a second external device 330 and the controller 300, transmitting, from the external device 320, a first set of instructions to the controller 300 over the first connection, transmitting, from the second external device 330, a first cryptographic hash or metadata corresponding to the first set of instructions to the controller 300, and, at the controller 300, verifying the integrity of the first set of instructions and the first cryptographic hash or metadata, based on the first cryptographic hash or metadata. The external device 320 may be separate from the second external device 330.

The first connections may be established between the controller 300 and a transceiver of the external communication unit 323. **In** some examples, the communication using the second connection is performed using a different protocol than a protocol used for communication using the first communication channel. **In** some examples, the first connection is a wireless connection and the second connection is an electrical connection. The second connection may, for example, be an electrical connection using the patient's body as a conductor (using 321). The protocols and ways of communicating may be any communication protocols described in this description with reference to C1, and WL1-WL4. The establishing of the first and second connections are performed according to the communication protocol used for each of the first and the second connections.

When using a cryptographic hash, the verifying the integrity of the first set of instructions may comprise calculating a second cryptographic hash for the received first set of instructions using a same cryptographic hash algorithm as the processor 306, and determining that the first set of instructions has been correctly received based on that the cryptographic hash and the second cryptographic hash are equal. The cryptographic hash may, for example, be a signature obtained by using a private key of the implantable medical device 10, and wherein the verifying comprises verifying the signature using a public key corresponding to the private key. **In** some examples, the cryptographic hash is a signature obtained by using a private key of the implantable medical device 10, and wherein the verifying comprises verifying the signature using a public key corresponding to the private key. The private keys and public keys, as well as the exchange or transmittal of keys have been described in this description. Alternatively, other well-known methods can be used for transmitting or exchanging a key or keys between the external device 320 and the controller 300.

When using a metadata, and wherein the verifying the integrity of the data may comprise obtaining a second metadata for the received first set of instructions, and determining that the first set of instructions has been correctly received based on that metadata and the second metadata are equal. The metadata may, for example, be any type of data relating to the data to be transmitted, in this example the first set of instructions. For example, the metadata may be a length of the data to be transmitted, a timestamp on which the data was transmitted or retrieved or obtained, a size, a number of packets, or a packet identifier.

In some examples, the controller 300 may transmit data to an external device 320 relating to the data information in order to verify that the received data is correct. The method may thus further comprise, transmitting, by the controller 300, information relating to the received first set of instructions, receiving, by the external device 320, the information, and verifying, by the external device 320, that the information corresponds to the first set of instructions sent by the external device 320. The information may, for example, comprise a length of the first set of instructions.

The method may further comprise, at the controller 300, verifying the authenticity of the first set of instructions by i. calculating a second cryptographic hash for the first set of instructions, ii. comparing the second cryptographic hash with the first cryptographic hash, iii. determining that the first set of instructions are authentic based on that the second cryptographic hash is equal to the first cryptographic hash, and upon verification of the authenticity of the first set of instructions, storing them at the controller 300.

In some examples, the first set of instructions comprises a cryptographic hash corresponding to a previous set of instruction, as described in other parts of this description.

In some examples, the first set of instructions may comprise a measurement relating to the patient of the body for authentication, as described in other parts of this description.

A system and a method for communication of instructions or control signals between an external device 320 and an implantable medical device 10 will now be described with reference to Figs. 22a - 22f.

The system shown in Figs. 22a - 22f comprises an implantable medical device 10, a first external device 320, and a second external device 330. The implantable medical device a controller 300. The controller 300 is adapted to receive an instruction from an external device 320 over the communication channel WL1, C1 and run the instruction to control a function of the medical device 10. The communication channel WL1, C1 may be any type of communication channel, such as a wireless connection WL1 or a conductive connection C1 described herein. For example, the wireless connection may comprise at least one of the following protocols: Radio Frequency type protocol, RFID type protocol, WLAN type protocol, Bluetooth type protocol, a BLE type protocol, a NFC type protocol, a 3G/4G/5G/6G type protocol, a GSM type protocol, and/or Bluetooth 5.

The first external device 320 is adapted to receive, such as through a user interface, or determine an instruction to be transmitted to the implantable medical device 10. The determination of the instruction may, for example, be based on received data from the implantable medical device 10, such as measurement data or data relating to a state of the implantable medical device 10, such as a battery status or a free memory status. The first external device 320 may be any type of device capable of transmitting information to the implantable medical device and capable of determining or receiving an instruction to be transmitted to the implantable medical device 10. In a preferred embodiment, the first external device 320 is a hand-held device, such as a smartphone, smartwatch, tablet etc. handled by the patient, having a user interface for receiving an instruction from a user, such as the patient or a caregiver.

The first external device 320 is further adapted to transmit the instruction to a second external device 330 via communication channel WL3. The second external device 320 is adapted to receive the instruction, encrypt the instruction using an encryption key, and then transmit the encrypted instruction to the implantable medical device 10. The implantable medical device 10 is configured to receive the instruction at the controller 300. The controller 300 thus comprises a wired transceiver or a wireless transceiver for receiving the instruction. The implantable medical device 10 is configured to decrypt the received instruction. The decryption may be performed using a decryption key corresponding to the encryption key. The encryption key, the decryption key and methods for encryption/decryption and exchange of keys may be performed as described in the "general definition of features" or as described with reference to Figs. 22a - 22f. Further, there are many known methods for encrypting data which the skilled person would understand to be usable in this example.

The second external device 330 may be any computing device capable of receiving, encrypting and transmitting data as described above. For example, the second external device 320 may be a network device, such as a network server, or it may be an encryption device communicatively coupled to the first external device.

The instruction may be a single instruction for running a specific function or method in the implantable medical device 10, a value for a parameter of the implantable medical device 10, or a set of sub-steps to be performed by the controller 300 comprised in the implantable medical device 10.

**In** this way, the instruction for controlling a function of the implantable medical device 10 may be received at the first external device 320 and transmitted to the implantable medical device 10 via the second external device 330. By having a second external device 330 encrypting the instruction before transmitting it to the implantable medical device 10, the instruction may be verified by the second external device 330 and the first external device 320 may function so as to relay the instruction. **In** some alternatives, the second external device 330 may transmit the instruction directly to the implantable medical device 10. This may provide an increased security as the instruction sent to the implantable medical device 10 may be verified by the second external device 330, which, for example, may be a proprietary device managed by the medical professional responsible for the implantable medical device 10. Further, by having the second external device 330 verifying and encrypting the instruction, the responsibility authenticity and/or correctness of the instruction may lie with the second external device 330, which may be beneficial for regulatory purposes, as the first external device 320 may not be considered as the instructor of the implantable medical device 10.

Further, the second external device 330 may verify that the instruction is correct before encrypting or signing and transmitting it to the implantable medical device 10. The second external device 330 may, for example, verify that the instruction is correct by comparing the instruction with a predetermined set of instructions, and if the instruction is comprised in the predetermined set of instructions determine that the instruction is correct. **If** the instruction comprises a plurality of sub-steps, the second external device 330 may determine that the instruction is correct if all the sub-steps are comprised in the predetermined set of instructions. If the instruction comprises a value for a parameter of the implantable medical device 10, the second external device 330 may verify that the value is within a predetermined range for the parameter. The second external device 320 may thus comprise a predetermined set of instructions, or a predetermined interval or threshold value for a value of a parameter, stored at an internal or external memory.

The second external device 330 may be configured to reject the instruction, i.e. to not encrypt and transmit the instruction to the implantable medical device 10, if the verification of the instruction would fail. For example, the second external device 330 determines that the instruction or any sub-step of the instruction is not comprised in the predetermined set of instructions, or if a value for a parameter is not within a predetermined interval, the second external device 330 may determine that the verification has failed.

**In** some embodiments, the implantable medical device 10 may be configured to verify the instruction. The verification of the instruction may be performed in the same way as described with reference to Figs. 22a - 22f. If the verification is performed by comparing the instruction or any sub-steps of the instruction with a predetermined set of instructions, the controller 300 may comprise a predetermined set of instructions. The predetermined set of instructions may, for example, be stored in an internal memory of the controller 300. Similarly, the controller 300 may store predetermined reference intervals for any parameter that can be set, and the controller 300 may be configured to compare a received value for a parameter to such a predetermined reference interval. If the verification of the instruction would fail, the controller 300 may be configured to reject the instruction, i.e. not run the instruction.

In an alternative to encrypting and decrypting the instruction, the instruction may be signed by the second external device 330 using a cryptographic hash, and the controller 300 may be configured to verify that the signature is correct before running the instruction.

A corresponding method for transmitting an instruction will now be described with reference to Figs. 22a - 22f. The instruction may relate to a function of the implantable medical device, such as an instruction to run a function or method of the implantable medical device, or to set a value of a parameter of the implantable medical device. The method comprises: transmitting an instruction for the implantable medical device from the first external device 300 to a second external device 320, the instruction relating to a function of the implantable medical device 10, encrypting, at the second external device 330 using a first encryption key, the instruction into an encrypted instruction, and transmitting the encrypted instruction from the second external device 330 to the implantable medical device 10, decrypting, at the implantable medical device, the instructions using a second encryption key corresponding to the first encryption key. The steps performed by or at the implantable medical device may be executed by the controller 300.

The instruction may be any type of instruction for controlling a function of the implantable medical device. For example, the instruction may be an instruction to run a function or method of the implantable medical device 10 or controller 300, an instruction comprising a plurality of sub-steps to be run at the controller 300, or a value for a parameter at the controller 300. The first external device 320 may, for example, receive the instruction from a user via a user interface displayed at or connected to the first external device 320. In another example, the first external device 320 may determine the instruction in response to data received from the implantable medical device 10, such as measurement data, or from another external device. Thus, in some examples, the method may further comprise receiving, at the first external device 320, an instruction to be transmitted to the implantable medical device 10. The method may further comprise displaying a user interface for receiving the instruction. In another example, the method comprises determining, at the first external device 320, an instruction to be transmitted to the implantable medical device 10.

In some embodiments, the transmitting of the encrypted instruction from the second external device 330 to the implantable medical device 10 comprises transmitting the encrypted instruction from the second external device 330 to the first external device 320, and transmitting the encrypted instruction from the first external device 320 to the controller 300 of the implantable medical device 10. In other words, the first external device 320 may relay the encrypted instruction from the second external device 330 to the controller 300, preferably without decrypting the instruction before transmitting it.

The method may further comprise to, at the controller 300, running the instruction or performing the instruction. The running of the instruction may be performed by an internal computing unit or a processor 306 comprised in the controller 300, and may, for example, cause the internal computing unit or processor 306 to instruct the implantable medical device 302 to perform an action.

The method may further comprise verifying, at the second external device 330, that the instructions are correct. The verifying may be performed as described above with reference to the corresponding system.

The method may further comprise verifying, at the controller 300, that the instructions are correct. The verifying may be performed as described above with reference to the corresponding system.

The method may further comprise authenticating the connection between the first external device 320 and the controller 300 over which the encrypted instruction is to be transmitted. The authentication may be performed as described herein.

As described above, a control program of the controller 300 may be updatable, configurable or replaceable. A system and a method for updating or configuring a control program of the controller 300 is now described with reference to figs. 22a - 22f. The controller may comprise an internal computing unit 306 configured to control a function of the implantable medical device 10, the internal computing unit 306 comprises an internal memory 307 configured to store: i. a first control program 310 for controlling the internal computing unit, and ii. a second, configurable or updatable, with predefined program steps, control program 312 for controlling said function of the implantable medical device 10, and iii. a set of predefined program steps for updating the second control program 312. The controller 300 is configured to communicate with an external device 320. The internal computing unit 306 is configured to receive an update to the second control program 312 via the controller 300, and a verification function of, connected to, or transmitted to the controller 300. The verification function is configured to verify that the received update to the second control program 312 comprises program steps comprised in the set of predefined program steps. In this way, the updating or programming of the second control program may be performed using predefined program steps, which may decrease the risk that the new or updated control program is incorrect or comprises malicious software, such as a virus, spyware or a malware.

The predefined program steps may comprise setting a variable related to a pressure, a time, a minimum or maximum temperature, a current, a voltage, an intensity, a frequency, an amplitude of electrical stimulation, a feedback mode (sensorics or other), a post-operative mode or a normal mode, a catheter mode, a fibrotic tissue mode (for example semi-open), an time open after urination, a time open after urination before bed-time.

The verification function may be configured to reject the update in response to the update comprising program steps not comprised in the set of predefined program steps and/or be configured to allow the update in response to the update only comprising program steps comprised in the set of predefined program steps.

The internal computing unit 306 may be configured to install the update in response to a positive verification, for example by a user using an external device, by a button or similarly pressed by a user, or by another external signal.

The authentication or verification of communications between the implant and an external device has been described above.

When updating a control program of the controller 300, it may be beneficial to transmit a confirmation to a user or to an external device or system. Such a method is now described with reference to figs. 22a - 22b.

The method for updating a control program of a controller 300 comprised in the implantable medical device 10 according to any of the embodiments herein. The controller 300 is adapted for communication with a first external device 320 and a second external device 330, which may comprise receiving, by the internal computing unit, an update or configuration to the control program from the first external device, wherein the update is received using a first communication channel; installing, by the internal computing unit 306, the update; and transmitting, by the internal computing unit, logging data relating to the receipt of the update or configuration and/or logging data relating to an installation of the update to the second external device 330 using the second communication channel; wherein the first and the second communication channels are different communication channels. By using a first and a second communication channels, in comparison to only using one, the security of the updating may be improved as any attempts to update the control program will be logged via the second communication channel, and thus, increasing the chances of finding incorrect or malicious update attempts.

The update or configuration comprises a set of instructions for the control program, and may, for examples comprise a set of predefined program steps as described above. The configuration or update may comprise a value for a predetermined parameter.

In some examples, the method further comprises confirming, by a user or by an external control unit, that the update or configuration is correct based on the received logging data.

The logging data may be related to the receipt of the update or configuration, and the controller 300 is configured to install the update or configuration in response to receipt of a confirmation that the logging data relates to a correct set of instructions. In this way, the controller 300 may receive data, transmit a logging entry relating to the receipt, and then install the data in response to a positive verification that the data should be installed.

In another example, or in combination with the one described above, the logging data is related to the installation or the update or configuration. In this example the logging data may be for information purposes only and not affect the installation, or the method may further comprise activating the installation in response to the confirmation that the update or configuration is correct.

If the update or configuration is transmitted to the controller 300 in one or more steps, the verification as described above may be performed for each of the steps.

The method may further comprise, after transmitting the logging data to the second external device, verifying the update via a confirmation from the second external device 330 via the second communication channel.

With reference to Fig. 22a - 22f there may further be provided an implantable controller 300. The controller 300 is connected to a sensor 351 wherein the sensor 351 is at least one microphone sensor 351 configured to record acoustic signals. For instance, the controller 300 may be configured to register a sound related to at least one of a bodily function of the patient and a function of the implantable medical device 10. The controller 300 comprises a computing unit 306 configured to derive at least one of a pulse of the patient from the registered sound related to a bodily function, such as information related to the patient swallowing, from the registered sound related to a bodily function. In the alternative, the controller 300 could be configured to derive information related to a functional status of the implantable medical device 10 from the registered sound, such as RPM of the motor. To this end the computing unit 306 may be configured to perform signal processing on the registered sound (e.g. on a digital or analog signal representing the registered sound) so as to derive any of the above mentioned information related to a bodily function of the patient or a function of the implantable medical device 10. The signal processing may comprise filtering the registered sound signals of the microphone sensor 351.

The implantable controller is placed in an implantable housing for sealing against fluid, and the microphone sensor 351 is placed inside of the housing. Accordingly, the controller and the microphone sensor 351 do not come into contact with bodily fluids when implanted which ensures proper operation of the controller and the microphone sensor 351.

In some implementations, the computing unit 306 is configured to derive information related to the functional status of an active unit 302 of the implantable medical device 10, from the registered sound related to a function of the implantable medical device 10. Accordingly, the computing unit 306 may be configured to derive information related to the functional status of at least one of: a motor, a pump and a transmission of the active unit 302 of the implantable medical device 10, from the registered sound related to a function of the implantable medical device 10.

The controller may comprise a transceiver 303,308 configured to transmit a parameter derived from the sound registered by the at least one microphone sensor 351 using the transceiver 303,308. For example, the transceiver 303,308 is a transceiver configured to transmit the parameter conductively (303) to an external device 320 or wirelessly (308) to an external device 320.

A method of authenticating the implantable medical device 10, the external device 320 or a communication signal or data stream between the external device 320 and the implantable medical device 10 is also described with reference to figs. 22a - 22f. The method comprises the steps of registering a sound related to at least one of a bodily function and a function of the implantable medical device 10, using the at least one microphone sensor 351, connected to the controller 300. The method could in a first authentication embodiment comprise transmitting a signal derived from the registered sound, using the transceiver 303,308, receiving the signal in the external device 320, using the receiver 323,328 and comparing, in the external device 320, a parameter derived from the received signal with a reference parameter, using the computing unit 306. The method could in a second authentication embodiment comprise receiving a signal in the controller 300, from the external device 320, using the transceiver 323,328 and deriving a reference parameter from the received signal, using the computing unit 306 of the controller 300, and comparing, in the controller 300, a parameter derived from the received signal with the derived reference parameter, using the computing unit 306 of the controller 300. The methods further comprise the steps of the implantable controller 300 authenticating the external device 320, or the external device 320 authenticating the implantable controller 300, on the basis of the comparison. The registered sound could for example be related to the patient eating.

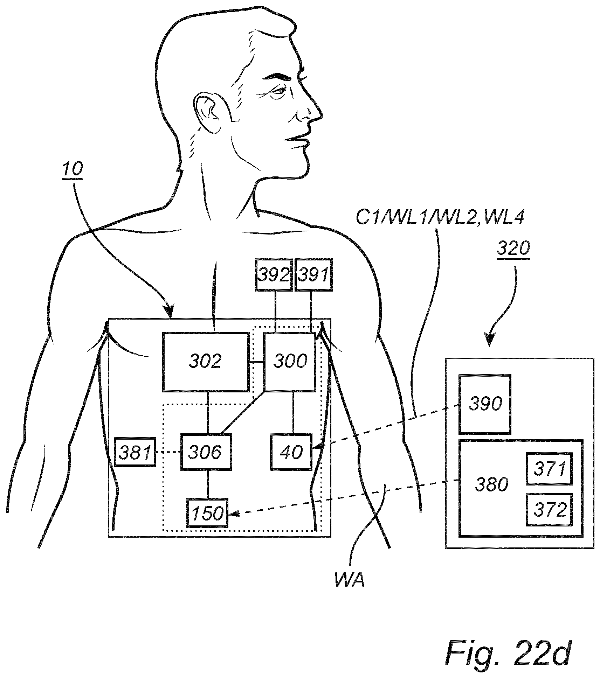

Embodiments relating to an implantable medical device 10 having a controller 300 having a processor 306 with a sleep mode and an active mode will now be described with reference to Fig. 22d. The implant, the internal communication unit and the external device(s) may have the features described above with reference to figs. 22a - 22f.

In an embodiment in which the controller 300 comprises a processor 306 having a sleep mode and an active mode, the controller 300 comprises or is connected to a sensor 150 and a processing unit 306 having a sleep mode and an active mode. The sensor 150 is configured to periodically measure a physical parameter of the patient, and the controller 300 is further configured to, in response to a sensor measurement preceding a predetermined value, setting the processing unit 306 in an active mode. That is, the controller 300 may "wake up" or be set in an active mode in response to a measurement from, for example, the body. A physical parameter of the patient could for example be a local or systemic temperature, saturation/oxygenation, blood pressure or a parameter related to an ischemia marker such as lactate.

By sleeping mode it is meant a mode with less battery consumption and/or processing power used in the processing unit 306, and by "active mode" it may be meant that the processing unit 306 is not restricted in its processing.

The sensor 150 may, for example, be a pressure sensor. The pressure sensor may be adapted to measure a pressure in an organ of a patient, a reservoir of the implant or a pressure exerted by at least one member. The sensor 150 may be an analog sensor or a digital sensor, i.e. a sensor 150 implemented in part in software. In some examples, the sensor is adapted to measure one or more of a battery or energy storage status of the implantable medical device 10 and a temperature of the implantable medical device 10. In this way, the sensor 150 may periodically sense a pressure of the implantable medical device 10 or of the patient, and set the processing unit 306 in an active mode if the measured pressure is above a predetermined value. Thus, less power, i.e. less of for example a battery or energy storage comprised in the implant, may be used, thereby prolonging the lifetime of the implantable medical device 10 or increasing the time between charging occasions of the implantable medical device 10.

In some examples, the processor 306, when in set in the active mode, may cause a sensation generator 381 connected to the implant, comprised in the implantable medical device 10 or comprised in an external device 320, 330, to generate a sensation detectable by a sense of the patient. For example, the processor may cause the sensation generator to generate a sensation in response to a measure battery status, for example that the battery is above or below a predetermined level, that a measured pressure is above or below a predetermined level, or that another measured parameter has an abnormal value, i.e. less than or exceeding a predetermined interval or level. The sensation generator has been described in further detail earlier in this description.

The processing unit 306 may be configured to perform a corrective action in response to a measurement being below or above a predetermined level. Such a corrective action may, for example, be increasing or decreasing a pressure, increasing or decreasing electrical stimulation, increasing or decreasing power.

The controller 300 may comprise a signal transmitter 320 connected to the processing unit, and wherein the processing unit is configured to transmit data relating to the measurement via the transceiver 308 of the controller 300 or an additional internal signal transmitter 392. The transmitted data may be received by an external device 320.

The external device may have an external communication unit 390. The external device 320 may comprise a signal provider 380 for providing a wake signal to the controller 300. In some examples, the signal provider comprises a coil or magnet 371 for providing a magnetic wake signal.

The controller 300 may implement a corresponding method for controlling an implantable medical device 10 when implanted in a patient. The method comprises measuring, with a sensor of the controller 300 connected to or comprised in the controller 300, a physiological parameter of the patient or a parameter of the implantable medical device 10, and, in response to a sensor measurement having an abnormal value, setting, by the controller 300, a processor 306 of the controller 300 from a sleep mode to an active mode. The measuring may be carried out periodically. By "abnormal value" it may be meant a measured value exceeding or being less than a predetermined value, or a measured value being outside a predetermined interval. The method may further comprise generating, with a sensation generator 381 as described above, a sensation detectable by the patient. In some examples, the generating comprises requesting, by the processor, the sensation generator 381 to generate the sensation.

The method may further comprise to perform a medical intervention in response to a sensor measurement having an abnormal value, preferably after the processing unit has been set in the active mode.

According to one embodiment described with reference to fig. 22a - 22c, the communication unit 300 or internal controller 300 or control unit 300 comprises a wireless transceiver 308 for communicating wirelessly with an external device, a security module 389, and a central unit, also referred to herein as a computing unit 306, which is to be considered as equivalent. The central unit 306 is configured to be in communication with the wireless transceiver 308, the security module 389 and the implantable medical device or active unit 302. The wireless transceiver 308 is configured to receive communication from the external device 320 including at least one instruction to the implantable energized medical device 10 and transmit the received communication to the central unit or computing unit 306. The central unit or computing unit 306 is configured to send secure communication to the security module 389, derived from the received communication from the external device 320, and the security module 389 is configured to decrypt at least a portion of the secure communication and verify the authenticity of the secure communication. The security module is further configured to transmit a response communication to the central unit or computing unit 306 and the central unit or computing unit is configured to communicate the at least one instruction to the active unit 302. In the embodiment shown in fig. 22a - 22c, the at least one instruction is based on the response communication, or a combination of the response communication and the received communication from the external device 320.

In the embodiment shown in fig. 22a - 22c, the security module 389 comprises a set of rules for accepting communication from the central unit or computing unit 306. In the embodiment shown in fig. 22a - 22c, the wireless transceiver 308 is configured to be able to be placed in an off-mode, in which no wireless communication can be transmitted or received by the wireless transceiver 308. The set of rules comprises a rule stipulating that communication from the central unit or computing unit 306 to the security module 389 or to the active unit 302 is only accepted when the wireless transceiver 308 is placed in the off-mode.

In the embodiment shown in fig. 22a - 22c, the set of rules comprises a rule stipulating that communication from the central unit or computing unit 306 is only accepted when the wireless transceiver 308 has been placed in the off-mode for a specific time period.

In the embodiment shown in fig. 22a - 22c, the central unit or computing unit 306 is configured to verify a digital signature of the received communication from the external device 320. The digital signature could be a hash-based digital signature which could be based on a biometric signature from the patient or a medical professional. The set of rules further comprises a rule stipulating that communication from the central unit 306 is only accepted when the digital signature of the received communication has been verified by the central unit 306. The verification could for example comprise the step of comparing the digital signature or a portion of the digital signature with a previously verified digital signature stored in the central unit 306. The central unit 306 may be configured to verify the size of the received communication from the external device and the set of rules could comprise a rule stipulating that communication from the central unit 306 is only accepted when the size of the received communication has been verified by the central unit 306. The central unit could thus have a rule stipulating that communication above or below a specified size range is to be rejected.

In the embodiment shown in fig. 22a - 22c, the wireless transceiver is configured to receive a message from the external device 320 being encrypted with at least a first and second layer of encryption. The central unit 306 the decrypts the first layer of decryption and transmit at least a portion of the message comprising the second layer of encryption to the security model 389. The security module 389 then decrypts the second layer of encryption and transmits a response communication to the central unit 306 based on the portion of the message decrypted by the security module 389.

In the embodiment shown in fig. 22a - 22c, the central unit 306 is configured to decrypt a portion of the message comprising a digital signature, such that the digital signature can be verified by the central unit 306, also the central unit 306 is configured to decrypt a portion of the message comprising message size information, such that the message size can be verified by the central unit 306.

In the embodiment shown in fig. 22a - 22c, the central unit 306 is configured to decrypt a first and second portion of the message, and the first portion comprises a checksum for verifying the authenticity of the second portion.

In the embodiment shown in fig. 22a - 22c, the response communication transmitted from the security module 389 comprises a checksum, and the central unit 306 is configured to verify the authenticity of at least a portion of the message decrypted by the central unit 306 using the received checksum, i.e. by adding portions of the message decrypted by the central unit 306 and comparing the sum to the checksum.

In the embodiment shown in fig. 22a - 22c, the set of rules further comprise a rule related to the rate of data transfer between the central unit 306 and the security module 389. The rule could stipulate that the communication should be rejected or aborted if the rate of data transfer exceeds a set maximum rate of data transfer, which may make it harder for unauthorized persons to inject malicious code or instructions to the medical implant.

In the embodiment shown in fig. 22a - 22c, the security module 389 is configured to decrypt a portion of the message comprising the digital signature being encrypted with the second layer of encryption, such that the digital signature can be verified by the security module 389. The security module 389 then transmits a response communication to the central unit 306 based on the outcome of the verification, which can be used by the central unit 306 for further decryption of the message or for determining if instructions in the message should be communicated to the active unit 302.

In the embodiment shown in fig. 22a - 22c, the central unit 306 is only capable of decrypting a portion of the received communication from the external device 320 when the wireless transceiver 308 is placed in the off-mode. In the alternative, or as an additional layer of security, the central unit 306 may be limited such that the central unit 306 is only capable of communicating instructions to the active unit 302 of the implantable energized medical device 10 when the wireless transceiver 308 is placed in the off-mode. This ensures that no attacks can take place while the central unit 306 is communicating with the active unit 302.

In the embodiment shown in fig. 22a - 22c, the implantable controller 300 is configured to receive, using the wireless transceiver 308, a message from the external device 320 comprising a first un-encrypted portion and a second encrypted portion. The implantable controller 300 (e.g. the central unit 306 or the security module 389) then decrypts the encrypted portion, and uses the decrypted portion to verify the authenticity of the un-encrypted portion. As such, computing power and thereby energy can be saved by not encrypting the entire communication, but rather only the portion required to authenticate the rest of the message (such as a checksum and/or a digital signature)

In the embodiment shown in fig. 22a - 22c, the central unit 306 is configured to transmit an encrypted portion to the security module 389 and receive a response communication from the security module 389 based on information contained in the encrypted portion being decrypted by the security module. The central unit 306 is then configured to use the response communication to verify the authenticity of the un-encrypted portion. The un-encrypted portion could comprise at least a portion of the at least one instruction to the implantable medical device 306.

In the embodiment shown in fig. 22a - 22c, the implantable controller 300 is configured to receive, using the wireless transceiver 308, a message from the external device 320 comprising information related to at least one of: a physiological parameter of the patient and a physical parameter of the implantable energized medical device 10, and use the received information to verify the authenticity of the message. The physiological parameter of the patient could be a parameter such as a parameter based on one or more of: a temperature, a heart rate and a saturation value.

The physical parameter of the implantable energized medical device 10 could comprise at least one of a current setting or value of the implantable energized medical device 10, a prior instruction sent to the implantable energized medical device 10 or an ID of the implantable energized medical device 10.

The portion of the message comprising the information related to the physiological parameter of the patient and/or physical or functional parameter of the implantable energized medical device 10 could be encrypted, and the central unit 306 may be configured to transmit the encrypted portion to the security module 389 and receive a response communication from the security module 389 based on the information having been decrypted by the security module 389.

In the embodiment shown in fig. 22a - 22c, the security module 389 is a hardware security module comprising at least one hardware-based key. The security module 389 may have features that provide tamper evidence such as visible signs of tampering or logging and alerting. It may also be so that the security module 389 is "tamper resistant", which makes the security module 389 inoperable in the event that tampering is detected. For example, the response to tampering could include deleting keys is tampering is detected. The security module 389 could comprise one or more secure cryptoprocessor chip. The hardware-based key(s) in the security module 389 could have a corresponding hardware-based key placeable in the external device 320. The corresponding external hardware-based key could be placed on a key-card connectable to the external device 320.

In alternative embodiments, the security module 389 is a software security module comprising at least one software-based key, or a combination of a hardware and software-based security module and key. The software-based key may correspond to a software-based key in the external device 320. The software-based key may correspond to a software-based key on a key-card connectable to the external device 320.

In the embodiment shown in fig. 22a - 22c, the external device 320 is a handheld external device, however, in alternative embodiments, the external device may be a remote external device or a cloud based external device

In the embodiment shown in fig. 22a - 22c, the at least one instruction to the implantable energized medical device 10 comprises an instruction for changing an operational state of the implantable energized medical device 10.

In the embodiment shown in fig. 22a - 22c, the wireless transceiver 308 is configured to communicate wirelessly with the external 320 device using electromagnetic waves at a frequency below 100 kHz, or more specifically below 40 kHz. The wireless transceiver 308 is thus configured to communicate with the external device 320 using "Very Low Frequency" communication (VLF). VLF signals have the ability to penetrate a titanium housing of the implantable energized medical device 10, such that the electronics of the implantable energized medical device 10 can be completely encapsulated in a titanium housing.

The wireless transceiver 308 is configured to communicate wirelessly with the external device 320 using a first communication protocol and the central unit 306 is configured to communicate with the security module 389 using a second, different, communication protocol. This adds an additional layer of security as security structures could be built into the electronics and/or software in the central unit 306 enabling the transfer from a first to a second communication protocol. The wireless transceiver 308 may be configured to communicate wirelessly with the external device using a standard network protocol, which could be one of an RFID type protocol, a WLAN type protocol,a Bluetooth (BT) type protocol,a BLE type protocol,an NFC type protocol, a 3G/4G/5G type protocol, and a GSM type protocol. In the alternative, or as a combination, the wireless transceiver 308 could be configured to communicate wirelessly with the external device 320 using a proprietary network protocol. The wireless transceiver 308 could comprises a Ultra-Wide Band (UWB) transceiver and the wireless communication between the implantable controller 300 and the external device 320 could thus be based on UWB. The use of UWB technology enables positioning of the remote control 320" which can be used by the implantable energized medical device 10 as a way to establish that the external device 320 is at a position which the implantable energized medical device 10 and/or the patient can acknowledge as being correct, e.g. in the direct proximity to the implantable energized medical device 10 and/or the patient, such as within reach of the patient and/or within 1 or 2 meters of the implantable energized medical device 10. In the alternative, a combination of UWB and BT could be used, in which case the UWB communication can be used to authenticate the BT communication, as it is easier to transfer large data sets using BT.

### Aspect 332SE - Variable Impedance

According to one embodiment described with reference to fig. 22a - 22c, the communication unit 300 or controller of the implantable energized medical device 10 comprises a receiving unit 305 or energy receiver 305 comprising a coil 192 (specifically shown in fig. 22b') configured for receiving transcutaneously transferred energy. The receiving unit further comprises a measurement unit 194 configured to measure a parameter related to the energy received by the coil 192 and a variable impedance 193 electrically connected to the coil 192. The receiving unit 305 further comprises a switch 195a placed between the variable impedance 193 and the coil 192 for switching off the electrical connection between the variable impedance 193 and the coil 192. The communication unit 300 or controller 300 is configured to control the variable impedance 193 for varying the impedance and thereby tune the coil 192 based on the measured parameter. The communication unit 300 or controller 300 is further configured to control the switch 195a for switching off the electrical connection between the variable impedance 193 and the coil 192 in response to the measured parameter exceeding a threshold value. The controller 300 may further be configured to vary the variable impedance in response to the measured parameter exceeding a threshold value. As such, the coil can be tuned or turned off to reduce the amount of received energy if the amount of received energy becomes excessive. The measurement unit 194 is configured to measure a parameter related to the energy received by the coil 192 over a time period and/or measure a parameter related to a change in energy received by the coil 192 by for example measure the derivative of the received energy over time. The variable impedance 193 is in the embodiment shown in fig. 22b' placed in series with the coil 192. **In** alternative embodiments it is however conceivable that the variable impedance is placed parallel to the coil 192.

The first switch 195a is placed at a first end portion 192a of the coil 192, and the implantable energized medical device 10 further comprises a second switch 195b placed at a second end portion of the coil 192, such that the coil 192 can be completely disconnected from other portions of the implantable energized medical device 10. The receiving unit 305 is configured to receive transcutaneously transferred energy in pulses according to a pulse pattern. The measurement unit 194 is in the embodiment shown in fig. 22b' configured to measure a parameter related to the pulse pattern. The controller 300 is configured to control the variable impedance in response to the pulse pattern deviating from a predefined pulse pattern. The controller 300 is configured to control the switch 195a for switching off the electrical connection between the variable impedance 193 and the coil 192 in response to the pulse pattern deviating from a predefined pulse pattern. The measurement unit is configured to measure a temperature in the implantable energized medical device 10 or in the body of the patient, and the controller 300 is configured to control the first and second switch 195a, 195b in response to the measured temperature.

The variable impedance 193 may comprise a resistor and a capacitor and/or a resistor and an inductor and/or an inductor and a capacitor. The variable impedance 193 may comprise a digitally tuned capacitor or a digital potentiometer. The variable impedance 193 may comprise a variable inductor. The first and second switch comprises a semiconductor, such as a MOSFET. The variation of the impedance is configured to lower the active power that is received by the receiving unit. As can be seen in fig. 22b', the variable impedance 193, the first and second switch 195a,195b and the measurement unit 194 are connected to the communication unit/controller 300 and the receiving unit 305 is connected to an energy storage unit 40 such that the energy storage unit 40 can store energy received by the receiving unit 305.

A system comprising an implantable medical device 10 having a controller 300 having a sleep mode and an active mode will now be described with reference to Fig. 22d. In one embodiment, the controller 300 comprises a sensor 150 adapted to detect a magnetic field and a processing unit 306 having a sleep mode and an active mode, now described with reference to figs. 22a - 22f. The external control unit 320 comprises a signal provider 380 adapted to provide a magnetic field detectable by the internal sensor 150. The controller 300 is further configured to, in response to a detected magnetic field exceeding a predetermined value, setting the processing unit 306 in an active mode. In this way, the external device 320 may cause a sleeping controller 300 or processor 306 to "wake up".

The sensor 150 may, for example, be a hall effect sensor, a fluxgate sensor, an ultra-sensitive magnetic field sensor, a magneto-resistive sensor, an AMR or GMR sensor, or the sensor may comprise a third coil having an iron core.

The magnetic field provider 380 may have an off state, wherein it does not provide any magnetic field, and an on state, wherein it provides a magnetic field. For example, the magnetic field provider 380 may comprise a magnet 371, a coil 371, a coil having a core 371, or a permanent magnet 371. In some embodiments, the magnetic field provider 380 may comprise a shielding means for preventing a magnet 371 or permanent magnet 371 from providing a magnetic field in the off state. In order to provide a substantially even magnetic field, the magnetic field provider may comprise a first and a second coil arranged perpendicular to each other.

After the processing unit 306 has been set in an active mode, i.e. when the processing unit 306 has been woken, the implant may determine a frequency for further communication between the controller 300 and the external device 320. The controller 300 may thus comprise a frequency detector 391 for detecting a frequency for communication between the controller 300 and the second communication unit 390. The frequency detector 391 is, for example, an antenna. The external device 320 may comprise a frequency indicator 372, for transmitting a signal indicative of a frequency. The frequency indicator 372, may, for example, be a magnetic field provider capable of transmitting a magnetic field with a specific frequency. In some examples the frequency indicator is comprised in or the same as the magnetic field provider 371. In this way, the frequency signal is detected using means separate from the sensor, and can, for example, be detected using a pin on a chip.

Alternatively, the controller 300 and the external device 320 may communicate using a predetermined frequency or a frequency detected by means defined by a predetermined method according to a predetermined protocol to be used for the communication between the controller 300 and the external device 320.

In some embodiments, the sensor 150 may be used for the communication. The communication may in these embodiments be performed with such that a frequency of the magnetic field generated by the coil is 9-315 kHz, or the magnetic field generated by the coil is less than or equal to 125kHz, preferably less than 58kHz. The frequency may be less than 50Hz, preferably less than 20Hz, more preferably less than 10Hz, in order to be transmittable through a titanium box.

In some embodiments, the controller 300 comprises a receiver unit 392, and the internal control unit and the external control unit are configured to transmit and/or receive data via the receiver unit 392 via magnetic induction. The receiver unit 392 may comprise a high-sensitivity magnetic field detector, or the receiver unit may comprise a fourth coil for receiving the magnetic induction.

The system may implement a method for controlling a medical implant implanted in a patient. The method comprises monitoring for signals by a sensor 150 comprised in the controller 300 communicatively coupled to the active unit 302, providing, from a signal provider 380 comprised in an external device 320, a wake signal, the external device 320 being adapted to be arranged outside of the patient's body, and setting, by the controller 300 and in response to a detected wake signal WS, a mode of a processing unit 306 comprised in the internal control unit from a sleep mode to an active mode.

The method may also comprise detecting, using a frequency detector 391, a frequency for data communication between the controller 300 and a second communication unit 390 being associated with the external device 320. The frequency detector 391 is communicatively coupled to the controller 300 or the external device 320. The detection may be performed using a detection sequence for detecting the frequency. This detection sequence may, for example, be a detection sequence defined in the protocol to be used for communication between the controller 300 and the second communication unit 390. Potential protocols that may be used for communication between the controller 300 and the external device 320 has been described earlier in this description. Thus, the method may comprise determining, using the frequency detector 391, the frequency for data communication, and initiating data communication between the controller 300 and the second communication unit 390. The data communication can, for example, comprise one or more control instructions for controlling the implantable medical device 10 transmitted from the external device 320, or, for example, comprise data related to the operation of the implantable medical device 10 and be transmitted from the controller 300.

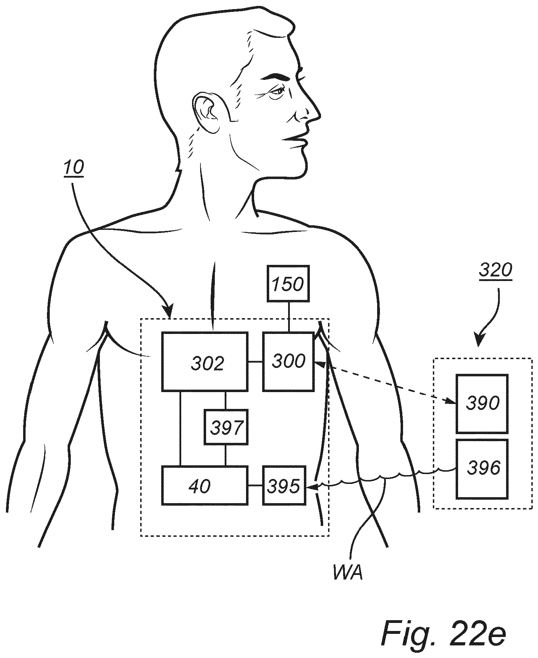

In some examples, the implantable medical device may comprise or be connected to a power supply for powering the implantable medical device 10. This will now be described with reference to fig. 22e. The medical device, the internal control unit, and the external device(s) may comprise all elements described with reference to figs. 22a - 22f. The power supply may comprise an implantable energy storage unit 40 for providing energy to the medical device, an energy provider 397 connected to the implantable energy storage unit 40 and connected to an energy consuming part of the implantable medical device 10, the energy provider 397 being configured to store energy to provide a burst of energy to the energy consuming part, wherein the energy provider 397 is configured to be charged by the implantable energy storage unit 40 and to provide the energy consuming part with electrical power during startup of the energy consuming part.

Alternatively, the implantable medical device 10 may comprise a first implantable energy storage unit 40 for providing energy to an energy consuming part of the implantable medical device 10, a second implantable energy storage unit 397 connected to the implantable energy storage unit 40 and connected to the energy consuming part, wherein the second implantable energy storage unit 397 is configured to be charged by the implantable energy storage unit 40 and to provide the energy consuming part with electrical power during startup of the energy consuming part. The second implantable energy storage unit 397 has a higher energy density than the first implantable energy storage unit 40. By having a "higher energy density" it may be meant that the second implantable energy storage unit 397 has a higher maximum energy output per time unit than the first implantable energy storage unit 40. The second energy storage 397 may be an energy provider as discussed below.

The energy consuming part may be any part of the implantable medical device 10, such as a motor for powering the hydraulic pump, a valve, a processing or computing unit, a communication unit, a device for providing electrical stimulation to a tissue portion of the body of the patient, a CPU for encrypting information, a transmitting and/or receiving unit for communication with an external unit (not shown as part of the energy consuming part in the drawings, that is, the communication unit may be connected to the energy storage unit 40 and to the energy provider 397), a measurement unit or a sensor, a data collection unit, a solenoid, a piezo-electrical element, a memory metal unit, a vibrator, a part configured to operate a valve comprised in the medical device, or a feedback unit.

In this way, an energy consuming part requiring a quick start or an energy consuming part which requires a high level or burst of energy for a start may be provided with sufficient energy. This may be beneficial as instead of having an idle component using energy, the component may be completely turned off and quickly turned on when needed. Further, this may allow the use of energy consuming parts needing a burst of energy for a startup while having a lower energy consumption when already in use. In this way, a battery or an energy storage unit having a slower discharging (or where a slower discharging is beneficial for the lifetime or health of the battery) may be used for the implant, as the extra energy needed for the startup is provided by the energy provider.

Energy losses may occur in a battery or energy storage unit of an implant if the battery or energy storage unit is discharged too fast. These energy losses may for example be in the form of heat, which may damage the battery or energy storage unit. By the apparatus described in these examples, energy may be provided from the battery or energy storage unit in a way that does not damage the battery or energy storage unit, which may improve the lifetime of the battery or energy storage unit and thereby the lifetime of the medical device.

In some examples, the discharging from the implantable energy storage unit 40 during startup of the energy consuming part is slower than the energy needed for startup of the energy consuming part, i.e. the implantable energy storage unit 40 is configured to have a slower discharging than the energy needed for startup of the energy consuming part. That is, there is a difference between the energy needed by the energy consuming part and the energy the implantable energy storage unit 40 is capable of providing without damaging the implantable energy storage unit 40. In other words, a maximum energy consumption of the energy consuming part may be higher than the maximum energy capable of being delivered by the implantable energy storage unit 40 without causing damage to the implantable energy storage unit, and the energy provider 397 may be adapted to deliver an energy burst corresponding to difference between the required energy consumption and the maximum energy capable of being delivered by the implantable energy storage unit 40. The implantable energy storage unit 40 may be configured to store a substantially larger amount of energy than the energy burst provider 397, but may be slower to charge.

The implantable energy storage unit 40 may be any type of energy storage unit suitable for an implant, such as a re-chargeable battery or a solid-state battery, such as a tionyl-chlorid battery. The implantable energy storage unit 40 may be connected to the energy consuming part and configured to power the energy consuming part after it has been started using the energy provider 397.

The energy provider 397 may be any type of part configured to provide a burst of energy for the energy consuming part. In some examples, the energy provider 397 is a capacitor, such as a start capacitor, a run capacitor, a dual run capacitor or a supercapacitor. The energy provider 397 may be connected to the implantable energy storage unit 40 and be adapted to be charged using the implantable energy storage unit 40. In some examples, the energy provider may be a second energy provider 397 configured to be charged by the implantable energy storage unit 40 and to provide the energy consuming part with electrical energy. The implantable medical device 10 may further comprising a temperature sensor for sensing a temperature of the capacitor and the temperature sensor may be integrated or connected to the controller 300 such that the sensed temperature can be used as input for controlling the implantable medical device 10 or as feedback to be sent to an external device 320.

A corresponding method for powering a medical device may also be contemplated. The method comprises the steps of initiating an energy consuming part 302 of the implant, the energy consuming part being connected to an implantable energy storage unit 40, providing an initial burst of energy to the energy consuming part using an energy provider 397 connected to the implantable energy storage unit 40 and to the energy consuming part 302, the energy provider 397 being adapted to provide a burst of energy to the energy consuming part, and subsequently powering the energy consuming part 302 using the implantable energy storage unit 40.

In some examples, a maximum energy consumption of the energy consuming part is higher than the maximum energy capable of being delivered by the implantable energy storage unit 40 without causing damage to the implantable energy storage unit 40, and the energy provider 397 is adapted to deliver an energy burst corresponding to difference between the required energy consumption and the maximum energy capable of being delivered by the implantable energy storage unit 40.

The method may further comprise the step of charging the energy provider 397 using the implantable energy storage unit 40.

Initiating an energy consuming part 302may comprise transitioning a control unit of the medical device from a sleep mode to an operational or active mode.

The implantable energy storage unit 40 may be adapted to be wirelessly charged and the implantable energy storage unit may be connected to an internal charger 395 for receiving wireless energy from an external device 320 via an external charger 396, and the method may comprise wirelessly charging the implantable energy storage unit 40. In some examples, the method comprises controlling a receipt of electrical power from an external energy storage unit at the internal charger 395. The internal energy storage unit 40 may be charged via the receipt of a transmission of electrical power from an external energy storage unit 396 by the internal charger 395.

The embodiments described herein may advantageously be combined. For example, all the embodiments relating to the communication and controlling of the medical device may be combined with the embodiments relating to the programming of the implant, the methods and systems for improving energy consumption or the power supply. The embodiments relating to the programming of the medical device may be combined with any of the embodiments relating to improving the energy consumption or the power supply. The embodiments relating to the power supply maybe combined with the methods and systems for improving the energy consumption.

A computer program product of, or adapted to be run on, an internal computing unit or an external device is also provided, which comprises a computer-readable storage medium with instructions adapted to make the internal computing unit and/or the external device perform the actions as described in any embodiment or example above.

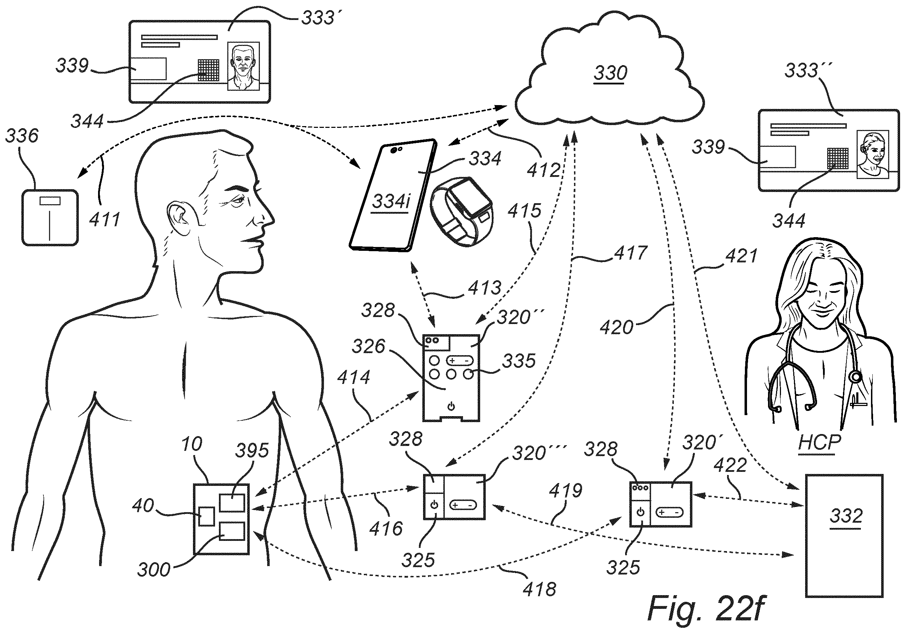

Fig. 22f shows one embodiment of a system for charging, programming and communicating with the controller 300 of the implanted medical device 10. Fig. 22f further describes the communication and interaction between different external devices which may be devices held and operated by the patient, by the health care provider (HCP) or by the Dedicated Data Infrastructure (DDI), which is an infrastructure supplier for example by the manufacturer of the implanted medical device 10 or the external devices 320',320",320‴. The system of the embodiment of fig. 22f comprises three external devices 320',320",320‴ capable of communicating with the controller 300. The basic idea is to ensure the security of the communication with, and the operation of, the medical device 10 by having three external devices 320',320",320‴ with different levels of authority. The lowest level of authority is given to the patient operated remote control 320". The remote control 320" is authorized to operate functions of the implanted medical device 10 via the implanted controller 300, on the basis of patient input. The remote control 320" is further authorized to fetch some necessary data from the controller 300. The remote control 320" is only capable of operating the controller 300 by communicating with the software currently running on the controller 300, with the currently settings or the software. The next level of authority is given to the Patient External Interrogation Device (P-EID) 320‴, which is a charging and communication unit which is held by the patient but is partially remotely operated by the Health Care Provider (HCP) (Usually a medical doctor with the clinic providing the treatment with help of the implanted medical device 10). The P-EID 320‴ is authorized to make setting changes to the software running on the controller 300 of the implanted medical device 10 when remotely operated by the HCP. The highest level of authority is given to the HCP-EID 320'. The HCP-EID 320' is a charging and communication unit which is held by the HCP physically at the clinic of the HCP. The HCP-EID 320' is authorized to freely alter or replace the software running on the controller 300, when the patient is physically in the clinic or the HCP.

Starting from the lowest level of authority, the remote control 320" comprises a wireless transceiver 328 for communicating with the implanted medical device 10. The remote control 320" is capable of controlling the operation of the implanted medical device 10 via the controller 300, by controlling pre-set functions of the implantable medical device 10, e.g. for operating an active portion of the implanted medical device 10 for performing the intended function of the implanted medical device 10. In the embodiment shown in fig. 22f, the wireless transceiver 328 comprises a Bluetooth (BT) transceiver, and the remote control 320" is configured to communicate with implanted medical device 10 using BT. In an alternative configuration, the remote control 320" communicates with the implanted medical device 10 using a combination of Ultra-Wide Band (UWB) wireless communication and BT. The use of UWB technology enables positioning of the remote control 320" which can be used by the implanted medical device 10 as a way to establish that the remote control 320" is at a position which the implanted medical device 10 and/or the patient can acknowledge as being correct, e.g. in the direct proximity to the medical device 10 and/or the patient, such as within reach of the patient and/or within 1 or 2 meters of the implanted medical device 10.

UWB communication is performed by the generation of radio energy at specific time intervals and occupying a large bandwidth, thus enabling pulse-position or time modulation. The information can also be modulated on UWB signals (pulses) by encoding the polarity of the pulse, its amplitude and/or by using orthogonal pulses. A UWB radio system can be used to determine the "time of flight" of the transmission at various frequencies. This helps overcome multipath propagation, since some of the frequencies have a line-of-sight trajectory, while other indirect paths have longer delay. With a cooperative symmetric two-way metering technique, distances can be measured to high resolution and accuracy. UWB is useful for real-time location systems, and its precision capabilities and low power make it well-suited for radio-frequency-sensitive environments.

In embodiments in which a combination of BT and UWB technology is used, the UWB technology may be used for location-based authentication of the remote control 320", whereas the communication and/or data transfer could take place using BT. The UWB signal could in some embodiments also be used as a wake-up signal for the controller 300, or for the BT transceiver, such that the BT transceiver in the implanted medical device 10 can be turned off when not in use, which eliminates the risk that the BT is intercepted, or that the controller 300 of the implanted medical device 10 is hacked by means of BT communication. In embodiments in which a BT/UWB combination is used, the UWB connection may be used also for the transmission of data. In the alternative, the UWB connection could be used for the transmission of some portions of the data, such as sensitive portions of the data, or for the transmission of keys for the unlocking of encrypted communication sent over BT.

The remote control 320" comprises computing unit 326 which runs a software application for communicating with the implanted medical device 10. The computing unit 326 can receive input directly from control buttons 335 arranged on the remote control 320" or may receive input from a control interface 334i displayed on a display device 334 operated by the patient. In the embodiments in which the remote control 320" receives input from a control interface 334i displayed on a display device 334 operated by the patient, the remote control 320" transmits the control interface 334i in the form of a web-view, i.e. a remote interface that run in a sandbox environment on the patient's display device 334. A sandbox environment means that it runs on the display device 334 but can only use a tightly controlled set of resources, such as storage and memory space as well as network access, the ability to inspect the host system and read or write from other input devices connected to the display device 334. The computing unit 326 is further configured to the control interface before transmission to the display device 334, and the control commands before transmission to the implanted medical device 10. The computing unit 326 is further configured to transform the received user input into control commands for wireless transmission to the implantable medical device 10.

The patient's display device 334 could for example be a mobile phone, a tablet or a smart watch. In the embodiment shown in fig. 22f, the patient's display device 334 communicates with the remote control 320" by means of BT. The control interface 334i in the form of a web-view is transmitted from the remote control 320" to the patient's display device 334 over BT. Control commands in the form of inputs from the patient to the control interface 334i is transmitted from the patient's display device 334 to the remote control 320", providing input to the remote control 320" equivalent to the input that may be provided using the control buttons 335. The control commands created in the patient's display device 334 is encrypted in the patient's display device 334 and transmitted to the remote control 320' using BT.

The patient's display device 334 may (in the case of the display device 334 being a mobile phone or tablet) comprise auxiliary radio transmitters for providing auxiliary radio connection, such as Wi-Fi or mobile connectivity (e.g. according to the 3G,4G or 5G standards). The auxiliary radio connection(s) may have to be disconnected to enable communication with the remote control 320". Disconnecting the auxiliary radio connections reduces the risk that the integrity of the control interface 334i displayed on the patient's display device 334 is compromised, or that the control interface 334i displayed on the patient's display device 334 is remote controlled by an unauthorized device.

In alternative embodiments, control commands are generated and encrypted by the patient's display device and transmitted to the DDI 330. The DDI 330 could either alter the created control commands to commands readable by the remote control 320" before further encrypting the control commands for transmission to the remote control 320" or could simply add an extra layer of encryption before transmitting the control commands to the remote control 320", or could simply act as a router for relaying the control commands from the patients' display device 334 to the remote control 320". It is also conceivable that the DDI 330 adds a layer of end-to-end encryption directed at the implanted medical device 10, such that only the implanted medical device 10 can decrypt the control commands to perform the command intended by the patient.

The patient's display device 334 could have a first and second application related to the implanted medical device 10. The first application is the control application displaying the control interface 334i for control of the implanted medical device 10, whereas the second application is a general application for providing the patient with general information of the status of the implanted medical device 10 or information from the DDI 330 or HCP, or for providing an interface for the patient to provide general input to the DDI 330 or HCP related to the general wellbeing of the patient, the lifestyle of the patient or related to general input from the patient concerning the function of the implanted medical device 10. The second application, which do not provide input to the remote control 320" and/or the implanted medical device 10 thus handles data which is less sensitive. As such, the general application could be configured to function also when all auxiliary radio connections are activated, whereas switching to the control application which handles the more sensitive control commands and communication with the implanted medical device 10 could require that the auxiliary radio connections are temporarily de-activated. It is also conceivable that the control application is a sub-application running within the general application, in which case the activation of the control application as a sub-application in the general application could require the temporary de-activation of auxiliary radio connections. In the embodiment shown in fig. 22f, access to the control application requires the use of the optical and/or NFC means of the hardware key 333' in combination with biometric input to the patient's display device, whereas accessing the general application only requires biometric input to the patient's display device and/or a pin code. In the alternative, a two-factor authentication solution, such as a digital key in combination with a pin code could be used for accessing the general application and/or the control application.

In the embodiments in which the patients display device 334 is configured to only display and interact with a web-view provided by another unit in the system, it is conceivable that the web-view is a view of a back-end provided on the DDI 330, and in such embodiments, the patient interacting with the control interface on the patient's display device is equivalent to the patient interacting with an area of the DDI 330.

Moving now to the P-EID 320‴. The P-EID 320‴ is an external device which communicates with, and charges, the implanted medical device 10. The P-EID 320‴ can be remotely controlled by the HCP to read information from the implanted medical device 10, control the operation of the implanted medical device 10, control the charging of the medical device 10, and adjusting the settings to the software running on the controller 300 of the implanted medical device 10, e.g. by adding or removing pre-defined program steps and/or by the selection of pre-defined parameters within a limited range. Just as the remote control 320", the P-EID 320‴ could be configured to communicate with the implanted medical device 10 using BT or UWB communication. Just as with the remote control 320", it is also conceivable to use a combination of UWB wireless communication and BT for enabling positioning of the P-EID 320" as a way to establish that the P-EID 320" is at a position which the implanted medical device 10 and/or patient and/or HCP can acknowledge as being correct, e.g. in the direct proximity to the correct patient and/or the correct medical device 10. Just as for the remote control 320", in embodiments in which a combination of BT and UWB technology is used, the UWB technology may be used for location-based authentication of the P-EID 320", whereas the communication and/or data transfer could take place using BT. The P-EID 320" comprises a wireless transmitter/transceiver 328 for communication and also comprises a wireless transmitter 325 configured for transferring energy wirelessly, in the form of a magnetic field, to a wireless receiver 395 of the implanted medical device 10 configured to receive the energy in the form of a magnetic field and transform the energy into electric energy for storage in an implanted energy storage unit 40, and/or for consumption in an energy consuming part of the implanted medical device 10 (such as the operation device, controller 300 etc.). The magnetic field generated in the P-EID YY and received in the implanted medical device 10 is denoted charging signal. In addition to enabling the wireless transfer of energy from the P-EID to the implanted medical implant 10, the charging signal may also function as a means of communication. E.g., variations in the frequency of the transmission, and/or the amplitude of the signal may be uses as signaling means for enabling communication in one direction, from the P-EID to the implanted medical device 10, or in both directions between the P-EID and the implanted medical device 10. The charging signal in the embodiment shown in fig. 22f is a signal in the range 120 - 140 kHz and the communication follow a proprietary communication signaling protocol, i.e., it is not based on an open standard. In alternative embodiments, BT could be combined with communication using the charging signal, or communication using the charging signal could be combined with an UWB signal.

Just as for the remote control 320", the UWB signal could in some embodiments also be used as a wake-up signal for the controller 300, or for the BT transceiver, such that the BT transceiver in the implanted medical device 10 can be turned off when not in use, which eliminates the risk that the BT is intercepted, or that the controller 300 of the implanted medical device 10 is hacked by means of BT communication. In the alternative, the charging signal could be used as a wakeup signal for the BT, as the charging signal does not travel very far. Also, as a means of location-based authentication, the effect of the charging signal or the RSSI could be assessed by the controller 300 in the implanted medical device 10 to establish that the transmitter is within a defined range. In the BT/UWB combination, the UWB may be used also for transmission of data. In some embodiments, the UWB and/or the charging signal could be used for the transmission of some portions of the data, such as sensitive portions of the data, or for the transmission keys for unlocking encrypted communication sent by BT.

UWB could also be used for waking up the charging signal transmission, to start the wireless transfer of energy or for initiating communication using the charging signal. As the signal for transferring energy has a very high effect in relation to normal radio communication signals, the signal for transferring energy cannot be active all the time, as this signal may be hazardous e.g., by generating heat.

The P-EID 320‴ communicates with the HCP over the Internet by means of a secure communication, such as over a VPN. The communication between the HCP and the P-EID 320‴ is preferably encrypted. The communication from the HCP to the implanted medical device 10 may be performed using an end-to-end encryption, in which case the communication cannot be decrypted by the P-EID 320"'. In such embodiments, the P-EID 320‴ acts as a router, only passing on encrypted communication from the HCP to the controller 300 of the implanted medical device 10. This solution further increases security as the keys for decrypting the information rests only with the HCP and with the implanted medical device 10, which reduces the risk that an unencrypted signal is intercepted by an unauthorized device.

When the implanted medical device 10 is to be controlled and/or updated remotely by the HCP, via the P-EID 320"', a HCP Dedicated Device (DD) 332 displays an interface in which predefined program steps or setting values are presented to the HCP. The HCP provides input to the HCP DD 332 by selecting program steps, altering settings and/or values or by altering the order in which pre-defined program steps is to be executed. The instructions/parameters inputted into the HCP DD 332 for remote operation is in the embodiment shown in fig. 22f routed to the P-EID 320‴ via the DDI 330, which may or may not be able to decrypt/read the instructions. The DDI 330 may store the instructions for a time period to later transfer the instructions in a package of created instructions to the P-EID 320‴. It is also conceivable that an additional layer of encryption is provided to the package by the DDI 330. The additional layer of encryption may be a layer of encryption to be decrypted by the P-EID 330, or a layer of encryption which may only be decrypted by the controller 300 of the implanted medical device 10, which reduces the risk that unencrypted instructions or packages are intercepted by unauthorized devices. The instructions/parameters are then provided to the P-EID 320", which then loads the instructions/parameters into the during the next charging/energy transfer to the implanted medical device 10 using any of the signal transferring means (wireless or conductive) disclosed herein.

The Health Care Provider EID (HCP EID) 320' have the same features as the P- EID 320" and can communicate with the implanted medical device 10 in the same alternative ways (and combinations of alternative ways) as the P-EID 320‴. However, in addition, the HCP EID 320' also enables the HCP to freely reprogram the controller 300 of the implanted medical device 10, including replacing the entire program code running in the controller 300. The idea is that the HCP EID 320' always remain with the HCP and as such, all updates to the program code or retrieval of data from the implanted medical device 10 using the HCP EID 320' is performed with the HCP present (i.e. not remote). The physical presence of the HCP is an additional layer of security for these updates which may be critical to the function of the implanted medical device 10.

In the embodiment shown in fig. 22f, the HCP communicates with the HCP EID 320' using a HCP Dedicated Device 332 (HCP DD), which is a display device comprising a control interface for controlling and communicating with the HCP EID 320'. As the HCP EID 320' always stays physically at the HCP's clinic, communication between the HCP EID 320' and HCP DD 332 does not have to be sent over the Internet. Instead, the HCP DD 332 and the HCP EID 320' can communicate using one or more of BT, a proprietary wireless communication channel, or a wired connection. The alteration to the programming is then sent to the implanted medical device 10 directly via the HCP EID 320'. Inputting into the HCP DD 332 for direct operation by means of the HCP EID 320' is the same as inputting directly into the HCP EID 320', which then directly transfers the instructions into the implanted medical device 10.

In the embodiment shown in fig. 22f, both the patient and the HCP has a combined hardware key 333',333". The combined keys 333',333" comprises a hardware component comprising a unique circuitry (providing the highest level of security), a wireless NFC-transmitter 339 for transmitting a specific code (providing mid-level security), and a printed QR-code 344 for optical recognition of the card (providing the lowest level of security).

The patient's key 333' in the embodiment shown in fig. 22f is in the form of a key card having an interface for communicating with the P-EID 320‴, such that the key card could be inserted into a key card slot in the P-EID 320". The NFC-transmitter 339 and/or the printed QR-code 344 can be used as means for accessing the control interface 334i of the display device 334. In addition, the display device 334 may require a pin-code and/or a biometric input, such as face recognition or fingerprint recognition.

The HCP's key 333" in the embodiment shown in fig. 22f is in the form of a key card having an interface for communicating with the HCP-EID 320', such that the key card could be inserted into a key card slot in the HCP-EID 320'. The NFC-transmitter 339 and/or the printed QR-code 344 can be used as means for accessing the control interface of the HCP DD 332. In addition, the HCP DD 332 may require a pin-code and/or a biometric input, such as face recognition or fingerprint recognition.

In alternative embodiments, it is however conceivable that the hardware key solution is replaced by a two-factor authentication solution, such as a digital key in combination with a PIN code or a biometric input (such as face recognition and/or fingerprint recognition).

In the embodiment shown in fig. 22f, communication over the Internet takes place over a Dedicated Data Infrastructure (DDI) 330, running on a cloud service. The DDI 330 handles communication between the HCP DD 332 and the P-EID 320‴, between the HCP and the remote control 320", between the HCP and the patient's display device 334, as well as between the HCP and auxiliary devices 336 (such as tools for following up the patient's treatments e.g. a scale in obesity treatment example or a blood pressure monitor in a blood pressure treatment example). In some embodiments, the HCP DD 332 also handles the communication between the patient's display device 334 and the remote control 335. In all examples, the communication from the HCP to: the P-EID 320"', the remote control 320", the patient's display device 334 and the auxiliary devices 336 may be performed using an end-to-end encryption. In embodiments with end-to-end encryption, the communication cannot be decrypted by the DDI 330. In such embodiments, the DDI 330 acts as a router, only passing on encrypted communication from the HCP to various devices. This solution further increases security as the keys for decrypting the information rests only with the HCP and with the device sending or receiving the communication, which reduces the risk that an unencrypted signal is intercepted by an unauthorized device.

In addition to acting as an intermediary or router for communication, the DDI 330 collects data on the implanted medical device 10, on the treatment and on the patient. The data may be collected in an encrypted form, in an anonymized form or in an open form. The form of the collected data may depend on the sensitivity of the data or on the source from which the data is collected. In the embodiment shown in fig. 22f, the DDI 330 sends a questionnaire to the patients display device 334. The questionnaire could comprise questions to the patient related to the general health of the patient, related to the way of life of the patient, or related specifically to the treatment provided by the implanted medical device 10 (such as for example a visual analogue scale for measuring pain). The DDI 330 could compile and/or combine input from several sources and communicate the input to the HCP which could use the provided information to create instructions to the various devices to be sent back over the DDI 330. The data collection performed by the DDI 330 could also be in the form a log to make sure that all communication between the units in the system can be back traced. Logging the communication ensures that all alterations to software or the settings of the software, as well as the frequency and operation of the implanted medical device 10 can be followed. Following the communication enables the DDI 330 or the HCP to follow the treatment and react it something in the communication indicates that the treatment does not provide the intended results or if something appears to be wrong with any of the components in the system.

In the specific embodiment disclosed in fig. 22f, the wireless connections between the different units are as follows. The wireless connection 411 between the auxiliary device 336 and the DDI 330 is based on WiFi or a mobile telecommunication regime and the wireless connection 411 between the auxiliary device 336 and the patient's display device 334 is based on BT. The wireless connection 412 between the patient's display device 334 and the DDI 330 is based on WiFi or a mobile telecommunication regime. The wireless connection 413 between the patient's display device 334 and the remote control 320" is based on BT. The wireless connection 414 between the remote control 320" and the implanted medical device 10 is based on BT and UWB. The wireless connection 415 between the remote control 320" and the DDI 330 is based on WiFi or a mobile telecommunication regime. The wireless connection 416 between the P-EID 320‴ and the implanted medical device 10 is based on BT, UWB and the charging signal. The wireless connection 417 between the P-EID 320‴ and the DDI 330 is based on WiFi or a mobile telecommunication regime. The wireless connection 418 between the HCP-EID 320' and the implanted medical device 10 is based on BT, UWB and the charging signal. The wireless connection 419 between the P-EID 320‴ and the HCP DD 332 is based on BT. The wireless connection 420 between the HPC-EID 320' and the DDI 330 is based on WiFi or a mobile telecommunication regime. The wireless connection 421 between the HPC DD 332 and the DDI 330 is based on WiFi or a mobile telecommunication regime. The wireless connection 422 between the HCP-EID 320' and the HCP DD 332 is based on BT.

The wireless connections specifically described in the embodiment shown in fig. 22f may however be replaced or assisted by wireless connections based on radio frequency identification (RFID), near field charge (NFC), Bluetooth, Bluetooth low energy (BLE), or wireless local area network (WLAN). The mobile telecommunication regimes may for example be 1G, 2G, 3G, 4G, or 5G. The wireless connections may further be based on modulation techniques such as amplitude modulation (AM), frequency modulation (FM), phase modulation (PM), or quadrature amplitude modulation (QAM). The wireless connection may further feature technologies such as time-division multiple access (TDMA), frequency-division multiple access (FDMA), or code-division multiple access (CDMA). The wireless connection may also be based on infra-red (IR) communication. The wireless connection may feature radio frequencies in the high frequency band (HF), very-high frequency band (VHF), and the ultra-high frequency band (UHF) as well as essentially any other applicable band for electromagnetic wave communication. The wireless connection may also be based on ultrasound communication to name at least one example that does not rely on electromagnetic waves.

Although wireless transfer is primarily described in the embodiment disclosed with reference to fig. 22f, the wireless communication between any of the external device may be substituted for wired communication. Also, some or all of the wireless communication between an external device and the implanted medical device 10 may be substituted for conductive communication using a portion of the human body as conductor (such as further described with reference to figs. 22a - 22f).

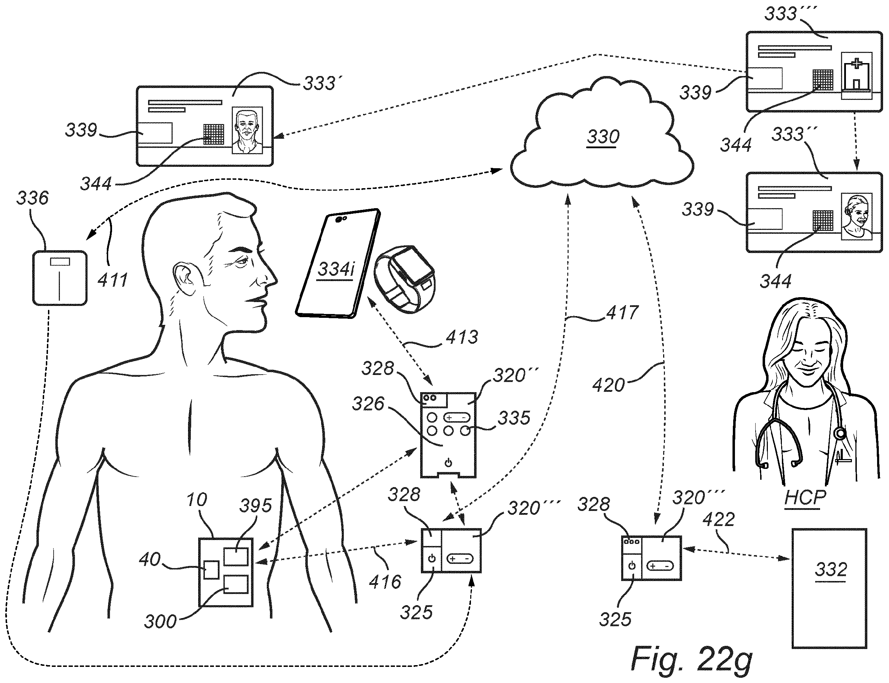

Fig. 22f and fig. 22g show one embodiment of a system for charging, programming and communicating with the controller 300 of the implantable energized medical device 10. Fig. 22f and 22g further describes the communication and interaction between different external devices which may be devices held and operated by the patient, by the health care provider (HCP) or by the Dedicated Data Infrastructure (DDI), which is an infrastructure supplier for example by the manufacturer of the implantable energized medical device 10 or the external devices 320',320",320‴. The system of the embodiment of fig. 22f and 22g comprises three external devices 320',320",320‴ capable of communicating with the controller 300. The basic idea is to ensure the security of the communication with, and the operation of, the medical device 100 by having three external devices 320',320",320‴ with different levels of authority. The lowest level of authority is given to the patient operated remote control 320". The remote control external device 320" is authorized to operate functions of the implantable energized medical device 10 via the implanted controller 300, on the basis of patient input. The remote control 320" is further authorized to fetch some necessary data from the controller 300. The remote control 320" is only capable of operating the controller 300 by communicating with the software currently running on the controller 300, with the currently settings of the software. The next level of authority is given to the Patient External Interrogation Device (P-EID) 320‴, which is a charging and communication unit which is held by the patient but is partially remotely operated by the Health Care Provider (HCP) (Usually a medical doctor with the clinic providing the treatment with help of the implantable energized medical device 10). The P-EID 320‴ is authorized to make setting changes by selecting pre-programmed steps of the software or hardware running on the controller 300 of the implantable energized medical device 10. The P-EID is remotely operated by the HCP, and receives input from the HCP, via the DDI. The highest level of authority is given to the HCP-EID 320' and its controller the HCP Dedicated Display Device (DDD). The HCP-EID 320' is a charging and communication unit which is held by the HCP physically at the clinic of the HCP. The HCP-EID 320' is authorized to freely alter or replace the software running on the controller 300, when the patient is physically in the clinic of the HCP. The HCP-EID 320' is controlled by the HCP DDD, which either acts on a "webview" portal from the HCP-EID or is a device closed down to any activities (which may include the absence of an internet connection) other than controlling and communicating with the HCP-EID. The webview portal does not necessarily mean internet based or HTML-protocol and the webview portal may be communicated over other communicating protocols such as Bluetooth or any other type of standard or proprietary protocol. The HCP DDD may also communicate with the HCP-EID over a local network or via Bluetooth or other standard or proprietary protocols.

Starting from the lowest level of authority, the patient remote control external device 320" comprises a wireless transceiver 328 for communicating with the implantable energized medical device 10. The remote control 320" is capable of controlling the operation of the implantable energized medical device 10 via the controller 300, by controlling pre-set functions of the implantable energized medical device 10, e.g. for operating an active portion of the implantable energized medical device 10 for performing the intended function of the implantable energized medical device 10. The remote control 320" is able communicate with implantable energized medical device 10 using any standard or proprietary protocol designed for the purpose. In the embodiment shown in fig. 22f and 22g, the wireless transceiver 328 comprises a Bluetooth (BT) transceiver, and the remote control 320" is configured to communicate with implantable energized medical device 10 using BT. In an alternative configuration, the remote control 320" communicates with the implantable energized medical device 10 using a combination of Ultra-Wide Band (UWB) wireless communication and BT. The use of UWB technology enables positioning of the remote control 320" which can be used by the implantable energized medical device 10 as a way to establish that the remote control 320" is at a position which the implantable energized medical device 10 and/or the patient can acknowledge as being correct, e.g. in the direct proximity to the medical device 100 and/or the patient, such as within reach of the patient and/or within 1 or 2 meters of the implantable energized medical device 10.

UWB communication is performed by the generation of radio energy at specific time intervals and occupying a large bandwidth, thus enabling pulse-position or time modulation. The information can also be modulated on UWB signals (pulses) by encoding the polarity of the pulse, its amplitude and/or by using orthogonal pulses. A UWB radio system can be used to determine the "time of flight" of the transmission at various frequencies. This helps overcome multipath propagation, since some of the frequencies have a line-of-sight trajectory, while other indirect paths have longer delay. With a cooperative symmetric two-way metering technique, distances can be measured to high resolution and accuracy. UWB is useful for real-time location systems, and its precision capabilities and low power make it well-suited for radio-frequency-sensitive environments, such as health care environments.

In embodiments in which a combination of BT and UWB technology is used, the UWB technology may be used for location-based authentication of the remote control 320", whereas the communication and/or data transfer could take place using BT or any other way of communicating different from the UWB. The UWB signal could in some embodiments also be used as a wake-up signal for the controller 300, or for the BT transceiver, such that the BT transceiver in the implantable energized medical device 10 can be turned off when not in use, which eliminates the risk that the BT is intercepted, or that the controller 300 of the implantable energized medical device 10 is hacked by means of BT communication. In embodiments in which a BT (or alternatives) / UWB combination is used, the UWB connection may be used also for the transmission of data. In the alternative, the UWB connection could be used for the transmission of some portions of the data, such as sensitive portions of the data, or for the transmission of keys for the unlocking of encrypted communication sent over BT.

The remote control 320" comprises computing unit 326 which runs a software application for communicating with the implantable energized medical device 10. The computing unit 326 can receive input directly from control buttons 335 arranged on the remote control 320" or may receive input from a control interface 334i displayed on a patient display device 334 operated by the patient. In the embodiments in which the remote control 320" receives input from a control interface 334i displayed on the patient display device 334 operated by the patient, the remote control 320" transmits the control interface 334i in the form of a web-view portal, i.e. a remote interface that run in a sandbox environment on the patient's display device 334. A sandbox environment means that it runs on the display device 334 but only displays what is presented from the remote control and can only use a tightly controlled set of commands and resources, such as storage and memory space as well as network access, the ability to inspect the host system and read or write from other input devices connected to the display device 334 is extremely limited. Any action or command generated by the patient display device is like controlling a webpage. All acting software is located on the remote control that only displays its control interface onto the patient display unit. The computing unit 326 is further configured to encrypt the control interface before transmission to the patient display device 334, and encrypt the control commands before transmission to the implantable energized medical device 10. The computing unit 326 is further configured to transform the received user input into control commands for wireless transmission to the implantable energized medical device 10.

The patient's display device 334 could for example be a mobile phone, a tablet or a smart watch. In the embodiment shown in fig. 22f and 22g, the patient's display device 334 communicates with the remote control 320" by means of BT. The control interface 334i in the form of a web-view portal is transmitted from the remote control 320" to the patient's display device 334 over BT. Control commands in the form of inputs from the patient to the control interface 334i is transmitted from the patient's display device 334 to the remote control 320", providing input to the remote control 320" equivalent to the input that may be provided using the control buttons 335. The control commands created in the patient's display device 334 is encrypted in the patient's display device 334 and transmitted to the remote control 320' using BT or any other communication protocol.

The remote control is normally not connected to the DDI or the Internet to increase security. In addition, the remote control 320" may in one embodiment have its own private key and in a specific embodiment the remote control 320" is activated by the patient's private key for a certain time period. This may activate the function of the patient's display device and the remote wed-view display portal supplied by the remote control to the patient's display device.

The patient's private key is supplied in a patient private key device compromising a smartcard that may be inserted or provided close to the remote control 320" to activate a permission to communicate with the implant 100 for a certain time period.

The patient's display device 334 may (in the case of the display device 334 being a mobile phone or tablet) comprise auxiliary radio transmitters for providing auxiliary radio connection, such as Wi-Fi or mobile connectivity (e.g. according to the 3G,4G or 5G standards). The auxiliary radio connection(s) may have to be disconnected to enable communication with the remote control 320". Disconnecting the auxiliary radio connections reduces the risk that the integrity of the control interface 334i displayed on the patient's display device 334 is compromised, or that the control interface 334i displayed on the patient's display device 334 is remote controlled by an unauthorized device.

In alternative embodiments, control commands are generated and encrypted by the patient's display device and transmitted to the DDI 330. The DDI 330 could either alter the created control commands to commands readable by the remote control 320" before further encrypting the control commands for transmission to the remote control 320" or could simply add an extra layer of encryption before transmitting the control commands to the remote control 320", or could simply act as a router for relaying the control commands from the patients' display device 334 to the remote control 320". It is also conceivable that the DDI 330 adds a layer of end-to-end encryption directed at the implantable energized medical device 10, such that only the implantable energized medical device 10 can decrypt the control commands to perform the commands intended by the patient. In the embodiments above, when the patient remote display device 334 is communicating with the DDI, the patient's display device 334 may be configured to only display and interact with a web-view portal provided by a section of the DDI and it is conceivable that the web-view portal is a view of a back-end provided on the DDI 330, and in such embodiments, the patient interacting with the control interface on the patient's display device 334 is equivalent to the patient interacting with an area of the DDI 330.

The patient's display device 334 could have a first and second application related to the implantable energized medical device 10. The first application is the control application displaying the control interface 334i for control of the implantable energized medical device 10, whereas the second application is a general application for providing the patient with general information of the status of the implantable energized medical device 10 or information from the DDI 330 or HCP, or for providing an interface for the patient to provide general input to the DDI 330 or HCP related to the general wellbeing of the patient, the lifestyle of the patient or related to general input from the patient concerning the function of the implantable energized medical device 10. The second application, which do not provide input to the remote control 320" and/or the implantable energized medical device 10 thus handles data which is less sensitive. As such, the general application could be configured to function also when all auxiliary radio connections are activated, whereas switching to the control application which handles the more sensitive control commands and communication with the implantable energized medical device 10 could require that the auxiliary radio connections are temporarily de-activated. It is also conceivable that the control application is a sub-application running within the general application, in which case the activation of the control application as a sub-application in the general application could require the temporary de-activation of auxiliary radio connections. In the embodiment shown in fig. 22f, access to the control application requires the use of the optical and/or NFC means of the hardware key 333' in combination with biometric input to the patient's display device, whereas accessing the general application only requires biometric input to the patient's display device and/or a pin code. In the alternative, a two-factor authentication solution, such as a digital key in combination with a pin code could be used for accessing the general application and/or the control application.

In general, a hardware key is needed to activate the patient display device 334 for certain time period to control the web-view portal of the remote control 320", displaying the control interface 334i for control of the implantable energized medical device 10.

In the embodiments in which the patients display device 334 is configured to only display and interact with a web-view provided by another unit in the system, it is conceivable that the web-view portal is a view of a back-end provided on the DDI 330, and in such embodiments, the patient interacting with the control interface on the patient's display device is equivalent to the patient interacting with an area of the DDI 330.

Moving now to the P-EID 320"'. The P-EID 320‴ is an external device used by the patient, patient external device, which communicates with, and charges, the implantable energized medical device 10. The P-EID 320‴ can be remotely controlled by the HCP to read information from the implantable energized medical device 10. The P-EID 320‴ controls the operation of the implantable energized medical device 10, control the charging of the medical device 100, and adjusts the settings on the controller 300 of the implantable energized medical device 10 by changing pre-defined pre-programed steps and/or by the selection of pre-defined parameters within a defined range., e.g. Just as the remote control 320", the P-EID 320‴ could be configured to communicate with the implantable energized medical device 10 using BT or UWB communication or any other proprietary or standard communication method. Since the device may be used for charging the implant, the charging signal and communication could be combined. Just as with the remote control 320", it is also conceivable to use a combination of UWB wireless communication and BT for enabling positioning of the P-EID 320" as a way to establish that the P-EID 320" is at a position which the implantable energized medical device 10 and/or patient and/or HCP can acknowledge as being correct, e.g. in the direct proximity to the correct patient and/or the correct medical device 100. Just as for the remote control 320", in embodiments in which a combination of BT and UWB technology is used, the UWB technology may be used for location-based authentication of the P-EID 320", whereas the communication and/or data transfer could take place using BT. The P-EID 320" comprises a wireless transmitter/transceiver 328 for communication and also comprises a wireless transmitter 325 configured for transferring energy wirelessly, which may be in the form of a magnetic field or any other signal such as electromagnetic, radio, light, sound or any other type of signal to transfer energy wirelessly to a wireless receiver 395 of the implantable energized medical device 10. The wireless receiver 395 of the implantable energized medical device 10 is configured to receive the energy in the form of the magnetic field and transform the energy into electric energy for storage in an implanted energy storage unit 40, and/or for consumption in an energy consuming part of the implantable energized medical device 10 (such as the operation device, controller 300 etc.). The magnetic field generated in the P-EID 320‴ and received in the implantable energized medical device 10 is denoted charging signal. In addition to enabling the wireless transfer of energy from the P-EID 320‴ to the implanted medical implant 10, the charging signal may also function as a means of communication. E.g., variations in the frequency of the transmission, and/or the amplitude of the signal may be uses as signaling means for enabling communication in one direction, from the P-EID 320‴ to the implantable energized medical device 10, or in both directions between the P-EID 320‴ and the implantable energized medical device 10. The charging signal in the embodiment shown in fig. 22f is a signal in the range 10 65kHz or 115 - 140 kHz and the communication follow a proprietary communication signaling protocol, i.e., it is not based on an open standard. In alternative embodiments, BT could be combined with communication using the charging signal, or communication using the charging signal could be combined with an UWB signal. The energy signal could also be used as a carrying signal for the communication signal.

Just as for the remote control 320", the UWB signal could in some embodiments also be used as a wake-up signal for the controller 300, or for the BT transceiver, such that the BT transceiver in the implantable energized medical device 10 can be turned off when not in use, which eliminates the risk that the BT is intercepted, or that the controller 300 of the implantable energized medical device 10 is hacked by means of BT communication. In the alternative, the charging signal could be used as a wakeup signal for the BT, as the charging signal does not travel very far. Also, as a means of location-based authentication, the effect of the charging signal or the RSSI could be assessed by the controller 300 in the implantable energized medical device 10 to establish that the transmitter is within a defined range. In the BT/UWB combination, the UWB may be used also for transmission of data. In some embodiments, the UWB and/or the charging signal could be used for the transmission of some portions of the data, such as sensitive portions of the data, or for the transmission keys for unlocking encrypted communication sent by BT. Wake-up could be performed with any other signal.

UWB could also be used for waking up the charging signal transmission, to start the wireless transfer of energy or for initiating communication using the charging signal. As the signal for transferring energy has a very high effect in relation to normal radio communication signals, the signal for transferring energy cannot be active all the time, as this signal may be hazardous e.g., by generating heat.

The P-EID 320‴ communicates with the HCP over the Internet by means of a secure communication, such as over a VPN. The communication between the HCP and the P-EID 320‴ is preferably encrypted. Preferably, the communication is sent via the DDI, which may only be relying the information. The communication from the HCP to the implantable energized medical device 10 may be performed using an end-to-end encryption, in which case the communication cannot be decrypted by the P-EID 320"'. In such embodiments, the P-EID 320‴ acts as a router, only passing on encrypted communication from the HCP to the controller 300 of the implantable energized medical device 10 (without full decryption). This solution further increases security as the keys for decrypting the information rests only with the HCP and with the implantable energized medical device 10, which reduces the risk that an unencrypted signal is intercepted by an unauthorized device. The P-EID 320‴ may add own encryption or information, specifically for security reasons. The P-EID 320‴ may hold its own private key and may be allowed to communicate with the implant 100 based on confirmation from the patient's private key, which may be provided as a smartcard to be inserted in a slot of the P-EID 320‴ or hold in close proximity thereto to be read by the P-EID 320"'. These two keys will add a high level of security to the performed communication between the Implant 100 and the P-EID 320‴ since the patient's hardware key in this example on the smartcard may activate and thereby allow the communication and action taken in relation to the implant. The P-EID 320‴ may as previously described change the treatment setting of the implant by selecting pre-programmed steps of the treatment possibilities. Such pre-programmed treatment options may include for example to change:
at least one of the position, frequency and level of compression of an implanted heart compression device,
the flow of an apparatus assisting the pump function of a heart of the patient,
the flow of an apparatus assisting the pump function comprising a turbine bump placed within a patient's blood vessel for assisting the pump function of the heart,
the function of an operable artificial heart valve,
at least one of the function of, the valve opening pressure and time for closure of an operable artificial heart valve for increasing the blood flow to the coronary arteries.
at least one of the functions of, the amount and/or concentration of a drug from an implantable drug delivery device,
at least one of the injection site and frequency as well as amount of drug delivered by an implantable drug delivery device for injecting directly into a blood vessel and change the position of the injection site, all from within the patient's body,
at least one of the injection site and frequency as well as amount of drug delivered by an implantable drug delivery device for injecting potency enhancing drugs into an erectile tissue of the patient,
at least one of the level of constriction, pressure or position of a hydraulic, mechanic, and/or electric constriction implant,
the volume of an operable volume filling device,
the constriction of an operable gastric band,
at least one of the level and time of stretching and when such stretching occur in relation to food intake of a patient for an operable implant for stretching the stomach wall of the patient for creating satiety,
when an action should be taken relating to an implant configured to sense the amount of food intake based on number of times a patient swallows solid food,
at least one of the size and shape of an operable cosmetic implant,
at least one of the shape and size in the breast region of a patient of an operable cosmetic implant for adjustment,
at least one of pressure, volume, sensor input or time of an implant controlling medical device for the emptying of a urinary bladder,
at least one of the closing pressure, the time to close after urinating, how much extra pressure would be allowed at exercise of an implant hindering urinary leakage,
at least one of the closing pressure, the time to close after revealing, how much extra pressure would be allowed at exercise of an implant hindering anal incontinence,
parameters of an implant controlling the emptying of fecal matter, such as pressure, volume, pump or motor position etc.,
parameters of an implant monitoring an aneurysm, such as pressure, aneurysm expansion, volume, reservoir volume, etc.,
parameters of an implant for hindering the expansion of an aneurysm, such as pressure, aneurysm expansion, volume, reservoir volume, etc.,
parameters of an implant lubricating a joint, such as volume, reservoir volume, etc.,
parameters of an implant for affecting the blood flow to an erectile tissue of the patient, such as the level of constriction, pressure or position of a hydraulic, mechanic, and/or electric constriction implant,
parameters of an implant for simulating the engorgement of an erectile tissue, such as the level of stimulation, frequency, or amplitude of an electrical stimulation,
parameters of an implant with a reservoir for holding bodily fluids, such as volume, reservoir volume, etc.,
parameters of an implant storing and/or emptying a bodily reservoir or a surgically created reservoir, such as stimulation parameters in a peristaltic wave, stretch or bending sensors, reservoir volume, etc.,
parameters of an implant communicating with a database outside the body, such as key handshake, new key pairing, signal amplitude etc.,
parameters of an implant able to be programmed from outside the body,
parameters of an implant able to be programmed from outside the body with a wireless signal,
parameters of an implant treating impotence, such as pressure, amount of drug delivered, time for erection period etc.,
parameters of an implant controlling the flow of eggs in the uterine tube, such as the level of constriction, time period, position of a hydraulic, mechanic, and/or electric constriction implant,
parameters of an implant controlling the flow of sperms in the uterine tube, such as the level of stimulation, frequency, or amplitude of an electrical stimulation,
parameters of an implant controlling the flow of sperms in the vas deferens, such as the level of constriction, time period, position of a hydraulic, mechanic, and/or electric constriction implant,
parameters of an implant for hindering the transportation of the sperm in the vas deferens, such as the level of constriction, time period, position of a hydraulic, mechanic, and/or electric constriction implant,
parameters of an implant treating osteoarthritis,
parameters of an implant performing a test of parameters inside the body,
parameters of an implant controlling specific treatment parameters from inside the body,
parameters of an implant controlling bodily parameters from inside the body,
parameters of an implant controlling the blood pressure,
parameters of an implant controlling the blood pressure by affecting the dilatation of the renal artery, such as heat and time period in relation to blood pressure,
parameters of an implant controlling a drug treatment parameter,
parameters of an implant controlling a parameter in the blood,
parameters of an implant for adjusting or replacing any bone part of a body of the patient,
parameters of an implant replacing an organ of the patient or part of an organ of the patient or the function thereof,
parameters of a vascular treatment device, such as bending, expanding sensor,
parameters of an implant adapted to move fluid inside the body of the patient, such as volume, pumping parameters,
parameters of an implant configured to sense a parameter related to the patient swallowing,
parameters of an implant configured to exercise a muscle with electrical or mechanical stimulation, such as stimulation parameters, amplitude frequency time period etc.,
parameters of an implant configured for emptying an intestine portion on command, such as electrical stimulation parameters, peristaltic wave adjustment etc.,
parameters of an operable implant configured to be invaginated in the stomach of the patient to reduce the volume of the stomach substantially more than the volume of the device, such as volume,
parameters of an implant configured for emptying the urinary bladder from within the patient's body by compressing the bladder, such as pressure, volume and time
parameters of an implant configured for draining fluid from within the patient's body,
parameters of an implant configured for the active lubrication of a joint with an added lubrication fluid, such as frequency and/or volume of the drug supplied,
parameters of an implant configured for removing clots and particles from the patient's blood stream,
parameters of an implant configured for elongating or straightening a bone in the patient, to reduce scoliosis, force, length etc.,
parameters of a device to stimulate the brain for a several position to a focused point,
parameters of an artificial stomach replacing the function of the natural stomach,
parameters of an implant configured for adjusting the position of a female's urinary tract or bladder neck,
parameters of an implant configured for stimulating the ampulla vas deference and creating temporary constriction.

When the implantable energized medical device 10 is to be controlled and/or updated remotely by the HCP, via the P-EID 320‴, a HCP Dedicated Device (DD) 332 displays an interface in which predefined program steps or setting values are presented to the HCP. The HCP provides input to the HCP DD 332 by selecting program steps, altering settings and/or values or by altering the order in which pre-defined program steps is to be executed. The instructions/parameters inputted into the HCP DD 332 for remote operation is in the embodiment shown in fig. 22f routed to the P-EID 320‴ via the DDI 330, which may or may not be able to decrypt/read the instructions. The DDI 330 may store the instructions for a time period to later transfer the instructions in a package of created instructions to the P-EID 320‴. It is also conceivable that an additional layer of encryption is provided to the package by the DDI 330. The additional layer of encryption may be a layer of encryption to be decrypted by the P-EID 330, or a layer of encryption which may only be decrypted by the controller 300 of the implantable energized medical device 10, which reduces the risk that unencrypted instructions or packages are intercepted by unauthorized devices. The instructions/parameters are then provided to the P-EID 320", which then loads the instructions/parameters into the during the next charging/energy transfer to the implantable energized medical device 10 using any of the signal transferring means (wireless or conductive) disclosed herein.

The Health Care Provider EID (HCP EID) 320' have the same features as the P- EID 320" and can communicate with the implantable energized medical device 10 in the same alternative ways (and combinations of alternative ways) as the P-EID 320"'. However, in addition, the HCP EID 320' also enables the HCP to freely reprogram the controller 300 of the implantable energized medical device 10, including replacing the entire program code running in the controller 300. The idea is that the HCP EID 320' always remain with the HCP and as such, all updates to the program code or retrieval of data from the implantable energized medical device 10 using the HCP EID 320' is performed with the HCP and patient present (i.e. not remote). The physical presence of the HCP is an additional layer of security for these updates which may be critical to the function of the implantable energized medical device 10.

In the embodiment shown in fig. 22f, the HCP communicates with the HCP EID 320' using a HCP Dedicated Display Device 332 (HCP DDD), which is a HCP display device comprising a control interface for controlling and communicating with the HCP EID 320'. As the HCP EID 320' always stays physically at the HCP's clinic, communication between the HCP EID 320' and HCP DDD 332 does not have to be sent over the Internet. Instead, the HCP DDD 332 and the HCP EID 320' can communicate using one or more of BT, a proprietary wireless communication channel, or a wired connection. The alteration to the programming is then sent to the implantable energized medical device 10 directly via the HCP EID 320'. Inputting into the HCP DDD 332 for direct operation by means of the HCP EID 320' is the same as inputting directly into the HCP EID 320', which then directly transfers the instructions into the implantable energized medical device 10.

In the embodiment shown in fig. 22f, both the patient and the HCP has a combined hardware key 333',333". The combined keys 333',333" comprises a hardware component comprising a unique circuitry (providing the highest level of security), a wireless NFC-transmitter 339 for transmitting a specific code (providing mid-level security), and a printed QR-code 344 for optical recognition of the card (providing the lowest level of security). The HCP private key is supplied by a HCP private key device 333" adapted to be provided to the HCP EID external device via at least one of; a reading slot or comparable for the HCP private key device 333", an RFID communication or other close distance wireless activation communication to both the HCP EID 320' and the HCP DDD 332 if used. The HCP DDD 332 will be activated by such HCP private key device 333", which for example may comprise at least one of, a smartcard, a key-ring device, a watch an arm or wrist band a neckless or any shape device.

The HCP EID external device may comprise at least one of;
a reading slot or comparable for the HCP private key device,
an RFID communication and
other close distance wireless activation communication means

The HCP external device 320' may further comprise at least one wireless transceiver 328 configured for communication with a data infrastructure server, DDI, through a first network protocol.

A dedicated data infrastructure server, DDI, is in one embodiment adapted to receive commands from said HCP external device 320' and may be adapted to rely the received commands without opening said commands directed to the patient external device 320", the DDI 330 comprising one wireless transceiver configured for communication with said patient external device 320".

The patient EID external device 320" is in one embodiment adapted to receive the commands relayed by the DDI, and further adapted to send these commands to the implantable energized medical device 10, which is adapted to receive commands from the HCP, Health Care Provider, via the DDI 330 to change the pre-programmed treatment steps of the implantable energized medical device 10. The patient EID is adapted to be activated and authenticated and allowed to perform the commands by the patient providing a patient private key device 333'. The patient's private key device is in one embodiment adapted to be provided to the patient external device by the patient via at least one of; a reading slot or comparable for the patient private key device 333', an RFID communication or other close distance wireless activation communication.

The patient EID external device, in one or more embodiments, comprises at least one of;
a reading slot or comparable for the HCP private key device,
an RFID communication, or
other close distance wireless activation communication

The patient EID external device may in one or more embodiments comprise at least one wireless transceiver configured for communication with the implanted medical device through a second network protocol.

The patient's key 333' is in the embodiment shown in fig. 22f in the form of a key card having an interface for communicating with the P-EID 320‴, such that the key card could be inserted into a key card slot in the P-EID 320". The NFC-transmitter 339 and/or the printed QR-code 344 can be used as means for accessing the control interface 334i of the display device 334. In addition, the display device 334 may require a pin-code and/or a biometric input, such as face recognition or fingerprint recognition.

The HCP's key 333", in the embodiment shown in fig. 22f is in the form of a key card having an interface for communicating with the HCP-EID 320', such that in one embodiment the key card could be inserted into a key card slot in the HCP-EID 320'. The NFC-transmitter 339 and/or the printed QR-code 344 can be used as means for accessing the control interface of the HCP DDD 332. In addition, the HCP DDD 332 may require a pin-code and/or a biometric input, such as face recognition or fingerprint recognition.

In alternative embodiments, it is however conceivable that the hardware key solution is replaced by a two-factor authentication solution, such as a digital key in combination with a PIN code or a biometric input (such as face recognition and/or fingerprint recognition). The key could also be a software key, holding similar advance key features, such as the Swedish Bank ID being a good example thereof.

In the embodiment shown in fig. 22f, communication over the Internet takes place over a Dedicated Data Infrastructure (DDI) 330, running on a cloud service. The DDI 330 in this case handles communication between the HCP DDD 332 and the P-EID 320"'. however, the more likely scenario is that the HCP DDD 332 is closed down, such that only the necessary functions of the control application can function on the HCP DDD 332. In the closed down embodiment, the HCP DDD 332 is only able to give the necessary commands to HCP EID 320' to further update the pre-programmed treatment steps of the Implant 100 via the P-EID 320‴ in direct contact, or more likely indirect contact via the DDI 332. If the patient is present locally, the HCP EID may communicate and act directly on the patient's implant. However, before anything is accepted by the implant, a patient private key device 333' has to be presented to the P EID 320‴ or HCP EID 320' for maximum security.

The DDI 330 is logging information of the contact between the HCP and the remote control 320" via implant feedback data supplied from the implant to P-EID 320"'. Data generated between the HCP and the patient's display device 334, as well as between the HCP and auxiliary devices 336 (such as tools for following up the patient's treatments e.g. a scale in obesity treatment example or a blood pressure monitor in a blood pressure treatment example) are logged by the DDI 330. In some embodiments, although less likely, the HCP DDD 332 may also handle the communication between the patient's display device 334 and the remote control 320". In fig. 22g, the and auxiliary devices 336 is connected to the P-EID as well and can thus provide input from the auxiliary devices 336 to the P-EID which can be used by the P-EID for altering the treatment or for follow up.

In all examples, the communication from the HCP to: the P-EID 320‴, the remote control 320", the patient's display device 334 and the auxiliary devices 336 may be performed using an end-to-end encryption. In embodiments with end-to-end encryption, the communication cannot be decrypted by the DDI 330. In such embodiments, the DDI 330 acts as a router, only passing on encrypted communication from the HCP to various devices. This solution further increases security as the keys for decrypting the information rests only with the HCP and with the device sending or receiving the communication, which reduces the risk that an unencrypted signal is intercepted by an unauthorized device. The P-EID 320‴ may also only pass on encrypted information.

In addition to acting as an intermediary or router for communication, the DDI 330 collects data on the implantable energized medical device 10, on the treatment and on the patient. The data may be collected in an encrypted form, in an anonymized form or in an open form. The form of the collected data may depend on the sensitivity of the data or on the source from which the data is collected. In the embodiment shown in fig. 22f, the DDI 330 sends a questionnaire to the patient's display device 334. The questionnaire could comprise questions to the patient related to the general health of the patient, related to the way of life of the patient, or related specifically to the treatment provided by the implantable energized medical device 10 (such as for example a visual analogue scale for measuring pain). The DDI 330 could compile and/or combine input from several sources and communicate the input to the HCP which could use the provided information to create instructions to the various devices to be sent back over the DDI 330. The data collection performed by the DDI 330 could also be in the form a log to make sure that all communication between the units in the system can be back traced. Logging the communication ensures that all alterations to software or the settings of the software, as well as the frequency and operation of the implantable energized medical device 10 can be followed. Following the communication enables the DDI 330 or the HCP to follow the treatment and react it something in the communication indicates that the treatment does not provide the intended results or if something appears to be wrong with any of the components in the system. If patient feedback from the patient display device 334 indicates that a new treatment step of the implant is needed, such information must be confirmed by direct contact between HCP and patient.

In the specific embodiment disclosed in fig. 22f, the wireless connections between the different units are as follows. The wireless connection 411 between the auxiliary device 336 and the DDI 330 is based on WiFi or a mobile telecommunication regime or may be sent to the DDI 330 via the P-EID 320‴ and the wireless connection 411 between the auxiliary device 336 and the patient's display device 334 is based on BT or any other communication pathway disclosed herein. The wireless connection 412 between the patient's display device 334 and the DDI 330 is based on WiFi or a mobile telecommunication regime. The wireless connection 413 between the patient's display device 334 and the remote control 320" is based on BT or any other communication pathway disclosed herein. The wireless connection 414 between the patient remote control 320" and the implantable energized medical device 10 is based on BT and UWB or any other communication pathway disclosed herein. The wireless connection 415 between the remote control 320" and the DDI 330 is likely to not be used, and if present be based on WiFi or a mobile telecommunication regime. The wireless connection 416 between the P-EID 320‴ and the implantable energized medical device 10 is based on BT, UWB and the charging signal or any other communication or energizing pathway disclosed herein. The wireless connection 417 between the P-EID 320‴ and the DDI 330 is based on WiFi or a mobile telecommunication regime. The wireless connection 418 between the HCP-EID 320' and the implantable energized medical device 10 is based on at least one of the BT, UWB and the charging signal. The wireless connection 419 between the P-EID 320‴ and the HCP DD 332 is based on BT or any other communication path disclosed herein. The wireless connection 420 between the HPC-EID 320' and the DDI 330 is based on WiFi or a mobile telecommunication regime. The wireless connection 421 between the HPC DD 332 and the DDI 330 is normally closed and not used and if so based on WiFi or a mobile telecommunication regime. The wireless connection 422 between the HCP-EID 320' and the HCP DD 332 is based on at least one of BT, UWB, local network or any other communication path disclosed herein.

The wireless connections specifically described in the embodiment shown in fig. 22f may however be replaced or assisted by wireless connections based on radio frequency identification (RFID), near field communication (NFC), Bluetooth, Bluetooth low energy (BLE), or wireless local area network (WLAN). The mobile telecommunication regimes may for example be 1G, 2G, 3G, 4G, or 5G. The wireless connections may further be based on modulation techniques such as amplitude modulation (AM), frequency modulation (FM), phase modulation (PM), or quadrature amplitude modulation (QAM). The wireless connection may further feature technologies such as time-division multiple access (TDMA), frequency-division multiple access (FDMA), or code-division multiple access (CDMA). The wireless connection may also be based on infra-red (IR) communication. The wireless connection may feature radio frequencies in the high frequency band (HF), very-high frequency band (VHF), and the ultra-high frequency band (UHF) as well as essentially any other applicable band for electromagnetic wave communication. The wireless connection may also be based on ultrasound communication to name at least one example that does not rely on electromagnetic waves.

Fig. 22g also discloses a master private key 333‴ device that allow issuance of new private key device wherein the HCP or HCP admin have such master private key 333‴ device adapted to be able to replace and pair a new patient private key 333' device or HCP private key device 333" into the system, through the HCP EID external device 320'.

A system configured for changing pre-programmed treatment settings of an implantable medical device, when implanted in a patient, from a distant remote location in relation to the patient, the system comprising:
Fig. 22g also discloses a scenario in which at least one health care provider, HCP, external device 320' is adapted to receive a command from the HCP to change said pre-programmed treatment settings of an implantable energized medical device 10, further adapted to be activated and authenticated and allowed to perform said command by the HCP providing a HCP private key device 333". The HCP EID external device 320' further comprising at least one wireless transceiver 328 configured for communication with a patient EID external device 320‴, through a first network protocol. The system comprises the patient EID external device 320‴, the patient EID external 320‴ device being adapted to receive command from said HCP external device 320', and to relay the received command without modifying said command to the implantable energized medical device 10. The patient EID external device 320‴ comprising one wireless transceiver 328. The patient EID 320‴ is adapted to send the command to the implantable energized medical device 10, to receive a command from the HCP to change said pre-programmed treatment settings of the implantable energized medical device 10, and further to be activated and authenticated and allowed to perform said command by the patient providing a patient private key 333' device comprising a patient private key.

Although wireless transfer is primarily described in the embodiment disclosed with reference to figs. 22f, 22g the wireless communication between any of the external device may be substituted for wired communication. Also, some or all of the wireless communication between an external device and the implantable energized medical device 10 may be substituted for conductive communication using a portion of the human body as conductor.

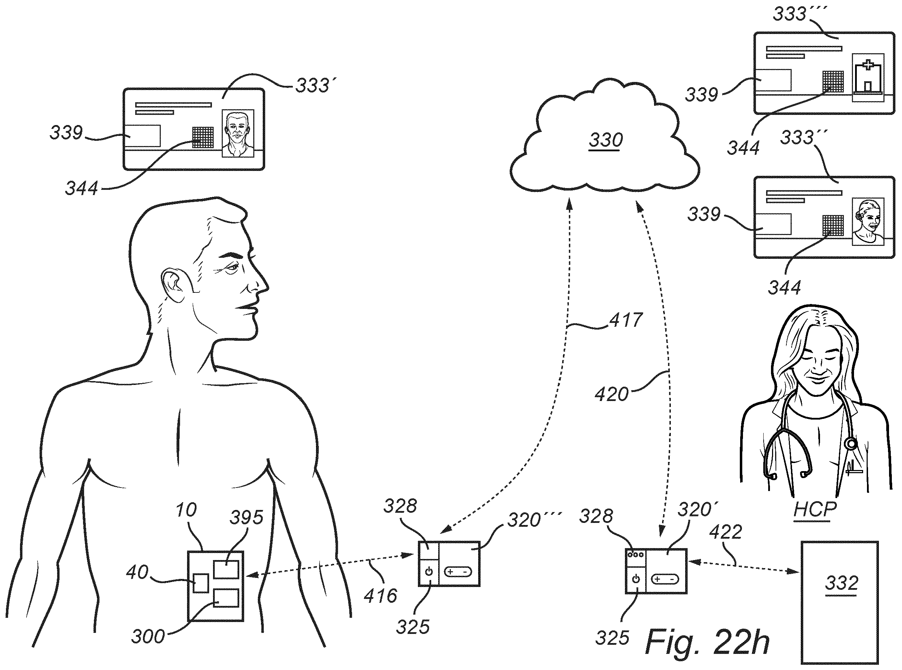

Fig. 22h shows a portion of fig. 22f, in which some of the components have been omitted to outline a specific scenario. In the scenario outlined in fig. 22h, the system is configured for changing pre-programmed treatment settings of an implantable energized medical device 10, when implanted in a patient, from a distant remote location in relation to the patient. The system if fig. 22h comprises at least one HCP EID 320' external device adapted to receive commands from the HCP to change said pre-programmed treatment settings of an implantable energized medical device 10. The HCP EID 320' external device is further adapted to be activated and authenticated and allowed to perform said command by the HCP providing a HCP private key device 333" adapted to be provided to the HCP EID external device 320'. The private key device 333" is adapted to be provided to the HCP EID external device 320' via at least one of: a reading slot or comparable for the HCP private key device 333", and an RFID communication or other close distance wireless activation communication. The HCP EID external device 320' comprises at least one of: a reading slot or comparable for the HCP private key device 333", an RFID communication, and other close distance wireless activation communication or electrical direct contact. The HCP EID external device 320' further comprises at least one wireless transceiver 328 configured for communication with a dedicated data infrastructure server (DDI) 330, through a first network protocol. The system further comprises a dedicated data infrastructure server (DDI) 330, adapted to receive command from said HCP EID external device 320', adapted to relay the received commands without modifying said command to a patient EID external device 320"'. The dedicated data infrastructure server (DDI) 330 further comprises a wireless transceiver 328 configured for communication with said patient external device. The system further comprises a patient EID external device 320‴ adapted to receive the command relayed by the dedicated data infrastructure server (DDI) 330 and further adapted to send commands to the implantable energized medical device 10 and further adapted to receive commands from the HCP EID external device 320' via the dedicated data infrastructure server (DDI) 330 to change said pre-programmed treatment settings of the implantable energized medical device 10. The patient EID external device 320‴, and further adapted to be activated and authenticated and allowed to perform said command by the patient providing a patient private key device 333' adapted to be provided to the patient EID external device 320‴ by the patient via at least one of: a reading slot or comparable for the patient private key device 333', an RFID communication or other close distance wireless activation communication or electrical direct contact. The patient EID external device 320‴ further comprises at least one of: a reading slot or comparable for the HCP private key device, an RFID communication and other close distance wireless activation communication or electrical direct contact. The patient EID external device 320‴ further comprises at least one wireless transceiver 328 configured for communication with the implantable energized medical device 10 through a second network protocol. The implantable energized medical device 10 is in turn configured to treat the patient or perform a bodily function.

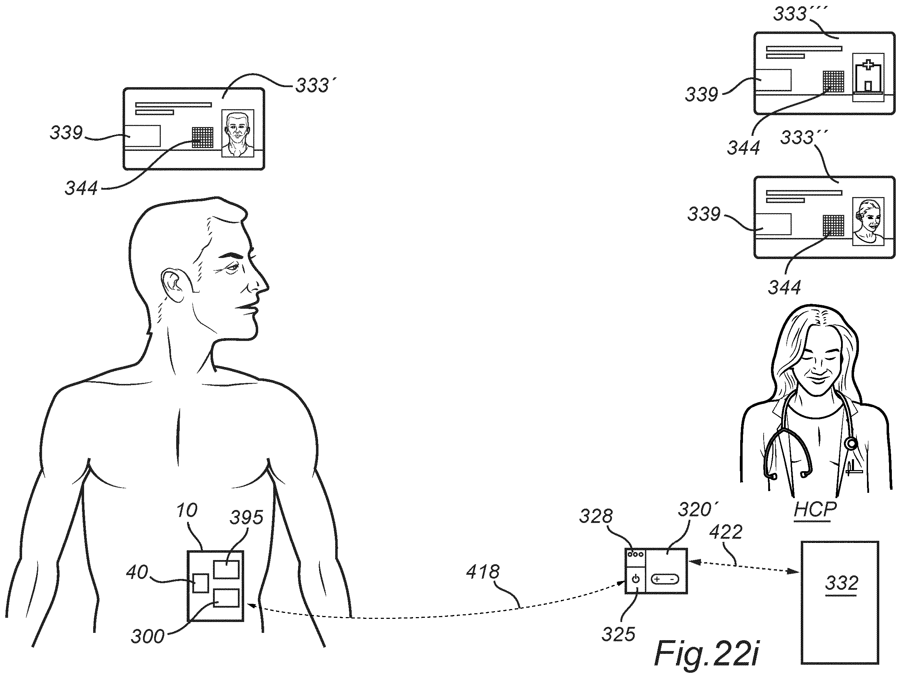

The scenario described with reference to fig. 22h may in alternative embodiments be complemented with additional units or communication connections, or combined with any of the scenarios described with reference to figures 22i - 22m.

Fig. 22i shows a portion of fig. 22f, in which some of the components have been omitted to outline a specific scenario. In the scenario outlined in fig. 22i, system configured for changing pre-programmed treatment settings of an implantable energized medical device 10 is disclosed. The changing pre-programmed treatment settings are performed by a health care provider (HCP) in the physical presence of the patient. The system comprises at least one HCP EID external device 320' adapted to receive commands from the HCP, directly or indirectly, to change said pre-programmed treatment settings in steps of an implantable energized medical device 10, when implanted. The HCP EID external device 320' is further adapted to be activated, authenticated, and allowed to perform said command by the HCP providing a HCP private key device 333" comprising a HCP private key. The HCP private key device in the embodiment of fig. 22i, comprises at least one of: a smart card, a keyring device, a watch, a arm or wrist band, a necklace, and any shaped device. The HCP EID external device 320' is adapted to be involved in at least one of: receiving information from the implant 100, receiving information from a patient remote external device 336, actuating the implantable energized medical device 10, changing pre-programmed settings, and updating software of the implantable energized medical device 10, when implanted. The HCP EID external device 320' is adapted to be activated, authenticated, and allowed to perform said command also by the patient, the system comprises a patient private key device 333' comprising a patient private key. The patient private key device 333' comprising at least one of: a smart card, a keyring device, a watch, a arm or wrist band, a necklace, and any shaped device. The HCP private key 333" and the patients private key are required for performing said actions by the HCP EID external device 320' to at least one of: receive information from the implant 100, to receive information from a patient remote external device 336, to actuate the implantable energized medical device 10, to change pre-programmed settings, and to update software of the implantable energized medical device 10, when the implantable medical device is implanted.

Fig. 22i also outlines a scenario in which the system is configured for changing pre-programmed treatment settings in steps of an implantable medical device, when implanted in a patient, by a health care provider, HCP, with the patient on remote on distance, the system comprising: at least one HCP EID external device 320' adapted to receive a command from the HCP direct or indirect, to change said pre-programmed treatment settings in steps of an implantable medical device, when implanted, wherein the HCP EID external device 320' is further adapted to be activated, authenticated, and allowed to perform said command by the HCP. The said action by the HCP EID external device 320' to change pre-programmed settings in the implant 100 and to update software of the implantable energized medical device 10, when the implantable energized medical device 10 is implanted, is adapted to be authenticated by a HCP private key device 333" and a patient private key device 333'.

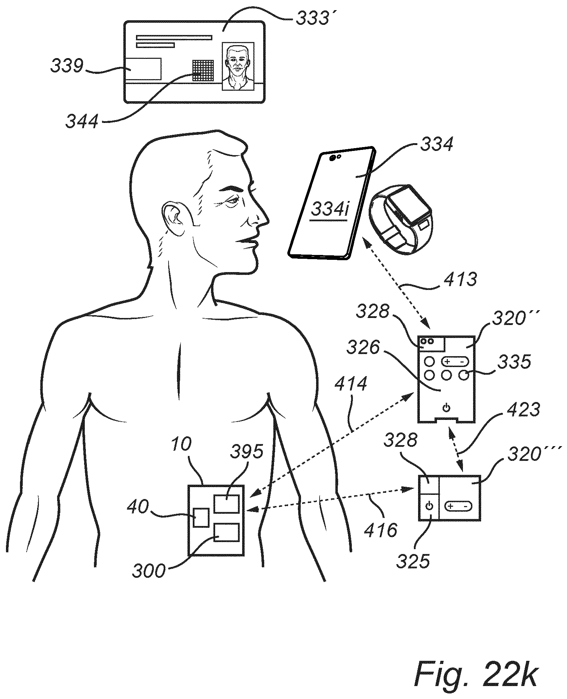

The scenario described with reference to fig. 22i may in alternative embodiments be complemented with additional units or communication connections, or combined with any of the scenarios described with reference to figures 22h, or 22k - 22m.

Fig. 22k shows a portion of fig. 22f, in which some of the components have been omitted to outline a specific scenario. In the scenario outlined in fig. 22k, a system configured to change pre-programmed and pre-selected treatment actions of an implantable energized medical device 10 by command from the patient is described. The system comprises an implantable energized medical device 10, a patient remote external device 320", and a wireless transceiver 328 configured for communication with the implantable energized medical device 10, when the medical device is implanted, through a second network protocol, The system further comprises a remote display portal interface 334i configured to receive content delivered from the patient remote external device 320" to expose buttons to express the will to actuate the functions of the implantable energized medical device 10 by the patient through the patient remote external device 320". The remote external device 320" is further configured to present the display portal remotely on a patient display device 334 allowing the patient to actuate the functions of the implantable energized medical device 10 through the display portal of the patient remote external device 320" visualised on the patient display device 334. In fig. 22k, a further wireless connection 423 between the patient remote external device 320" and the patient EID external device 320‴ is provided. This further wireless connection 423 could be a wireless connection according to any one of the wireless signaling methods and protocols described herein, and the communication can be encrypted.

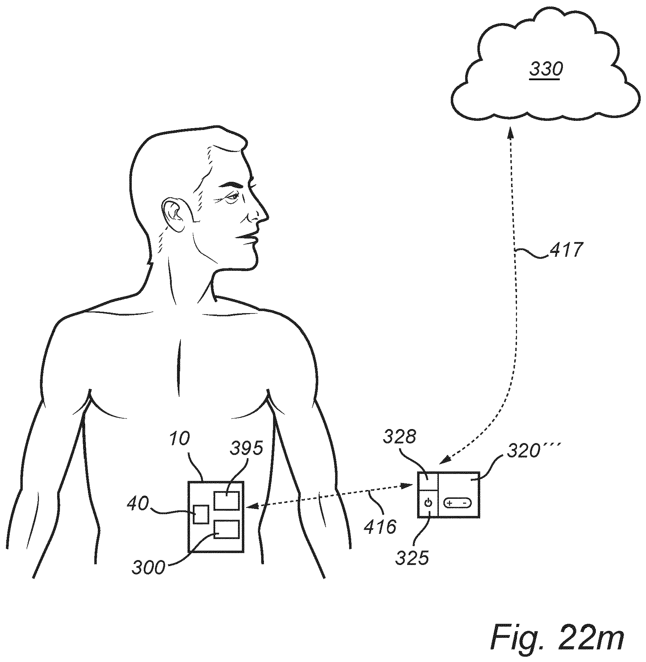

The scenario described with reference to fig. 22k may in alternative embodiments be complemented with additional units or communication connections, or combined with any of the scenarios described with reference to figures 22h, 22i, or 22m.

Fig. 22m shows a portion of fig. 22f, in which some of the components have been omitted to outline a specific scenario. In the scenario outlined in fig. 22m, a system configured for providing information from an implantable energized medical device 10, when implanted in a patient, from a distant remote location in relation to the patient is described. The system comprises at least one patient EID external device 320‴ adapted to receive information from the implant 100, and adapted to send such information further on to a server or dedicated data infrastructure, DDI, 330. The patient EID external device 320‴ is further adapted to be activated and authenticated and allowed to receive said information from the implantable energized medical device 10 by the patient providing a private key, The patient private key device comprises the private key adapted to be provided to the patient EID external device 320‴ via at least one of; a reading slot or comparable for the patient private key device, an RFID communication or other close distance wireless activation communication or direct electrical connection, The patient EID external device 320‴ comprises at least one of; a reading slot or comparable for the patient private key device, an RFID communication and other close distance wireless activation communication or direct electrical contact, The patient EID external device 320‴ further comprises at least one wireless transceiver 328 configured for communication with the DDI 330, through a first network protocol.

The scenario described with reference to fig. 22m may in alternative embodiments be complemented with additional units or communication connections, or combined with any of the scenarios described with reference to figures 22h - 22k.

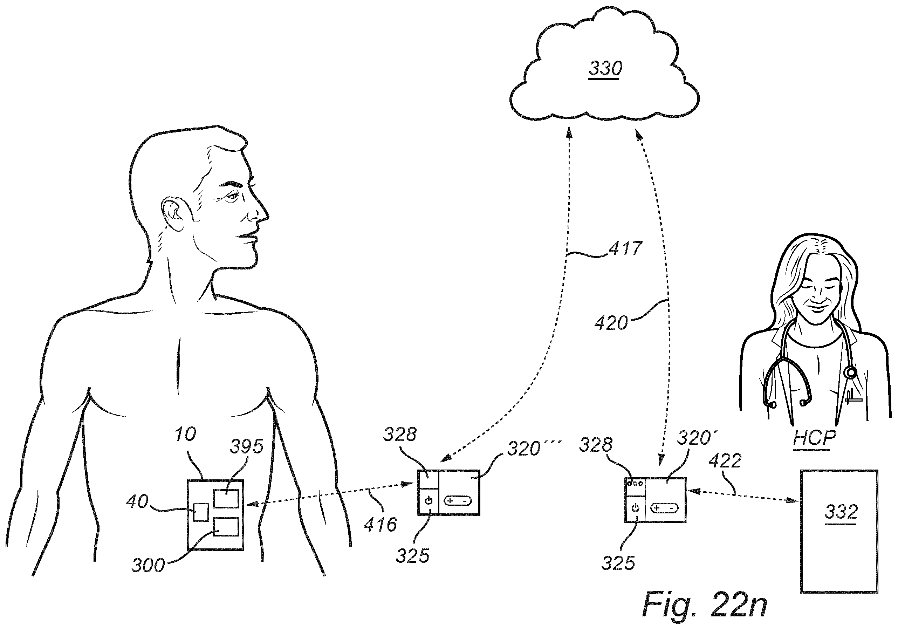

Fig. 22n shows a portion of fig. 22f, in which some of the components have been omitted to outline a specific scenario. In the scenario outlined in fig. 22n a system configured for changing pre-programmed treatment settings in steps of an implantable energized medical device 10, when implanted in a patient, by a health care provider, HCP, either in the physical presence of the patient or remotely with the patient on distance is described. The system comprising at least one HCP EID external device 320' adapted to receive a command directly or indirectly from the HCP to change said pre-programmed treatment settings in steps of the implantable energized medical device 10, when implanted, wherein the HCP EID external device 320' is further adapted to be activated, authenticated, and allowed to perform said command by the HCP providing a HCP private key device comprising a HCP private key, comprising at least one of: a smart card, a keyring device, a watch, a arm or wrist band, a necklace, and any shaped device. The system further comprises a patient private key device comprising a patient private key comprising at least one of: a smart card, a keyring device, a watch, a arm or wrist band, a necklace, and any shaped device. Both the HCP and patient private key is required for performing said action by the HCP EID external device 320' to change the pre-programmed settings in the implant 100 and to update software of the implantable energized medical device 10, when the implantable energized medical device 10 is implanted. The patient private key is adapted to activate, be authenticated, and allowed to perform said command provided by the HCP, either via the HCP EID external device or when the action is performed remotely via a patient EID external device 320'. In the embodiment shown in fig. 22n, the communication is routed over the DDI server 330.

The scenario described with reference to fig. 22n may in alternative embodiments be complemented with additional units or communication connections, or combined with any of the scenarios described with reference to figures 22h - 22m.

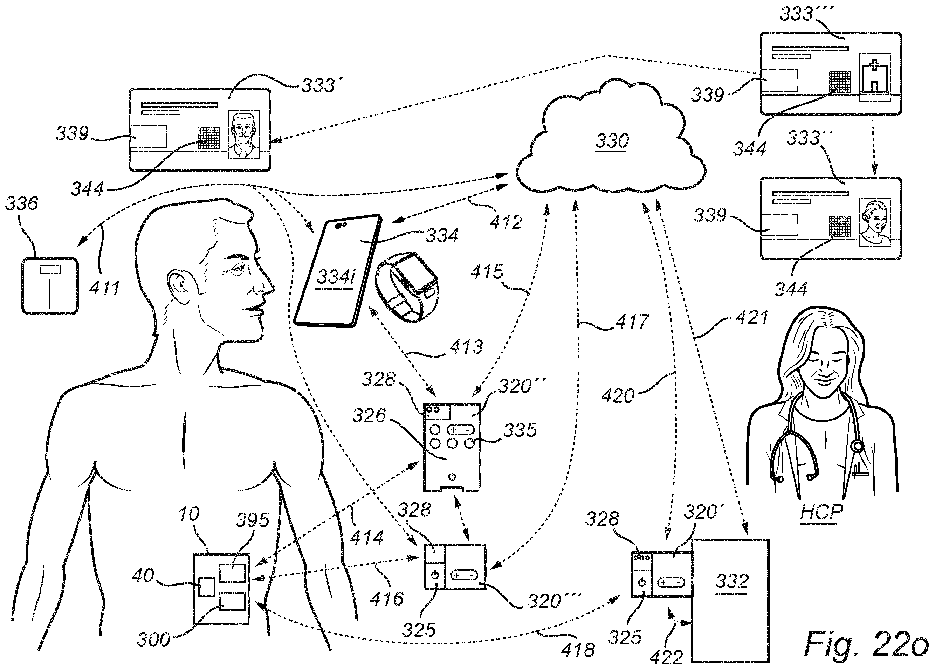

Fig. 22o shows an overview of an embodiment of the system, similar to that described with reference to fig. 22g, the difference being that the HCP EID and the HCP DDD are combined into a single device.

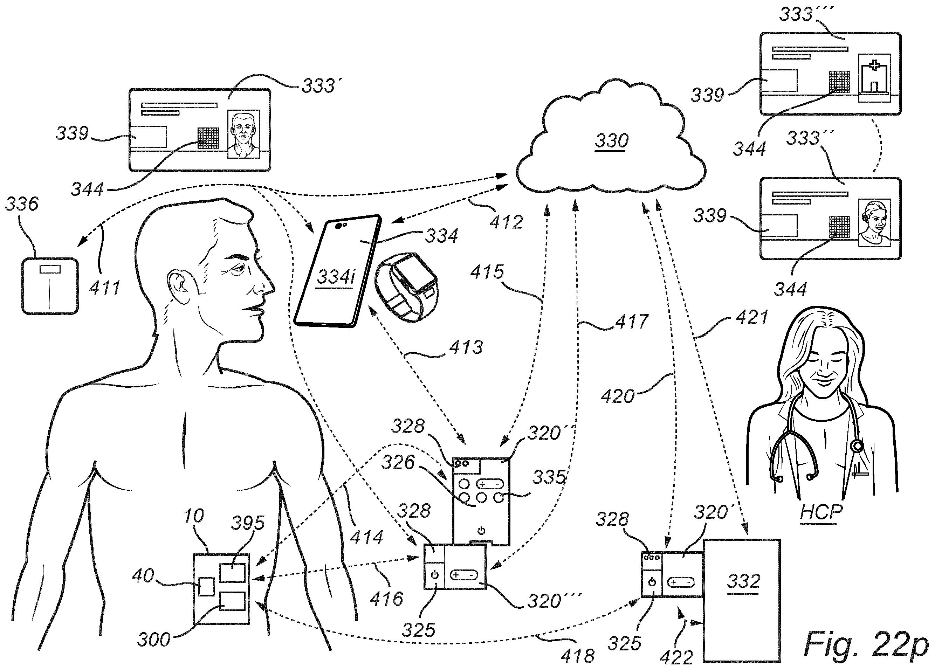

Fig. 22p shows an overview of an embodiment of the system, similar to that described with reference to fig. 22g, the difference being that the HCP EID 320‴ and the HCP DDD 332 are combined into a single device and the P EID 320‴ and the patient remote control external device 320" are combined into a single device.

One probable scenario / design of the communication system is for the purpose of changing pre-programmed treatment settings of an implantable medical device, when implanted in a patient, from a distant remote location in relation to the patient. The system comprises at least one health care provider, HCP, external device 320' adapted to receive a command from the HCP to change said pre-programmed treatment settings of an implanted medical device, further adapted to be activated and authenticated and allowed to perform said command by the HCP providing a HCP private key device 333" adapted to be provided to an HCP EID external device via at least one of; a reading slot or comparable for the HCP private key device, a RFID communication or other close distance wireless activation communication. The HCP EID external device comprising at least one of: a reading slot or comparable for the HCP private key device, a RFID communication, and other close distance wireless activation communication or electrical direct contact. The HCP EID external device further comprises at least one wireless transceiver configured for communication with a patient EID external device, through a first network protocol, wherein the system comprises the patient EID external device, the patient EID external device being adapted to receive command from said HCP external device, and to relay the received command without modifying said command to the implanted medical device. The patient EID external device comprising one wireless transceiver configured for communication with said patient external device. The patient EID is adapted to send the command to the implanted medical device, to receive a command from the HCP to change said pre-programmed treatment settings of the implanted medical device, and further to be activated and authenticated and allowed to perform said command by the patient providing a patient private key device comprising a patient private key.

Although the different scenarios outlined in figures 22h - 22o are described with specific units and method of signaling, these scenarios may very well be combined with each other or complemented with additional units or communication connections.

Aspect 330SE eHealth General Communication Housing

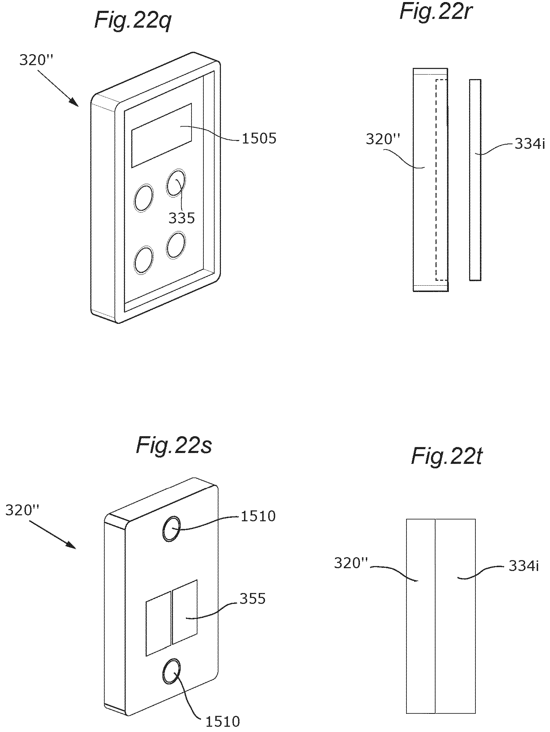

As have been discussed before in this application, communication with a medical implant needs to be reliable and secure. For this purpose, it is desirable to have a standalone device as an external remote control (for example described as 320" in figs 22a -22i, 22k, and 22m - 22p) for the medical implant, such that no other programs or applications run on the same device which may disturb or corrupt the communication to the medical implant. However, the smartphone or tablet (for example described as 334 in figs 22f,g,k,o,p) has become an integrated part of everyday life for most people. This means that we almost always have our smartphones at hand. For this reason, it would have been convenient for the patient to communicate with the medical implant directly using the smartphone, such that no additional standalone device would have to be carried. However, as a lot of other applications are running on the smartphone, it does not fulfill the requirement of being a secure and reliable communication tool without interference from other communication. It is therefore desirable to split the tasks of providing secure communication between the external device and the implant from the task of communicating with the Internet and providing a familiar and intuitive user interface. For this purpose, and external device providing secure communication and tamperproof soft- and hardware, where the display device allows for intuitive and easy use is provided. In the embodiments described with reference to figs. 22q-22u a device fulfilling these combinatory needs will be described in the form of a standalone remote control external device integrated in a housing unit 320" connectable to a smartphone or another display device 334, such as a smart watch or a tablet.

Figs. 22q shows the housing unit 320" in an elevated perspective view form the left, and fig. 22r shows the housing unit 320" in a plain view from the left. In the embodiment shown in fig. 22q, the housing unit 320" has a rectangular shape with rounded edges, having a height 1521 which is more than 1,5 times the width 1522. The housing unit 320" comprises recess 1525 configured to receive a display device 334, in the form of a smartphone, configured to be fitted in the housing unit 320" for mechanically, disconnectably connecting the display device 334 to the housing unit 320". The boundaries of the recess 1525 in the housing unit 320" forms an edge 1528 configured to encircle the display device 334, when the display device 334 is inserted into the recess 1525. In the embodiment shown in fig. 22q, the recess 1525 has a depth 1526 configured to allow the display device 334 to be entirely inserted into the recess 1525. As such, the depth 1526 of the recess 1525 exceeds the depth 1531 of the display device 334. In the embodiment shown in figs. 22q and 22r, the edge is relatively thin, and has a width 1527 which is in the range 1/8 - 1/100 of the width of the display device 334, as such, the housing unit 320" has a width in the range 1,02 - 1,25 times the width 1522 of the housing unit 320". In the same way, the housing unit 320" has a height 1521 in the range 1,01 - 1,25 times the height 1521 of the display device 334. In the embodiment shown in figs.22q - 22r, the edges 1528 are configured to clasp the display device 334 and thereby mechanically fixate the display device 334 in the housing unit 320". The minimum bounding box of the housing unit 320" and the display device 334 when mechanically connected, is no more than, 10 % wider, 10 % longer or 100 % higher, than the minimum bounding box of the display device 334.

For creating a clasping fixation, the edges of the housing unit 320" is made from an elastic material crating a tension between the edge 1528 and the display device 334 holding the display device 334 in place. The elastic material could be an elastic polymer material, or a thin sheet of elastic metal. For the purpose of further fixating the display device 334 in the housing unit 320", the inner surface of the edges 1528 may optionally comprise a recess or protrusion (not shown) corresponding to a recess or protrusion of the outer surface of the display device 334. The edges 1528 may in the alterative comprise concave portions for creating a snap-lock clasping mechanical fixation between the housing unit 320" and the display device 334.

In the embodiment shown in figs. 22q and 22r, the housing unit 320" functions as a remote control for communicating with an implanted medical device, including receiving information from, and providing instructions and updates to, the implanted medical device. Information could be information related to a state of the implanted medical device including any functional parameter of the implanted medical device or could be related to a state of the patient, including any physiological parameter pertaining to the body of the patient (further described on other sections of this disclosure). For the purpose of providing input to the implanted medical device and controlling and updating the functions of the housing unit 320", the housing unit 320" comprises a control interface comprising switches in the form of control buttons 335. The control buttons 335 are configured to be used when the external device is disconnected from the display device 334. The control interface further comprises a display 1505, which is a smaller and typically less sophisticated display 1505 than the display of the display device 334. In an alternative embodiment, the control buttons 335 and display 1505 are integrated into a single touch-responsive (touchscreen) display on which the control buttons may be displayed. In the embodiment shown in figs. 22q and 22r, one of the control buttons 335 is a control button for activating the implanted medical device and another of the control buttons 335 is a control button for deactivating the implanted medical device. When the display device 334 is attached to the housing unit 320", the control buttons 335 and the display is covered by the display device 334 and are as such not in an operational state. In the embodiment shown in figs 22q and 22r, the housing unit 320" is configured to transmit information pertaining to the display of the user interface to the display device 334 and the display device 334 is configured to receive input pertaining to communication to or from the implantable medical device from the patient, and transmit signals based on the received input to the housing unit 320". The input may be a command to change the operational state of the implantable medical device. The display device 334 comprises a touch screen configured to display the user interface and receive the input from the patient. The display of the display device 334 may comprise one or more OLEDs or IPS LCDs elements. When the display device 334 is connected to the housing unit 320", the display device 334 is configured to display a control interface which is used to communicate with the housing unit 320", i.e. providing input to and receiving information from the housing unit 320". The input provided the housing unit 320" is then relayed to the implanted medical device - and in the same way information communicated from the implanted medical device to the housing unit 320" may be relayed or displayed on the display device 334. Having an external device comprising a combination of a housing unit 320" comprising the communication means for communicating with the implanted medical device and a display device 334 basically only functioning as and Input/Output device connected to the housing unit 320" makes it possible to have a secure communication between the housing unit 320" and the display device 334, which is out of reach from the Internet connection of the display device 334, which makes it much harder for an external attacker to get access to any of the vital communication portions of the housing unit 320". The communication between the housing unit and the display device 334 is very restricted and the only communication allowed from the display device 334 to the housing unit 320" is input from the patient or a healthcare professional, and authentication parameters created by an authentication application running on the display device 334. The authentication application running on the display device 334 could be a number-generating authenticator or a biometric authenticator for authenticating the patient or health care professional, and the authentication parameters could for example be parameters derived from a facial image or a fingerprint. In the opposite direction, i.e. from the housing unit 320" to the display device 334, the communication could be restricted to only communication needed for displaying information and/or a graphical user interface on the display device 334. The communication restrictions could for example be based on size of the communication packages or the frequency with which the communication takes place which reduces the risk that an un-authorized person makes multiple attempts to extract information from, or transit information to, the hand-held device.

In the embodiment shown with reference to figs. 22q and 22r, the housing unit 320" comprises a first communication unit providing a wireless connection 413 to the display device 334. The wireless connection 413 is in the embodiment shown in figs- 22q and 22r based on NFC, but could in alternative embodiment be based on Bluetooth or any other communication pathway disclosed herein. The housing unit 320" further comprises a second communication unit providing a wireless connection with the implanted medical device. The wireless communication between the housing unit 320" and the implanted medical device is in the embodiment shown in figs. 22q and 22r based on Bluetooth, but could in alternative embodiments be based on NFC or UWB or any other communication pathway disclosed herein.

As mentioned, in the embodiment shown in figs. 22q and 22r, the wireless communication between the housing unit 320" and the display device 334 is based on NFC, while the wireless communication between the housing unit 320" and the is based on Bluetooth. As such, the first communication unit of the housing unit 320" is configured to communicate wirelessly with the display device 334' using a first communication frequency and the second communication unit of the housing unit 320" is configured to communicate wirelessly with the implantable medical device using a second different communication frequency. For this purpose, the first communication unit of the housing unit 320" comprises a first antenna configured for NFC-based wireless communication with the display device 334, and the second communication unit comprises a second antenna configured for Bluetooth-based wireless communication with the implantable medical device. The first and second antennae may be a wire-based antennae or a substrate-based antennae. As such, the first communication unit is configured to communicate wirelessly with the display device 334 on a first frequency and the second communication unit is configured to communicate wirelessly with the implantable medical device using a second different communication frequency. Also, first communication unit of the housing unit 320' is configured to communicate wirelessly with the display device 334 using a first communication protocol (the NFC-communication protocol), and the second communication unit is configured to communicate wirelessly with the implantable medical device using a second communication protocol (the Bluetooth communication protocol). The first and second communication protocols are different which adds an additional layer of security as security structures could be built into the electronics and/or software enabling the transfer from a first to a second communication protocol.

In an alternative embodiment, the second communication unit may be configured to communicate wirelessly with the implantable medical device using electromagnetic waves at a frequency below 100 kHz, or preferably at a frequency below 40 kHz. The second communication unit may thus be configured to communicate with the implantable medical device using "Very Low Frequency" communication (VLF). VLF signals have the ability to penetrate a titanium housing of the implant, such that the electronics of the implantable medical device can be completely encapsulated in a titanium housing. In yet further embodiments, the first and second communication units may be configured to communicate by means of an RFID type protocol, a WLAN type protocol, a BLE type protocol, a 3G/4G/5G type protocol, or a GSM type protocol.

In yet other alternative embodiments, it is conceivable that the mechanical connection between the housing unit 320" and the display device 334 comprises an electrical connection for creating a wire-based communication channel between the housing unit 320" and the display device 334. The electrical connection could also be configured to transfer electric energy from the display device 334 to the housing unit, such that the housing unit 320" may be powered or charged by the display device 334. A wired connection is even harder to access for a non-authorized entity than an NFC-based wireless connection, which further increases the security of the communication between the housing unit 320" and the display device 334.

In the embodiment shown with reference to figs. 22q and 22r, the display device 334 comprises a first communication unit providing a wireless connection 413 to the housing unit 320" based on NFC. The display device 334 further comprises a second communication unit providing a wireless connection with a further external device and/or with the Internet. The second external device may be far away, for example at a hospital or a place where a medical professional practice. The wireless communication between the display device 334 and a further external device is in the embodiment shown in figs. 22q and 22r based on WiFi, but could in alternative embodiments be based on for example Bluetooth.

As mentioned, in the embodiment shown in figs. 22q and 22r, the wireless communication between the display device 334 and the housing unit 320" is based on NFC, while the wireless communication between the display device and a further external unit is based on WiFi. As such, the first communication unit of the display device 334 is configured to communicate wirelessly with the housing unit 320" using a first communication frequency and the second communication unit of the display device 334 is configured to communicate wirelessly with a further external device using a second different communication frequency. For this purpose, the first communication unit of the display device 334 comprises a first antenna configured for NFC-based wireless communication with the housing unit 320", and the second communication unit comprises a second antenna configured for WiFi-based wireless communication with a further external device. The first and second antennae may be wire-based antennae or substrate-based antennae. As such, the first communication unit is configured to communicate wirelessly with the housing unit 320" on a first frequency and the second communication unit is configured to communicate wirelessly with the further external device using a second different communication frequency. Also, the first communication unit of the display device 334 is configured to communicate wirelessly with the housing unit 320" using a first communication protocol (the NFC communication protocol), and the second communication unit is configured to communicate wirelessly with the further external device using a second communication protocol (the WiFi communication protocol). The first and second communication protocols are different which adds an additional layer of security as security structures could be built into the electronics and/or software enabling the transfer from a first to a second communication protocol.

In alternative embodiments, the second communication unit of the display device 334 may be configured to communicate with the further external device by means of, a WLAN type protocol, or a 3G/4G/5G type protocol, or a GSM type protocol.

In the embodiment shown in figs. 22q and 22r, the communication range of the first communication unit of the housing unit 320" is less than a communication range of the second communication unit of the housing unit 320', such that the communication distance between the housing unit 320" and the medical implant may be longer than the communication distance between the housing unit 320" and the display device 334. In the embodiment shown in figs. 22q and 22r, the communication range of the first communication unit may be constrained to a length that is less than five times the longest dimension of the minimal bounding box of the display device 334, or more precisely constrained to a length that is less than three times the longest dimension of the minimal bounding box of the display device 334.

In the embodiment shown in figs. 22q and 22r, communication between the housing unit 320" and the display device 334 is only enabled when the housing unit 320" is connected to the display device 334. I.e. at least one of the housing unit 320" and the display device 334 is configured to allow communication between the housing unit 320" and the display device 334 on the basis of the distance between the housing unit 320" and the display device 334. In the alternative, the housing unit 320" and/or the display device 334 may comprise a sensor configured to estimate whether the housing unit 320" is attached to the display device 334 or not, such as a mechanically activated switch or a photo resistive sensor which providing sensor input when the housing unit 320" and display device 334 are mechanically connected to each other. The signal from the at least one sensor then may be used to permit usage of the communication unit configured for communication with the display device 334.

In the embodiment shown in figs. 22q and 22r, communication between the housing unit 320" and the implantable medical device is only enabled on the basis of a distance between the housing unit 320" and the implantable medical device. In the embodiment shown in figs. 22q and 22r, the distance should be less than twenty times the longest dimension of the minimal bounding box of the display device, or more specifically less than ten times the longest dimension of the minimal bounding box of the display device. The distance between the housing unit 320" and the medical implant may be measured using electromagnetic waves, or acoustic waves. The process of measuring the distance may comprise triangulation.

In the embodiment shown in figs. 22q and 22r, the second communication unit of the display device 334 need to be disabled to enable communication between the display device 334 and the housing unit 320", and further the second communication unit of the display device 334 needs to be disabled to enable communication between the housing unit 320" and the medical implant. Also, the second communication unit of the housing unit 320" needs to be disabled to enable communication between the housing unit 320" and the medical implant.

In the embodiment shown in figs. 22q and 22r, the housing unit 320" further comprises an encryption unit configured to encrypt communication received from the display device 334 before transmitting the communication to the implanted medical device. The encryption unit may for example be based on one of the following algorithms: AES, Blowfish, DES, Kalyna, Serpent or Twofish. For the purpose for handling the communication, I/O and encryption, the housing unit 320" comprises a processor which could be a general-purpose microprocessor and/or an instruction set processor and/or related chips sets and/or special purpose microprocessors such as ASICs (Application Specific Integrated Circuit). The processor also comprise memory for storing instruction and/or data.

Figs. 22s and 22t shows an embodiment of the external unit similar to the embodiment described with reference to figs. 22q and 22r. The difference being that in the embodiment of figs. 22s and 22t, the housing unit 320" does not clasp the display device 334. Instead, the housing unit comprises two magnets 1510 for magnetically fixating the display device 334 to the housing unit 320". In alternative embodiments, it is equally conceivable that the external device comprises an intermediate portion, which is fixedly fixated to the housing unit for providing a detachable connection with the display device 334. In the alternative, the intermediate device could be fixedly fixated to the display device 334 and provide a detachable connection with the housing unit 320".

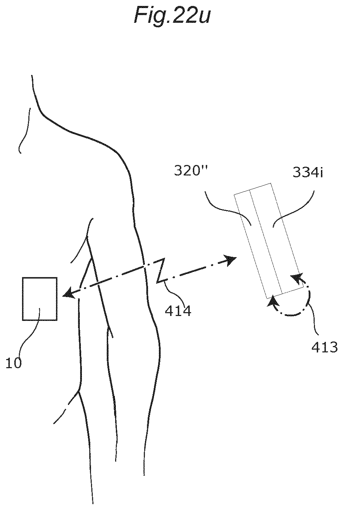

Fig. 22u shows a system overview of the external device (which could be the external device of the embodiment described with reference to figs 22q and 22r, or of the embodiment described with reference to figs 22s and 22t). The housing unit 320" is connected to the display device 334. A wireless connection 413 is provided between the housing unit 320" and the display device 334, and a further wireless connection 413 is provided between the housing unit 320" and the implantable energized medical device 10, such that the housing unit can send instructions and updates to the implantable energized medical device 10, and receive information, parameters (such as sensor values) and alarms from the implantable energized medical device 10. The communication between the external device and the medical implant 100 is further described in other portions of this disclosure.

The implantable energized medical device 10 may be an active and/or operable implantable energized medical device 10 which may be an implantable medical device configured to exert a force on a body portion of the patient. The body portion of the patient may be a fluid carrying vessel, an organ, a joint, a membrane, a muscle, a bone or a nerve. The implantable energized medical device 10 may comprises an electrical motor and a controller for controlling the electrical motor and instructions transmitted to the implantable energized medical device 10 could be instructions pertaining to the control of the electrical motor. The controller may control, the velocity, the acceleration or the torque of the motor. The implantable energized medical device 10 could for example comprises at least one of: an external heart compression device, an apparatus assisting the pump function of a heart of the patient, an apparatus assisting the pump function comprising a turbine bump placed within a patient's blood vessel for assisting the pump function of the heart, an operable artificial heart valve, an operable artificial heart valve for increasing the blood flow to the coronary arteries. an implantable drug delivery device, an implantable drug delivery device for injecting directly into a blood vessel and change the position of the injection site, all from within the patient's body, an implantable drug delivery device for injecting potency enhancing drugs into an erectile tissue of the patient, a hydraulic, mechanic, and/or electric constriction implant, an operable volume filling device, an operable gastric band, an operable implant for stretching the stomach wall of the patient for creating satiety, an implant configured to sense the frequency of the patient ingesting food, an operable cosmetic implant, an operable cosmetic implant for adjust the shape and/or size in the breast region of a patient, an implant controlling medical device for the emptying of a urinary bladder, an implant hindering urinary leakage, an implant hindering anal incontinence, an implant controlling the emptying of fecal matter, an implant monitoring an aneurysm, an implant for hindering the expansion of an aneurysm, an implant lubricating a joint, an implant for affecting the blood flow to an erectile tissue of the patient, an implant for simulating the engorgement of an erectile tissue, an implant with a reservoir for holding bodily fluids, an implant storing and/or emptying a bodily reservoir or a surgically created reservoir, an implant communicating with a database outside the body, an implant able to be programmed from outside the body, an implant able to be programmed from outside the body with a wireless signal, an implant treating impotence, an implant controlling the flow of eggs in the uterine tube, an implant controlling the flow of sperms in the uterine tube, an implant controlling the flow of sperms in the vas deferens, an implant for hindering the transportation of the sperm in the vas deferens, an implant treating osteoarthritis, an implant performing a test of parameters inside the body, an implant controlling specific treatment parameters from inside the body, an implant controlling bodily parameters from inside the body, an implant controlling the blood pressure, an implant controlling the blood pressure by affecting the dilatation of the renal artery, an implant controlling a drug treatment parameter, an implant controlling a parameter in the blood, an implant for adjusting or replacing any bone part of a body of the patient, an implant replacing an organ of the patient or part of an organ of the patient or the function thereof, a vascular treatment device, an implant adapted to move fluid inside the body of the patient, an implant configured to sense a parameter related to the patient swallowing, an implant configured to exercise a muscle with electrical or mechanical stimulation, an implant configured for emptying an intestine portion on command, an operable implant configured to be invaginated in the stomach of the patient to reduce the volume of the stomach substantially more than the volume of the device, an implant configured for emptying the urinary bladder from within the patient's body by compressing the bladder, an implant configured for draining fluid from within the patient's body, an implant configured for the active lubrication of a joint with an added lubrication fluid, an implant configured for removing clots and particles from the patient's blood stream, an implant configured for elongating or straightening a bone in the patient, to reduce scoliosis, a device to stimulate the brain for a several position to a focused point, an artificial stomach replacing the function of the natural stomach, an implant configured for adjusting the position of a female's urinary tract or bladder neck, an implant configured for stimulating the ampulla vas deference and creating temporary constriction.

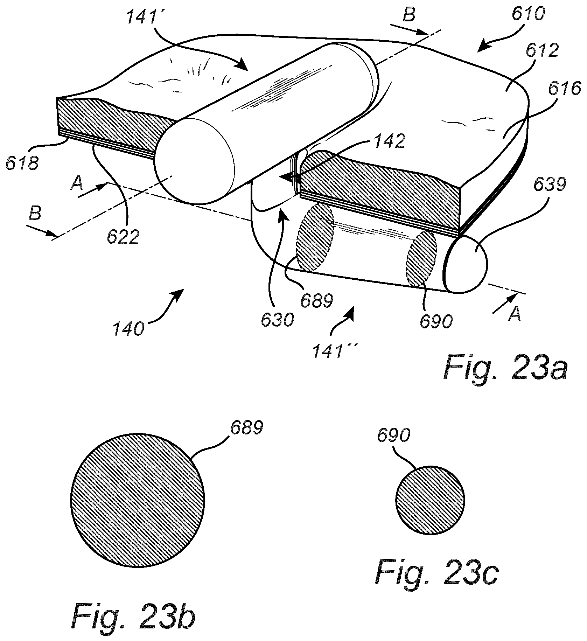

With reference to Figs. 23a-c, 24, 25, 26, and 27a-c, embodiments of an implantable energized medical device 140, which may be referred to as a remote unit in other parts of the present disclosure, will be described. As illustrated, these implantable energized medical devices have a second portion being shaped in a particular manner in order to facilitate removal of the implantable energized medical device once it has been implanted for a period of time and fibrotic tissue has begun to form around the second portion. It is hereby disclosed that these types of second portions, as illustrated in Figs. 23a-c, 24, 25, 26 and 27a-c, and as disclosed below, may be combined with any of the other features of the implantable energized medical device discussed in the present disclosure.

The device 140 is configured to be held in position by a tissue portion 610 of a patient. The device 140 comprises a first portion 141' configured to be placed on a first side 612 of the tissue portion 610, the first portion 141' having a first cross-sectional area in a first plane and comprising a first surface configured to face and/or engage a first tissue surface 616 of the first side 612 of the tissue portion 610. The device 140 further comprises a second portion 141" configured to be placed on a second side 618 of the tissue portion 610, the second side 618 opposing the first side 612, the second portion 141" having a second cross-sectional area in a second plane and comprising a second surface configured to engage a second tissue surface 622 of the second side 618 of the tissue portion 610. The device 140 further comprises a connecting portion 142 configured to be placed through a hole in the tissue portion 610 extending between the first and second sides 612, 618 of the tissue portion 610. The connecting portion 142 here has a third cross-sectional area in a third plane. The connecting portion 142 is configured to connect the first portion 141' to the second portion 141". In the illustrated embodiment, a connecting interface 630 between the connecting portion 142 and the second portion 141" is arranged at an end of the second portion 141".

The first portion 141' may have an elongated shape. Similarly, the second portion 141" may have an elongated shape. However, the first portion 141' and/or second portion 141" may assume other shapes, such as a flat disk e.g. having a width and length being larger than the height, a sphere, an ellipsoid, or any other polyhedral or irregular shape, some of these being exemplified in Figs. 9-11.

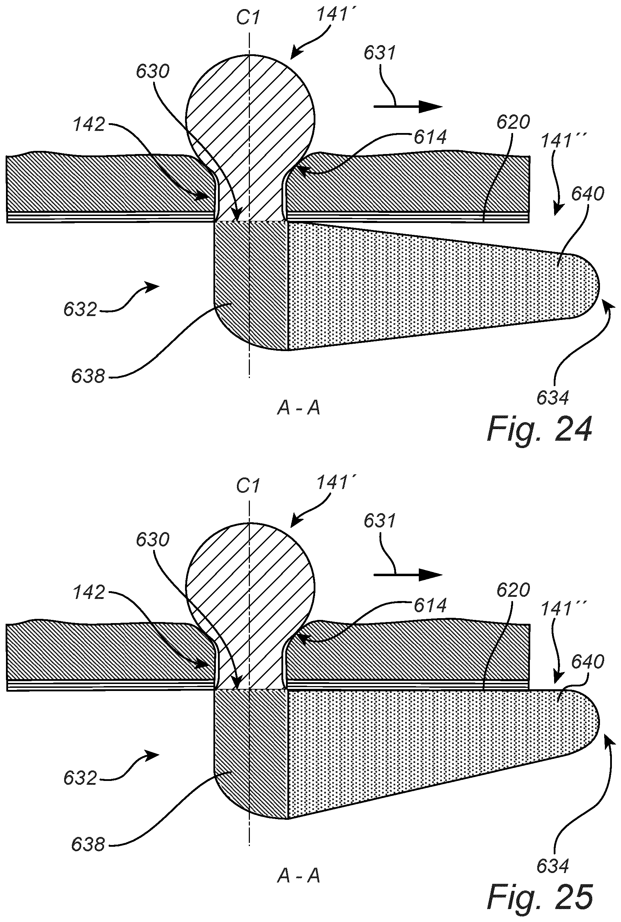

To provide a frame of reference for the following disclosure, and as illustrated in Figs. 24, 25 and 26, a first direction 631 is here parallel to the line A-A, to the second plane, and to a length of the second portion 141". A second direction 633 is here parallel to the line B-B, to the second plane, and to a width of the second portion 141". The second portion 141" has a first end 632 and a second end 634 opposing the first end 632. The length of the second portion 141" is defined as the length between the first end 632 and the second end 634. The length of the second portion 141" is furthermore extending in a direction being different to the central extension C1 of the connecting portion 142. The first end 632 and second end 634 are separated in a direction parallel to the second plane. Similarly, the first portion 141' has a length between a first and a second end, the length extending in a direction being different to the central extension C1 of the connecting portion 142.

The first portion 141', connecting portion 142 and second portion 141" may structurally form one integral unit. It is however also possible that the first portion 141' and the connecting portion 142 structurally form one integral unit, while the second portion 141" form a separate unit, or, that the second portion 141" and the connecting portion 142 structurally form one integral unit, while the first portion 141' form a separate unit.

Additionally, or alternatively, the second portion 141" may comprise a removable and/or interchangeable portion 639 as described in other parts of the present disclosure.

In the following paragraphs, some features and properties of the second portion 141" will be described. It is however to be understood that these features and properties may also apply to the first portion 141'.

The second portion 141" has an intermediate region 638, and a distal region 640. A proximal region may be present, as described in other parts of the present disclosure, The intermediate region 638 is defined by the connecting interface 630 between the connecting portion 142 and the second portion 141", and the distal region 640 extends from the connecting interface 630 between the connecting portion 142 and the second portion 141" to the second end 634.

The first surface 614 configured to face and/or engage the first tissue surface 616 of the first side 612 of the tissue portion 610 may be substantially flat. In other words, the first portion 141' may comprise a substantially flat side facing towards the tissue portion 610. Furthermore, an opposing surface of the first portion 141', facing away from the tissue portion 610, may be substantially flat. Similarly, the second surface 620 configured to engage the second tissue surface 622 of the second side 618 of the tissue portion 610 may be substantially flat. In other words, the second portion 141" may comprise a substantially flat side facing towards the tissue portion 610. Furthermore, an opposing surface of the second portion 141", facing away from the tissue portion 610, may be substantially flat.

The second portion 141" may be tapered from the first end 632 to the second end 634, thus giving the second portion 141" different heights and/or widths along the length of the second portion 141". The second portion may also be tapered from each of the first end 632 and second end 634 towards the intermediate region 638 of the second portion 141".

Still referring to Figs. 23a-c, 24, 25, 26, and 27a-c, the second portion 141" and connecting portion 142 here form a connecting interface 630. Furthermore, the second portion 141" has a lengthwise cross-sectional area along the first direction, wherein a second lengthwise cross-sectional area 690 is smaller than a first lengthwise cross-sectional area 689 and wherein the first lengthwise cross-sectional area 689 is located closer to the connecting interface 630 with regard to the first direction 631. Hereby, a tapered second portion is formed, being tapered towards the second end 634. The lengthwise cross-sectional area of the second portion 141" may decrease continuously from an end of the intermediate region 638 towards the second end 634, as illustrated for example in Fig. 24. The decrease may be linear, as illustrated for example in Fig. 24. However, other types of decreasing lengthwise cross-sectional areas are possible, such as a parabolic, exponential, stepwise, or stepwise with radiused edges between each step thus forming a smooth rounded contour.

Figs. 23b and 23c illustrate how the lengthwise cross-sectional area decrease over the length of the second portion 141" towards the second 634, as viewed along the line A-A. Fig. 23b illustrate the first lengthwise cross-sectional area 689, and Fig. 23c illustrate the second lengthwise cross-sectional area 690.

In some embodiments, the lengthwise cross-sectional area may decrease over a majority of the length of the second portion towards the second end 634. In some embodiments, a decrease of the lengthwise cross-sectional area over at least ¼ of the length of the second portion towards the second end 634 may be sufficient. In the example illustrated in Fig. 24, the lengthwise cross-sectional area decrease over about 85% of the length of the second portion.

With the second portion 141" having rotational symmetry along the first direction 631, as illustrated for example in Fig. 23a, the shape of the second portion 141" may be conical.

As illustrated in Fig. 25, the second portion 141" may have an upper surface, which include the second surface 620 configured to engage a second tissue surface of the second side of the tissue portion as discussed in other parts of the present disclosure, wherein the upper surface or second surface 620 is substantially flat and parallel to the second plane. In some embodiments the upper surface may be substantially perpendicular to the central extension C1 of the connecting portion 142. Hereby, the second surface may be configured to lay flat against the second side of the tissue portion. In such embodiments, a lower surface of the second portion 141", opposite the second surface 620 and facing away from the first portion 141', may be configured to taper towards the second end 634, thus achieving the decreasing lengthwise cross-sectional area along the first direction 631 towards the second end 634.

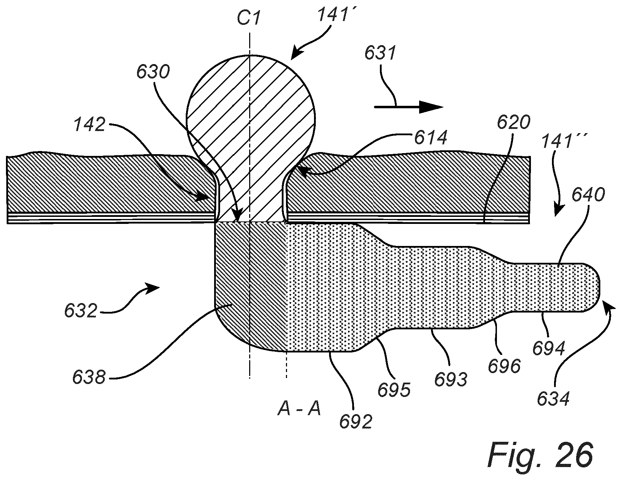

Fig. 26 illustrate an embodiment wherein the lengthwise cross-sectional area decrease in a stepwise manner towards the second end 634 of the second portion 141". Here, the second portion 141" has three major segments 692, 693, 694 having substantially constant diameter and each respective diameter being smaller moving towards the second end 634, being connected by intermediate segments 695, 696, wherein the diameter decreases along the first direction 631. Other variations of major segments having substantially constant diameter, and intermediate segments, having a decreasing diameter along the first direction 632, are possible, such as at least two major segments connected by a single intermediate segment with decreasing diameter, at least four major segments connected by three intermediate segments with decreasing diameter, and so on.

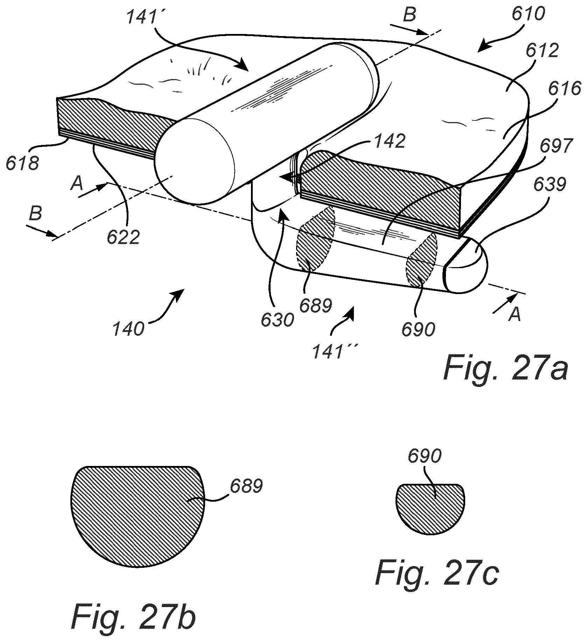

Referring now to Figs. 27a-c, an implantable energized medical device similar to the one illustrated in Fig. 25 is illustrated. As can be seen in the perspective view of Fig. 27a, the second portion 141" has a decreasing lengthwise cross-sectional area towards the second end. The upper surface 697 is also visible in this view, being substantially flat and providing a contact area to the second tissue surface 622. The first lengthwise cross-sectional area 689 is larger than the second cross-sectional area 690, as can be seen in Figs. 27b-c, and the first lengthwise cross-sectional area 689 is located closer to the connecting interface between the connecting portion 142 and the second portion 141" with regard to the first direction.

## Claims

1. An implantable energized medical device configured to be held in position by a tissue portion of a patient, the medical device comprising:
a first portion configured to be placed on a first side of the tissue portion, the first portion having a first cross-sectional area in a first plane and comprising a first surface configured to face a first tissue surface of the first side of the tissue portion,
a second portion configured to be placed on a second side of the tissue portion, the second side opposing the first side, the second portion having a second cross-sectional area in a second plane and comprising a second surface configured to engage a second tissue surface of the second side of the tissue portion, and
a connecting portion configured to be placed through a hole in the tissue portion extending between the first and second sides of the tissue portion, the connecting portion having a third cross-sectional area in a third plane and being configured to connect the first portion to the second portion,
wherein:
the first, second, and third planes are parallel to each other,
the third cross-sectional area is smaller than the first and second cross-sectional areas, such that the first portion and second portion are prevented from travelling through the hole in the tissue portion in a direction perpendicular to the first, second and third planes, and
the first portion comprises a first wireless energy receiver for receiving energy transmitted wirelessly by an external wireless energy transmitter, **characterised in that**: the first portion further comprises an internal wireless energy transmitter configured to transmit energy wirelessly to the second portion, and
the second portion comprises a second wireless energy receiver configured to receive energy transmitted wirelessly by the internal wireless energy transmitter.

2. The implantable energized medical device according to claim 1, wherein the first portion comprises a first energy storage unit connected to the first wireless energy receiver.

3. The implantable energized medical device according to any one of claims 1 and 2, wherein the second portion comprises a second energy storage unit connected to the second wireless energy receiver.

4. The implantable energized medical device according to claim 3, wherein at least one of the first and second energy storage unit is a solid-state battery.

5. The implantable energized medical device according to claim 4, wherein the solid-state battery is a thionyl-chloride battery.

6. The implantable energized medical device according to any one of claims 3 - 5, wherein:
the first wireless energy receiver is configured to receive energy transmitted wirelessly by the external wireless energy transmitter and store the received energy in the first energy storage unit,
the internal wireless energy transmitter is configured to wirelessly transmit energy stored in the first energy storage unit to the second wireless energy receiver, and
the second wireless energy receiver is configured to receive energy transmitted wirelessly by the internal wireless energy transmitter and store the received energy in the second energy storage unit.

7. The implantable energized medical device according to any one of the preceding claims, wherein the first portion comprises a first controller comprising at least one processing unit.

8. The implantable energized medical device according to any one of the preceding claims, wherein the second portion comprises a second controller comprising at least one processing unit.

9. The implantable energized medical device according to any one of claims 7 and 8, wherein at least one of the first and second controller is connected to a wireless transceiver for communicating wirelessly with an external device.

10. The implantable energized medical device according to any one of claims 7 and 8, wherein:
the first controller is connected to a first wireless communication receiver in the first portion for receiving wireless communication from an external device,
the first controller is connected to a first wireless communication transmitter in the first portion for transmitting wireless communication to a second wireless communication receiver in the second portion.

11. The implantable energized medical device according to claim 10, wherein the second controller is connected to the second wireless communication receiver for receiving wireless communication from the first portion.

12. The implantable energized medical device according to any one of the preceding claims, wherein the first wireless energy receiver comprises a first coil and the wireless energy transmitter comprises a second coil.

13. The implantable energized medical device according to any one of the preceding claims, wherein the first portion comprises a combined coil, wherein the combined coil is configured to receive energy wirelessly from an external wireless energy transmitter, and transmit energy wirelessly to the second wireless receiver of the second portion.

14. The implantable energized medical device according to any one of claims 12 and 13, wherein at least one of the coils are embedded in a ceramic material.

15. The implantable energized medical device according to any one of the preceding claims, further comprising a housing configured to enclose at least the first portion, and wherein a first portion of the housing is made from titanium and a second portion of the housing is made from a ceramic material.

## Patentansprüche

1. Implantierbare energetisierte medizinische Vorrichtung, die dazu ausgelegt ist, durch einen Gewebeabschnitt eines Patienten in Position gehalten zu werden, wobei die medizinische Vorrichtung umfasst:
einen ersten Abschnitt, der dazu ausgelegt ist, auf einer ersten Seite des Gewebeabschnitts platziert zu werden, wobei der erste Abschnitt eine erste Querschnittsfläche in einer ersten Ebene aufweist und eine erste Oberfläche umfasst, die dazu ausgelegt ist, zu einer ersten Gewebeoberfläche der ersten Seite des Gewebeabschnitts zu weisen,
einen zweiten Abschnitt, der dazu ausgelegt ist, auf einer zweiten Seite des Gewebeabschnitts platziert zu werden, wobei die zweite Seite der ersten Seite gegenüberliegt, wobei der zweite Abschnitt eine zweite Querschnittsfläche in einer zweiten Ebene aufweist und eine zweite Oberfläche umfasst, die dazu ausgelegt ist, mit einer zweiten Gewebeoberfläche der zweiten Seite des Gewebeabschnitts in Eingriff zu kommen, und
einen Verbindungsabschnitt, der dazu ausgelegt ist, durch ein Loch in dem Gewebeabschnitt platziert zu werden, das sich zwischen der ersten und der zweiten Seite des Gewebeabschnitts erstreckt, wobei der Verbindungsabschnitt eine dritte Querschnittsfläche in einer dritten Ebene aufweist und dazu ausgelegt ist, den ersten Abschnitt mit dem zweiten Abschnitt zu verbinden,
wobei:
die erste, die zweite und die dritte Ebene parallel zueinander sind,
die dritte Querschnittsfläche kleiner als die erste und zweite Querschnittsfläche ist, so dass verhindert wird, dass sich der erste Abschnitt und der zweite Abschnitt durch das Loch in dem Gewebeabschnitt in einer Richtung rechtwinklig zu der ersten, zweiten und dritten Ebene bewegen, und
der erste Abschnitt einen ersten drahtlosen Energieempfänger zum Empfangen von Energie umfasst, die drahtlos durch einen externen drahtlosen Energiesender übertragen wird, **dadurch gekennzeichnet, dass**: der erste Abschnitt ferner einen internen drahtlosen Energiesender umfasst, der dazu ausgelegt ist, Energie drahtlos zu dem zweiten Abschnitt zu übertragen, und
der zweite Abschnitt einen zweiten drahtlosen Energieempfänger umfasst, der dazu ausgelegt ist, drahtlos durch den internen drahtlosen Energiesender übertragene Energie zu empfangen.

2. Implantierbare energetisierte medizinische Vorrichtung nach Anspruch 1, wobei der erste Abschnitt eine erste Energiespeichereinheit umfasst, die mit dem ersten drahtlosen Energieempfänger verbunden ist.

3. Implantierbare energetisierte medizinische Vorrichtung nach einem der Ansprüche 1 und 2, wobei der zweite Abschnitt eine zweite Energiespeichereinheit umfasst, die mit dem zweiten drahtlosen Energieempfänger verbunden ist.

4. Implantierbare energetisierte medizinische Vorrichtung nach Anspruch 3, wobei mindestens eine von der ersten und der zweiten Energiespeichereinheit eine Festkörperbatterie ist.

5. Implantierbare energetisierte medizinische Vorrichtung nach Anspruch 4, wobei die Festkörperbatterie eine Thionylchloridbatterie ist.

6. Implantierbare energetisierte medizinische Vorrichtung nach einem der Ansprüche 3-5, wobei:
der erste drahtlose Energieempfänger dazu ausgelegt ist, drahtlos durch den externen drahtlosen Energiesender übertragene Energie zu empfangen und die empfangene Energie in der ersten Energiespeichereinheit zu speichern,
der interne drahtlose Energiesender dazu ausgelegt ist, in der ersten Energiespeichereinheit gespeicherte Energie drahtlos an den zweiten drahtlosen Energieempfänger zu übertragen, und
der zweite drahtlose Energieempfänger dazu ausgelegt ist, drahtlos durch den internen drahtlosen Energiesender übertragene Energie zu empfangen und die empfangene Energie in der zweiten Energiespeichereinheit zu speichern.

7. Implantierbare energetisierte medizinische Vorrichtung nach einem der vorhergehenden Ansprüche, wobei der erste Abschnitt eine erste Steuerung umfasst, die mindestens eine Verarbeitungseinheit umfasst.

8. Implantierbare energetisierte medizinische Vorrichtung nach einem der vorhergehenden Ansprüche, wobei der zweite Abschnitt eine zweite Steuerung umfasst, die mindestens eine Verarbeitungseinheit umfasst.

9. Implantierbare energetisierte medizinische Vorrichtung nach einem der Ansprüche 7 und 8, wobei mindestens eine von der ersten und zweiten Steuerung mit einem drahtlosen Sendeempfänger zum drahtlosen Kommunizieren mit einer externen Vorrichtung verbunden ist.

10. Implantierbare energetisierte medizinische Vorrichtung nach einem der Ansprüche 7 und 8, wobei:
die erste Steuerung mit einem ersten drahtlosen Kommunikationsempfänger in dem ersten Abschnitt zum Empfangen von drahtlosen Kommunikation von einer externen Vorrichtung verbunden ist,
die erste Steuerung mit einem ersten drahtlosen Kommunikationssender in dem ersten Abschnitt zum Übertragen von drahtloser Kommunikation zu einem zweiten drahtlosen Kommunikationsempfänger in dem zweiten Abschnitt verbunden ist.

11. Implantierbare energetisierte medizinische Vorrichtung nach Anspruch 10, wobei die zweite Steuerung mit dem zweiten drahtlosen Kommunikationsempfänger zum Empfangen von drahtloser Kommunikation von dem ersten Abschnitt verbunden ist.

12. Implantierbare energetisierte medizinische Vorrichtung nach einem der vorhergehenden Ansprüche, wobei der erste drahtlose Energieempfänger eine erste Spule umfasst, und der drahtlose Energiesender eine zweite Spule umfasst.

13. Implantierbare energetisierte medizinische Vorrichtung nach einem der vorhergehenden Ansprüche, wobei der erste Abschnitt eine kombinierte Spule umfasst, wobei die kombinierte Spule dazu ausgelegt ist, Energie drahtlos von einem externen drahtlosen Energiesender zu empfangen und Energie drahtlos zu dem zweiten drahtlosen Empfänger des zweiten Abschnitts zu übertragen.

14. Implantierbare energetisierte medizinische Vorrichtung nach einem der Ansprüche 12 und 13, wobei mindestens eine der Spulen in ein Keramikmaterial eingebettet ist.

15. Implantierbare energetisierte medizinische Vorrichtung nach einem der vorhergehenden Ansprüche, ferner umfassend ein Gehäuse, das dazu ausgelegt ist, mindestens den ersten Abschnitt zu umschließen, und wobei ein erster Abschnitt des Gehäuses aus Titan hergestellt ist und ein zweiter Abschnitt des Gehäuses aus einem Keramikmaterial hergestellt ist.

## Revendications

1. Dispositif médical excité implantable configuré pour être maintenu en position par une partie de tissu d'un patient, le dispositif médical comprenant :
une première partie configurée pour être placée sur un premier côté de la partie de tissu, la première partie ayant une première aire en coupe transversale dans un premier plan et comprenant une première surface configurée pour faire face à une première surface de tissu du premier côté de la partie de tissu,
une seconde partie configurée pour être placée sur un second côté de la partie de tissu, le second côté étant opposé au premier côté, la seconde partie ayant une deuxième aire en coupe transversale dans un deuxième plan et comprenant une seconde surface configurée pour entrer en prise avec une seconde surface de tissu du second côté de la partie de tissu, et
une partie de liaison configurée pour être placée à travers un trou dans la partie de tissu s'étendant entre les premier et second côtés de la partie de tissu, la partie de liaison ayant une troisième aire en coupe transversale dans un troisième plan et étant configurée pour relier la première partie à la seconde partie,
dans lequel :
les premier, deuxième et troisième plans sont parallèles entre eux,
la troisième aire en coupe transversale est plus petite que les première et deuxième aires en coupe transversale, de telle sorte que la première partie et la seconde partie soient empêchées de se déplacer à travers le trou dans la partie de tissu dans une direction perpendiculaire aux premier, deuxième et troisième plans, et
la première partie comprend un premier récepteur d'énergie sans fil destiné à recevoir l'énergie émise sans fil par un émetteur d'énergie sans fil externe, **caractérisé en ce que** : la première partie comprend en outre un émetteur d'énergie sans fil interne configuré pour émettre l'énergie sans fil vers la seconde partie, et
la seconde partie comprend un second récepteur d'énergie sans fil configuré pour recevoir l'énergie émise sans fil par l'émetteur d'énergie sans fil interne.

2. Dispositif médical excité implantable selon la revendication 1, dans lequel la première partie comprend une première unité de stockage d'énergie connectée au premier récepteur d'énergie sans fil.

3. Dispositif médical excité implantable selon l'une quelconque des revendications 1 et 2, dans lequel la seconde partie comprend une seconde unité de stockage d'énergie connectée au second récepteur d'énergie sans fil.

4. Dispositif médical excité implantable selon la revendication 3, dans lequel au moins une des première et seconde unités de stockage d'énergie est une batterie à semi-conducteurs.

5. Dispositif médical excité implantable selon la revendication 4, dans lequel la batterie à semi-conducteurs est une batterie au chlorure de thionyle.

6. Dispositif excité implantable selon l'une quelconque des revendications 3 à 5, dans lequel :
le premier récepteur d'énergie sans fil est configuré pour recevoir l'énergie émise sans fil par l'émetteur d'énergie sans fil externe et stocker l'énergie reçue dans la première unité de stockage d'énergie,
l'émetteur d'énergie sans fil interne est configuré pour émettre sans fil l'énergie stockée dans la première unité de stockage d'énergie vers le second récepteur d'énergie sans fil, et
le second récepteur d'énergie sans fil est configuré pour recevoir l'énergie émise sans fil par l'émetteur d'énergie sans fil interne et stocker l'énergie reçue dans la seconde unité de stockage d'énergie.

7. Dispositif médical excité implantable selon l'une quelconque des revendications précédentes, dans lequel la première partie comprend un premier dispositif de commande comprenant au moins une unité de traitement.

8. Dispositif médical excité implantable selon l'une quelconque des revendications précédentes, dans lequel la seconde partie comprend un second dispositif de commande comprenant au moins une unité de traitement.

9. Dispositif médical excité implantable selon l'une quelconque des revendications 7 et 8, dans lequel au moins l'un des premier et second dispositifs de commande est connecté à un émetteur-récepteur sans fil pour communiquer sans fil avec un dispositif externe.

10. Dispositif médical excité implantable selon l'une quelconque des revendications 7 et 8, dans lequel :
le premier dispositif de commande est connecté à un premier récepteur de communication sans fil dans la première partie pour recevoir une communication sans fil provenant d'un dispositif externe,
le premier dispositif est connecté à un premier émetteur de communication sans fil dans la première partie pour émettre une communication sans fil vers un second récepteur de communication sans fil dans la seconde partie.

11. Dispositif médical excité implantable selon la revendication 10, dans lequel le second dispositif de commande est connecté au second récepteur de communication sans fil pour recevoir une communication sans fil provenant de la première partie.

12. Dispositif médical excité implantable selon l'une quelconque des revendications précédentes, dans lequel le premier récepteur d'énergie sans fil comprend une première bobine et l'émetteur d'énergie sans fil comprend une seconde bobine.

13. Dispositif médical excité implantable selon l'une quelconque des revendications précédentes, dans lequel la première partie comprend une bobine combinée, dans lequel la bobine combinée est configurée pour recevoir l'énergie sans fil en provenance d'un émetteur d'énergie sans fil externe, et pour émettre l'énergie sans fil vers le second récepteur sans fil de la seconde partie.

14. Dispositif médical excité implantable selon l'une quelconque des revendications 12 et 13, dans lequel au moins l'une des bobines est noyée dans un matériau céramique.

15. Dispositif médical excité implantable selon l'une quelconque des revendications précédentes, comprenant en outre un boîtier configuré pour enfermer au moins la première partie, et dans lequel une première partie du boîtier est réalisée en titane et une seconde partie du boîtier est réalisée en matériau céramique.
